(19) Europäisches Patentamt
European Patent Office
Office européen des brevets

(11) EP 0 222 491 B2

(12) NEW EUROPEAN PATENT SPECIFICATION

(45) Date of publication and mention of the opposition decision:
**13.08.2003 Bulletin 2003/33**

(45) Mention of the grant of the patent:
**15.03.1995 Bulletin 1995/11**

(21) Application number: **86307586.7**

(22) Date of filing: **02.10.1986**

(51) Int Cl.[7]: **C12N 15/16**, C07K 14/575, C12P 21/02, A61K 38/22, A61K 39/395

# (54) Nucleic acid encoding the alpha or beta chains of inhibin and method for synthesizing polypeptides using such nucleic acid

Die alpha- oder beta-Ketten von Inhibin codierende Nukleinsäure und Verfahren zur Synthese von Polypeptiden unter Verwendung einer solchen Nukleinsäure

Acide nucléique codant les chaînes alpha ou bêta d'inhibine et méthode de synthèse de polypeptides par utilisation d'un tel acide nucléique

(84) Designated Contracting States:
**AT BE CH DE ES FR GB GR IT LI LU NL SE**

(30) Priority: **03.10.1985 US 783910**
**07.02.1986 US 827710**
**12.09.1986 US 906729**

(43) Date of publication of application:
**20.05.1987 Bulletin 1987/21**

(73) Proprietor: **GENENTECH, INC.**
**South San Francisco California 94080 (US)**

(72) Inventors:
- **Mason, Anthony John**
  **San Francisco California 94122 (US)**
- **Seeburg,Peter Horst**
  **69 Heidelberg (DE)**

(74) Representative: **Stuart, Ian Alexander et al**
**MEWBURN ELLIS**
**York House**
**23 Kingsway**
**London WC2B 6HP (GB)**

(56) References cited:
**WO-A-86/00078** **WO-A-86/06076**

- **Biochem. Biophys. Res. Commun., vol. 129 (1985), pages 396-403, K. Miyamoto**
- **PROCEEDINGS OF THE NATIONAL ACADEMY OF SCIENCES OF THE UNITED STATES OF AMERICA, vol. 83, no.16, August 1986, Baltimore, USA; K.E. MAYO et al., pages 5849-5853**
- **NATURE, vol. 318, 19/26 Dec. 1985, New York, London; A.J. MASON et al, pages 659-663**
- **NATURE, vol. 321, 19 June 1986, New York, London; W. VALE et al., pages 776-779**
- **NATURE, vol. 321, 19 June 1986, New York, London, N.LING et al., pages 779-782**
- **PROCEEDINGS OF THE NATIONAL ACADEMY OF SCIENCES OF THE UNITED STATES OF AMERICA, vol. 82, no. 12, June 1985, Baltimore, USA; C.H.LI et al., pages 4041-4044**
- **FEBS LETTERS, vol. 175, no. 2, October 1984, Amsterdam; N.G. SEIDAH et al., pages 349-355**

EP 0 222 491 B2

## Description

### BACKGROUND

This invention relates to methods for making proteins in recombinant cell culture which contain the α or β chains of inhibin. In particular, it relates to methods for obtaining and using DNA which encodes inhibin, and for making inhibin variants that depart from the amino acid sequence of natural animal or human inhibins and the naturally-occurring alleles thereof.

Inhibin is a protein produced in the gonad which acts specifically at the pituitary level to inhibit the secretion of follicle-stimulating hormone (FSH). The existence of inhibin was first postulated by McCullagh in 1932 ("Science" 76: 19-20). Such preferential regulation of the gonadotropin secretion has generated a great deal of interest and has prompted many laboratories in the past fifty years to attempt to isolate and characterize this substance from extracts of testis, spermatozoa, rete testis fluid, seminal plasma and ovarian follicular fluid, using various bioassays. Although many reports have appeared in the literature claiming the purification of inhibin-like material with molecular weights ranging from 5,000 to 100,000 daltons, subsequent studies have shown that these substances were not homogenous, did not have the high specific activity expected of true inhibin and/or failed to exhibit the molecular characteristics of inhibin as described herein (de Jong, Inhibin-Factor Artifact, "Molecular & Cellular Endocrin." 13: 1-10 (1979); Sheth et al., 1984, "F.E.B.S." 165(1) 11-15; Seidah et al., 1984, "F.E.B.S." 175(2):349-355; Lilja et al., March 1985, "F.E.B. S." 182(1):181-184: Li et al., June 1985, "Proc. Nat. Acad. Sci. USA" 82:4041-4044; Seidah et al., "F.E.B.S." 167(1): 98-102; and Beksac et al., 1984, "Intern. J. Andrology" 7:389-397).

A polypeptide having inhibin activity was purified from bovine or ovine follicular fluid (PCT 86/00078, published January 3, 1986). This protein was reported to have a molecular weight of 56,000±1,000 on SDS-PAGE and was dissociable into two subunits having apparent molecular weights of 44,000±3,000 and 14,000±2,000. Amino terminal sequences for each subunit were described.

Miyamoto, K. et al., Biochem. Biophys. Res. Comm., 129(2), 1985, 396-403 disclose isolation of at least four inhibin-like proteins from porcine follicular fluid. One of Mr 32K was further separated into bands I, II and III. Band II was reduced and dissociated into two bands of Mr 20K and 13K having N-terminal sequences Ser-Thr-Ala-Pro and Gly-Leu-Glu-Cys respectively.

EP-A-210 461 (Ajinomoto) which is part of the state of the art by virtue of Art 54(3)EPC discloses isolation of a polypeptide from malignant human monocytic cells. The polypeptide had a molecular weight of 25±1KD under non-reducing, and 16±1KD under reducing SDS electrophoresis. The amino terminal sequence and that of a cyanogen bromide cleavage fragment of the 16KD polypeptide were determined.

WO86/06076 (Biotechnology Australia) which is part of the state of the art by virtue of Art. 54(3)EPC discloses DNA sequences encoding inhibin.

Two proteins both having a molecular weight of about 32,000 daltons and having inhibin activity have been successfully isolated from porcine follicular fluid. Purification of porcine inhibin to substantial homogeneity, ie. about 90% by weight of total protein in the fraction, was achieved through a combination of protein separation procedures including heparin-Sepharose affinity chromatography, gel filtration and reverse-phase, high-performance liquid chromatography (RP-HPLC).

These proteins were isolated to substantial homogeneity from material obtained from swine and are referred to as Protein A and Protein B. Each protein has a molecular weight of about 32,000 daltons (32K) and is composed of two polypeptide chains having molecular weights of 18,000 and 14,000 daltons, respectively, the chains being linked together in the hormonally-active protein by disulfide bonding. The amino-terminal amino acid residue sequence of the 18,000 daltons (18K) or alpha chain of both proteins was determined to be

**Ser-Thr-Ala-Pro-Leu-Pro-Trp-**
**Pro-Trp-Ser-Pro-Ala-Ala-Leu-Arg-Leu-Leu-Gln-Arg-Pro-Pro-Glu-Glu-**
**Pro-Ala-Val.**

The amino-terminal amino acid residue sequence of the 14,000 dalton (14K) or beta chain of Protein A was determined to be

Gly-Leu-Glu-X-Asp-Gly-Lys-Val-Asn-Ile-X-X-Lys-Lys-Gln-Phe-Phe-Val-Ser-Phe-Lys-Asp-Ile-Gly-Trp-Asn-Asp-Trp-Ile-Ile-Ala

and of Protein B was determined to be

Gly-Leu-Glu-X-Asp-Gly-Arg-Thr-Asn-Leu-X-X-Arg-Gln-Gln-Phe-Phe-Ile-Asp-Phe-Arg-Leu.

Proteins A and B have been completely characterized. Each 32K protein exhibits inhibin activity in that it specifically inhibits the basal secretion of FSH but does not inhibit secretion of luteinizing hormone (LH). The individual chains were not hormonally-active.

After the filing of the parent application hereto, inhibin B-chain dimers were shown to exist in follicular fluid as naturally-occurring substances, termed activin, which are capable of stimulating FSH release by rat anterior pituitary cells (Vale et al., 1986, "Nature" 321:776-779 and Ling et al., 1986, "Nature" 321:779-782).

The amino acid sequence of the α and β chains of inhibin from humans remained unknown until the invention herein. The large quantities of human follicular fluid required to parallel the studies conducted with animal inhibins are not readily available, nor is there any assurance that human and animal inhibins would be sufficiently similar that purification using a parallel procedure would be effective. Accordingly, methods are needed for determining the characteristics and amino acid sequence for human inhibin.

Also needed are economical methods for making the α and β chains of inhibin in large quantities, preferably entirely and completely free of proteins from the species homologous to the inhibin in question, which inhibin preferably also is biologically active.

These and other objects will be apparent from consideration of the invention as a whole. The scope of protection is as determined by the appended claims. Subject to that, the following represents a description of the invention.

## SUMMARY

Nucleic acid now has been isolated and cloned in replicable vectors which encodes the mature porcine and human a and chains of inhibin and their precursor prepro and pro forms. Sequencing of inhibin-encoding cDNA has led to the identification of prodomain regions located N-terminal to the mature inhibin chains that represent coordinately expressed biologically active polypeptides. The prodomain regions or prodomain immunogens are useful in monitoring preproinhibin processing in transformant cell culture or in experiments directed at modulating the clinical condition or reproductive physiology of animals. Thus α or β chain nucleic acid is used to prepare prodomain sequences from the precursor forms of the inhibin chains, to transform host cells for the recombinant expression of mature inhibin α and/or β chains, and in diagnostic assays. In particular, regions from inhibins α and/or β chains are expressed in recombinant cell culture by a method comprising ligating the nucleic acid encoding the region into a replicable vector under the control of a promoter, transforming a host cell with the vector, culturing the host cell and recovering the prodomain, activin or inhibin from the cultured cell. Inhibin, activin and prodomains produced by the method of this invention are entirely free of homologous source proteins and can be produced in biologically active form.

The nucleic acids identified herein encode the α, $β_A$ and $β_B$ chains of porcine or human inhibin. Recombinant cells are transformed to express $αβ_A$ or $αβ_B$ inhibins, or to express β-chain heterodimers or homodimers (which are collectively referred to in the literature as activin). β-chain dimers as products of recombinant cells expression are free of homologous proteins with which they ordinarily are associated in nature.

Inhibin or activin and their nontoxic salts, combined with a pharmaceutically acceptable carrier to form a pharmaceutical composition, are administered to mammals, including humans, for control of fertility. Administration of inhibin decreases fertility in female mammals and decreases spermatogenesis in male mammals, and administration of a sufficient amount induces infertility. Inhibin is also useful in tests to diagnose infertility. Activin has been shown in the literature to be capable of stimulating FSH release from pituitary cells and accordingly is useful as a fertility inducing therapeutic.

The method of this invention also facilitates the convenient preparation of inhibin, activin and prodomain variants having primary amino acid sequences and/or glycosylation differing from the native analogues, in particular fusions of immunogenic peptides with inhibin, activin or prodomain sequences.

Brief Description of the Drawings

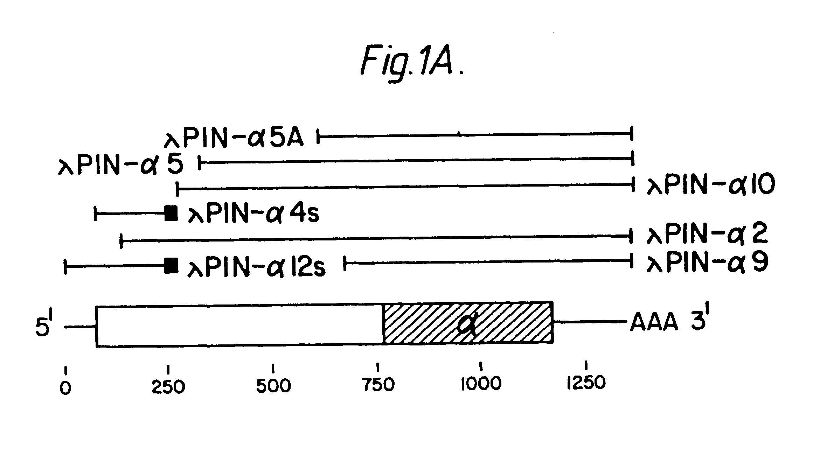

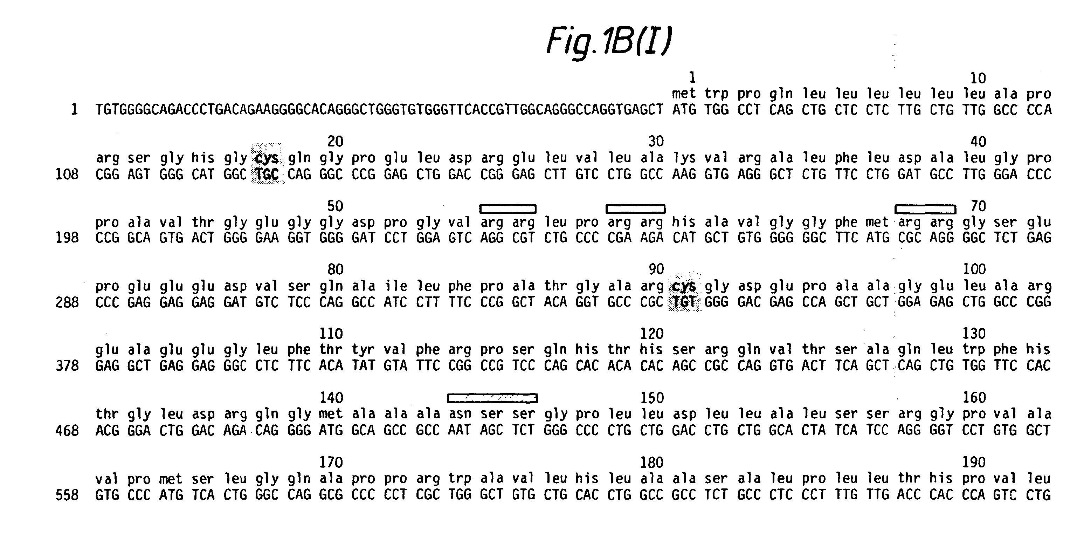

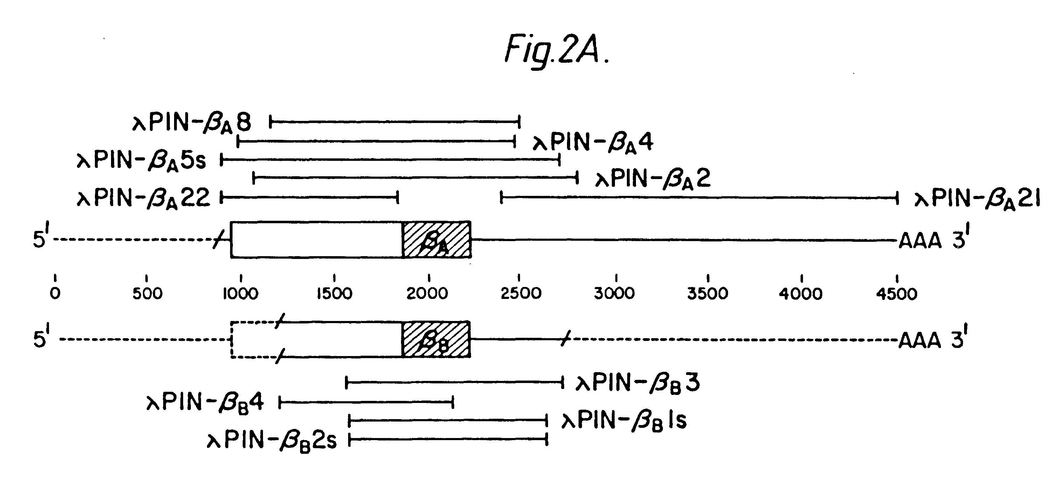

Fig. 1A is a schematic representation of the porcine α-chain mRNA. Overlapping cDNA clones used in the sequence determination are shown above the diagram of the mRNA structure. Black boxes on the 3' ends of λ clones indicate that these clones were obtained by specific priming. Untranslated sequences are represented by a line, coding sequences are boxed. The unfilled portion represents the coding region for the signal peptide and pro-sequences, and the cross-hatched areas indicates the 134 amino acid α-chain. The scale is in nucleotides from the 5' end of the longest cDNA clone.

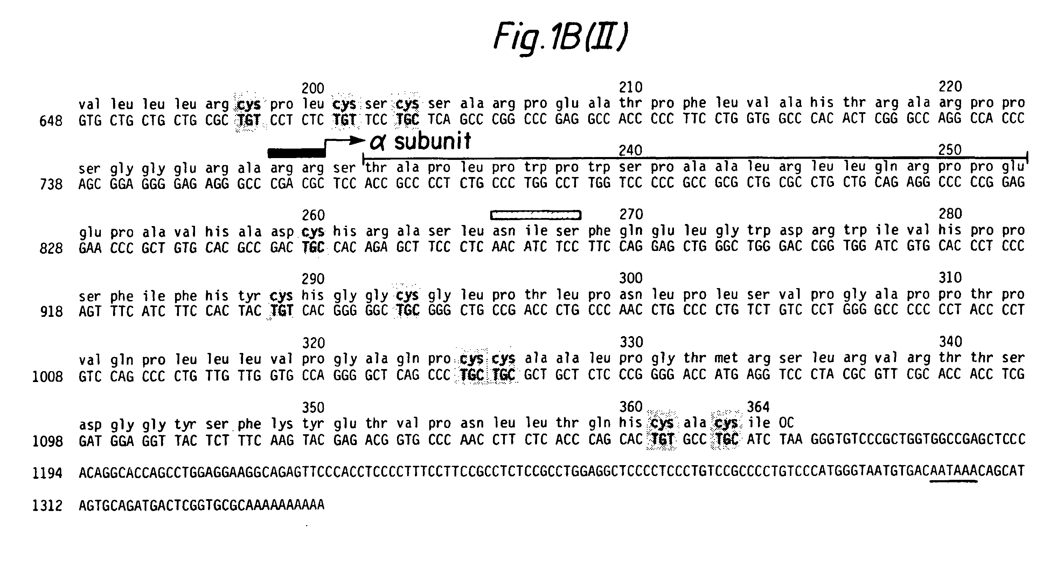

Fig. 1B shows the nucleotide and predicted amino acid sequence of the porcine α-chain precursor. Nucleotides are numbered at the left and amino acids are numbered throughout. The amino acid sequence underlined was used to design a long synthetic DNA probe. The 364 amino acid precursor includes a hydrophobic signal sequence, a pro-region, and the mature α-chain (amino acids 231-364). The proteolytic processing site Arg-Arg (black bar) immediately precedes the NH2-terminus of the mature alpha chain. Several other putative dibasic processing sites present in the pro-region are indicated by open bars. The two potential N-linked glycosylation sites are shown by the cross-hatched bars. The AATAAA box close to the 3' end of the mRNA is underlined.

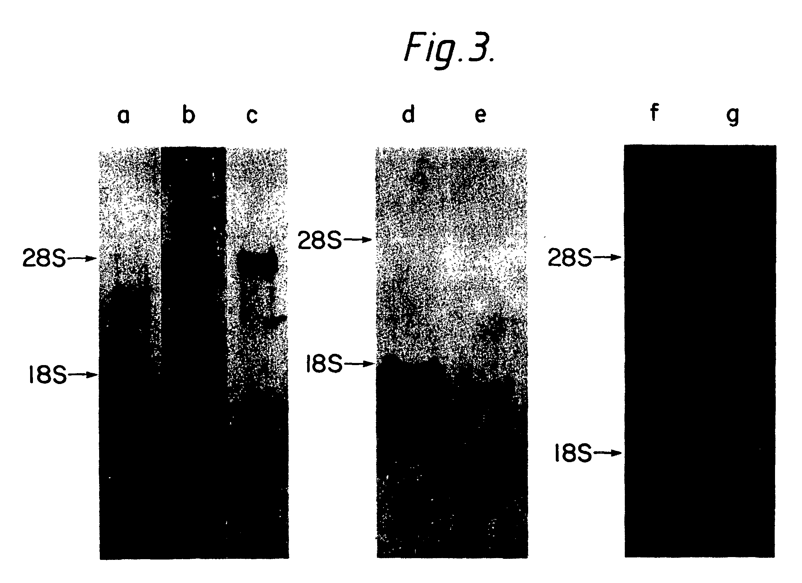

Fig.2A is a schematic representation of the porcine $\beta_A$ and $\beta_B$ subunit mRNAs with coding sequences boxed. The $\beta_A$ and $\beta_B$ subunits (dashed) are encoded towards the 3' of the coding sequences. The 3' and 5' untranslated regions are shown as a line. The length of the 5' and 3' untranslated region of the βB subunit mRNA is inferred from the size of the mRNA (Fig. 3) and its obvious similarity to the $\beta_A$ mRNA. Tentative regions of the cDNAs are shown as dashed in the diagram. The relative positions of the overlapping oligo-dT primed cDNA clones and the randomly primed clones (λPIN$\beta_A$5s, λPIN$\beta_B$1s, and λPIN$\beta_B$2s) are indicated. The scale is in nucleotides from the 5' end of the 4.5 kb mRNA.

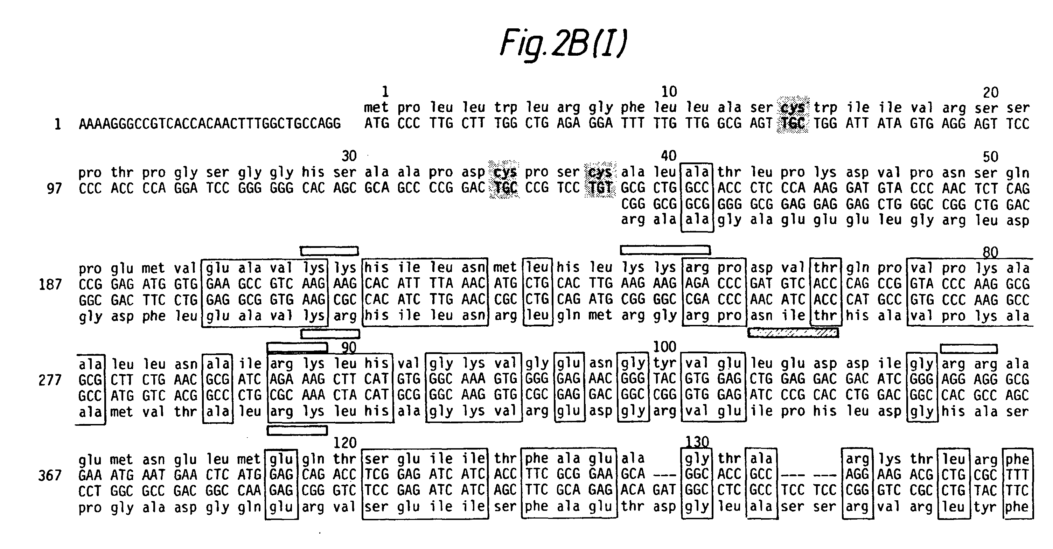

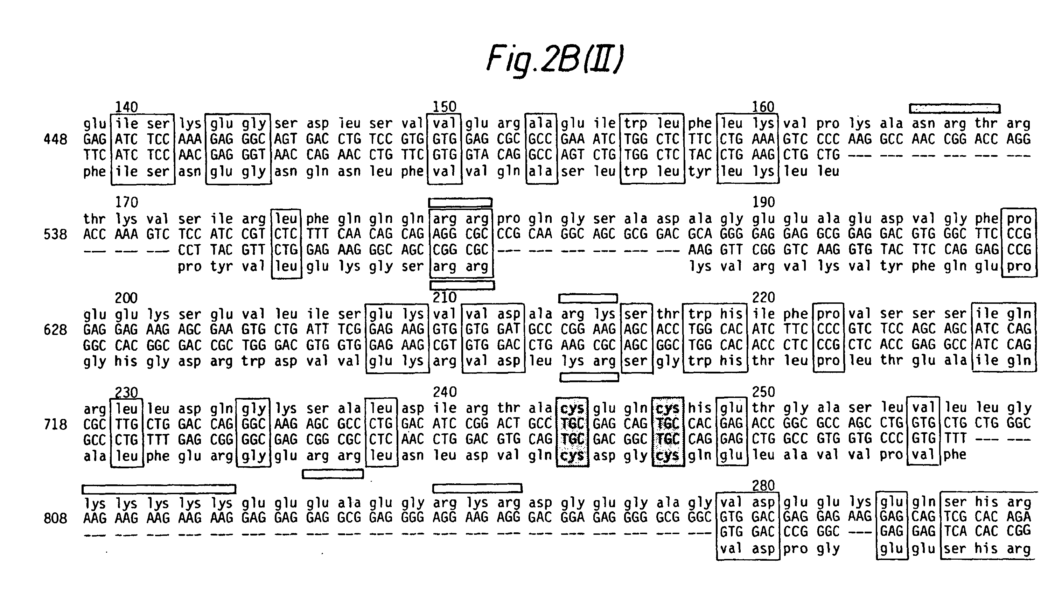

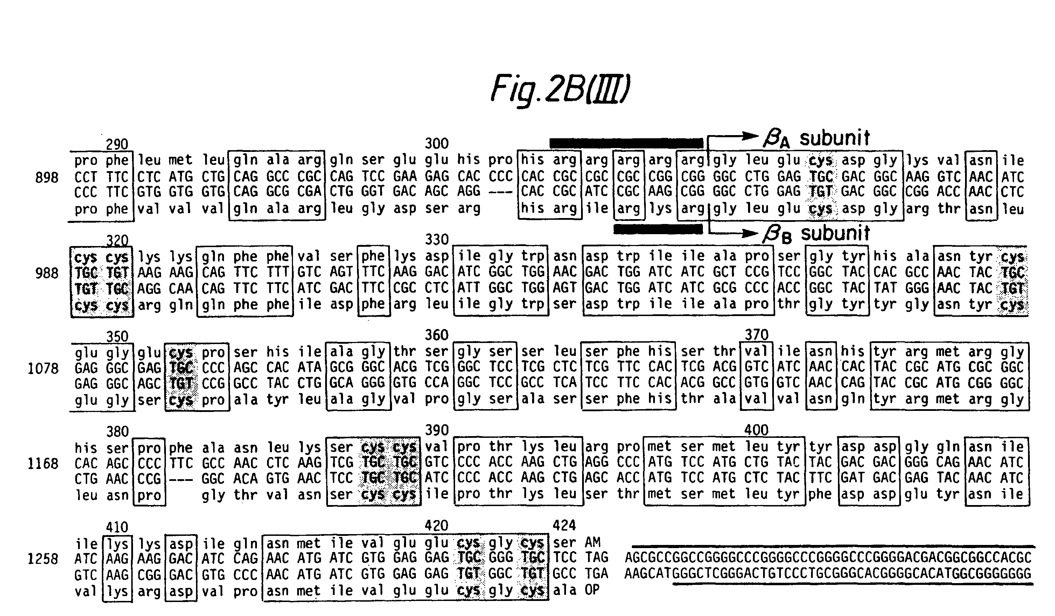

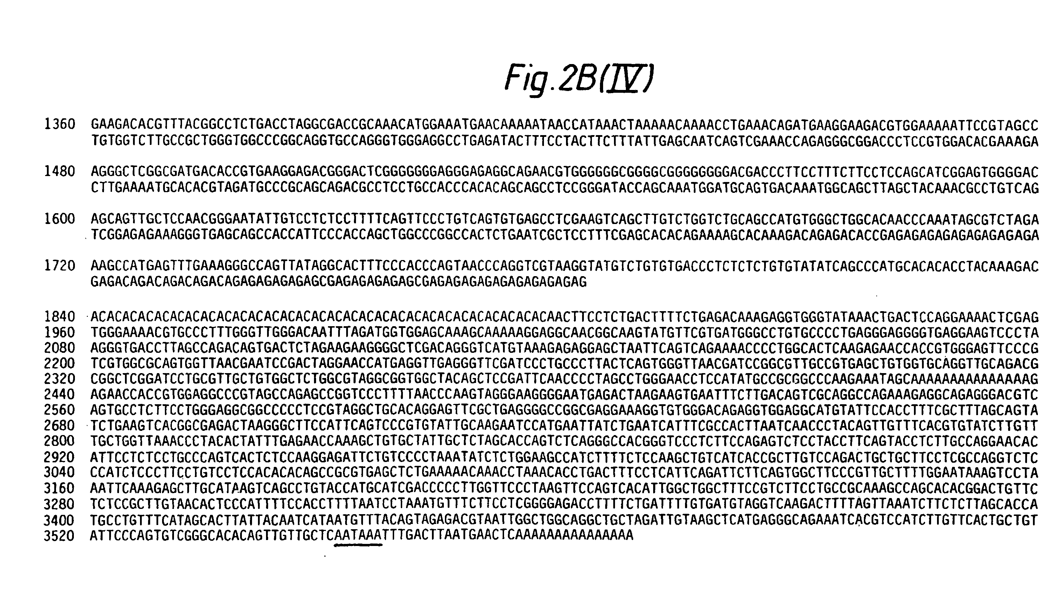

Fig. 2B is the nucleotide sequence and deduced amino acid sequence of the porcine inhibin β-subunit precursors. The $\beta_B$ sequence is aligned with the $\beta_A$ sequence for maximum homology. The $NH_2$-terminal of the β-subunit precursors are indicated by bracket and arrows. Cysteine residues are shaded, possible processing sites are indicated by open bars, and a potential glycosylation site is shown by the cross-hatched box. A very GC-rich region present 3' to the termination codon intron sequences is underlined and overlined in both sequences. Amino acid sequences used to design oligonucleotide probes are underlined, as is the AATAAA polyadenylation signal. There was one nucleotide difference between λPIN-$\beta_A$8 and other clones covering this area. A G-to-A change causes a change of amino acid 278 from a glycine to a serine. The proteolytic processing site Arg Arg Arg Arg Arg (black bar) immediately precedes the $NH_2$ terminus of the mature $\beta_A$ subunit, with the prosequences located upstream. The amino acids for the $\beta_A$ subunit only are numbered.

Fig. 3 is a Northern blot analysis of porcine ovarian mRNA with α, $\beta_A$ and $\beta_B$ subunit cDNA hybridization probes. Lanes a, b, c, d, and f are polyA$^+$ mRNA and e and g are total RNA. The position of the 28S and 18S ribosomal RNAs are shown. Lanes a, d, and e were hybridized with an α-subunit cDNA probe; lanes d, e and g with a $\beta_A$ subunit specific probe, and lane c with a $\beta_B$ subunitspecific probe. The α-subunit mRNA is approximately 1.5 kb, the $\beta_A$ subunit mRNAs are approximately 4.5 kb. The hybridizations shown in lanes a, b, and c were performed with probes of approximately equal length and specific activity in order to judge relative mRNA levels.

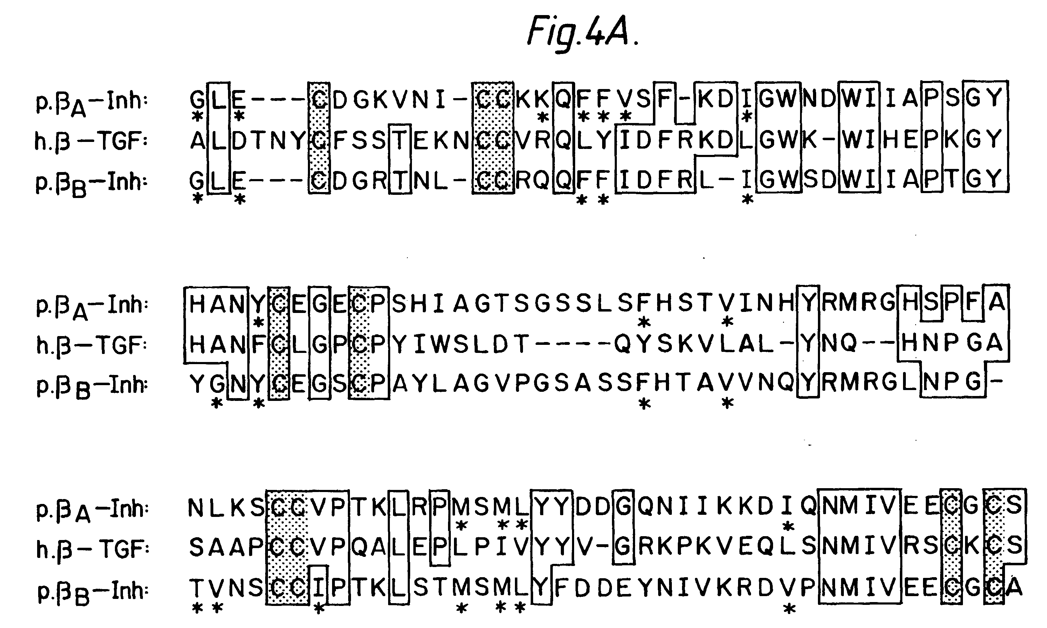

Fig. 4A is a comparison of the human β-TGF amino acid sequence and porcine inhibin $\beta_A$ and $\beta_B$ amino acid sequences. The sequences were aligned around the cysteine residues. Identical residues are boxed, while conservative changes are designated by an asterisk.

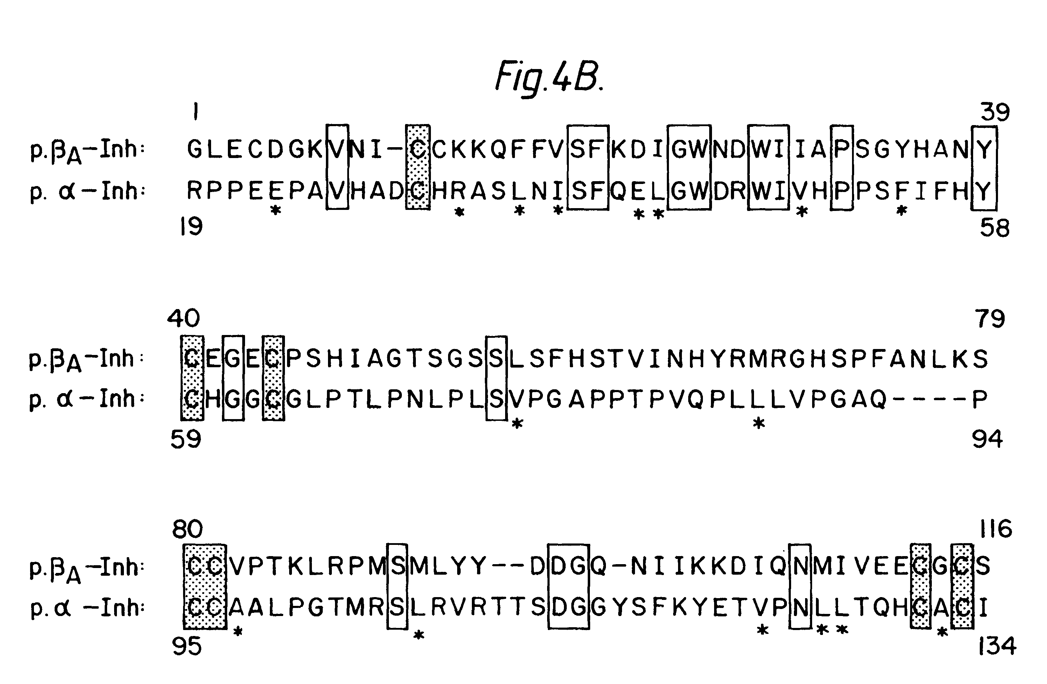

Fig. 4B compares the α-subunit sequence with the $\beta_A$-inhibin sequence.

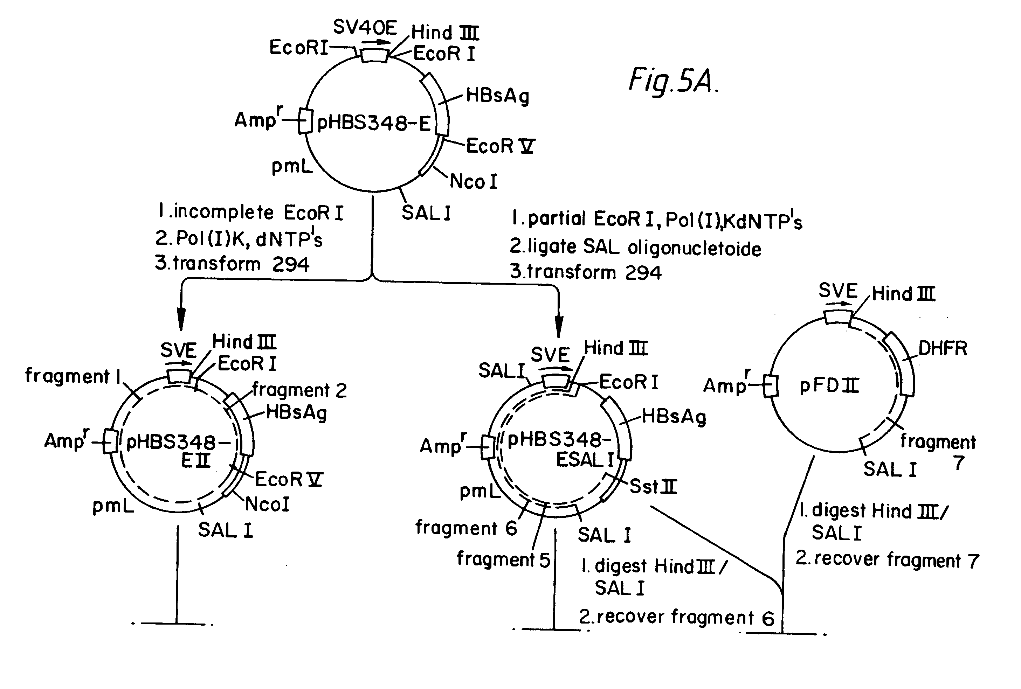

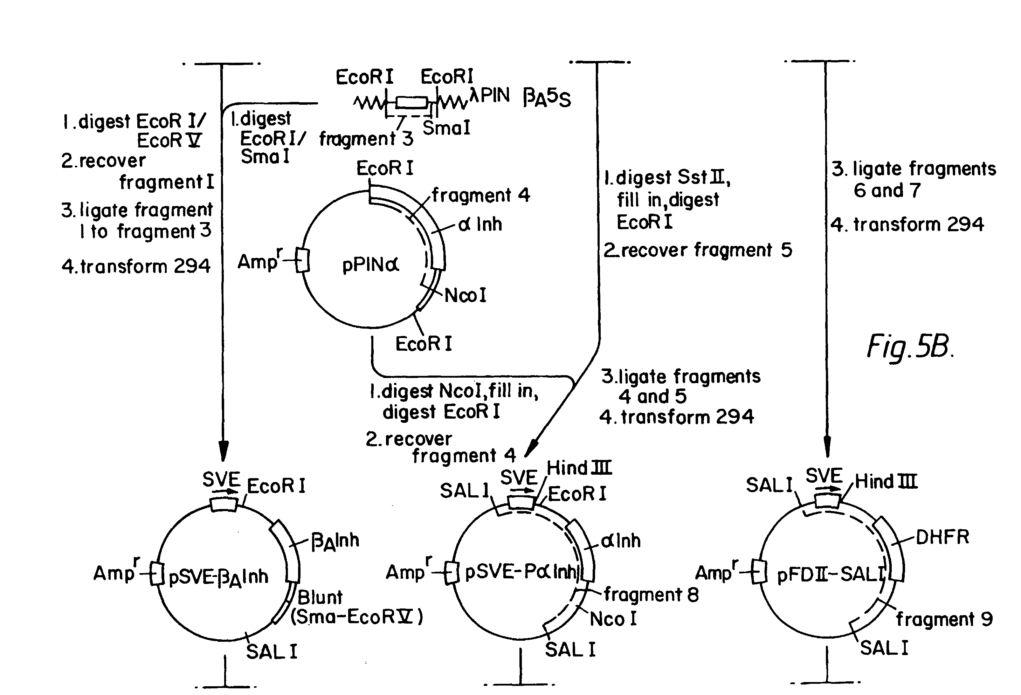

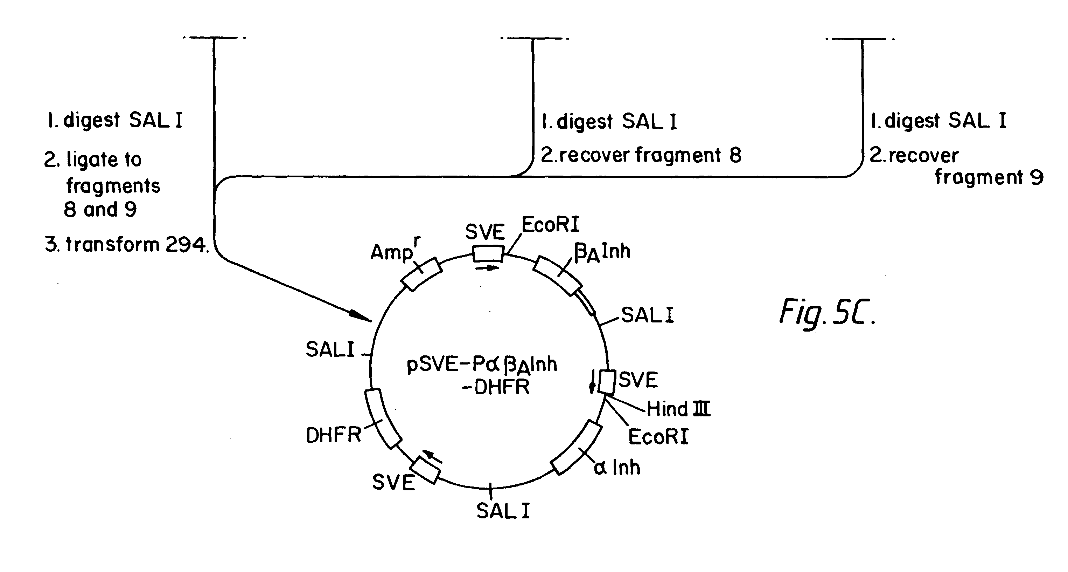

Fig. 5 depicts the construction of a representative recombinant expression plasmid for porcine inhibin.

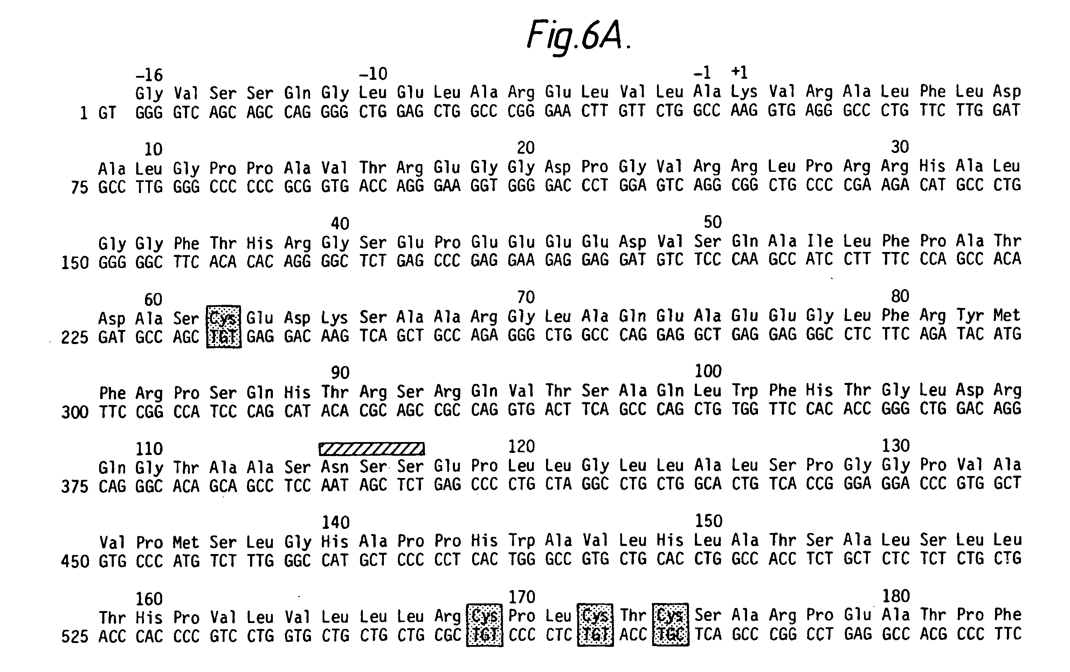

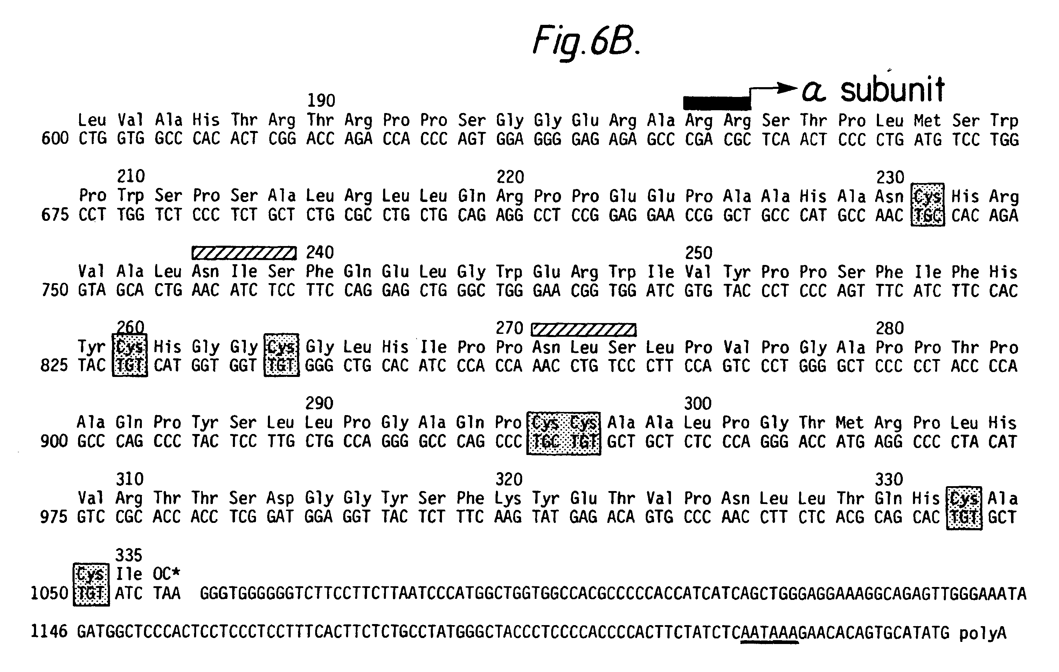

Fig. 6 shows the nucleotide sequence and deduced amino acid sequence of the human α-inhibin cDNA. The 335 amino acid pro-or inhibin sequence is numbered from the hypothesized signal cleavage site. Sixteen amino acids of the signal sequence are numbered -1 through -16. Homology with the porcine sequence predicts a further 12 amino acid residues in the signal sequence. In this and other figures, putative dibasic processing sites are shown by the open bars, glycosylation sites indicated by cross-hatched bars, and amino terminal mature chain processing sites are depicted as black bars. The poly(A) additional signal sequence is underlined. Cysteine residues are shaded.

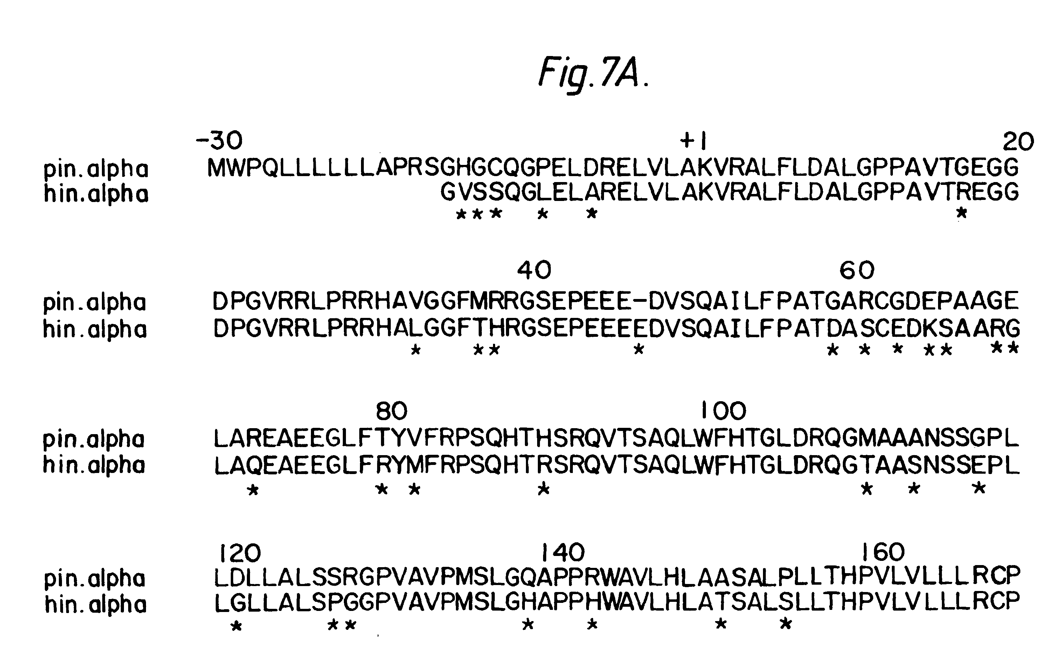

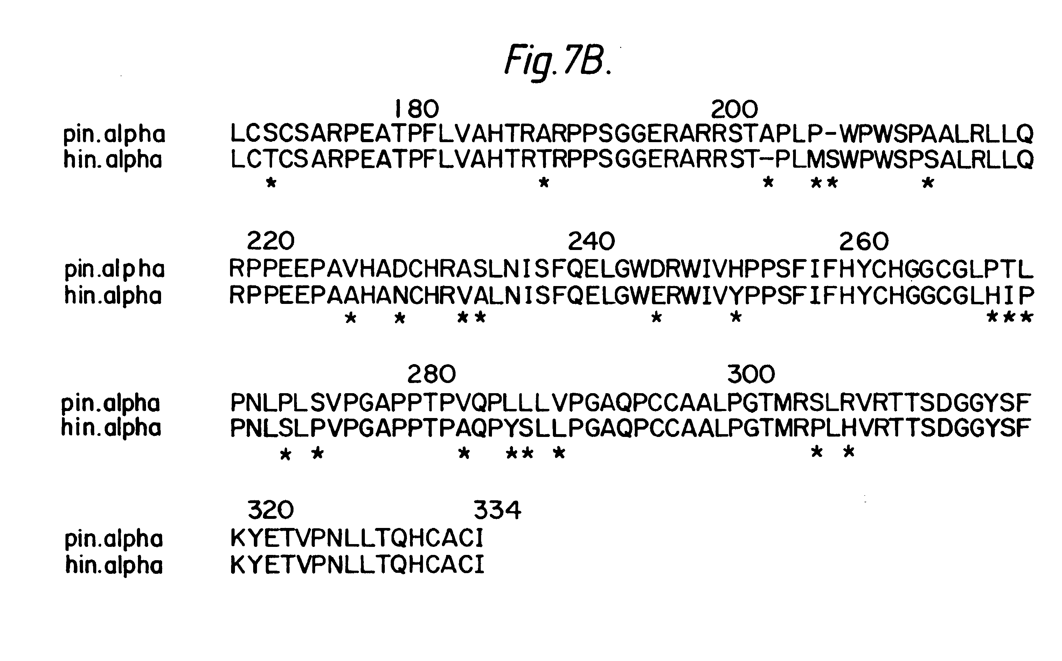

Fig. 7 is a comparison of the human and porcine α-inhibin protein sequences. Spaces are introduced to maximize the homology; positions of non-identity are indicated by stars. Numbering is as for the porcine sequence, which is one amino acid shorter than the human.

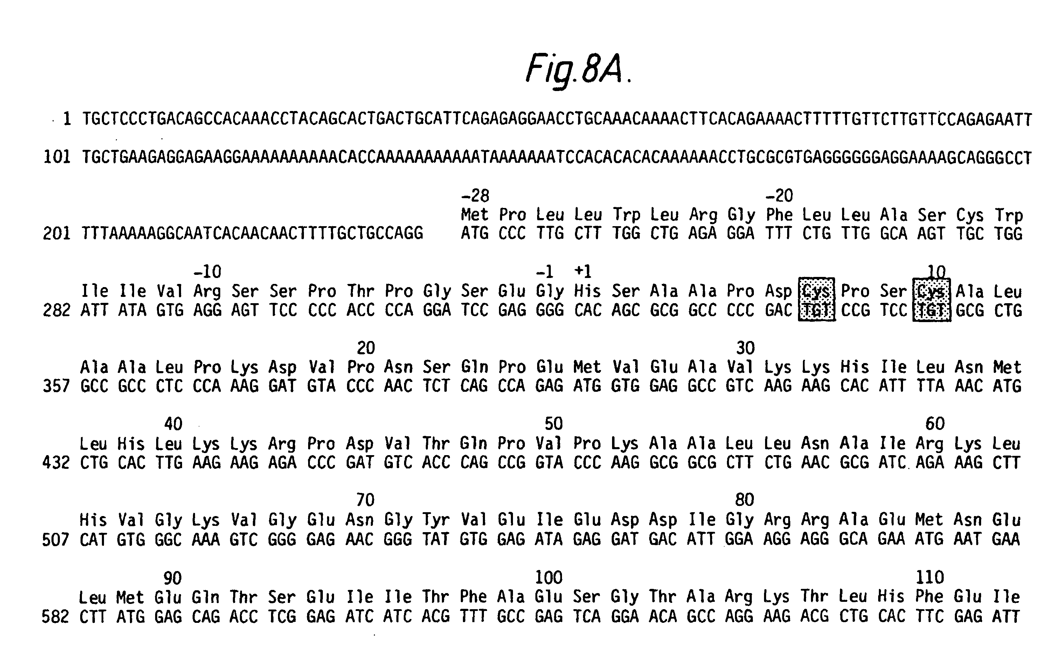

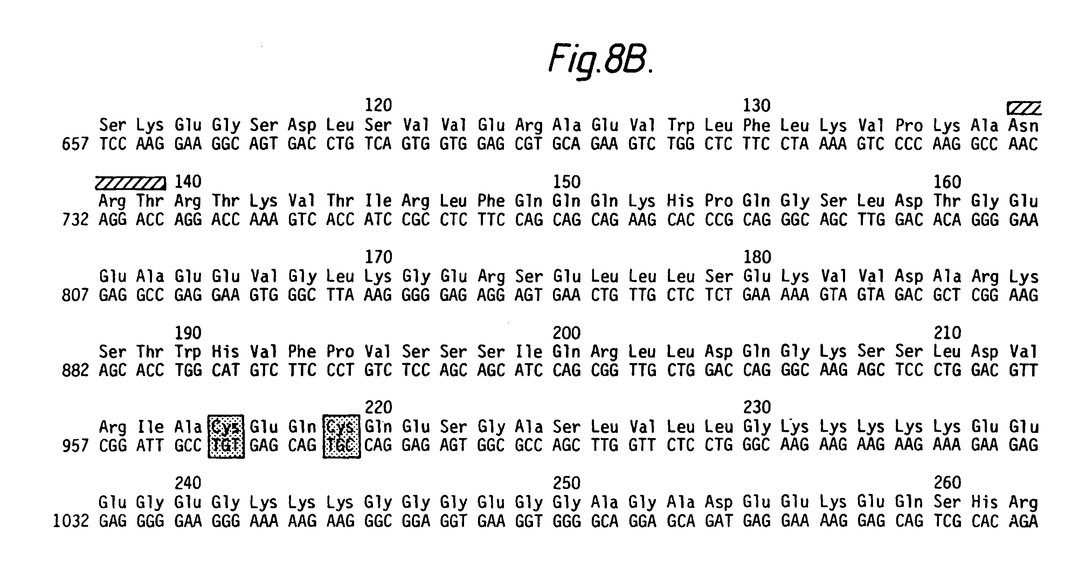

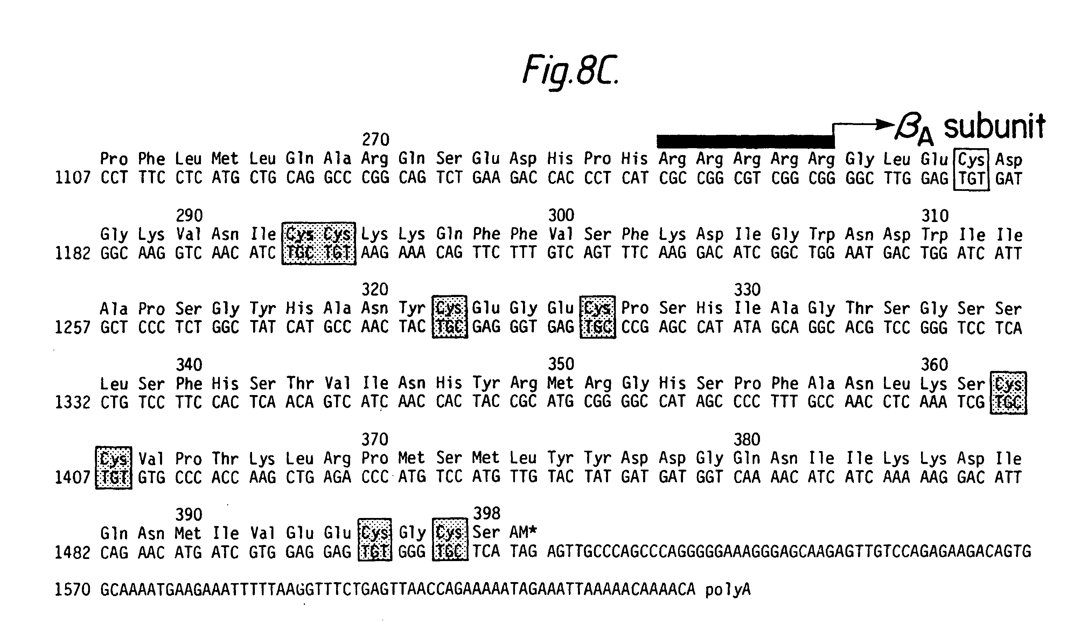

Fig. 8 shows that the nucleotide and deduced amino acid sequence of the human $\beta_A$ inhibin signal sequence (residues -28 through -1) is 28 amino acids with the precursor being 378 amino acids in length. The basic processing site is indicated by a black bar, and a potential glycosylation site in the precursor is indicated by a cross-hatched bar above the sequence. Cysteine residues are shaded.

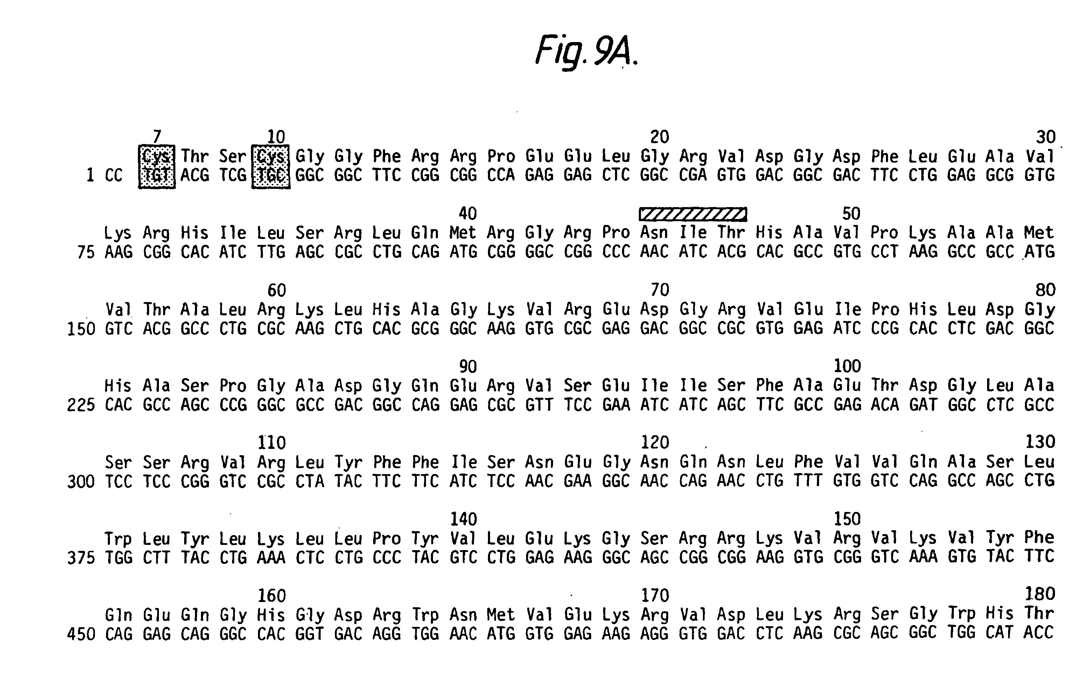

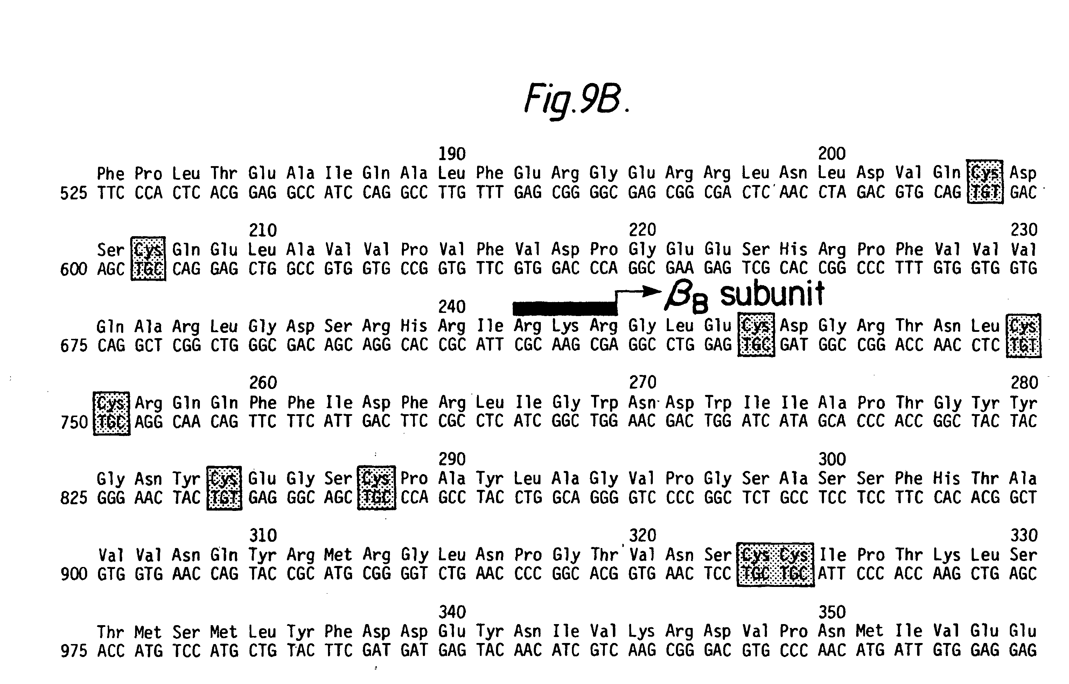

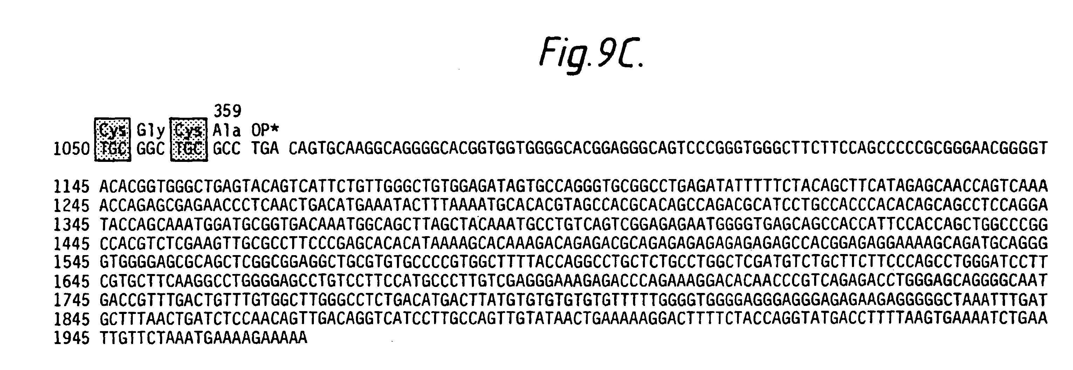

Fig. 9 illustrates the nucleotide and deduced amino acid sequence of human $\beta_B$ inhibin cDNA. The sequence commences at a cysteine residue (position 7), which lines up with the cysteine present at residue 7 in the $\beta_A$ sequence (see Fig. 8). The processing site for the mature $\beta_B$ inhibin is shown as a black bar and a potential glycosylation site as a cross-hatched bar. Cysteine residues are shaded.

## DETAILED DESCRIPTION

The polypeptides of this invention are the α and β chains of inhibin, as well as their multimer forms (activin and inhibin), their prepro forms and their prodomains, together with glycosylation and/or amino acid sequence variants of each chain or form thereof. Inhibin (including alleles) from human or animal sources inhibits the basal release of FSH but not of LH from anterior pituitary cells while activin does the opposite (hereinafter referred to as "hormonally active" activin or inhibin).

Generally, amino acid sequence variants will be substantially homologous with the relevant portion of the porcine or human α or β chain sequences set forth in Figs. 1B, 2B, 6, 8 and 9. Substantially homologous means that greater than about 70% of the primary amino acid sequence of the candidate polypeptide corresponds to the sequence of the porcine or human chain when aligned in order to maximize the number of amino acid residue matches between the two proteins. Alignment to maximize matches of residue includes shifting the amino and/or carboxyl terminus, introducing gaps as required and/or deleting residues present as inserts in the candidate. For example, see Figs. 2B and 7 where the $\beta_A$ and $\beta_B$ subunits or human and porcine α-inhibin sequences are aligned for maximum homology. Typically, amino acid sequences variants will be greater than about 90% homologous with the corresponding native sequences shown in Figs. 1 B, 2B, 6, 8 and 9.

Variants that are not hormonally-active fall within the scope of this invention, and include polypeptides that may or may not be substantially homologous with either a mature inhibin chain or prodomain sequence, but which are immunologically cross-reactive with antibodies raised against the native counterpart.

Hormonally inactive variants are produced by the recombinant or organic synthetic preparation of fragments, in particular the isolated β chains of inhibin, or by introducing amino acid sequence variations so that the molecules no longer demonstrate hormonal activity as defined above.

Immunological cross-reactivity means that the candidate polypeptide is capable of competitively inhibiting the binding of the hormonally-active analogue to polyclonal antisera raised against the hormonally-active analogue. Such antisera are prepared in conventional fashion by injecting goats or rabbits S.C. with the hormonally-active analogue or derivative in complete Freunds adjuvant, followed by booster intraperitoneal or S.C. injections in incomplete Freunds.

Variants that are not hormonally active but which are capable of cross-reacting with antisera to hormonally-active inhibin, activin, or prodomains are useful (a) as reagents in diagnostic assays for the native analogues or their antibodies, (b) when insolubilized in accord with known methods, as an agent for purifying anti-native analogue antibodies from antisera, and (c) as an immunogen for raising antibodies to hormonally-active analogues.

This invention includes the pro and/or prepro sequences of the inhibin α or β chain precursors, or their immunologically or biologically active fragments, substantially free of the corresponding mature inhibin chains. These sequences for porcine and human inhibin are shown in Figs. 1B, 2B, 6, 8 and 9. The prepro sequence for the porcine α subunit precursor is the polypeptide comprised by residues 1 to about 230, while the $\beta_A$ subunit pro sequence is comprised by residues 1 to about 308. These sequences shall be referred to herein as encompassing prodomain sequences.

The α and β subunit prodomain sequences are comprised of several domains bounded by proteolysis sites, any one of which is synthesized herein separately or in combination with other domains. The principal porcine $\beta_A$ domains fall within residues 1 to about 70 (domain I), about 70 to about 110 (domain II), about 110 to about 180 (domain III), about 180 to about 260 (domain IV), and about 270 to about 309 (domain V). In particular, the porcine $\beta_A$ domains are

GHSAAPDCPSCALATLPKDVPNSQPEMVEAV,

HILNMLHLKKRPDVTQPVPKAALLNAI, LHVGKVGENGYVELEDDIG,

AEMNELMEQTSEIITFAEAGRARKTLRFEISKEGSDLSVVERAEIWLFKVPKANRTRTKV

SIRLFQQQ, PQGSADAGEEAEDVGFPEEKSEVLISEKVVDA,

STWHIFPVSSSIQRLLDQGKSALDIRTACEQCHETGASLVLLG, and

GHSAAPDCPSCALATLPKDVPNSQPEMVEAVKKHILNMLHLKKRPDVTQPVPKAALLNAI.

The porcine $\beta_B$ domains comprise RAAHILLHAVRVSGWLNL as well as homologous β domains having the same sequences. The porcine α domains comprise

GPELDRELVLAKVRALFLDALGPPAVTGEGGDPGV and

GSEPEEEDVSQAILFPATGARCGAEPAAGELAREAEEGLFTYVGRPSQHTHSRQVTSAQLWFHTGL DRQGMAAANSSGPLLDLLALSSRGPVAVPMSLGQAPPRWAVLHLAASALPLLTHPVLVLLLRCPLC SCSARPEATPFLVAHTRARPPSGGERA.

A typical combination domain polypeptide would be $\beta_A$ domain II linked at its C-terminus to the $NH_2$-terminus of $\beta_A$ domain III. In addition, these domains are fused together by the proteolysis sites found in the sequences shown in Figs. 1B or 2B, by 1 to 4 residue polypeptides that are resistant to hydrolysis (for example, glutaminyl or histidyl residues), or are directly fused, whereby, in all three instances, combination domain polypeptides are produced.

Principal human α chain prodomains are approximately residues 30-199 and 1 to 29, human βA prodomains are approximately residues 1-30, 32-40, 43-59, 62-80, 83-185 and 186-230 while human $\beta_B$ prodomains are approximately residues 1-13, 15-30, 32-59, 62-145, 148-195 and 198-241 (referring to the numbering system adopted in Figs. 6, 8 and 9, respectively). Combination prodomain polypeptides are within the scope hereof, for example, the $\beta_A$ prodomain at about 43-80, and $\beta_B$ prodomains at about 1-30 and about 32-145. The preferred human α, $\beta_A$ and $\beta_B$ chain prodomains are about residues 1-29, about 43-80 and about 1-30, respectively.

The intact isolated prepro or prodomain $\beta_A$, $\beta_B$ or α sequences are best synthesized in recombinant-cell culture. The individual subcomponent domains are synthesized by routine methods of organic chemistry or by recombinant cell culture. They then are labelled with a radioisotope or other detectable group such as an enzyme or fluorophore in accord with known methods and used in standard competitive immunoassays to detect the levels of prepro or pro forms of inhibin, including individual domains, in transformants with DNA encoding such forms or their precursors. This assay is useful in determining whether proteolytic hydrolysis of proinhibin is occurring in the host transformants or their culture media. The assay also is useful in determining whether a rate limiting step in recombinant synthesis is translation of mRNA into the prepro forms or processing of the prepro forms into mature inhibin. For example, high levels of prepro or pro inhibin in cell lysates, but relatively low levels of secreted mature inhibin, would suggest that the host cell is adequately transcribing and translating the inhibin DNA, but is not processing the precursors at an adequate rate. Thus, in this case one would select an alternate host cell rather than concentrating on improving the transcription or translation efficiency of the transforming plasmid, e.g., by selecting an alternate promoter. The prodomain sequences also are believed to be involved in coordinate modulation of animal physiology in reproductive cycles and fertility.

Amino acid sequence variants are any one of 1) hormonally-active, or 2) cross reactive with antibodies raised against mature inhibin or prodomain α or β chain sequences, but are characterized by a primary amino acid sequence that departs from the sequence of natural inhibins or prodomain sequences. These derivatives ordinarily are prepared by introducing insertions, deletions or substitutions of nucleotides into the DNA encoding the target DNA to be modified in order to encode the variant, and thereafter expressing the DNA in recombinant cell culture. Polypeptides having up to about 100-150 residues also are conveniently prepared by in vitro synthesis. Such variants are characterized by the predetermined nature of the variation, a feature that sets them apart from naturally occurring allelic or interspecies variation. The variants may exhibit the same qualitative biological activity as the naturally-occurring analogue or may act antagonistically towards such analogues.

While the site for introducing a sequence variation is predetermined, it is unnecessary that the mutation per se be predetermined. For example, in order to optimize the performance of mutation at a given site, random mutagenesis may be conducted at the target codon or region and the expressed inhibin mutants screened for the optimal combination of desired activity. Techniques for making substitution mutations at predetermined sites in DNA having a known sequence is well known, for example M13 primer mutagenesis.

Mutagenesis is conducted by making amino acid insertions, usually on the order of about from 1 to 10 amino acid residues, or deletions of about from 1 to 30 residues. Deletions or insertions preferably are made in adjacent pairs, i.e. a deletion of 2 residues or insertion of 2 residues. Substitutions, deletions, insertions or any subcombination may be combined to arrive at a final construct. Insertions include amino or carboxyl-terminal fusions, e.g. a hydrophobic extension added to the carboxyl terminus. Preferably, however, only substitution mutagenesis is conducted. Obviously, the mutations in the encoding DNA must not place the sequence out of reading frame and preferably will not create complementary regions that could produce secondary mRNA structure.

Not all mutations in the DNA which encode the polypeptides herein will be expressed in the final secreted product. For example, a major class of DNA substitution mutations are those in which a different secretory leader or signal has been substituted for the native porcine or human α or β chain secretory leader, either by deletions within the leader sequence or by substitutions, wherein most or all of the native leader is exchanged for a leader more likely to be recognized by the intended host. For example, in constructing a procaryotic expression vector the porcine or human α or β chain secretory leader is deleted in favor of the bacterial alkaline phosphatase or heat stable enterotoxin II leaders, and for yeast the leader is substituted in favor of the yeast invertase, alpha factor or acid phosphatase leaders. However, the porcine and human secretory leaders are recognized by many heterologous higher eukaryotic cells. When the secretory leader is "recognized" by the host, the host signal peptidase is capable of cleaving a fusion of the leader polypeptide fused at its C-terminus to the mature inhibin or prodomain such that mature inhibin or prodomain polypeptide is secreted.

Another major class of DNA mutants that are not expressed in final form as amino acid sequence variations are nucleotide substitutions made in the DNA to enhance expression, primarily to avoid 5' stem and loop structures in the transcribed mRNA (see de Boer et al., EP 75,444A) or to provide codons that are more readily transcribed by the selected host, e.g. the well-known preference codons for E. coli or yeast expression. These substitutions may or may not encode substituted amino acid residues, but preferably do not.

Insertional and deletional amino acid sequence variants are proteins in which one or more amino acid residues are introduced into or removed from a predetermined site in the target inhibin, activin, prodomain or proform of inhibin or activin. Most commonly, insertional variants are fusions of heterologous proteins or polypeptides to the amino or carboxyl terminus of the α or β chains, the prodomains or other inhibin derivatives. Immunogenic derivatives are made by fusing an immunogenic polypeptide to the target sequence, e.g. a prodomain polypeptide, by synthesis in vitro or by recombinant cell culture transformed with DNA encoding the fusion. Such immunogenic polypeptides preferably are bacterial polypeptides such as trpLE, beta-galactosidase and-the like, together with their immunogenic fragments. Other insertions entail inserting heterologous eukaryotic (e.g. the herpes virus gD signal) or microbial secretion signal or protease processing sequences upstream from the $NH_2$-terminus of the protein to be secreted. Deletions of cysteine or other labile residues also may be desirable, for example in increasing the oxidative stability of the α or β chain. Deletion derivatives will produce α or β chain fragments. Such fragments, when biologically or immunologically active, are within the scope herein. For instance, a fragment comprising $\beta_B$ or $\beta_A$ residues about from 11 to 45 (numbered from mature $Gly_1$) is to be included within the scope herein.

Immunogenic conjugates of prodomain polypeptides, inhibin and activin are readily synthesized in recombinant cell culture as fusions with immunogenic polypeptides, e.g. beta-lactamase or viral antigens such as herpes gD protein, or by preparation of the polypeptides in unfused form (by recombinant or in vitro synthetic methods) followed by covalent cross-linking to an immunogenic polypeptide such as keyhole limpet hemocyanin or STI using a divalent cross-linking agent. The immunogenic polypeptides are formulated with a vaccine adjuvant, e.g. alum or Freunds. Methods for preparing proteins in adjuvants and for cross-linking are well-known per se and would be employed by one skilled in the art, as are methods for vaccinating animals. The immunogenic conjugates are useful in preparing antibodies to the prodomain region for use in monitoring inhibin manufacture or for in vivo vaccination with the objective of raising antibodies capable of modulating animal physiology in reproductive cycles and fertility. Typically, the prodomain or its immunogen is administered in varied doses to fertile laboratory animals or swine and the reproductive cycles and fertility of the animals monitored, together with assays of serum levels of anti-immunogen or prodomain by routine competitive or sandwich immunoassay.

Substitution derivatives are produced by mutating the DNA in a target codon, so that thereafter a different amino acid is encoded by the codon, with no concomitant change in the number of residues present in the molecule expressed from the mutated DNA. Substitutions or deletions are useful for example in increasing the stability of the proteins herein by eliminating proteolysis sites, wherein residues are substituted within or adjacent to the sites or are deleted from the sites, or by introducing additional disulfide bonds through the substitution of cysteine for other residues. Substitutions are useful for facilitating the synthesis or recovery of mature or prodomain α or β chains. For example, methionine residues within the mature inhibin sequences are substituted or deleted, prepro sequences deleted, methionine inserted at the -1 site immediately $NH_2$ terminal to the mature $NH_2$ terminal residue and another sequence inserted N-terminal to the exogenous methionine. The inhibin derivative in this case is expressed as a fusion having an intermediate methionyl residue, which in turn is cleaved at this residue by cyanogen bromide in accordance with known practice. The mature inhibin derivative released from the fusion is recovered.

Exemplary porcine inhibin derivatives are [$Asn_{266}$->Gln]Inhα (to remove the putative glycosylation site), [$Cys_{325}$ or $Cys_{324}$->Δ]Inhα, [$Cys_{361}$ or $Cys_{363}$->Δ]Inhα, [$Lys_{321}$ or $Lys_{322}$->Δ]Inh$\beta_A$ or [$Lys_{322}$->His or Ser]Inh$\beta_A$ (to inactivate a potential proteolysis site), [$Lys_{315}$->Arg; $Val_{316}$->Thr] Inh$\beta_A$ (to create a $\beta_A/\beta_B$ hybrid), [$Cys_{388}$ or $Cys_{390}$->Δ]Inh$\beta_A$, [$Lys_{411}$->Gln]Inh$\beta_A$, [$Arg_{315}$->Lys, $Val_{316}$->Thr]Inh$\beta_B$ (to create a $\beta_B/\beta_A$ hybrid), [$Cys_{319}$ or $Cys_{320}$->Δ]Inh$\beta_B$ [$Pro_{381}$ $Gly_{382}$-> Pro Phe Gly]Inh$\beta_B$, and [$Arg_{395}$->Gln]Inh$\beta_B$, wherein Inh is an abbreviation for inhibin and the residue members for Inh$\beta_B$ are those used for the corresponding Inh$\beta_A$ residue (see Fig. 2B).

The Inh$\beta_A$ amino acid positions which are principal candidates for mutational substitution or deletion (or adjacent to which residues may be inserted) include residues 293-297, 364-376 and 387-398 (Fig. 8). Preferably, the proline, cysteine and glycine residues within these sequences are not modified. Candidates having greater potency than inhibin or activin, or which serve as inhibin or activin antagonists, are identified by a screening assay wherein the candidate is diluted into solutions containing constant amounts of inhibin or activin and the compositions assayed in the rat pituitary cell assay. Candidates which neither antagonize or agonize inhibin or activin are screened for utility in immunoassays for inhibin or activin by measuring competitive immunodisplacement of labelled inhibin or activin of the native hormones from polyclonal antibody directed against the native hormones. Exemplary contemplated sequence variants of Inh$\beta_A$ include $Phe_{302}$->Ile or Leu; $Gln_{297}$->Asp or Lys; $Trp_{307}$->Tyr or Phe; $Trp_{310}$->Tyr or Phe; $Ile_{311}$->Phe or Val; $Tyr_{317}$->Trp or Thr; $His_{318}$->Lys; $Ala_{319}$->Ser; $Asn_{320}$->Gln, Tyr or His; $Tyr_{321}$->Thr or Asp, $Phe_{340}$->Tyr (a TGF-$\beta/\beta_A$ intrachain hybrid); $His_{353}$->Asp; $His_{353}$->Lys (a $\beta_A/\beta_B$ hybrid); $Phe_{356}$->Tyr; $Val_{364}$->Phe; $Val_{364}$->Leu; $Tyr_{375}$->Thr; $Tyr_{376}$->Trp; $Asn_{389}$->Gln, His or Lys; $Ile_{391}$->Leu or Thr; $Met_{390}$ ->Leu or Ser; $Val_{392}$->Phe, Glu, Thr or Ile. Comparable modifications are made in the human $\beta_B$ chain. For example, Inh$\beta_A$ contains a phenylalanyl residue at position 302, and Inh$\beta_B$ also contains a phenylalanyl residue at a homologous position (264, Fig. 9) when aligned in the same fashion as is shown for porcine $\beta_B$ in Fig. 4A. Thus, since the $Phe_{302}$residue of $\beta_A$ is described above as substituted by isoleucinyl or leucinyl, the $Phe_{264}$ of $\beta_B$ is substituted with the same residues.

A factor in establishing the identity of a polypeptide as inhibin, activin or an inhibin variant is the ability of antisera which are capable of substantially neutralizing the hormonal activity of mature inhibin or activin to also substantially neutralize the hormonal activity of the polypeptide in question. However it will be recognized that immunological identity and hormonal activity are not necessarily coextensive. For example, a neutralizing antibody for inhibin may not bind a candidate protein because the neutralizing antibody happens to not be directed to specifically bind a site on inhibin that is critical to its activity. Instead, the antibody may bind an innocuous region and exert its neutralizing effect by steric hinderance. Therefore a candidate protein mutated in this innocuous region might no longer bind the neutralizing antibody, but it would nonetheless be inhibin in terms of substantial homology and biological activity.

It is important to observe that characteristics such as molecular weight, isoelectric point and the like for a native or wild type mature inhibin or activin obtained from follicular fluid or other tissue sources are descriptive only for the native form. Variants contemplated by the foregoing definition will include other polypeptides which will not exhibit all of the characteristics of native analogue. For example, inhibin derivatives like the insertion mutants, deletion mutants, or fusion proteins described above will bring inhibin outside of the molecular weight established for the corresponding native inhibin because fusion proteins with mature inhibin or proinhibin itself as well as insertion mutants will have a greater molecular weight than native, mature inhibin. On the other hand deletion mutants of native, mature inhibin will have a lower molecular weight. Finally, post-translational processing of preproinhibin chains in heterologous cell lines may not be accomplished with the fidelity exercised by the homologous host cell, thereby resulting in some variation in the amino termini of the $\alpha$ and/or $\beta$ chains. This variation may be encountered as residual prosequence remaining with the mature protein, or the loss of several mature residues that are cleaved off with the prosequence. The same is true with processing of the preprotein in heterologous recombinant cells.

Covalent modifications of inhibin, activin or prodomains are included within the scope hereof and include covalent or aggregative conjugates with other chemical moieties. Covalent derivatives are prepared by linkage of functionalities to groups which are found in the inhibin amino acid side chains or at the N-or C-termini, by means known in the art. For example, these derivatives will include: aliphatic esters or amides of the carboxyl terminus or residues containing carboxyl side chains, e.g., aspartyl residues; O-acyl derivatives of hydroxyl group-containing residues such as seryl or alanyl; and N-acyl derivatives of the amino terminal amino acid or amino-group containing residues, e.g. lysine or arginine. The acyl group is selected from the group of alkyl-moieties (including C3 to C10 normal alkyl), thereby forming alkanoyl species, and carbocyclic or heterocyclic compounds, thereby forming aroyl species. The reactive groups preferably are difunctional compounds known per se for use in cross-linking proteins to insoluble matrices through reactive side groups, e.g. m-Maleimidobenzoyl-N-hydroxy succinimide ester. Preferred derivatization sites are at histidine residues.

Covalent or aggregative derivatives of mature inhibin, activin or prodomain sequences are useful as reagents in immunoassay or for affinity purification procedures. For example, inhibin or prodomain is insolubilized by covalent bonding to cyanogen bromide-activated Sepharose® by methods known per se or adsorbed to polyolefin surfaces (with or without glutaraldehyde cross-linking) for use in the assay or purification of anti-inhibin or anti-prodomain antibodies or cell surface receptors. Inhibin or a prodomain sequence also is labelled with a detectable group, e.g., radioiodinated by the chloramine T procedure, covalently bound to rare earth chelates or conjugated to another fluorescent moiety for use in diagnostic assays, especially for diagnosis of inhibin or prodomain levels in biological samples by competitive-type immunoassays.

DNA which encodes the complete $\alpha$ and $\beta$ chains of inhibin/activin is obtained by chemical synthesis, by screening reverse transcripts of mRNA from ovary, or by screening genomic libraries from any cell. It may be more efficient to simply synthesize portions of the DNA desired since screening is required to identify DNA to cDNA or genomic libraries that encode the $\alpha$ and $\beta$ chains. Synthesis also is advantageous because unique restriction sites can be introduced at the time of preparing the DNA, thereby facilitating the use of the gene in vectors containing restriction sites otherwise not present in the native sequence, and steps can be taken to enhance translational efficiency as discussed above, without the need to further modify the DNA as by mutagenesis or the like. cDNA encoding the α or β chains is free of untranslated intervening sequences (introns) as well as free of flanking DNA encoding other proteins homologous to their source.

DNA encoding the α and β chains is obtained from other sources than porcine and human by (a) obtaining a cDNA library from the ovary of the target animal, (b) conducting Southern analysis with labelled DNA encoding porcine or human α and β chains or fragments thereof (generally, greater than 100 bp) in order to detect clones in the cDNA library that contain homologous sequences, (c) analyzing the clones by restricting enzyme analysis and nucleic acid sequencing so as to identify full-length clones and, if full length clones are not present in the library, recovering appropriate fragments from the various clones and ligating them at restriction sites common to the clones to assemble a clone encoding-the full-length molecule. As shown infra, any sequences missing from the library can be obtained by the 3' extension on ovarian mRNA of synthetic oligodeoxynucleotides complementary to cDNA identified by screening the library, or homologous sequences are supplied from known animal cDNAs. This is particularly useful in constructing pre or prepro inhibin sequences to facilitate processing of preproinhibin to mature inhibin from the desired species.

Porcine and human ovarian cDNA libraries initially were probed for DNA encoding inhibin sequences using labelled oligonucleotides whose sequence was based on the partial amino acid sequence determined from analysis of purified porcine inhibin or, in the case of human cDNA, porcine cDNA probes. However, once having described cDNA encoding human and porcine inhibin and prodomains, one skilled in the art would realize that precisely hybridizing probes can be prepared from the described sequences in order to readily obtain the remainder of the desired human or porcine gene.

Nucleotide sequence analyses of identified porcine and human cDNA clones revealed the structures of the biosynthetic precursors of both forms of inhibin. Interestingly, the two inhibin chains are not derived from a single processed precursor. Instead, the two chains are translated from separate mRNAs and then assembled into the disulfide crosslinked two-chain molecule.

Figs. 1B and 2B and 6, 8 and 9 depict the DNA encoding the polypeptide chains constituting porcine and human preproinhibin and preproactivin. Obviously, degenerate codons may be substituted for those disclosed in these figures where the same amino acid is encoded. The DNA of Figs. 1B, 2B, 6, 8 and 9 is mutated in order to encode the amino acid variants of the α and βchains described above. In particular, the prepro sequences are deleted and a start codon inserted immediately 5' to the mature chain in question so that the chain is expressed directly in recombinant culture. The DNA also is labelled, e.g. with radioactive phosphorous, and used to screen ovarian cDNA libraries from other species to identify α or β chain encoding DNA from such other species as is generally described above.

Covalent labelling of this DNA is accomplished with a detectable substance such as fluorescent group, a radioactive atom or a chemiluminescent group by methods known per se. The labelled DNA is then used in conventional hybridization assays. Such assays are employed in identifying vectors and transformants as described in examples infra, or for in vitro diagnosis such as detection of mRNA in tissues.

Lengthy sequences desirably are synthesized in host cells transformed with vectors containing DNA encoding them, e.g. inhibin or prodomain sequence. Vectors are used to amplify the DNA which encodes the chains, either in order to prepare quantities of DNA for further processing (cloning vectors) or for expression of the chains (expression vectors). An expression vector is a replicable DNA construct in which a DNA sequence encoding an α or β chain is operably linked to suitable control sequences capable of effecting their expression in a suitable host. Cloning vectors need not contain expression control sequences. Such control sequences include a transcriptional promoter, an optional operator sequence to control transcription, a sequence encoding suitable mRNA ribosomal binding sites (for prokaryotic expression), and sequences which control termination of transcription and translation. The vector should include a selection gene to facilitate the stable expression of the desired polypeptide and/or to identify transformants. However, the selection gene for maintaining α and/or β chain expression can be supplied by a separate vector in cotransformation systems using eukaryotic host cells.

Vectors comprise plasmids, viruses (including phage), and integratable DNA fragments i.e., fragments that are integratable into the host genome by recombination. The vectors described herein for use in eukaryotic cell expression of inhibin α and/or β chains contain plasmid sequences for cloning in microbes, where the plasmid replicates autonomously from the host genome, but the DNA is believed to integrate into the eukaryotic host cell genome upon transformation. Similarly, bacillus vectors that genomically integrate by homologous recombination in bacillus also are useful. However, all other forms of vectors which serve an equivalent function and which are, or become, known in the art are suitable for use herein.

Suitable vectors generally will contain replicon (origins of replication, for use in non-integrative vectors) and control sequences which are derived from species compatible with the intended expression host. Transformed host cells are cells which have been transformed or transfected with vectors containing inhibin α and/or β chain encoding DNA. Transformed host cells contain cloned DNA and, when transformed with an expression vector, also express the α and/or β chains. The expressed polypeptides will be deposited intracellularly or secreted into either the periplasmic space or the culture supernatant, depending upon the host cell selected and the presence of suitable processing signals in the expressed protein, e.g. homologous or heterologous signal sequences.

DNA regions are operably linked when they are functionally related to each other. For example, DNA for a presequence or secretory leader is operably linked to DNA for a polypeptide if it is expressed as a preprotein which participates in the secretion of the polypeptide; a promoter is operably linked to a coding sequence if it controls the transcription of the sequence; or a ribosome binding site is operably linked to a coding sequence if it is positioned so as to permit translation. Generally, operably linked means that the DNA sequences being linked are contiguous and, in the case of secretory leaders, contiguous and in reading phase.

Suitable host cells are prokaryotes, yeast or higher eukaryotic cells. Prokaryotes include gram negative or gram positive organisms, for example E. coli or Bacilli. Higher eukaryotic cells include established cell lines of mammalian origin as described below. A preferred host cell is E. coli 294 (ATCC 31,446) although other prokaryotes such as E. coli B, E. coli X1776 (ATCC 31 537), E. coli W3110 (ATCC 27 325), pseudomonas species, or Serratia Marcesans are suitable.

Expression vectors for host cells ordinarily include an origin of replication (where extrachromosomal amplification is desired, as in cloning, the origin will be a bacterial origin), a promoter located upstream from the inhibin coding sequences, together with a ribosome binding side (the ribosome binding or Shine-Dalgarno sequence is only needed for prokaryotic expression), RNA splice site (if the inhibin DNA contains genomic DNA containing one or more introns), a polyadenylation site, and a transcriptional termination sequence. As noted, the skilled artisan will appreciate that certain of these sequences are not required for expression in certain hosts. An expression vector for use with microbes need only contain an origin of replication recognized by the intended host, a promoter which will function in the host and a phenotypic selection gene, for example, a gene encoding proteins conferring antibiotic resistance or supplying an auxotrophic requirement. Inhibin DNA is typically cloned in E. coli using pBR322, a plasmid derived from an E. coli species (Bolivar, et al., 1977 "Gene" 2: 95). pBR322 contains genes for ampicillin and tetracycline resistance and thus provides easy means for identifying transformed cells.

Expression vectors, unlike cloning vectors, must contain a promoter which is recognized by the host organism. This is generally a promotor homologous to the intended host. Promoters most commonly used in recombinant DNA construction include the β-lactamase (penicillinase) and lactose promoter systems (Chang et al., 1978, "Nature", 275: 615; and Goeddel et al., 1979, "Nature" 281: 544), a tryptophan (trp) promoter system (Goeddel et al., 1980, "Nucleic Acids Res." 8: 4057 and EPO Appl. Publ. No. 36,776) and the tac promoter [H. De Boer et al., 1983, "Proc. Nat'l. Acad. Sci. U.S.A." 80: 21-25]. While these are the most commonly used, other known microbial promoters are suitable. Details concerning their nucleotide sequences have been published, enabling a skilled worker operably to ligate them to DNA encoding inhibin in plasmid vectors (Siebenlist et al., 1980, "Cell" 20: 269) and the DNA encoding inhibin or its derivative. Promoters for use in prokaryotic expression systems also will contain a Shine-Dalgarno (S.D.) sequence operably linked to the DNA encoding the inhibin, i.e., the S.D. sequence is positioned so as to facilitate translation. Generally, this means that the promoter and S.D. sequences located upstream from the second codon of a bacterial structural gene are substituted for the sequences of prepro inhibin located 5' to the mature α and/or β chains.

In addition to prokaryotes, eukaryotic microbes such as yeast cultures are transformed with inhibin-encoding vectors. Saccharomyces cerevisiae, or common baker's yeast, is the most commonly used among lower eukaryotic host microorganisms. However, a number of other strains are commonly available and useful herein. Yeast vectors generally will contain an origin of replication from the 2 micron yeast plasmid or an autonomously replicating sequence (ARS), a promoter, DNA encoding the α and/or β chain sequences for polyadenylation and transcription termination, and a selection gene. A suitable plasmid for expression in yeast is YRp7, (Stinchcomb et al., 1979, "Nature", 282: 39; Kingsman et al., 1979, "Gene", 7: 141; Tschemper et al., 1980, "Gene", 10:157). This plasmid already contains the trp1 gene which provides a selection marker for a mutant strain of yeast lacking the ability to grow in tryptophan, for example ATCC No. 44076 or PEP4-1 (Jones, 1977, "Genetics", 85: 12). The presence of the trpl lesion in the yeast host cell genome then provides an effective environment for detecting transformation by growth in the absence of tryptophan.

Suitable promoting sequences in yeast vectors include the promoters for metallothionein, 3-phosphoglycerate kinase (Hitzeman et al., 1980, "J. Biol. Chem.", 255: 2073) or other glycolytic enzymes (Hess et al., 1968, "J. Adv. Enzyme Reg.", 7: 149; and Holland et al., 1978, "Biochemistry", 17, 4900), such as enolase, glyceraldehyde-3-phosphate dehydrogenase, hexokinase, pyruvate decarboxylate, phosphofructokinase, glucose-6-phosphate isomerase, 3-phosphoglycerate mutase, pyruvate kinase, triosephosphate isomerase, phosphoglucose isomerase, and glucokinase. Suitable vectors and promoters for use in yeast expression ar further described in R. Hitzeman et al., EP 73,657A.

Other yeast promoters, which have the additional advantage of transcription controlled by growth conditions, are the promoter regions for alcohol dehydrogenase 2, isocytochrome C, acid phosphatase, degradative enzymes associated with nitrogen metabolism, and the aforementioned metallothionein and glyceraldehyde-3- phosphate dehydrogenase, as well as enzymes responsible for maltose and galactose utilization. In constructing suitable expression plasmids, the termination sequences associated with these genes are also ligated into the expression vector 3' of the inhibin or derivative coding sequences to provide termination and polyadenylation of the mRNA.

Cultures of cells derived from multicellular organisms are the preferred host cells herein because it is believed that expression of hormonally active inhibin or activin will only occur in such cells, with microbial expression resulting at most only in immunological cross-reactivity. In principle, any higher eukaryotic cell culture is workable, whether from vertebrate or invertebrate culture. Propagation of vertebrate cells in culture per se has become a routine procedure in recent years [Tissue Culture, Academic Press, Kruse and Patterson, editors (1973)].

Suitable host cells for expressing α or β chains in higher eukaryotes include: monkey kidney CVI line transformed by SV40 (COS-7, ATCC CRL 1651); baby hamster kidney cells (BHK, ATCC CRL 10); chinese hamster ovary-cells-DHFR (described by Urlaub and Chasin, PNAS (USA) 77: 4216, [1980]); mouse sertoli cells (TM4, Mather, J.P., Biol. Reprod. 23: 243-251 [1980]); monkey kidney cells (CVI ATCC CCL 70); african green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060652, ATCC CCL 51); rat hepatoma cells (HTC, M1, 54, Baumann, M., et al., J. Cell Biol. 85: 1-8 [1980]) and TRI cells (Mather, J.P. et al., Annals N.Y. Acad. Sci. 383: 44-68 [1982]).

The transcriptional and translation control sequences in vertebrate cell expression vectors preferably are provided from viral sources. For example, commonly used promoters are derived from polyoma, Adenovirus 2, and most preferably Simian Virus 40 (SV40). The early and late promoters of SV40 are particularly useful because both are obtained easily from the virus as a fragment which also contains the SV40 viral origin of replication (Fiers et al., 1978, "Nature", 273: 113). Smaller or larger SV40 fragments may also be used, provided the approximately 250 bp sequence extending from the Hind III site toward the Bgl I site located in the viral origin of the replication is included. Further, it is also possible to utilize the genomic promoters, control and/or signal sequences normally associated with the α or β-chains, provided each control sequences are compatible with and recognized by the host cell.

An origin of replication may be provided either by construction of the vector to include an exogenous origin, such as may be obtained from SV40 or other viral (e.g. Polyoma, Adenovirus, VSV, or BFV) source, or may be provided by the host cell chromosomal replication mechanism. If the vector is integrated into the host cell chromosome, the latter is often sufficient.

Rather than using vectors which contain viral origins of replication, mammalian cells are cotransformed with DNA encoding a selectable marker and DNA encoding the α and/or β chains. An example of a suitable selectable marker is dihydrofolate reductase (DHFR) or thymidine kinase. Such markers are proteins, generally enzymes that enable the identification of transformant cells, i.e., cells which had been competent to take up exogenous DNA. Generally, identification is by survival of transformants in culture medium that is toxic to untransformed cells or from which the cells cannot obtain a critical nutrient without having taken up the marker protein.

In selecting a preferred host mammalian cell for transfection by vectors which comprise DNA sequences encoding both inhibin and DHFR, it is appropriate to select the host according to the type of DHFR protein employed. If wild type DHFR protein is employed, it is preferable to select a host cell which is deficient in DHFR thus permitting the use of the DHFR coding sequence as marker for successful transfection in selective medium which lacks hypoxanthine, glycine, and thymidine ($hgt^-$). An appropriate host cell in this case is the Chinese hamster ovary (CHO) cell line deficient in DHFR activity, prepared and propagated as described by Urlaub and Chasin, 1980, "Proc., Nat'l. Acad. Sci." (USA) 77: 4216.

On the other hand, if DNA encoding DHFR protein with low binding affinity for methotrexate (MTX) is used as the controlling sequence, it is not necessary to use DHFR resistant cells. Because the mutant DHFR is resistant to MTX, MTX containing media can be used as a means of selection provided that the host cells are themselves MTX sensitive. Most eukaryotic cells which are capable of absorbing MTX appear to be methotrexate sensitive. One such useful cell line is a CHO line, CHO-KI (ATCC No. CCL 61). Preferably, transformants are first selected for neomycin resistance (the transfection is conducted together with DNA encoding the neomycin resistance gene), followed by MTX amplification of the α and/or β chain expression as the case may be. See Kim et al., "Cell" 42: 129-138 (1985) and EP 160,457A.

Other methods suitable for adaptation to the synthesis of α and/or β chains in recombinant vertebrate cell culture are described in M-J. Gething et al., "Nature" 293: 620-625 (1981); N. Mantei et al., "Nature" 281: 40-46; and A. Levinson et al., EP 117,060A and 117,058A.

The inhibin α chain is expressed in recombinant cell culture with or without either of the β-chain molecules. Similarly, host cells are transformed with DNA encoding either or both of the mature β-chains. Based on analogy to TGF-β, the mature β-chains are capable of forming homodimers or $\beta_A/\beta_B$ heterodimers upon expression in recombinant culture. These structures are not inhibin and will be referred to herein as β-chain dimers or activin. These are useful in the preparation of active inhibin, serving as sources of the β-chain, or are used as gel electrophoresis standards to detect the diversion into β-chain dimers of β-chains synthesized in α and β chain cotransformants. As will be seen in Example 4, this is not a hypothetical problem. Of course, the dimers are also useful in modulating reproduction as noted above.

β-chain hetero or homodimers are separated by in vitro unfolding of the individual chains followed by oxidative disulfide bond formation with the α-chain in accord with processes generally known per se. Preferably, however, in preparing mature inhibin the recombinant host is transformed with DNA encoding both the α and either of the β-chains. The intact hormonally active molecule is then assembled by the host cell in vivo, and it is thus unnecessary to combing the two chains by in vitro processing. The DNA encoding the α and β-chains is preferably located on the same vector, and under the control of the same promoter, but this is not essential.

Certain β-chain amino acid sequence variants identified in the screening procedure will not bind to pituitary cell surface receptors nor as a consequence will they exhibit hormonal activity. Such variants, when expressed as homodimers in recombinant cell culture, are useful in immunoassays for activin when they bear immunological epitopes cross-reactive with the native β-chain. In addition, such variants are coexpressed with DNA encoding hormonally active β-chain to yield a hybrid bearing native and variant β-chain. In this case the variant serves to stabilize the structure of the native β-chain. This form of β-chain heterodimer is useful, like the homodimer, in immunoassays for activin. It may also function as an activin antagonist.

The activin/inhibin β-chains also are coexpressed with TGF-β in order to produce β-chain/TGF-β hybrids. Vectors and methods for the expression of TGF-β are known. For example, see Derynck et al., Human Transforming Growth Factor-β Complementary DNA Sequence and Expression in Normal and Transformed Cells "Nature" 316: 701-705 (1985). Contransformation of mammalian host cells by vectors bearing the TGF-β gene as described by Derynck et al. together with the $\beta_A$ or $\beta_B$ chains of activin/inhibin will result in secretion of a proportion of a β-chain/TGF-β hybrid dimers. This hybrid is useful in preparing TGF-β/β-chain immunogens or in immunoassays.

Inhibin, activin or prodomain sequences are recovered from transformed cells in accord with per se known procedures. When a polypeptide is expressed in recombinant bacteria as a refractile body, the desired polypeptide is recovered and refolded by conventional methods. Alternatively, the culture supernatants from transformed cells that secrete activin or inhibin, preferably mammalian cells, are simply separated from the cells by centrifugation. Then the inhibin generally is purified by successive purification procedures that include heparin-Sepharose affinity chromatography, gel filtration and at least one and preferably several RP-HPLC (reverse phase high pressure liquid chromatography) steps using different conditions in the stationary phase and/or mobile phase. Prodomain sequences produced by in vitro synthesis will be purified by conventional methods.

The prodomain polypeptides that are preferred for use herein are recovered from the culture media of recombinant cells transformed to synthesize the α and/or β chains as appropriate for the desired prodomain. Specifically, they are recovered by separating the culture medium polypeptides on native electrophoresis gel, excising bands having the predicted molecular weight and thereafter purifying the eluted polypeptides further, for example by FPLC or HPLC, followed by amino acid sequence determination for the substantially homogeneous separated polypeptides. Purified prodomain polypeptides then are used to raise antibodies, e.g., in rabbits, which when used in immunoaffinity purification will simplify the recovery of the prodomains.

In the preferred procedure for isolating porcine hormonally active inhibin, clarified transformant culture supernatant or cell lysate is first purified by heparin-Sepharose® affinity chromatography, next by gel filtration on Sephacryl® S-200 gel and then with four successive RP-HPLCs using different mobile phase gradients and/or derivatized silica supports. Preferably, stationary phases having relatively low hydrophobicity are used, with C3-C8 columns being preferred and C3-C5 and phenyl columns being particularly preferred. Solute specificity of the mobile phase is preferably adjusted by varying the concentration of an organic component, particularly acetonitrile. Although a single RP-HPLC fractionation significantly increases the purity relative to the gel-filtrated material, two or more, and preferably four, RP-HPLC purifications are generally performed subsequent to successive treatment by heparin-Sepharose chromatography and gel filtration. This method has been found to be adaptable to the purification of human inhibin from recombinant cell culture as well.

The first step of the purification is heparin-Sepharose affinity chromatography, in which the protein is adsorbed to the Sepharose-bound heparin moieties under application conditions, and the adsorbed inhibin material is recovered by 1M NaCl elution. This step greatly expedites the purification procedure for crude extracts because it allows a relatively large volume of a crude extract to be processed fairly rapidly while recovering an amount of protein exhibiting total inhibin activity equal to at least 90% of that of the crude extract.

For the detection of inhibin activity in the various column fractions, aliquots ranging from 0.01% to 0.1% by volume are removed, and after added 100 μg human serum albumin in 100 μl water, the solvents were evaporated in a Speed-Vac concentrator (Savant, Hicksville, N.Y.). The residue as redissolved in 3 ml 1% fetal bovine serum in HDMEM, filtered through a Millex-GS 0.22 μm filter (Millipore Corp., Bedford, MA) and assayed in duplicate. To speed up the bioassays during the purification process, only basal inhibition of FSH secretion exerted by the inhibin activity is determined and plotted in the region where the inhibin proteins were expected to migrate in the chromatograms.

To perform the heparin-Sepharose affinity chromatography, cell debris is spun down in a Beckman J2-21 centrifuge (Beckman Instruments, Inc., Palo Alto, CA.) using a JA-20 rotor at 10,000 rpm for 30 minutes. One half of the supernatant is diluted in 10 times its volume by the addition of 0.01 M Tris-HCl containing 0.1 M NaCl, pH 7, in an Erlenmeyer flask and pumped simultaneously via silastic tubes (0.76 mm ID) into heparin-Sepharose® (Pharmacia Fine Chemicals, Piscataway, N.J.) columns (3.5 x 9 cm) by two Rabbit 4-channel peristaltic pumps (Rainin Instrument Co., Inc., Emeryville, CA) at 40 ml/hr per column. After all the fluid has been pumped through the heparin-Sepharose, the eight columns are washed simultaneously with 0.01 M Tris-HCl, pH 7, containing 0.1 M NaCl in the same manner. The adsorbed proteins with inhibin activity are removed by washing the eight columns simultaneously with 0.01 M Tris-HCl containing 1 M NaCl, pH 7, as above, and the wash is collected into fractions. The inhibin activity is monitored by the in vitro bioassay described above. The columns are regenerated by further washing with 2M NaCl in 0.01 M Tris-HCl, pH 7, and re-equilibrated with 0.01 M Tris-HCl containing 0.1 M NaCl for purification of remaining extract.

Next, the material is fractionated by gel filtration to separate proteins generally according to their molecular weights. The fractions having inhibin activity extracted by the heparin-Sepharose columns were pooled and dialyzed overnight to remove NaCl in a 28.6 mm cylinder diameter Spectrapor No. 3 membrane tubing with $M_r$ cutoff at 3,500 (Spectrum Medical Industries, Inc., Los Angeles, CA.) against 30% acetic acid. The retained fluid is centrifuged, as above, to remove a white precipitate, and the supernatant is divided into equal portions for applying to 5 x 100 cm Sephacryl S-200 superfine columns (Pharmacia Fine Chemicals, Piscataway, N.J.). Each column is eluted with 30% acetic acid at 20 ml for 22 min., and the column fractions are monitored by UV absorption at 280 nm and by bioassay.

The bioassay-positive protein from the S-200 columns is pooled and lyophilized. The lyophilized material is dissolved in 0.2N acetic acid (1 ml/ml) and filtered through a Millex-HA 0.45 μm filter (Millipore Corp., Bedford, MA.). The filtrate is applied directly onto a 1 x 25 cm Vydac 5-μm particle-size C4 column (The Separations Group Hesperia, CA.) and developed with a gradient of TEAP buffer. In the TEAP system, buffer A consists of 0.25 N triethylammonium phosphate pH 3, and buffer B is 80% acetonitrile in buffer A. After all the filtrate had been loaded, the column is washed with the aqueous buffer A until the UV absorption reached baseline. The fractions exhibiting inhibin activity are separated in a Beckman 332 gradient liquid chromatography system (Beckman Instruments, Inc., Berkeley, CA.) equipped with a Spectroflow 757 UV detector (Kratos Analytical Instruments, Ramsey, N.J.), a Soltec 220 recorder (Soltec Corp,, Sun Valley, CA.) and a Redirac 2112 fraction collector (LKB Instruments, Inc., Gathersburg, MD). Zones of inhibin activity are detected by bioassay.

Inhibin protein containing the $\beta_B$ chain is further purified free of inhibin containing the $\beta_A$ species, if desired, by two more RP-HPLC steps. The first step uses a 1 x 25 cm Vydac 5-μm-particle-size C4 column and a trifluoroacetic acid (TFA) buffer system and the second step employs a 1 x 25 cm Vydac 5-μm-particle-size Phenyl column and the TEAP buffer system. In the TFA system, buffer A contains 1 ml trifluoroacetic acid in 999 ml water and buffer B is 1 ml trifluoroacetic acid in 199 ml water and 800 ml acetonitrile. The two inhibin species elute separately. Inhibin accumulated from a few batches was concentrated by RP-HPLC using a 0.46 x 25 cm Aquapore RF-300 10 μm-particle-size column (Brownlee Labs., Santa Clara, CA.) and the TFA buffer system. Ordinarily, however, this purification step will not be used with cell culture supernatants from transformants with DNA encoding only the $\beta_A$ or $\beta_B$ chains.

Inhibin, activin, prodomain sequences or their variants are administered in the form of pharmaceutically acceptable nontoxic salts, such as acid addition salts or metal complexes e.g., with zinc, iron or the like (which are considered as salts for purposes of this application). Illustrative of such acid addition salts are hydrochloride, hydrobromide, sulphate, phosphate, maleate, acetate, citrate, benzoate, succinate, malate, ascorbate, tartrate and the like. Intravenous administration in isotonic saline, phosphate buffer solutions or the like is suitable.

The polypeptide herein should be administered under the guidance of a physician, and pharmaceutical compositions will usually contain an effective amount of the peptide in conjunction with a conventional, pharmaceutically-acceptable carrier. The dosage will vary depending upon the specific purpose for which the protein is being administered, and dosage levels in the range of about 0.1 to about 1 milligram per Kg. of body weight may be used when inhibin is administered on a regular basis as a male contraceptive.

Inhibin, activin, prodomain sequences or their variants desirably are administered from an implantable or skin-adhesive sustained-release article. Examples of suitable systems include copolymers of L-glutamic acid and gamma ethyl-L-glutamate (U. Sidman et al., 1983, "Biopolymers" 22(1): 547-556), poly (2-hydroxyethyl-methacrylate) (R. Langer et al., 1981, "J. Biomed. Mater. Res." 15: 167-277 and R. Langer, 1982, "Chem. Tech." 12: 98-105) ethylene vinyl acetate (R. Langer et al., Id.), or poly-D-(-)-3- Hydroxybutyric acid (EP 133,988A). Such articles are implanted subcutaneously or are placed into contact with the skin or mucous membranes.

In order to simplify the Examples certain frequently occurring methods will be referenced by shorthand phrases.

Plasmids are designated by a low case p preceded and/or followed by capital letters and/or numbers. The starting plasmids herein are commercially available, are publicly available on an unrestricted basis, or can be constructed from publicly available plasmids or DNA in accord with published procedures. In addition, other equivalent plasmids are known in the art and will be apparent to the ordinary artisan.

"Digestion" of DNA refers to catalytic cleavage of the DNA with an enzyme that acts only at certain locations in the DNA. Such enzymes are called restriction enzymes, and the sites for which each is specific is called a restriction site. "Partial" digestion refers to incomplete digestion by a restriction enzyme, i.e, conditions are chosen that result in cleavage of some but not all of the sites for a given restriction endonuclease in a DNA substrate. The various restriction enzymes used herein are commercially available and their reaction conditions, cofactors and other requirements as established by the enzyme suppliers were used. Restriction enzymes commonly are designed by abbreviations composed of a capital letter followed by other letters and then, generally, a number representing the microorganism from which each restriction enzyme originally was obtained. In generally, about 1 μg of plasmid or DNA fragment is used with about 1 unit of enzyme in about 20 μl of buffer solution. Appropriate buffers and substrate amounts for particular restriction enzymes are specified by the manufacturer. Incubation times of about 1 hour at 37 °C are ordinarily used, but may vary in accordance with the supplier's instructions. After incubation, protein is removed by extraction with phenol and chloroform, and the digested nucleic acid is recovered from the aqueous fraction by precipitation with ethanol. Digestion with a restriction enzyme infrequently is followed with bacterial alkaline phosphatase hydrolysis of the terminal 5' phosphates to prevent the two restriction cleaved ends of a DNA fragment from "circularizing" or forming a closed loop that would impede insertion of another DNA fragment at the restriction site. Unless otherwise stated, digestion of plasmids is not followed by 5' terminal dephosphorylation. Procedures and reagents for dephosphorylation are conventional (T. Maniatis et al., 1982, Molecular Cloning pp. 133-134).

"Recovery" or "isolation" of a given fragment of DNA from a restriction digest means separation of the digest on polyacrylamide gel electrophoresis, identification of the fragment of interest by comparison of its mobility versus that of market DNA fragments of known molecular weight, removal of the gel section containing the desired fragment, and separation of the gel from DNA. This procedure is known generally. For example, see R. Lawn et al., 1981, "Nucleic Acids Res." 9: 6103-6114, and D. Goeddel et al., 1980, "Nucleic Acids Res." 8: 4057.

"Southern Analysis" is a method by which the presence of DNA sequences in a digest or DNA-containing composition is confirmed by hybridization to a known, labelled oligonucleotide or DNA fragment. For the purposes herein, unless otherwise provided, Southern analysis shall mean separation of digests on 1 percent agarose, denaturation and transfer to nitrocellulose by the method of E. Southern, 1975, "J. Mol. Biol." 98: 503-517, and hybridization as described by T. Maniatis et al., 1978, "Cell" 15: 687-701.

"Transformation" means introducing DNA into an organism so that the DNA is replicable, either as an extrachromosomal element or chromosomal integrant. Unless otherwise provided, the method used herein for transformation of E. coli is the $CaCl_2$ method of Mandel et al., 1970, "J. Mol. Biol." 53: 154.

"Ligation" refers to the process of forming phosphodiester bonds between two double stranded nucleic acid fragments (T. Maniatis et al., Id., p. 146). Unless otherwise provided, ligation may be accomplished using known buffers and conditions with 10 units of T4 DNA ligase ("ligase") per 0.5 μg of approximately equimolar amounts of the DNA fragments to be ligated.

"Preparation" of DNA from transformants means isolating plasmid DNA from microbial culture. Unless otherwise provided, the alkaline/SDS method of Maniatis et al., Id. p. 90., may be used.

"Oligonucleotides" are short length single or double stranded polydeoxynucleotides which are chemically synthesized by known methods and then purified on polyacrylamide gels.

## EXAMPLE 1

### Isolation of Cloned Inhibin α-Subunit cDNAs

The strategy for identification of clones containing coding sequences for the porcine inhibin subunits was based on the "long-probe" approach, successful in some previous instances (Anderson et al., 1983, "Proc. Nat. Acad. Sci. USA" 80:6836-6842 and Ullrich et al., 1984, "Nature" 309:418-425). Briefly, a high-complexity cDNA library constructed in λgt10 and derived from porcine ovarian mRNA by oligo-dT-primed cDNA synthesis was screened with a single 64-base-long synthetic oligodeoxynucleotide directed against the N-terminal amino acid sequence of the α-chain of porcine inhibin. It was found that the library is to be prepared from fresh ovarian tissue because the inhibin chain mRNA was apparently quite labile. Approximately 1 in 2,000 plaques hybridized with this probe, and sequence analysis of several hybridizing cloned cDNAs confirmed correct probe identification. This analysis revealed that none of the characterized cDNAs contained sufficient sequence information to predict the complete structure of the α-chain precursor protein. Rather than analyzing more clones from the same cDNA library, a second library was constructed by 3' extension on ovarian mRNA of a synthetic oligodeoxynucleotide complementary to a sequenced region encoding α precursor residues 60-64 (Fig. 1A). This library was screened with a suitable restriction fragment from a previously analyzed cDNA and yielded several isolates which specified the remainder of the DNA sequences encoding the N-terminal region of the α precursor. Completeness of the coding sequence was judged from the presence of a long reading frame which specifies the porcine α-chain peptide sequence and starts with a methionine codon preceded by an in-frame stop codon and followed by a hydrophobic sequence bearing the hallmarks of a signal peptide. The full sequences for the precursor protein and its cDNA are shown in Fig. 1B. The complete protein including signal peptide has an Mr of ~40K consisting of 364 amino acids, of which the C-terminal 134 ($M_r$ ~14.5K) constitute the porcine inhibin α-chain. There are several Arg-Arg sequences in the proregion of the precursor, one of them directly preceding the α subunit. We believe that this latter pair of basic residues is the processing site for the proteolytic release of the α peptide. The deduced precursor sequence predicts two N-linked glycosylation sites, one within the α chain proper.

In addition to the coding region, the cDNA sequence contains a 3'-untranslated sequence of 167 nucleotides, including the canonical AATAAA polyadenylation signal, and a 5'-untranslated region, the proper length of which is presently unknown.

The detailed method was as follows:

Polyadenylated mRNA was prepared from freshly frozen porcine ovaries (Kaplan et al., "J. Biochem." 183: 181-184) An oligo-dT-primed cDNA library of ~6x$10^6$ clones in λgt10 (Huynh et al., 1984, DNA Cloning Techniques., Ed. D. Clover) was prepared form 5 μg polyA+ mRNA as described by Wood et al., "Nature" 312: 330-337 (1984), except that the EcoRI adaptors used had the sequence

5'-AATTCACTCGAGACGC-3'
3'-GTGAGCTCTGCG-5'P.

Approximately 1 x $10^6$ unamplified cDNA clones were screened with 5 α-subunit oligonucleotide

5'-ACCGCCCCTTTGCCTTGGCCTTGGTCCCCTGCTGCTCTGAGACTGCTGCAGAGACCTCCTGAGG-3',

based on the amino acid sequence underlined in Fig. 1B. Hybridization was carried out with the phosphorylated $^{32}P$-labelled probe in 5xSSC, 40% formamide at 37°C. Filters were washed at 50°C with 1xSSC, 0.1% SDS. Approximately 500 hybridization positive clones were obtained, twelve of which were purified and examined for insert size. The EcoR1 inserts of five of these (λPIN-α2, -α5A, -α5, -α9, -α10) were subcloned into M13 derivatives (Messing et al., 1981 "Nucl. Acids Res." 9:309-321) and sequenced by the dideoxy chain termination method of Sanger et al.,"Proc. Nat. Acad. Sci. USA" 74:5463-5467 (1977). A specifically primed library was prepared by priming 5 μg of polyA$^+$ mRNA with the oligonucleotide 5'-CCCCACAGCATGTCTT-3' (complementary to nucleotides 248-263) and subsequent cloning into λgt10. Approximately 2x$10^5$ clones of the 1x$10^6$ clones obtained were screened with the 5' 100bp EcoRI-BamHI fragment prepared from λPIN-α2. Twelve of the 170 hybridization positive clones obtained were purified and two (λPIN-S12s, -S4s) were sequenced by the dideoxy method. The complete nucleotide sequences of the α-subunit cDNAs were obtained by subcloning various restriction fragments with the different X isolates into the M13 phage derivatives. Compressions were resolved by the use of deoxyinosine mixes in combination with the E. coli single stranded binding protein (Pharmacia).

Isolation of Cloned Inhibin β Subunit cDNAS

The cDNA sequences encoding the precursors of the inhibin β subunits were obtained from the same cDNA libraries used for the α subunit. Overlapping cDNA clones were isolated by screening first with single long synthetic oligodeoxynucleotide probes based on the two N-terminal β subunit sequences and subsequently with suitable restriction fragments derived from characterized cDNA clones which served as probes for "walking" in both 5' and 3' directions (Fig. 2A).

In more detail, approximately 2x$10^5$ oligo-dT primed ovarian cDNA clones were screened with the 5' end labelled $\beta_A$ oligonucleotide,

5'-AAGAAGCAGTTCTTTGTGTCCTTCAAGGACATTGGCTGGAATGACTGGATCATTGC-3'

based on the amino acid sequence of residues 321-339. Five hybridization positives were obtained, of which three proved to contain $\beta_A$ coding sequences (λPIN-βA2, -$\beta_A$4, -$\beta_A$8). A 5' end 154 bp EcoRI-HindIII (nucleotides 158-297) fragment and a 3' end 213 bp EcoRI-Pst fragment (nucleotides 1679-1892) derived from λPINβA2 were used to screen 2x$10^6$ oligo-dT primed cDNA clones and 2x$10^5$ clones from the α-chain specifically primed library. Out of the sixteen clones analyzed in detail two were found to have longer 5' ends (λPIN-$\beta_A$5s, -$\beta_A$22) and one clone λPIN-$\beta_A$21 contained the entire 3'-untranslated region. Porcine inhibin $\beta_B$ subunit cDNA clones were isolated by screening 2x$10^5$ clones from the specifically primed library with the $\beta_B$ oligonucleotide

5'-GGCCTGGAGTGTGATGGGAGAACCAACCTGTCCTGCCGCCAGGAATTTTTCATCGATTTCAGGCT-3',

which was based on the $NH_2$-terminal sequence described in Fig. 1A. Positive clones were further screened with the oligonucleotide inosine probe 5'-AAITCTATIAAIAA$^{T}_{C}$ TG$^{T}_{C}$ -3' ("I" in this sequence stands for inosine), which covers all the possibilities in the non-coding strand for the amino acid sequence QQFFIDF. Two clones (λPIN$\beta_B$-1s, -2s) were isolated and sequenced and found to code for the $\beta_B$ subunit. A 230 bp EcoRI-Sma (nucleotides 21-251) fragment was isolated from λPIN$\beta_B$-I and used as a hybridization probe to screen 2x$10^6$ oligo-dT primed cDNA clones. Two positives were obtained (λPIN$\beta_B$-3,4). The nucleotide sequence of these overlapping clones was used to construct the sequence shown. All sequences were obtained by subcloning specific fragments into M13 phage vectors (Messing et al., op cit.). The EcoRI restriction sites referred to above are all contained within the cDNA adaptor fragment, and do not refer to sequences present in the cDNA.

We noted that only very few clones from the oligo-dT-primed library (4 out of 2x$10^5$) hybridized with the synthetic probe for the β-subunit of inhibin A. Although most of these proved correct by DNA sequence analysis, none contained a full 3'-untranslated region, as judged by the absence of a polyA homopolymer at their 3' ends. Absence of polyA tails suggested the existence of a very long 3'-untranslated sequence in this mRNA species and/or structural region(s) which prove difficult to copy by the polymerases used for library construction. Unexpectedly, a higher abundance (~10-fold) of inhibin $\beta_A$ subunit coding sequences was found in the cDNA library made by specific priming on α-subunit mRNA. This library was screened with the synthetic probe for the β-chain of inhibin A on the subsequently refuted theory that the α precursor mRNA might also encode the β subunit. The high abundance of inhibin $\beta_A$ cDNA in this library was later traced to fortuitous complementarity of the specific α chain primer to a region in the 3'-untranslated portion of the corresponding mRNA.

Only four cloned cDNAs encoding the β subunit of inhibin B were found in our libraries. The sequence information obtained from these clones failed to reveal the complete structure of the corresponding precursor protein and its cDNA. The sequences of cDNAs and deduced protein structures for the precursors of the β subunits are compared in Fig. 2B. The nucleotide sequence of inhibin $\beta_A$ subunit cDNA is 3.6 kb in length and contains an open reading frame for a protein of 425 amino acids (Mr ~46K), the C-terminal 116 residues of which represent the β subunit proper (Mr ~13K). This reading frame begins with a methionine codon followed by a sequence that codes for a characteristic signal peptide, the true length of which is believed to be 29 residues. The encoded β subunit is preceded by a string of 5 arginines at which it is presumably proteolytically cleaved from the precursor. Similar to the α subunit precursor, this β precursor contains several additional pairs of basic residues at which hitherto unknown biologically active peptide entities are believed to be released. It also contains one possible site of N-linked glycosylation in the proregion (Asn, residue 165).

The deduced protein sequence for the β subunit of inhibin B shows high homology with the $\beta_A$ subunit sequence. 71 amino acid residues are identical and most changes are conservative in nature. Sequence homology, although of a lesser degree, is also found in the proregion of both β subunit precursors. Interestingly, an extremely purine-rich sequence rarely seen in coding regions but present in the cDNA encoding the inhibin $\beta_A$ precursor and resulting in a curious amino acid sequence is not found in the cDNA which codes for the homologous $\beta_B$ precursor. This results in a gap of 22 amino acid residues from the $\beta_B$ precursor of inhibin when protein sequences are aligned for maximal homology. Such alignment also brings about a perfect match in the cysteine positions of both precursors (see Fig. 2B).

Northern Analysis of α and chain Precursor mRNAS

Ovarian total and polyadenylated RNAs were analyzed by the Northern procedure using the sequenced cDNAs as probes to assess size and relative abundance of the mRNAs which encode the peptide subunits α and β and $\beta_B$ of the heterodimeric inhibin molecule. Polyadenylated mRNA (2 μg: lanes a, b, c, and f; 8 μg: lane d) and total RNA (10 μg: lanes e and g) were electrophoresed into a formaldehyde 1.2% agarose gel and blotted onto nitro-cellulose filters. The following $^{32}$P-labelled cDNA fragments were used as hybridization probes under stringent conditions. Lane a: 240 bp EcoRI-SmaI (nucleotides 134-371) from α subunit cDNA; b: 154 pb EcoRI-HindIII (nucleotides 158-297) from $\beta_A$ subunit cDNA; c: 230 bp EcoRI-Sma (nucleotides 21-251) from $\beta_B$ subunit cDNA; d and e: EcoRI insert of λPIN-α2; f and g: EcoRI insert of λPIN-$\beta_A$5. Filters were washed for 2 hours with 3 changes of 0.1xSSC, 0.1% SDS at 60°C.

Analysis showed (Fig. 3) that α and β mRNAs are of different size and abundance, as indicated by results obtained from cDNA cloning. From their respective band intensities the α precursor mRNA is estimated to be at least of 10-fold higher abundance than the mRNA for the $\beta_A$ precursor, and approximately 20-fold higher than the mRNA for the $\beta_B$ precursor.

Using ribosomal RNAs as size standards, the α precursor mRNA, which is a single species, is ~1500 nucleotides in length, a size in good agreement with the cloned cDNA sequence (Fig. 1B). $β_A$ precursor mRNA sequences are represented by two main species of ~4.5 and ~7.2 kb in length. The relatively higher intensity of both species in polyadenylated than total RNA suggests that the 4.5 kb species does not represent 28S RNA which hybridized to the cDNA probe. Thus, the β precursor cDNA sequences shown in Fig. 2B are thought to represent the 4.5 kb mRNA, suggesting that the 5' untranslated region for the $β_A$ mRNA is approximately 900 nucleotides long. The $β_B$ precursor is encoded on one mRNA, of approximately 4.5 kb in size, which is present at roughly half the level of the two $β_A$ mRNAs. Since the two β mRNAs are closely related, one can predict that both mRNAs have a similar structure and thus the $β_B$ mRNA presumably possesses a long 5' and 3' untranslated region equivalent to that shown for the $β_A$ mRNA. Choice of a different polyadenylation signal might explain the existence of the 7.2 kb species.

Homology To Transforming Growth Factor-β

The mature α and β inhibin subunits contain seven and nine cysteine residues respectively. Upon alignment of the cysteine residues it is apparent that the two subunits share a similar cysteine distribution and some sequence homology exists around these residues (Fig. 4), suggesting that both types of subunits derive from one ancestral gene. Surprisingly, significant homology was found between the β chain and the primary structure of human TGF-β recently determined. As outlined in Fig. 4, both peptides are of nearly equal length (inhibin $β_A$ subunit, 116, $β_B$ subunit 115; TGFS, 116 residues) and show a strikingly similar distribution of their nine cysteine residues. Using this cysteine "skeleton" for alignment, the $β_A$ and TGF-β sequences have an additional 31 residues in identical positions and show conservative changes in nine homologous places. Similar high homologies are seen upon comparison of the $β_B$ and β-TGF. Some gaps were introduced for better alignment (Fig. 4). The overall homology reaches 35%, but approaches 60% in certain sections (cf. porcine inhibin $β_A$ chain residues 11-45 and TGF residues 15-49), a very high degree of homology considering the difference in species. Interestingly, this homology extends beyond the termination codon for protein synthesis in the respective cDNAs. Thus, the cDNAs for TGF-β and both inhibin β subunits contain a highly G and C rich sequence in this region, and they also possess unusually long 5' and 3' untranslated regions.

One can discount the suggestion that the β subunit of inhibin is the porcine equivalent of human TGF-β, since there is almost absolute homology between human and murine β-TGFs. These findings strongly indicate that both inhibin subunits and TGF-β have a common ancestor and belong to one gene family. All three peptides are derived from similarly-sized precursors ($M_r$ ~40K) where they occupy the C-terminal 110 or so residues and are released by proteolytic cleavage at pairs of arginines. They form homo- or heterodimers, and subunits in the biologically active complex are linked by disulfide bridges. However, there is little sequence homology between TGF-β and the β subunits in the pro parts of their precursors, although the regions comprising the odd residues which precede the β subunit and TGF peptides display limited but significant sequence relatedness.

EXAMPLE 2

Recombinant Synthesis of Porcine Inhibin

The plasmid used for recombinant synthesis of porcine inhibin was pSVE-Pα$B_A$Inh-DHFR. The procedure to construct this plasmid is shown in Fig. 5. This plasmid was constructed as follows:

pHBS348-E (EP 0073656A) was partially digested with EcoRI, blunted with E. coli DNA polymerase I (Klenow fragment) and the four dNTPs, ligated and the ligation mixture was transformed into E. coli 294 (ATCC 31446). The transformed culture was plated on ampicillin media plates and resistant colonies selected. Plasmids were screened for the loss of the EcoRI site preceding the SV40 early promoter. A plasmid having the site deleted is referred to as pHBS348-EII.

pHBS348-EII was digested with EcoRI and EcoRI to produce two fragments, fragment I containing the SV40 early promoter, pmL-$Amp^r$ sequences and the HBsAg 3' untranslated region and fragment 2 containing the HBsAg (hepatitis B antigen) coding sequences.

λPIN$β_A 5_S$ containing the coding region for the porcine inhibin $β_A$ subunit was digested with EcoRI and SmaI and the 1335 bp fragment (fragment 3) containing the $β_A$ coding region recovered by polyacrylamide gel electrophoresis. Fragment I, recovered by agarose gel electrophoresis, was ligated to fragment 3 and the ligation mixture transformed into E. coli strain 294 (ATCC 31446). The transformed culture was plated on ampicillin media plates and resistant colonies selected. Plasmid DNA was prepared from transformants and checked by restriction analysis for the presence of the correct DNA fragments. This plasmid is referred to as pSVE-p$β_A$Inh.

pHBS348-E (EP 0073656A) was partially digested with EcoRI, blunted with E. coli DNA polymerase I (Klenow fragment) and the four dNTPs, ligated to the synthetic oligonucleotide 5' GGTCGACC-3' containing the SalI rec-

ognition site. The transformed culture was plated on ampicillin media plates and resistant colonies selected. Plasmids were screened for the presence of the extra Sail restriction site. Plasmid DNA is prepared from this construction (pHBS348-ESalI).

λPINα-12s and λPINα-2 were digested with EcoRI and BamHI. A 104 bpEcoRI-BamHI fragment from λPINα-12s containing the 5' coding region and a 1246 bp EcoRI-BamHI fragment from λPINα-2 containing the middle and 3' coding region were recovered and ligated together. The ligation mixture was digested with EcoRI, the enzyme heat denatured, and the mixture ligated to EcoRI-digested pUC9 (BRL). Recombinants were selected and confirmed by restriction analysis. DNA was prepared from the correct plasmid (pPINα).

pPINα, containing the complete coding region for porcine α-inhibin was digested with NcoI and EcoRI, filled in by Pol(I)K in the presence of 4dNTP's, the 1280 bp fragment (fragment 4) was recovered by gel electrophoresis. pHBS348-ESalI was digested with SstII and HindIII, filled in by Pol(I)K in the presence of 4dNTP's, and fragment 5 containing the PML-Amp$^r$ region, SV40 early promoter and HBsAg 3' untranslated region was recovered by gel electrophoresis. Fragments 4 and 5 were ligated together and the ligation mixture used to transform E. coli 294 (ATCC 31446). Recombinants were selected by growing on Ampicillin media plates. The desired recombinant is called pSVE-PαInh.

pHBS348-ESalI was digested with SalI and HindIII and fragment 6 containing the pML-Amp$^r$, and SV40 early promoter was recovered by gel electrophoresis. pFD II (EP 117,060A) was digested with SalI and HindIII and fragment 7 was recovered which contains the normal mouse DHFR gene fused to the HBsAg 3' untranslated region. Fragments 6 and 7 were ligated, and the ligation mixture transformed into E. coli strain 294 (ATCC 31446). The transformed culture was plated on ampicillin media plates and resistant colonies selected. Plasmid DNA was prepared from transformants and checked by restriction analysis for the presence of the correct DNA fragments. This construction is referred to as pFDII-Sal1.

pSVE-PαInh was digested with SalI and fragment 8 was recovered which contains the SV40 early promoter and the α-inhibin coding region fused to the HBsAg 3'-untranslated region. pFDII-SalI was digested with SalI and fragment 9 containing the SV40 early promoter and the mouse DHFR coding region linked to the HBsAg 3'-untranslated region was recovered. PSVE-$\beta_A$Inh was linearized by SalI digestion and ligated to fragments 8 and 9 in a three part ligation. The ligation mixture was transformed into E. coli strain 294 (ATCC 31446). The transformed culture is plated on ampcillin media plates and resistant colonies selected. Transformants were screened for the presence of fragments 8 and 9 in the correct orientation such that transcription from the three SV40 early promoters will proceed in the same direction. This final plasmid is designated pSVE-Pα$\beta_A$Inh-DHFR.

Plasmid pSVE-Pα$\beta_A$Inh-DHFR was transfected into DHFR deficient CHO cells (Urlaub and Chasin, 1980, PNAS 77,4216-4220). However, any DHFR- mammalian host cell is suitable for use with this plasmid. Alternatively, any mammalian host cell is useful when the host cell is cotransformed with a plasmid encoding neomycin resistance, and transformants identified by their ability to grow in neomycin-containing medium.

The transfected CHO cells were selected by culturing in 15 HGT- medium. The cells were allowed to grow to confluency in 15cm diameter plates. The cells thereafter were cultured in serum free medium for 48 hours prior to harvest. 50ml of supernatant medium was lyophilized after the addition of 100mg human serum albumin. The residue was redissolved in 3ml 1% fetal bovine serum in HDMEM (GIBCO Laboratories, Santa Clara, CA), filtered through a Millex-GS 0.22mM filter (Millipore Corp., Bedford, MA) and assayed in duplicate.

The inhibin hormonal activity in the transformant supernatants was determined by an in vitro bioassay using rat anterior pituitary monolayer culture, Vale, W. et al. Endocrinology, 91, 562-572 (1972). In brief, 21-day-old female rat anterior pituitaries were collected, enzymatically dispersed and plated in 10% fetal bovine serum in HDMEM (GIBCO Laboratories, Santa Clara, CA) into 24-well tissue culture plates (Falcon Plastic, Oxnard, CA) on day 1. On day 2, the medium was changed to 1% fetal bovine serum in HDMEM, and the transformant medium sample was added. Incubation was continued for another 48 hours. The monolayer medium was then harvested, and the LH and FSH contents were determined by radioimmunoassay (RIA) using materials provided by The Pituitary Hormone Program of NIADDKD. In this assay, the inhibin-containing CHO cell culture inhibits the basal release of FSH but not LH, as compared to control pituitary cells that received the incubation medium only. The amount of porcine inhibin detected in transformant supernatants was 20 ng/ml and exhibited a dose response curve parallel to that obtained with pure porcine ovarian inhibin.

Immunological cross-reactivity is assayed by a sandwich-type radioimmunoassay. Rabbit antisera are raised against purified porcine follicular inhibin by s.c. immunization of rabbits with the porcine inhibin in Freund's complete adjuvant. The presence of anti-inhibin in the antiserum is detected by incubation of the antiserum with purified porcine inhibin and assaying for the formation of an immune complex by conventional techniques, e.g. gel filtration. An aliquot of the antisera is coated onto goat-anti-rabbit IgG precoated polystyrene test tubes. The recombinant culture supernatant or extract is diluted into phosphate buffered saline and added to the coated tubes, incubated overnight and washed. Another aliquot of the rabbit antiserum is added to the test tubes, incubated and washed. Radioiodinated goat antirabbit IgG is added to the tubes, incubated and unbound goat antiserum removed by washing. The recombinant produced inhibin cross-reacts with the rabbit antiserum, as evidenced by bound counts on the test tubes which exceed those of controls incubated with culture medium or extracts from untransformed host cells.

EXAMPLE 3

Construction of Human Inhibin Vector and Expression of Human Inhibin in Recombinant Cell Culture-I

Expression of human inhibin $\alpha\beta_A$ facilitated by the discovery that the mature porcine and human $\beta_A$ chains are identical. Thus, construction of a vector for the expression of human inhibin can proceed from plasmid PSVE-$\beta_A$-Inh from Example 1, which contains the porcine $\beta_A$-encoding cDNA.

A $\lambda$gt 10 library of human ovarian cDNA made from 10 $\mu$g of ovarian mRNA was subjected to Southern analysis using radiophosphate labelled porcine cDNA encoding $\alpha$, $\beta_A$ and $\beta_B$ chains. $\lambda$HIN$\alpha$-2 was identified as containing coding regions for the human $\alpha$ inhibin chain. The prevalence of hybridizing clones in the case of human $\alpha$ inhibin was considerably less than that found for porcine $\alpha$ inhibin, on the order of 1 in 100,000 human clones hybridized to the 685 bp SmaI fragment of the porcine cDNA for $\alpha$Inh. The $\beta$ chain clones were also rare, with the $\beta_B$ clones being present at about 3 times the level of $\beta_A$ (1 and 3 out of about 1,000,000 clones, respectively). None of the $\beta$ chain clones were full length. They were supplemented with a primed cDNA library and assembled generally as described above for the porcine cDNA. The $\lambda$ inserts were recovered by EcoR1 digestion.

Plasmids pHIN$\alpha$-2 is digested with NcoI and SmaI, and the 1049 bp 15 fragment (fragment 10) is recovered by gel electrophoresis. pPin$\alpha$ (Example 2) is digested with EcoRI and PvuII. The 98 bp fragment (fragment 11) is recovered by gel electrophoresis. Fragments 10 and 11 are ligated to adaptor I

5'-CTGCTCCTCTTGCTGTTGGCCCCACGGAGTGGGCATGGCTGCCAGGGCCCGGAGCTGGACC-3',

in combination with adaptor II which is the complement of adaptor I. The resulting 1208 bp fragment (fragment 12) is treated with Klenow fragment of Pol(I) and the 4 dNTP's and ligated to pHBS348-ESalI which has been restricted with HindIII and SacII and blunt-ended as described in Example 1. Alternatively, pPin$\alpha$ was digested with EcoRI and HpaII with the fragment encoding upstream from the HpaII site (that is, the first 21 residues of the porcine sequence) being recovered. The adaptor used in this alternative approach was

5'CGGAGCTCGACC 3'
3'  CTCGAGCTGG 5'.

A plasmid pSVE-H$\alpha$Inh having the correct orientation of fragment 12 is identified by sequence analysis of transformants. This construction (pSVE-H$\alpha$Inh) thus contains the first 24 residues of the porcine signal sequence with the remainder being prepro human inhibin. Plasmid pSVE-H$\alpha$Inh is digested with SalI. The fragment containing the SV40 promoter and human inhibin sequence is ligated to fragment 9 and SalI digested pSVE-$\beta_A$Inh (Example 2). This final plasmid designated pSVE-h$\alpha\beta_A$Inh-DHFR1 is transfected into DHFR-deficient CHO cells and selected as described in Example 2. The culture supernatant contains hormonally active human inhibin.

EXAMPLE 4

Construction of Human Inhibin Vector and Expression of Human Inhibin in Recombinant Cell Culture-II

This example is similar to Example 3 except that the pro sequence of human inhibin $\beta_B$ was employed in the place of the porcine $\beta_B$ prepro domain.

The lambda gt10 library of Example 3 yielded $\lambda$HIN$\alpha$2, as described in example 3, together with $\lambda$HIN$\beta_A$-5 and -14. The latter two phage were employed to construct the full length $\beta_A$ coding cDNA by ligating the 311 bp EcoRI-HindIII fragment (fragment 13) of $\lambda$HIN$\beta_A$-5 to the 1101 bp HindIII-HpaI fragment (fragment 14) of $\lambda$HIN$\beta_A$-14 and ligating this mixture in an EcoR1-SmaI digested mp18 vector (Biolabs). Clones were selected and screened for the appropriate sized insert. An mp18 vector containing the correct insert was treated with DNA polymerase(I) and the four dNTPs in order to render it double stranded, and thereafter digested with XbaI (which cleaves in the mp18 polylinker sequence), blunted with DNA polymerase I and the four dNTPs, and digested with EcoR1. A 1320 bp fragment (fragment 15) was ligated to the EcoR1-EcoRV fragment 1 from Example 2. This ligation mixture was used to transform E. coli 294 cells. Clones were screened by Southern Hybridization and confirmed by restriction analysis. The clone containing the hInh$\beta_A$ coding sequence was designated pSVE-hum$\beta_A$Inh. A plasmid containing the human $\beta_A$ coding sequences and the human α-inhibin sequences together with the DHFR gene is constructed from plasmids pSVE-hum$\beta_A$Inh, pSVE-HαInh and pFDIISalI as outlined above. Specifically, the Sal fragments from pSVE-HαInh and pFDIISalI which contain the human alpha inhibin and the DHFR genes were ligated with SalI digested pSVE-hum$\beta_A$Inh and a clone containing all three genes was identified. This plasmid, designated pSVE-humα$\beta_A$Inh-DHFR2 was transfected into DHFR- CHO cells and selected by culture in ght$^-$ medium. 24 clones were picked, grown to confluency in ght$^-$ medium under conditions conventional for CHO cells for two days, allowed to rest for 2 more days and thereafter the culture media were assayed for inhibin and activin activity using the rat pituitary cell assay described above. 4 clones were found to secrete significant levels of human α$\beta_A$ inhibin (hα$\beta_A$-8, 12, 14, and 18). The levels in the culture medium for each clone were, respectively, 125, 125, 200 and 250 ng/ml. Another clone (hα$\beta_A$-11) produced activin as the $\beta_A\beta_A$ homodimer, but no detectable inhibin, as determined by biological activity and the lack of α chain immunoreactivity in the culture medium for this clone. Clone hα$\beta_A$-16 secreted only α chain and was devoid of activin or inhibin activity.

EXAMPLE 5

Recombinant Expression of Human Activin

As reported by Vale et al. (Id.) and Ling et al. (Id.), homodimers and heterodimers of the β chains A and/or B have the opposite effect of inhibin on the pituitary, introducing rather than inhibiting FSH secretion. These proteins, collectively termed activin, are made in α and β chain cotransformants as described in Example 4. However, somewhat less screening for an appropriate transformant is needed if the initial transfection is conducted with a vector or vectors that do not contain the α chain gene. A suitable vector is readily constructed from the above-described vectors by excising the α chain gene. Plasmid pSVE-hum$\beta_A$Inh from Example 4 is digested with SalI and ligated to fragment 9 (Example 2) containing the DHFR gene. The ligation mixture was used to transfect E. coli 294 cells and colonies selected on the basis of failure to hybridize to the α chain sequence but which did hybridize to the β chain DNA. A clone pSVE-hum$\beta_A$Inh-DHFR was identified from which the α chain DNA has been deleted. This clone is transfected into DHFR- CHO cells as described above. Transformants are identified that secrete activin into the culture medium. Similarly, an expression vector containing a $\beta_B$ coding sequence (reconstituted by ligating DNA encoding the first 34 amino acids of human $\beta_A$ to the remaining coding sequence of the human $\beta_B$ chain) is readily constructed and contransfected with pSVE-hum$\beta_A$Inh-DHFR to produce the heterodimer. The reconstructed human $\beta_B$ gene also is used in the forgoing plasmids in order to produce α$\beta_B$-inhibin which,

**Claims**

**Claims for the following Contracting States : BE, CH, DE, FR, GB, IT, LI, NL, SE**

1. A method comprising culturing a host cell transformed with a vector which includes nucleic acid encoding a human or porcine inhibin α chain and/or a human or porcine inhibin β chain the amino acid sequences of which are as depicted in Fig 1B (porcine α chain), Fig 2B (porcine β chains), Fig 6A (human α chain) and Figs 8 and 9 (human β chains), or an amino acid sequence variant by way of insertion, deletion or substitution of a said depicted sequence, the variant being substantially homologous with a polypeptide of a depicted sequence but excluding bovine inhibin α chain and the partial bovine inhibin β chain of the sequence

Gly-Leu-Glu-Cys-Asp-Gly-Lys-Val-Asn-Ile-Cys-Cys-Lys-Lys-Gln-Phe-Phe-Val-Ser-Phe-Lys-Asp-Ile-Gly-Trp-Asn-Asp-Trp-Ile-Ile-Ala-Pro-Ser-Gly-Tyr-His-Ala-Asn-Tyr-Cys-Glu-Gly-Glu-Cys-Pro-Ser-His-Ile-Ala-Gly-Thr-Ser-Gly-Ser-Ser-Leu-Ser-Phe-His-Ser-Thr-Val-Ile-Asn-His-Tyr-Arg-Met-Arg-Gly-His-Ser

and

1) being cross reactive with antibodies raised against a polypeptide of a depicted sequence; or 2) having like hormonal activity to a polypeptide of a depicted sequence.

**2.** A method according to claim 1 wherein the nucleic acid encodes a human or porcine inhibin α chain and/or a human or porcine β chain the amino acid sequences of which are as depicted in Fig 1B (porcine α chain), Fig 2B (porcine β chains), Fig 6A (human α chain) and Figs 8 and 9 (human β chains).

**3.** A method according to Claim 1 wherein the vector encodes an inhibin β chain, or an amino acid sequence variant thereof, other than native human or porcine $\beta_A$ or the partial bovine inhibin β chain specified in claim 1.

**4.** A method according to claim 1 wherein the nucleic acid encodes a variant selected from: porcine inhibin derivatives ($Asn_{266}$→Gln]Inhα; [$Cys_{325}$ or $Cys_{324}$→Δ]Inhα; [$Cys_{361}$ or $Cys_{363}$→Δ]Inhα; [$Lys_{321}$ or $Lys_{322}$→Δ]Inh$\beta_A$;[$Lys_{322}$→His or Ser]Inh$\beta_A$; [$Lys_{315}$→Arg; $Val_{316}$→Thr] Inh$\beta_A$;[$Cys_{388}$ or $Cys_{390}$→Δ]-Inh$\beta_A$, [$Lys_{411}$→Gln]Inh$\beta_A$; [$Arg_{315}$→Lys; $Val_{316}$→Thr]Inh$\beta_B$; [$Cys_{319}$ or $Cys_{320}$-Δ]Inh$\beta_B$; [$Pro_{381}$ $Gly_{382}$→ Pro Phe Gly]Inh$\beta_B$; [$Arg_{395}$→Gin]Inh$\beta_B$, wherein Inh is an abbreviation of inhibin and the residue numbers for Inh$\beta_B$ are those used for the corresponding Inh$\beta_A$ residue (see Fig. 2B); human inhibin $\beta_A$ chain variants having variations which are substitution or deletion at, or insertion next to, a residue selected from residues 293-297, 364-376 and 387-398 of the sequence depicted in Fig. 8; and variants of human α chain which are greater than 90% homologous with the sequence depicted in Fig. 6A.

**5.** A method according to claim 1 wherein the nucleic acid encodes a variant of a human $\beta_A$ inhibin chain selected from $Phe_{302}$→Ile or Leu; $Gln_{297}$→Asp or Lys; $Trp_{307}$→Tyr or Phe; $Trp_{310}$→Tyr or Phe; $Ile_{311}$→Phe or Val; $Tyr_{317}$→Trp or Thr; $His_{318}$→Lys; $Ala_{319}$→Ser; $Asn_{320}$→Gln, Tyr or His; $Tyr_{321}$→Thr or Asp, $Phe_{340}$→Tyr; $His_{353}$→Asp; $His_{353}$→Lys (a $\beta_A$/$\beta_B$ hybrid); $Phe_{356}$→Tyr; $Val_{364}$→Phe; $Val_{364}$→Leu; $Tyr_{375}$→Thr; $Tyr_{376}$→Trp; $Asn_{389}$→Gln, His or Lys; $Ile_{391}$→Leu or Thr; $Met_{390}$ →Leu or Ser; $Val_{392}$→Phe, Glu, Thr or Ile; or comparably modified human $\beta_B$ chain

**6.** The method of any one of the preceding claims wherein the nucleic acid encoding the ihhibin α chain and/or β chain is operably linked to a promoter recognized by the host cell and including the further step of recovering inhibin or a β chain dimer from the culture medium.

**7.** The method of any one of the preceding claims wherein the cell is a prokaryote.

**8.** The method of any preceding claim wherein the vector comprises nucleic acid encoding the prepro form of an inhibin α chain or an inhibin β chain.

**9.** The method of claim 8 wherein the vector comprises nucleic acid encoding the prepro form of both the inhibin α chain and an inhibin β chain.

**10.** The method of any one of claims 1 to 6, 8 or 9 wherein the cell is a cell from a multicellular organism and hormonally active inhibin is produced.

**11.** The method of claim 6 wherein the promoter is a viral promoter.

**12.** The method of claim 11 wherein the promoter is an SV40 promoter.

**13.** The method of any preceding claim wherein mature porcine or human inhibin is recovered.

**14.** The method of any of claims 1 to 7 or 10 to 12 wherein the vector comprises nucleic acid encoding the prepro form of an inhibin β chain and a mature β-chain dimer is recovered free of the α -chain.

**15.** The method of claim 10 or 13 wherein the β chain is the $β_A$ chain arid the inhibin is present in a concentration greater than about 20 ng/ml in the culture medium.

**16.** A composition comprising human or porcine inhibin made up of an α and a β chain, the amino acid sequences of said α and β chains being selected from those depicted in Fig 1B (porcine α chain), Fig 2B (porcine β chains), Fig 6A (human α chain) and Figs 8 and 9 (human β chains), and amino acid sequence variants by way of insertion, deletion or substitution of a polypeptide of a said depicted sequence, which variants are substantially homologous with a polypeptide of a depicted sequence, but excluding bovine inhibin α chain and 1) are cross reactive with antibodies raised against a polypeptide of a depicted sequence; or 2) have like hormonal activity to a polypeptide of a depicted sequence; which composition is completely free of unidentified human or porcine proteins.

**17.** A composition according to Claim 16 wherein the sequences of said α and β chains are selected from those depicted in Fig 1 B (porcine α chain), Fig 2B (porcine β chains), Fig 6A (human α chain) and Figs 8 and 9 (human β chains).

**18.** A composition according to claim 16 wherein said variants are selected from: porcine inhibin derivatives ($Asn_{266}$→In]Glnhα; [$Cys_{325}$ or $Cys_{324}$→Δ]Inhα; [$Cys_{361}$ or $Cys_{363}$→Δ]Inhα; [$Lys_{321}$ or $Lys_{322}$→Δ]Inh$β_A$; [$Lys_{322}$→His or Ser]Inh$β_A$; [$Lys_{315}$-Arg; $Val_{316}$→Thr] Inh$β_A$; [$Cys_{388}$ or $Cys_{390}$→Δ]Inh$β_A$, [$Lys_{411}$→Gln]Inh$β_A$; [$Arg_{315}$→Lys; $Val_{316}$→Thr]Inh$β_B$; [$Cys_{319}$ or $Cys_{320}$→Δ]Inh$β_B$ [$Pro_{381}$ $Gly_{382}$→Pro Phe Gly]Inh$β_B$; [$Arg_{395}$→Gln] Inh$β_B$, wherein Inh is an abbreviation of inhibin and the residue numbers for Inh$β_B$ are those used for the corresponding Inh$β_A$ residue (see Fig. 2B); human inhibin $β_A$ chain variants having variations which are substitution or deletion at, or insertion next to, a residue selected from residues 293-297, 364-376 and 387-398 of the sequence depicted in Fig. 8; and variants of human α chain which are greater than 90% homologous with the sequence depicted in Fig. 6A.

**19.** A composition according to claim 16 including a variant of a human $β_A$ inhibin chain selected from $Phe_{302}$→Ile or Leu; $Gln_{297}$→Asp or Lys; $Trp_{307}$→Tyr or Phe; $Trp_{310}$→Tyr or Phe; $Ile_{311}$→Phe or Val; $Tyr_{317}$→Trp or Thr; $His_{318}$→Lys; $Ala_{319}$→Ser; $Asn_{320}$→Gln, Tyr or His; $Tyr_{321}$→Thr or Asp, $Phe_{340}$→Tyr; $His_{353}$→Asp; $His_{353}$→Lys (a $β_A$/$β_B$ hybrid); $Phe_{356}$→Tyr; $Val_{364}$→Phe; $Val_{364}$→Leu; $Tyr_{375}$→Thr; $Tyr_{376}$→Trp; $Asn_{389}$→Gln, His or Lys; $Ile_{391}$→Leu or Thr; $Met_{390}$ →Leu or Ser; $Val_{392}$→Phe, Glu, Thr or Ile; or comparably modified human $β_B$ chain.

**20.** A composition comprising a prodomain of human or porcine α or $β_B$ inhibin as depicted in Fig 1B (porcine α chain), Fig 2B (porcine $β_B$ chain), Fig 6A (human α chain), and Fig 9 (human $β_B$ chain) unassociated with native glycosylation.

**21.** A composition comprising a homodimer of mature human or porcine inhibin $β_A$ or $β_B$ chains, said chains being as depicted in Fig 2B (porcine β chains) and Figs 8 and 9 (human β chains) or of an amino acid sequence variant by way of insertion, deletion or substitution of a polypeptide of a said depicted sequence the variant being substantially homologous with a polypeptide of a depicted sequence and 1) being cross reactive with antibodies raised against a polypeptide of a depicted sequence; or 2) having like hormonal activity to a polypeptide of a depicted sequence; which composition is free of the inhibin α chain.

**22.** A composition according to Claim 21 which is a homodimer of mature human or porcine $β_B$ chains or a said amino acid sequence variant thereof.

**23.** A composition comprising a heterodimer of mature human or porcine inhibin $β_A$ with mature human or porcine inhibin $β_B$, said chains being as depicted in Fig 2B (porcine β chains) and Figs 8 and 9 (human β chains) or of an amino acid sequence variant by way of insertion, deletion or substitution of a said depicted sequence the variant being substantially homologous with a polypeptide of a depicted sequence and 1) being cross reactive with antibodies raised against a polypeptide of a depicted sequence; or 2) having like hormonal activity to a polypeptide of a depicted sequence; which composition is free of the inhibin α chain.

**24.** Non-chromosomal DNA encoding a human or porcine inhibin α or a human or porcine inhibin β chain the amino acid sequences of which are as depicted in Fig 1B (porcine α chain), Fig 2B (porcine β chains), Fig 6A (human α chain) and Figs 8 and 9 (human β chains) or an amino acid sequence variant by way of insertion, deletion or substitution of a polypeptide of a said depicted sequence, the variant being substantially homologous with a polypeptide of a depicted sequence excluding bovine inhibin α chain and the partial bovine inhibin β chain of the sequence

Gly-Leu-Glu-Cys-Asp-
Gly-Lys-Val-Asn-Ile-Cys-Cys-Lys-Lys-Gln-Phe-Phe-Val-Ser-
Phe-Lys-Asp-Ile-Gly-Trp-Asn-Asp-Trp-Ile-Ile-Ala-Pro-Ser-
Gly-Tyr-His-Ala-Asn-Tyr-Cys-Glu-Gly-Glu-Cys-Pro-Ser-His-
Ile-Ala-Gly-Thr-Ser-Gly-Ser-Ser-Leu-Ser-Phe-His-Ser-Thr-
Val-Ile-Asn-His-Tyr-Arg-Met-Arg-Gly-His-Ser

and

1) being cross reactive with antibodies raised against a polypeptide of a depicted sequence; or 2) having like hormonal activity to a polypeptide of a depicted sequence.

**25.** The DNA of claim 24 encoding a human or porcine inhibin α or a human or porcine inhibin β chain whose amino acid sequence is as depicted in Fig 1B (porcine α chain), Fig 2B (porcine β chains), Fig 6A (human α chain) and Figs 8 and 9 (human β chains).

**26.** The DNA of claim 24 which encodes a variant selected from: porcine inhibin derivatives ($Asn_{266}$→Gln]-Inhα;[$Cys_{325}$ or $Cys_{324}$→Δ]Inhα; [$Cys_{361}$ or $Cys_{363}$→Δ]Inhα; [$Lys_{321}$ or $Lys_{322}$→Δ]Inh$\beta_A$; [$Lys_{322}$→His or Ser]Inh$\beta_A$; [$Lys_{315}$→Arg; $Val_{316}$→Thr] Inh$\beta_A$; [$Cys_{388}$ or $Cys_{390}$→Δ]Inh$\beta_A$,[$Lys_{411}$→Gln]Inh$\beta_A$; [$Arg_{315}$→Lys; $Val_{316}$→Thr]Inh$\beta_B$; [$Cys_{319}$ or $Cys_{320}$→Δ]Inh$\beta_B$; [$Pro_{381}$ $Gly_{382}$→ Pro Phe Gly]-Inh$\beta_B$;[$Arg_{395}$→Gln]Inh$\beta_B$, wherein Inh is an abbreviation of inhibin and the residue numbers for Inh$\beta_B$ are those used for the corresponding Inh$\beta_A$ residue (see Fig. 2B); human inhibin $\beta_A$ chain variants having variations which are substitution or deletion at, or insertion next to, a residue selected from residues 293-297, 364-376 and 387-398 of the sequence depicted in Fig. 8; and variants of human α chain which are greater than 90% homologous with the sequence depicted in Fig. 6A.

**27.** The DNA of claim 24 which encodes a variant of a human β inhibin chain selected from $Phe_{302}$→Ile or Leu; $Gln_{297}$→Asp or Lys; $Trp_{307}$→Tyr or Phe; $Trp_{310}$→Tyr or Phe; $Ile_{311}$→Phe or Val; $Tyr_{317}$→Trp or Thr; $His_{318}$→Lys; $Ala_{319}$→Ser; $Asn_{320}$→Gln, Tyr or His; $Tyr_{321}$→Thr or Asp, $Phe_{340}$→Tyr; $His_{353}$→Asp; $His_{353}$→Lys (a $\beta_A$/$\beta_B$ hybrid); $Phe_{356}$→Tyr; $Val_{364}$→Phe; $Val_{364}$→Leu; $Tyr_{375}$→Thr; $Tyr_{376}$→Trp; $Asn_{389}$→Gln, His or Lys; $Ile_{391}$→Leu or Thr; $Met_{390}$ →Leu or Ser; $Val_{392}$→Phe, Glu, Thr or lie; or comparable modified human $\beta_B$ chain

**28.** The DNA of any of claims 24 to 27 which is free of intervening untranslated sequences.

**29.** The DNA of any one of claims 24 to 28 which is labelled with a detectable moiety.

**30.** A replicable vector comprising a DNA of any of claims 21 to 27.

**31.** The vector of claim 30 comprising a viral promoter operably linked to the DNA encoding the inhibin α and/or β chains.

**32.** The vector of claim 30 or 31 which contains DNA encoding both an inhibin α and an inhibin β chain.

**33.** The vector of claim 30 or 31 which contains DNA encoding an inhibin β chain but not the inhibin α chain.

**34.** A host cell transformed with a replicable vector comprising DNA encoding the human or porcine inhibin α and/or an inhibin β chain the amino acid sequences of which are as depicted in Fig 1B (porcine α chain), Fig 2B (porcine β chains), Fig 6A (human α chain) and Figs 8 and 9 (human β chains) or an amino acid sequence variant by way of insertion, deletion or substitution of a polypeptide of a said depicted sequence, the variant being substantially homologous with a polypeptide of a depicted sequence, excluding bovine inhibin α chain, and the partial bovine inhibin β chain of the sequence

Gly-Leu-Glu-Cys-Asp-Gly-
Lys-Val-Asn-Ile-Cys-Cys-Lys-Lys-Gln-Phe-Phe-Val-Ser-Phe-
Lys-Asp-Ile-Gly-Trp-Asn-Asp-Trp-Ile-Ile-Ala-Pro-Ser-Gly-
Tyr-His-Ala-Asn-Tyr-Cys-Glu-Gly-Glu-Cys-Pro-Ser-His-Ile-
Ala-Gly-Thr-Ser-Gly-Ser-Ser-Leu-Ser-Phe-His-Ser-Thr-Val-
Ile-Asn-His-Tyr-Arg-Met-Arg-Gly-His-Ser

and

1) being cross reactive with antibodies raised against a polypeptide of a depicted sequence; or 2) having like hormonal activity to a polypeptide of a depicted sequence.

**35.** A host cell according to claim 34 wherein the DNA encodes a human or porcine inhibin α and/or an inhibin β chain, the amino acid sequences of which are as depicted in Fig 1B (porcine α chain), Fig 2B (porcine β chains), Fig 6A (human α chain) and Figs 8 and 9 (human β chains).

**36.** A host cell according to claim 34 wherein the DNA encodes a variant selected from: porcine inhibin derivatives ($Asn_{266}$→Gln]Inhα; [$Cys_{325}$ or $Cys_{324}$→Δ]Inhα; [$Cys_{361}$ or $Cys_{363}$→Δ]Inhα; [$Lys_{321}$ or $Lys_{322}$→Δ]Inh$β_A$;[$Lys_{322}$→His or Ser]Inh$β_A$; [$Lys_{315}$→Arg; $Val_{316}$→Thr] Inh$β_A$;[$Cys_{388}$ or $Cys_{390}$→Δ]-Inh$β_A$, [Lys $_{411}$→Gln]Inh$β_A$; [$Arg_{315}$→Lys; $Val_{316}$→Thr]Inh$β_B$; [$Cys_{319}$ or $Cys_{320}$→Δ]Inh$β_B$; [$Pro_{381}$ $Gly_{382}$→ Pro Phe Gly]Inh$β_B$; [$Arg_{395}$→Gln]Inh$β_B$, wherein Inh is an abbreviation of inhibin and the residue numbers for Inh$β_B$ are those used for the corresponding Inh$β_A$ residue (see Fig. 2B); human inhibin $β_A$ chain variants having variations which are substitution or deletion at, or insertion next to, a residue selected from residues 293-297, 364-376 and 387-398 of the sequence depicted in Fig. 8; and variants of human α chain which are greater than 90% homologous with the sequence depicted in Fig. 6A.

**37.** A host cell according to claim 34 wherein the DNA encodes a variant of a human $β_A$ inhibin chain selected from $Phe_{302}$→Ile or Leu; $Gln_{297}$→Asp or Lys; $Trp_{307}$→Tyr or Phe; $Trp_{310}$→Tyr or Phe; $Ile_{311}$→Phe or Val; $Tyr_{317}$→Trp or Thr; $His_{318}$→Lys; $Ala_{319}$→Ser; $Asn_{320}$→Gln, Tyr or His; $Tyr_{321}$→Thr or Asp, $Phe_{340}$→Tyr; $His_{353}$→Asp; $His_{353}$→Lys (a $β_A$/$β_B$ hybrid); $Phe_{356}$→Tyr; $Val_{364}$→Phe; $Val_{364}$→Leu; $Tyr_{375}$→Thr; $Tyr_{376}$→Trp; $Asn_{389}$→Gln, His or Lys; $Ile_{391}$→Leu or Thr; $Met_{390}$ →Leu or Ser; $Val_{392}$→Phe, Glu, Thr or Ile; or comparably modified human $β_B$ chain

**38.** The cell of any of claims 34 to 37 which is a eukaryotic cell.

**39.** A cell-free composition that is free of mature α chain polypeptide containing a human or porcine inhibin α chain prodomain polypeptide sequence as depicted in Fig 1B (porcine α chain) or Fig 6A (human α chain) or an amino acid sequence variant by way of insertion, deletion or substitution of a said prodomain sequence, the variant being substantially homologous with a polypeptide of a said sequence and being cross reactive with antibodies raised against a polypeptide of a depicted sequence.

**40.** A cell-free composition containing

a) a polypeptide comprising the human inhibin $β_A$ chain prodomain sequence

HSAAPDCPSCALAALPKDVPNSQPEMVEAVKKHILNMLHL (Amino acids 1-40 of Fig 8), PDVTQPVPKAALLNAIRKLHVGKVGENGYVEIEDDIG (amino acids 44-80 of Fig 8.),

AEMNELMEQTSEIITFAESGTARKTLHFEISKEGSDLSVVERAEVWLFLKVPKAN-RTRTKVTIRLFQQQKHPQGSLDTGEEAEEVGLKGERSELLLSEKVVDA (amino acids 83-185 of Fig 8), STWHVFPVSSSIQRLLD-QGKSSLDVRIACEQCQESGASLVLLG (amino acids 188-230 of Fig 8),

or naturally occurring mammalian amino acid sequence variants thereof;

b) a polypeptide comprising the human inhibin $\beta_B$ chain prodomain sequence

CTSCGGFRRPEELGRVDGDFLEAV, (amino acids 7-30 of Fig 9),

HILSRLQMRGRPNITHAVPKAAMVTALRKLHAGKVREDGRVEIPHLDGHASPGAD-GQERVSEIISFAETDGLASSRVRLYFFISNEGNQNLFVVQASLWLYLKLLPYVLEKGS (amino acids 33-145 of Fig 9),

VRVKVYFQEQGHGDRWNMVEKRVDLKRSGWHTFPLTEAIQALFERGE (amino acids 149 - 195 of Fig 9),

LNLDVQCDSCQELAVVPVFVDPGEESHRPFVVVQARLGDSRHRI (amino acids 198-241 of Fig 9),

or naturally occurring mammalian amino acid sequence variants thereof; or

c) a polypeptide free of the mature α chain amino acid sequence comprising the human inhibin α chain prodomain sequences

KVRALFLDALGPPAVTREGGDPGV (amino acids 1 - 24 of Fig 6),

HALGGFTHRGSEPEEEEDVSQAILFPATDASCEDKSAARGLAQEAEEGLFRYMFR-

PSQHTRSRQVTSAQLWFHTGLDRQGTAASNSSEPLLGLLALSPGGPVAVPMSLGH-

APPHWAVLHLATSALSLLTHPVLVLLLRCPLCTCSARPEATPFLVAHTRTRPPSGGERA

(amino acids 32 - 199 of Fig 6),

or naturally occurring mammalian amino acid sequence variants thereof.

**41.** , A cell-free composition containing a human or porcine inhibin β chain prodomain polypeptide of a sequence as depicted in Fig 2B (porcine β chains) and Figs 8 and 9 (human β chains) or an amino acid sequence variant by way of insertion, deletion, or substitution of a said prodomain sequence, the variant being substantially homologous with a polypeptide of a said sequence, and being cross reactive with antibodies raised against a polypeptide of a depicted sequence.

**42.** The composition of claim 41 wherein the β chain is the $\beta_A$ chain and the composition is free of mature $\beta_A$ chain sequence.

**43.** The composition of claim 41 wherein the β chain is the $\beta_B$ chain and the composition is free of mature $\beta_B$ chain sequence.

**44.** The composition of claim 40 wherein the variant is the corresponding porcine amino acid sequence, the composition containing:

a) a polypeptide comprising the porcine inhibin $\beta_A$ chain prodomain sequence depicted at: amino acids 28 to 58; amino acids 61 to 87; amino acids 90 to 108; amino acids 111 to 179; amino acids 182 to 213; amino acids 216 to 258; or amino acids 28 to 87 of Fig 2B; or
b) a polypeptide free of the mature α chain amino acid sequence comprising the porcine inhibin α chain prodomain polypeptide sequences depicted at: amino acids 20 to 54; or amino acids 70 to 228 of Fig 1B.

**45.** The composition of any of claims 39 to 44 wherein the polypeptide is unaccompanied by native glycosylation.

**46.** The composition of any of claims 39 to 45 which is sterile and wherein the polypeptide further comprises an immunogenic polypeptide.

**47.** The use of a composition of any of claims 39 to 46 in the preparation of an antibody capable of binding said polypeptide.

**48.** The composition of any of claims 39 to 46 wherein the polypeptide is conjugated to a detectable group.

**49.** The composition of claim 48 wherein the group is an enzyme, fluorophore or radioisotope.

**50.** The composition of any of claims 39 to 44, 48 or 49 which is insolubilized by non-covalent absorption or covalent cross-linking to a water insoluble support.

**51.** The composition of any of claims 39 to 46, 48 or 49 further comprising a physiologically acceptable implantable matrix for controlled release of the polypeptide into the tissues of an animal.

**Claims for the following Contracting State : LU**

**1.** A method comprising culturing a host cell transformed with a vector which includes nucleic acid encoding a human or porcine inhibin α chain and/or a human or porcine inhibin β chain the amino acid sequences of which are as depicted in Fig 1B (porcine α chain), Fig 2B (porcine β chains), Fig 6A (human α chain) and Figs 8 and 9 (human β chains), or an amino acid sequence variant by way of insertion, deletion or substitution of a said depicted sequence, the variant being substantially homologous with a polypeptide of a depicted sequence but excluding bovine inhibin α chain and 1) being cross reactive with antibodies raised against a polypeptide of a depicted sequence; or 2) having like hormonal activity to a polypeptide of a depicted sequence.

**2.** A method according to Claim 1 wherein the nucleic acid encodes a human or porcine inhibin α chain and/or a human or porcine β chain the amino acid sequences of which are as depicted in Fig 1B (porcine α chain), Fig 2B (porcine β chains), Fig 6A (human α chain) and Figs 8 and 9 (human β chains).

**3.** A method according to Claim 1 wherein the vector encodes an inhibin β chain, or an amino acid sequence variant thereof, other than native human or porcine $\beta_A$.

**4.** A method according to claim 1 wherein the nucleic acid encodes a variant selected from: porcine inhibin derivatives ($Asn_{266}\rightarrow$Gln]Inhα; [$Cys_{325}$ or $Cys_{324}\rightarrow\Delta$]Inhα; [$Cys_{361}$ or $Cys_{363}\rightarrow\Delta$]Inhα; [$Lys_{321}$ or $Lys_{322}\rightarrow\Delta$]$Inh\beta_A$;[$Lys_{322}\rightarrow$His or Ser]$Inh\beta_A$; [$Lys_{315}\rightarrow$Arg; $Val_{316}\rightarrow$Thr] $Inh\beta_A$;[$Cys_{388}$ or $Cys_{390}\rightarrow\Delta$]-$Inh\beta_A$, [$Lys_{411}\rightarrow$Gln]$Inh\beta_A$; [$Arg_{315}\rightarrow$Lys; $Val_{316}\rightarrow$Thr]$Inh\beta_B$; [$Cys_{319}$ or $Cys_{320}\rightarrow\Delta$]$Inh\beta_B$; [$Pro_{381}$ $Gly_{382}\rightarrow$ Pro Phe Gly]$Inh\beta_B$; [$Arg_{395}\rightarrow$Gln]$Inh\beta_B$, wherein Inh is an abbreviation of inhibin and the residue numbers for $Inh\beta_B$ are those used for the corresponding $Inh\beta_A$ residue (see Fig. 2B); human inhibin $\beta_A$ chain variants having variations which are substitution or deletion at, or insertion next to, a residue selected from residues 293-297, 364-376 and 387-398 of the sequence depicted in Fig. 8; and variants of human α chain which are greater than 90% homologous with the sequence depicted in Fig. 6A.

**5.** A method according to claim 1 wherein the nucleic acid encodes a variant of a human $\beta_A$ inhibin chain selected from $Phe_{302}\rightarrow$Ile or Leu; $Gln_{297}\rightarrow$Asp or Lys; $Trp_{307}\rightarrow$Tyr or Phe; $Trp_{310}\rightarrow$Tyr or Phe; $Ile_{311}\rightarrow$Phe or Val; $Tyr_{317}\rightarrow$Trp or Thr; $His_{318}\rightarrow$Lys; $Ala_{319}\rightarrow$Ser; $Asn_{320}\rightarrow$Gln, Tyr or His; $Tyr_{321}\rightarrow$Thr or Asp, $Phe_{340}\rightarrow$Tyr; $His_{353}\rightarrow$Asp; $His_{353}\rightarrow$Lys (a $\beta_A/\beta_B$ hybrid); $Phe_{356}\rightarrow$Tyr; $Val_{364}\rightarrow$Phe; $Val_{364}\rightarrow$Leu; $Tyr_{375}\rightarrow$Thr; $Tyr_{376}\rightarrow$Trp; $Asn_{389}\rightarrow$Gln, His or Lys; $Ile_{391}\rightarrow$Leu or Thr; $Met_{390}\rightarrow$Leu or Ser; $Val_{392}\rightarrow$Phe, Glu, Thr or Ile; or comparably modified human $\beta_B$ chain

**6.** The method of any one of the preceding claims wherein the nucleic acid encoding the inhibin α chain and/or β chain is operably linked to a promoter recognized by the host cell and including the further step of recovering inhibin or a β chain dimer from the culture medium.

**7.** The method of any one of the preceding claims wherein the cell is a prokaryote.

**8.** The method of any preceding claim wherein the vector comprises nucleic acid encoding the prepro form of an inhibin α chain or an inhibin β chain.

**9.** The method of claim 8 wherein the vector comprises nucleic acid encoding the prepro form of both the inhibin α chain and an inhibin β chain.

**10.** The method of any one of claims 1 to 6, 8 or 9 wherein the cell is a cell from a multicellular organism and hormonally active inhibin is produced.

**11.** The method of claim 6 wherein the promoter is a viral promoter.

**12.** The method of claim 11 wherein the promoter is an SV40 promoter.

**13.** The method of any preceding claim wherein mature porcine or human inhibin is recovered.

**14.** The method of any of claims 1 to 7 or 10 to 12 wherein the vector comprises nucleic acid encoding the prepro form of an inhibin β chain and a mature β-chain dimer is recovered free of the α -chain.

**15.** The method of claim 10 or 13 wherein the β chain is the $\beta_A$ chain and the inhibin is present in a concentration greater than about 20 ng/ml in the culture medium.

**16.** A composition comprising human or porcine inhibin made up of an α and a β chain, the amino acid sequences of said α and β chains being selected from those depicted in Fig 1B (porcine α chain), Fig 2B (porcine β chains), Fig

6A (human $\alpha$ chain) and Figs 8 and 9 (human $\beta$ chains), and amino acid sequence variants by way of insertion, deletion or substitution of a polypeptide of a said depicted sequence, which variants are substantially homologous with a polypeptide of a depicted sequence, but excluding bovine inhibin $\alpha$ chain and 1) are cross reactive with antibodies raised against a polypeptide of a depicted sequence; or 2) have like hormonal activity to a polypeptide of a depicted sequence; which composition is completely free of unidentified human or porcine proteins.

**17.** A composition according to Claim 16 wherein the sequences of said $\alpha$ and $\beta$ chains are selected from those depicted in Fig 1B (porcine $\alpha$ chain), Fig 2B (porcine $\beta$ chains), Fig 6A (human $\alpha$ chain) and Figs 8 and 9 (human $\beta$ chains).

**18.** A composition according to claim 16 wherein said variants are selected from: porcine inhibin derivatives ($Asn_{266}$→In]GlnhInh$\alpha$; [$Cys_{325}$ or $Cys_{324}$→$\Delta$]Inh$\alpha$; [$Cys_{361}$ or $Cys_{363}$→$\Delta$]Inh$\alpha$; [$Lys_{321}$ or $Lys_{322}$→$\Delta$]Inh$\beta_A$; [$Lys_{322}$→His or Ser]Inh$\beta_A$; ($Lys_{315}$→Arg; $Val_{316}$→Thr] Inh$\beta_A$; [$Cys_{388}$ or $Cys_{390}$→$\Delta$]Inh$\beta_A$, [$Lys_{411}$→Gln]Inh$\beta_A$; [$Arg_{315}$→Lys; $Val_{316}$→Thr]Inh$\beta_B$; [$Cys_{319}$ or $Cys_{320}$→$\Delta$]Inh$\beta_B$ [$Pro_{381}$ $Gly_{382}$→Pro Phe Gly]Inh$\beta_B$; [$Arg_{395}$→Gln] Inh$\beta_B$, wherein Inh is an abbreviation of inhibin and the residue numbers for Inh$\beta_B$ are those used for the corresponding Inh$\beta_A$ residue (see Fig. 2B); human inhibin $\beta_A$ chain variants having variations which are substitution or deletion at, or insertion next to, a residue selected from residues 293-297, 364-376 and 387-398 of the sequence depicted in Fig. 8; and variants of human $\alpha$ chain which are greater than 90% homologous with the sequence depicted in Fig. 6A.

**19.** A composition according to claim 16 including a variant of a human $\beta_A$ inhibin chain selected from $Phe_{302}$→Ile or Leu; $Gln_{297}$→Asp or Lys; $Trp_{307}$→Tyr or Phe; $Trp_{310}$→Tyr or Phe; $Ile_{311}$→Phe or Val; $Tyr_{317}$→Trp or Thr; $His_{318}$-Lys; $Ala_{319}$→Ser; $Asn_{320}$→Gln, Tyr or His; $Tyr_{321}$→Thr or Asp, $Phe_{340}$→Tyr; $His_{353}$→Asp; $His_{353}$→Lys (a $\beta_A/\beta_B$ hybrid); $Phe_{356}$→Tyr; $Val_{364}$→Phe; $Val_{364}$→Leu; $Tyr_{375}$→Thr; $Tyr_{376}$→Trp; $Asn_{389}$→Gln, His or Lys; $Ile_{391}$→Leu or Thr; $Met_{390}$ →Leu or Ser; $Val_{392}$→Phe, Glu, Thr or Ile; or comparably modified human $\beta_B$ chain.

**20.** A composition comprising a prodomain of human or porcine $\alpha$ or $\beta_B$ inhibin as depicted in Fig 1B (porcine $\alpha$ chain), Fig 2B (porcine $\beta_B$ chain), Fig 6A (human $\alpha$ chain), and Fig 9 (human $\beta_B$ chain) unassociated with native glycosylation.

**21.** A composition comprising a homodimer of mature human or porcine inhibin $\beta_A$ or $\beta_B$ chains, said chains being as depicted in Fig 2B (porcine $\beta$ chains) and Figs 8 and 9 (human $\beta$ chains) or of an amino acid sequence variant by way of insertion, deletion or substitution of a polypeptide of a said depicted sequence the variant being substantially homologous with a polypeptide of a depicted sequence and 1) being cross reactive with antibodies raised against a polypeptide of a depicted sequence; or 2) having like hormonal activity to a polypeptide of a depicted sequence; which composition is free of the inhibin $\alpha$ chain.

**22.** A composition according to Claim 21 which is a homodimer of mature human or porcine $\beta_B$ chains or a said amino acid sequence variant thereof.

**23.** A composition comprising a heterodimer of mature human or porcine inhibin $\beta_A$ with mature human or porcine inhibin $\beta_B$, said chains being as depicted in Fig 2B (porcine $\beta$ chains) and Figs 8 and 9 (human $\beta$ chains) or of an amino acid sequence variant by way of insertion, deletion or substitution of a said depicted sequence the variant being substantially homologous with a polypeptide of a depicted sequence and 1) being cross reactive with antibodies raised against a polypeptide of a depicted sequence; or 2) having like hormonal activity to a polypeptide of a depicted sequence; which composition is free of the inhibin $\alpha$ chain.

**24.** Non-chromosomal DNA encoding a human or porcine inhibin $\alpha$ or a human or porcine inhibin $\beta$ chain the amino acid sequences of which are as depicted in Fig 1B (porcine $\alpha$ chain), Fig 2B (porcine $\beta$ chains), Fig 6A (human $\alpha$ chain) and Figs 8 and 9 (human $\beta$ chains) or an amino acid sequence variant by way of insertion, deletion or substitution of a polypeptide of a said depicted sequence, the variant being substantially homologous with a polypeptide of a depicted sequence excluding bovine inhibin $\alpha$ chain and 1) being cross reactive. with antibodies raised against a polypeptide of a depicted sequence; or 2) having like hormonal activity to a polypeptide of depicted sequence.

**25.** The DNA of claim 24 encoding a human or porcine inhibin $\alpha$ or a human or porcine inhibin $\beta$ chain whose amino acid sequence is as depicted in Fig 1B (porcine $\alpha$ chain), Fig 2B (porcine $\beta$ chains), Fig 6A (human $\alpha$ chain) and Figs 8 and 9 (human $\beta$ chains).

**26.** The DNA of claim 24 which encodes a variant selected from: porcine inhibin derivatives ($Asn_{266}$→Gln]-Inhα;[$Cys_{325}$ or $Cys_{324}$→Δ]Inhα; [$Cys_{361}$ or $Cys_{363}$→Δ]Inhα; [$Lys_{321}$ or $Lys_{322}$→Δ]Inh$\beta_A$; [$Lys_{322}$→His or Ser]Inh$\beta_A$; [$Lys_{315}$→Arg; $Val_{316}$→Thr] Inh$\beta_A$; [$Cys_{388}$ or $Cys_{390}$→Δ]Inh$\beta_A$,[$Lys_{411}$→Gln]Inh$\beta_A$; [$Arg_{315}$→Lys; $Val_{316}$→Thr]Inh$\beta_B$; [$Cys_{319}$ or $Cys_{320}$→Δ]Inh$\beta_B$; [$Pro_{381}$ $Gly_{382}$→ Pro Phe Gly]-Inh$\beta_B$;[$Arg_{395}$→Gln]Inh$\beta_B$, wherein Inn is an abbreviation of inhibin and the residue numbers for Inh$\beta_B$ are those used for the corresponding Inh$\beta_A$ residue (see Fig. 2B); human inhibin $\beta_A$ chain variants having variations which are substitution or deletion at, or insertion next to, a residue selected from residues 293-297, 364-376 and 387-398 of the sequence depicted in Fig. 8; and variants of human α chain which are greater than 90% homologous with the sequence depicted in Fig. 6A.

**27.** The DNA of claim 24 which encodes a variant of a human β inhibin chain selected from $Phe_{302}$→Ile or Leu; $Gln_{297}$→Asp or Lys; $Trp_{307}$→Tyr or Phe; $Trp_{310}$→Tyr or Phe; $Ile_{311}$→Phe or Val; $Tyr_{317}$→Trp or Thr; $His_{318}$→Lys; $Ala_{319}$→Ser; $Asn_{320}$→Gln, Tyr or His; $Tyr_{321}$→Thr or Asp, $Phe_{340}$→Tyr; $His_{353}$→Asp; $His_{353}$→Lys (a $\beta_A$/$\beta_B$ hybrid); $Phe_{356}$→Tyr; $Val_{364}$→Phe; $Val_{364}$→Leu; $Tyr_{375}$→Thr; $Tyr_{376}$→Trp; $Asn_{389}$→Gln, His or Lys; $Ile_{391}$→Leu or Thr; $Met_{390}$ →Leu or Ser; $Val_{392}$→Phe, Glu, Thr or Ile; or comparable modified human $\beta_B$ chain

**28.** The DNA of any of claims 24 to 27 which is free of intervening untranslated sequences.

**29.** The DNA of any one of claims 24 to 28 which is labelled with a detectable moiety.

**30.** A replicable vector comprising a DNA of any of claims 21 to 27.

**31.** The vector of claim 30 comprising a viral promoter operably linked to the DNA encoding the inhibin α and/or β chains.

**32.** The vector of claim 30 or 31 which contains DNA encoding both an inhibin α and an inhibin β chain.

**33.** The vector of claim 30 or 31 which contains DNA encoding an inhibin β chain but not the inhibin α chain.

**34.** A host cell transtormed with a replicable vector comprising DNA encoding the human or porcine inhibin α and/or an inhibin β chain the amino acid sequences of which are as depicted in Fig 1B (porcine α chain), Fig 2B (porcine β chains), Fig 6A (human α chain) and Figs 8 and 9 (human β chains) or an amino acid sequence variant by way of insertion, deletion or substitution of a polypeptide of a said depicted sequence, the variant being substantially homologous with a polypeptide of a depicted sequence, excluding bovine inhibin α chain, and 1) being cross reactive with antibodies raised against a polypeptide of a depicted sequence; or 2) having like hormonal activity to a polypeptide of a depicted sequence.

**35.** A host cell according to claim 34 wherein the DNA encodes a human or porcine inhibin α and/or an inhibin β chain, the amino acid sequences of which are as depicted in Fig 1B (porcine α chain), Fig 2B (porcine β chains), Fig 6A (human α chain) and Figs 8 and 9 (human β chains).

**36.** A host cell according to claim 34 wherein the DNA encodes a variant selected from: porcine inhibin derivatives ($Asn_{266}$→Gln]Inhα; [$Cys_{325}$ or $Cys_{324}$→Δ]Inhα; [$Cys_{361}$ or $Cys_{363}$→Δ]Inhα; [$Lys_{321}$ or $Lys_{322}$→Δ]Inh$\beta_A$;[$Lys_{322}$→His or Ser]Inh$\beta_A$; [$Lys_{315}$→Arg; $Val_{316}$→Thr] Inh$\beta_A$;[$Cys_{388}$ or $Cys_{390}$→Δ]-Inh$\beta_A$, [Lys $_{411}$→Gln]Inh$\beta_A$; [$Arg_{315}$→Lys; $Val_{316}$→Thr]Inh$\beta_B$; [$Cys_{319}$ or $Cys_{320}$→Δ]Inh$\beta_B$; [$Pro_{381}$ $Gly_{382}$→ Pro Phe Gly]Inh$\beta_B$; [$Arg_{395}$→Gln]Inh$\beta_B$, wherein Inh is an abbreviation of inhibin and the residue numbers for Inh$\beta_B$ are those used for the corresponding Inh$\beta_A$ residue (see Fig. 2B); human inhibin $\beta_A$ chain variants having variations which are substitution or deletion at, or insertion next to, a residue selected from residues 293-297, 364-376 and 387-398 of the sequence depicted in Fig. 8; and variants of human α chain which are greater than 90% homologous with the sequence depicted in Fig. 6A.

**37.** A host cell according to claim 34 wherein the DNA encodes a variant of a human $\beta_A$ inhibin chain selected from $Phe_{302}$→Ile or Leu; $Gln_{297}$→Asp or Lys; $Trp_{307}$→Tyr or Phe; $Trp_{310}$→Tyr or Phe; $Ile_{311}$→Phe or Val; $Tyr_{317}$→Trp or Thr; $His_{318}$→Lys; $Ala_{319}$→Ser; $Asn_{320}$→Gln, Tyr or His; $Tyr_{321}$→Thr or Asp, $Phe_{340}$→Tyr; $His_{353}$→Asp; $His_{353}$→Lys (a $\beta_A$/$\beta_B$ hybrid); $Phe_{356}$→Tyr; $Val_{364}$→Phe; $Val_{364}$→Leu; $Tyr_{375}$→Thr; $Tyr_{376}$→Trp; $Asn_{389}$→Gln, His or Lys; $Ile_{391}$→Leu or Thr; $Met_{390}$→Leu or Ser; $Val_{392}$→Phe, Glu, Thr or Ile; or comparably modified human $\beta_B$ chain

**38.** The cell of any of claims 34 to 37 which is a eukaryotic cell.

**39.** A cell-free composition that is free of mature α chain polypeptide containing a human or porcine inhibin α chain prodomain polypeptide sequence as depicted in Fig 1B (porcine α chain) or Fig 6A (human α chain) or an amino acid sequence variant by way of insertion, deletion or substitution of a said prodomain sequence, the variant being substantially homologous with a polypeptide of a said sequence and being cross reactive with antibodies raised against a polypeptide of a depicted sequence.

**40.** A cell-free composition containing

a) a polypeptide comprising the human inhibin $\beta_A$ chain prodomain sequence

HSAAPDCPSCALAALPKDVPNSQPEMVEAVKKHILNMLHL (Amino acids 1-40 of Fig 8), PDVTQPVPKAALLNAIRKLHVGKVGENGYVEIEDDIG (amino acids 44-80 of Fig 8.),

AEMNELMEQTSEIITFAESGTARKTLHFEISKEGSDLSVVERAEVWLFLKVPKAN-RTRTKVTIRLFQQQKHPQGSLDTGEEAEEVGLKGERSELLLSEKVVDA (amino acids 83-185 of Fig 8), STWHVFPVSSSIQRLLD-

QGKSSLDVRIACEQCQESGASLVLLG (amino acids 188-230 of Fig 8),

or naturally occurring mammalian amino acid sequence variants thereof;

b) a polypeptide comprising the human inhibin $\beta_B$ chain prodomain sequence

CTSCGGFRRPEELGRVDGDFLEAV, (amino acids 7-30 of Fig 9),

HILSRLQMRGRPNITHAVPKAAMVTALRKLHAGKVREDGRVEIPHLDGHASPGAD-GQERVSEIISFAETDGLASSRVRLYFFISNEGNQNLFVVQASLWLYLKLLPYVLEKGS (amino acids 33-145 of Fig 9),

VRVKVYFQEQGHGDRWNMVEKRVDLKRSGWHTFPLTEAIQALFERGE (aminc acids 149 - 195 of Fig 9),

LNLDVQCDSCQELAVVPVFVDPGEESHRPFVVVQARLGDSRHRI (amino acids 198-241 of Fig 9),

or naturally occurring mammalian amino acid sequence variants thereof; or

c) a polypeptide free of the mature α chain amino acid sequence comprising the human inhibin α chain pro-

domain sequences

KVRALFLDALGPPAVTREGGDPGV (amino acids 1 - 24 of Fig 6),

HALGGFTHRGSEPEEEEDVSQAILFPATDASCEDKSAARGLAQEAEEGLFRYMFR-PSQHTRSRQVTSAQLWFHTGLDRQGTAASNSSEPLLGLLALSPGGPVAVPMSLGH-APPHWAVLHLATSALSLLTHPVLVLLLRCPLCTCSARPEATPFLVAHTRTRPPSGGERA (amino acids 32 - 199 of Fig 6),

or naturally occurring mammalian amino acid sequence variants thereof.

**41.** A cell-free composition containing a human or porcine inhibin β chain prodomain polypeptide of a sequence as depicted in Fig 2B (porcine β chains) and Figs 8 and 9 (human β chains) or an amino acid sequence variant by way of insertion, deletion, or substitution of a said prodomain sequence, the variant being substantially homologous with a polypeptide of a said sequence, and being cross reactive with antibodies raised against a polypeptide of a depicted sequence.

**42.** The composition of claim 41 wherein the β chain is the $\beta_A$ chain and the composition is free of mature $\beta_A$ chain sequence.

**43.** The composition of claim 41 wherein the β chain is the $\beta_B$ chain and the composition is free of mature $\beta_B$ chain sequence.

**44.** The composition of claim 40 wherein the variant is the corresponding porcine amino acid sequence, the composition containing:

a) a polypeptide comprising the porcine inhibin $\beta_A$ chain prodomain sequence depicted at: amino acids 28 to 58; amino acids 61 to 87; amino acids 90 to 108: amino acids 111 to 179; amino acids 182 to 213; amino acids 216 to 258; or amino acids 28 to 87 of Fig 2B; or
b) a polypeptide free of the mature α chain amino acid sequence comprising the porcine inhibin α chain prodomain polypeptide sequences depicted at: amino acids 20 to 54; or amino acids 70 to 228 of Fig 1B.

**45.** The composition of any of claims 39 to 44 wherein the polypeptide is unaccompanied by native glycosylation.

**46.** The composition of any of claims 39 to 45 which is sterile and wherein the polypeptide further comprises an immunogenic polypeptide.

**47.** The use of a composition of any of claims 39 to 46 in the preparation of an antibody capable of binding said polypeptide.

**48.** The composition of any of claims 39 to 46 wherein the polypeptide is conjugated to a detectable group.

**49.** The composition of claim 48 wherein the group is an enzyme, fluorophore or radioisotope.

**50.** The composition of any of claims 39 to 44, 48 or 49 which is insolubilized by non-covalent absorption or covalent cross-linking to a water insoluble support.

**51.** The composition of any of claims 39 to 46, 48 or 49 further comprising a physiologically acceptable implantable matrix for controlled release of the polypeptide into the tissues of an animal.

**Claims for the following Contracting State : GR**

1. A method comprising culturing a host cell transformed with a vector which includes nucleic acid encoding a human or porcine inhibin $\alpha$ chain and/or a human or porcine inhibin $\beta$ chain the amino acid sequences of which are as depicted in Fig 1B (porcine $\alpha$ chain), Fig 2B (porcine $\beta$ chains), Fig 6A (human $\alpha$ chain) and Figs 8 and 9 (human $\beta$ chains), or an amino acid sequence variant by way of insertion, deletion or substitution of a said depicted sequence, the variant being substantially homologous with a polypeptide of a depicted sequence and 1) being cross, reactive with antibodies raised against a polypeptide of a depicted sequence; or 2) having like hormonal activity to a polypeptide of a depicted sequence.

2. A method according to Claim 1 wherein the nucleic acid encodes a human or porcine inhibin $\alpha$ chain and/or a human or porcine $\beta$ chain the amino acid sequences of which are as depicted in Fig 1B (porcine $\alpha$ chain), Fig 2B (porcine $\beta$ chains), Fig 6A (human $\alpha$ chain) and Figs 8 and 9 (human $\beta$ chains).

3. A method according to Claim 1 wherein the vector encodes an allelic variant of human or porcine inhibin $\alpha$ and/or $\beta$ chain.

4. The method of any one of the preceding claims wherein the nucleic acid encoding the inhibin $\alpha$ chain and/or $\beta$ chain is operably linked to a promoter recognized by the host cell and including the further step of recovering inhibin $\alpha$ chain, inhibin $\beta$ chain, inhibin or a $\beta$ chain dimer from the culture medium.

5. The method of any one of the preceding claims wherein the cell is a prokaryote.

6. The method of any one of the preceding claims wherein the vector comprises nucleic acid encoding the prepro form of an inhibin $\alpha$ chain or an inhibin $\beta$ chain.

7. A method according to claim 6 wherein the vector comprises nucleic acid encoding the prepro forms of both the inhibin $\alpha$ chain and an inhibin $\beta$ chain.

8. The method of any one of claim 1 to 4, 6 or 7 wherein the cell is a cell from a multicellular organism and hormonally active inhibin is produced.

9. The method of claim 4 wherein the promoter is a viral promoter.

10. The method of claim 9 wherein the promoter is an SV40 promoter.

11. The method of any preceding claims wherein mature porcine or human inhibin is recovered.

12. The method of any of claims 1 to 5 or 8 to 10 wherein the vector comprises nucleic acid encoding the prepro form of an inhibin $\beta$ chain and a mature $\beta$-chain dimer is recovered free of the $\alpha$-chain.

13. The method of claim 8 or 11 wherein the $\beta$ chain is the $\beta_A$ chain and the inhibin is present in a concentration greater than about 20 ng/ml in the culture medium.

14. A method according to Claim 4 wherein the inhibin is human or porcine inhibin made up of an $\alpha$ and a $\beta$ chain, amino acid sequences of said $\alpha$ and $\beta$ chain being selected from those depicted in Fig 1B (porcine $\alpha$ chain), Fig 2B (porcine $\beta$ chains), Fig 6A (human $\alpha$ chain) and Figs 8 and 9 (human $\beta$ chains), and amino acid sequence variants by way of insertion, deletion or substitution of a polypeptide of a said depicted sequence, which variants are substantially homologous with a polypeptide of a depicted sequence and 1) are cross reactive with antibodies raised against a polypeptide of a depicted sequence; or 2) have like hormonal activity to a polypeptide of a depicted sequence; which composition is completely free of unidentified human or porcine proteins.

15. A method according to Claim 14 wherein the inhibin is human or porcine inhibin made up of an $\alpha$ and a $\beta$ chain, the amino acid sequences of said $\alpha$ and $\beta$ chain being selected from those depicted in Fig 1B (porcine $\alpha$ chain), Fig 2B (porcine $\beta$ chains), Fig 6A (human $\alpha$ chain) and Figs 8 and 9 (human $\beta$ chains).

16. A method according to Claim 4 wherein the recovered inhibin chain is a prodomain of human or porcine $\alpha$ or $\beta_B$ inhibin as depicted in Fig 1B (porcine $\alpha$ chain), Fig 2B (porcine $\beta_B$ chain), Fig 6A (human $\alpha$ chain), and Fig 9

(human $\beta_B$ chain) unassociated with native glycosylation.

**17.** A method according to Claim 4 wherein a β chain dimer is recovered which is a homodimer of mature human or porcine inhibin $\beta_A$ or $\beta_B$ chains, said chains being as depicted in Fig 2B (porcine β chains) and Figs 8 and 9 (human β chains) or an amino acid sequence variant by way of insertion, deletion or substitution of a polypeptide of a said depicted sequence the variant being substantially homologous with a polypeptide of a depicted sequence and 1) cross reactive with antibodies raised against a polypeptide of a depicted sequence; or 2) having like hormonal activity to a polypeptide of a depicted sequence; which dimer is free of the inhibin α chain.

**18.** A method according to Claim 17 wherein a homodimer of mature human or porcine $\beta_B$ chains or a said amino acid sequence variant thereof is recovered.

**19.** A method according to Claim 4 wherein a β chain dimer is recovered which is a heterodimer of mature human or porcine inhibin $\beta_A$ with mature human or porcine inhibin $\beta_B$, said chains being as depicted in Fig 2B (porcine β chains) and Figs 8 and 9 (human β chains) or an amino acid sequence variant by way of insertion, deletion or substitution of a polypeptide of a said depicted sequence the variant being substantially homologous with a polypeptide of a depicted sequence and 1) being cross reactive with antibodies raised against a polypeptide of a depicted sequence; or 2) having like hormonal activity to a polypeptide of a depicted sequence; which dimer is free of the inhibin α chain.

**20.** Non chromosomal DNA encoding a human or porcine inhibin α or a human or porcine inhibin β chain the amino acid sequences of which are as depicted in Fig 1B (porcine α chain), Fig 2B (porcine β chains), Fig 6A (human α chain) and Figs 8 and 9 (human β chains) or an amino acid sequence variant by way of insertion, deletion or substitution of a polypeptide of a said depicted sequence, the variant being substantially homologous with a polypeptide of a depicted sequence and 1) being cross reactive with antibodies raised against a polypeptide of a depicted sequence; or 2) having like hormonal activity to a polypeptide of a depicted sequence.

**21.** A DNA according to Claim 20 encoding a human or porcine inhibin α or a human or porcine inhibin β chain whose amino acid sequence. is as depicted in Fig 1B (porcine α chain), Fig 2B (porcine β chains), Fig 6A (human α chain) and Figs 8 and 9 (human β chains).

**22.** The DNA of claim 20 or 21 which is free of intervening untranslated sequences.

**23.** The DNA of any one of Claims 20 to 22 which is labelled with a detectable moiety.

**24.** A replicable vector comprising a DNA of claim 20 or 21.

**25.** The vector of Claim 24 comprising a viral promoter operably linked to the DNA encoding the inhibin α and/or β chains.

**26.** The vector of claim 24 or 25 which contains DNA encoding both an inhibin α and an inhibin β chain.

**27.** The vector of claim 24 or 25 which contains DNA encoding an inhibin β chain but not the inhibin α chain.

**28.** A host cell transformed with a replicable vector comprising DNA encoding the human or porcine inhibin α and/or an inhibin β chain the amino acid sequences of which are as depicted in Fig 1B (porcine α chain), Fig 2B (porcine β chains), Fig 6A (human α chain) and Figs 8 and 9 (human β chains) or an amino acid sequence variant by way of insertion, deletion or substitution of a polypeptide of a said depicted sequence, the variant being substantially homologous with a polypeptide of a depicted sequence and 1) being cross reactive with antibodies raised against a polypeptide of a depicted sequence; or 2) having like hormonal activity to a depicted sequence.

**29.** A host cell according to Claim 28 wherein the DNA encodes a human or porcine inhibin α and/or an inhibin β chain, the amino acid sequences of which are as depicted in Fig 1B (porcine α chain), Fig 2B (porcine β chains), Fig 6A (human α chain) and Figs 8 and 9 (human β chains).

**30.** The cell of claim 28 or 29 which is a eukaryotic cell.

**31.** A method according to Claim 4 wherein the recovered inhibin chain is a cell-free composition, free of mature α chain sequence, containing a human or porcine inhibin α chain prodomain polypeptide sequence as depicted in Fig 1B (porcine α chain) or Fig 6A (human α chain) or an amino acid sequence variant by way of insertion, deletion or substitution of a said prodomain sequence, the variant being substantially homologous with a polypeptide of a a said sequence and being cross reactive with antibodies raised against a polypeptide of a depicted sequence.

**32.** A method according to claim 4 wherein the recovered inhibin chain is a cell-free composition containing

a) a polypeptide comprising the human inhibin $\beta_A$ chain prodomain sequence

HSAAPDCPSCALAALPKDVPNSQPEMVEAVKKHILNMLHL (amino acids 1-40 of Fig 8), PDVTQPVPKAALLNAIRKLHVGKVGENGYVEIEDDIG (amino acids 44-80 of Fig 8).

AEMNELMEQTSEIITFAESGTARKTLHFEISKEGSDLSVVERAEVWLFLKVPKAN-RTRTKVTIRLFQQQKHPQGSLDTGEEAEEVGLKGERSELLLSEKVVDA (amino acids 83-185 of Fig 8),

STWHVFPVSSSIQRLLDQGKSSLDVRIACEQCQESGASLVLLG (amino acids 188-230 of Fig 8),

or naturally occurring mammalian amino acid sequence variants thereof;

b) a polypeptide comprising the human inhibin $\beta_B$ chain prodomain sequence

CTSCGGFRRPEELGRVDGDFLEAV, (amino acids 7-30 of Fig 9),

HILSRLQMRGRPNITHAVPKAAMVTALRKLHAGKVREDGRVEIPHLDGHASPGAD-GQERVSEIISFAETDGLASSRVRLYFFISNEGNQNLFVVQASLWLYLKLLPYV-LEKGS (amino acids 33-145 of Fig 9),

VRVKVYFQEQGHDRWNMVEKRVDLKRSGWHTFPLTEAIQALFERGE (amino acids 149 - 195 of Fig 9),

LNLDVQCDSCQELAVVPVFVDPGEESHRPFVVVQARLGDSRHRI (amino acids 198-241 of Fig 9),

or naturally occurring mammalian amino acid sequence variants thereof; or

c) a polypeptide free of the mature α chain amino acid sequence comprising the human inhibin α chain prodomain sequences

KVRALFLDALGPPAVTREGGPGV (amino acids 1 - 24 of Fig 6),

HALGGFTHRGSEPEEEEDVSQAILFPATDASCEDKSAARGLAQEAEEGLFRYMFR-PSQHTRSRQVTSAQLWFHTGLDRQGTAASNSSEPLLGLLALSPGGPVAVPMSLGH-APPHWAVLHLATSALSLLTHPVLVLLLRCPLCTCSARPEATPFLVAHTRTRPPSG-GERA (amino acids 32 - 199 of Fig 6),

or naturally occurring mammalian amino acid sequence variants thereof.

**33.** A method according to Claim 4 wherein the recovered inhibin chain is a cell-free composition containing a human or porcine inhibin β chain prodomain polypeptide of a sequence as depicted in Fig 2B (porcine β chains) and Figs 8 and 9 (human β chains) or an amino acid sequence variant by way of insertion, deletion, or substitution of a said prodomain polypeptide of a sequence, the variant being substantially homologous with a polypeptide of a said sequence and being cross reactive with antibodies raise against a polypeptide of a depicted sequence.

**34.** The method of claim 33 wherein the β chain is the $\beta_A$ chain and the composition is free of mature $\beta_A$ chain sequence.

**35.** The method of claim 33 wherein the β chain is the $\beta_B$ chain and the composition is free of mature $\beta_B$ chain sequence.

**36.** The method of claim 32 wherein the variant is the corresponding porcine amino acid sequence, the composition containing:

a) a polypeptide comprising the porcine inhibin $\beta_A$ chain prodomain sequence depicted at amino acids 28 to 58; amino acids 61 to 87; amino acids 90 to 108; amino acids 111 to 179; amino acids 182 to 213; amino acids 216 to 258; or amino acids 28 to 87 of Fig 2B; or

b) a polypeptide free of the mature α chain amino acid sequence comprising the porcine inhibin α chain prodomain polypeptide sequences depicted at: amino acids 20 to 54; or amino acids 70 to 228 of Fig 1B.

**37.** The method of any of claims 31 to 36 wherein the polypeptide is unaccompanied by native glycosylation.

**38.** The method of any one of claims 31 to 37 wherein the composition is sterile and further comprising coupling the polypeptide with an immunogenic polypeptide.

**39.** The use of a composition of any of claims 31 to 38 in the preparation of an antibody capable of binding said polypeptide.

**40.** The method of any of claims 31 to 38 further comprising conjugating the polypeptide to a detectable group.

**41.** The method of claim 40 wherein the group is an enzyme, fluorophore or radioisotope.

**42.** The method of any of claims 31 to 38, 40, or 41 wherein the composition is insolubilized by non-covalent absorption or covalent cross-linking to a water insoluble support.

**43.** The method of any of claims 31 to 38 or 40 to 41 further comprising mixing the composition with a physiologically acceptable implantable matrix for controlled release of the polypeptide into the tissues of an animal.

**Claims for the following Contracting States : AT, ES**

1. A method comprising culturing a host cell transformed with a vector which includes nucleic acid encoding a human or porcine inhibin α chain and/or a human or porcine inhibin chain the amino acid sequences of which are as depicted in Fig is (porcine α chain), Fig 2B (porcine β chains), Fig 6A (human α chain) and Figs 8 and 9 (human β chains), or an amino acid sequence variant by way of insertion, deletion or substitution of a said depicted sequence, the variant being substantially homologous with a polypeptide of a depicted sequence and 1) being cross reactive with antibodies raised against a polypeptide of a depicted sequence; or 2) having like hormonal activity to a polypeptide of a depicted sequence.

2. A method according to claim 1 wherein the nucleic acid encodes a human or porcine inhibin α chain and/or a human or porcine β chain the amino acid sequences of which are as depicted in Fig 1B (porcine α chain), Fig 2B (porcine β chains), Fig 6A (human α chain) and Figs 8 and 9 (human β chains).

3. A method according to claim 1 wherein the vector encodes an allelic variant of human or porcine inhibin α and/or β chain.

4. The method of any one of the preceding claims wherein the nucleic acid encoding the inhibin α chain and/or β chain is operably linked to a promoter recognized by the host cell and including the further step of recovering inhibin α chain, inhibin β chain, inhibin or a β chain dimer from the culture medium.

5. The method of any one of the preceding claims wherein the cell is a prokaryote.

6. The method of any one of the preceding claims wherein the vector comprises nucleic acid encoding the prepro form of an inhibin α chain or an inhibin β chain.

7. A method according to claim 6 wherein the vector comprises nucleic acid encoding the prepro forms of both the inhibin α chain and an inhibin β chain.

8. The method of any one of claim 1 to 4, 6 or 7 wherein the cell is a cell from a multicellular organism and hormonally active inhibin is produced.

9. The method of claim 4 wherein the promoter is a viral promoter.

10. The method of claim 9 wherein the promoter is an SV40 promoter.

11. The method of any preceding claims wherein mature porcine or human inhibin is recovered.

12. The method of any of claims 1 to 5 or 8 to 10 wherein the vector comprises nucleic acid encoding the prepro form of an inhibin β chain and a mature β-chain dimer is recovered free of the α-chain.

13. The method of claim 8 or 11 wherein the β chain is the $\beta_A$ chain and the inhibin is present in a concentration greater than about 20 ng/ml in the culture medium.

14. A method according to claim 4 wherein the inhibin is human or porcine inhibin made up of an α and a β chain, the amino acid sequences of said α and β chain being selected from those depicted in Fig 1B (porcine α chain), Fig 2B (porcine β chains), Fig 6A (human α chain) and Figs 8 and 9 (human β chains), and amino acid sequence variants by way of insertion, deletion or substitution of a polypeptide of a said depicted sequence, which variants are substantially homologous with a polypeptide of a depicted sequence and 1) are cross reactive with antibodies raised against a polypeptide of a depicted sequence; or 2) have like hormonal activity to a polypeptide of a depicted sequence; which composition is completely free of unidentified human or porcine proteins.

15. A method according to claim 14 wherein the inhibin is human or porcine inhibin made up of an α and a β chain, the amino acid sequences of said α and chain being selected from those depicted in Fig 1B (porcine α chain), Fig 2B (porcine β chains), Fig 6A (human α chain) and Figs 8 and 9 (human β chains).

**16.** A method according to claim 4 wherein the recovered inhibin chain is a prodomain of human or porcine $\alpha$ or $\beta_B$ inhibin as depicted in Fig 1B (porcine $\alpha$ chain), Fig 2B (porcine $\beta_B$ chain), Fig 6A (human $\alpha$ chain), and Fig 9 (human $\beta_B$ chain) unassociated with native glycosylation.

**17.** A method according to claim 4 wherein a $\beta$ chain dimer is recovered which is a homodimer of mature human or porcine inhibin $\beta_A$ or $\beta_B$ chains, said chains being as depicted in Fig 2B (porcine $\beta$ chains) and Figs 8 and 9 (human $\beta$ chains) or an amino acid sequence variant by way of insertion, deletion or substitution of a polypeptide of a said depicted sequence the variant being substantially homologous with a polypeptide of a depicted sequence and 1) being cross reactive with antibodies raised against a polypeptide of a depicted sequence; or 2) having like hormonal activity to a polypeptide of a depicted sequence; which dimer is free of the inhibin $\alpha$ chain.

**18.** A method according to claim 17 wherein a homodimer of mature human or porcine $\beta_B$ chains or a said amino acid sequence variant thereof is recovered.

**19.** A method according to claim 4 wherein a $\beta$ chain dimer is recovered which is a heterodimer of mature human or porcine inhibin $\beta_A$ with mature human or porcine inhibin $\beta_B$, said chains being as depicted in Fig 2B (porcine $\beta$ chains) and Figs 8 and 9 (human $\beta$ chains) or an amino acid sequence variant by way of insertion, deletion or substitution of a polypeptide of a said depicted sequence the'variant being substantially homologous with a polypeptide of a depicted sequence and 1) being cross reactive with antibodies raised against a polypeptide of a depicted sequence, or 2) having like hormonal activity to a polypeptide of a depicted sequence; which heterodimer is free of the inhibin $\alpha$ chain.

**20.** A method for the production of non chromosomal DNA encoding a human or porcine inhibin $\alpha$ or a human or porcine inhibin $\beta$ chain as depicted in Fig 1B (porcine $\alpha$ chain), Fig 2B (porcine $\beta$ chains), Fig 6A (human $\alpha$ chain) and Figs 8 and 9 (human $\beta$ chains) or an amino acid sequence variant by way of insertion, deletion or substitution of a polypeptide of a said depicted sequence, the variant being substantially homologous with a polypeptide of a depicted sequence and 1) being cross reactive with antibodies raised against a polypeptide of a depicted sequence; or 2) having like hormonal activity to a polypeptide of a depicted sequence; the method comprising chemical synthesis, screening mRNA from ovary, or screening genomic libraries of any cell.

**21.** A method according to claim 20 wherein the DNA encodes a human or porcine inhibin $\alpha$ or a human or porcine inhibin $\beta$ chain whose amino acid sequence is as depicted in Fig 1B (porcine $\alpha$ chain), Fig 2B (porcine $\beta$ chains), Fig 6A (human $\alpha$ chain) and Figs 8 and 9 (human $\beta$ chains).

**22.** The method of claim 20 or 21 wherein the non-chromosomal DNA is free of intervening untranslated sequences.

**23.** The method of any one of claims 20. to 22 further comprising the step of labelling the non-chromosomal DNA with a detectable moiety.

**24.** A method for the production of a replicable vector, the method comprising inserting a DNA of claim 20 or 21 in a cloning vector.

**25.** The method of claim 24 wherein the DNA is inserted into a vector which has a viral promoter operably linked to the DNA encoding the inhibin $\alpha$ and/or $\beta$ chains thereby to produce an expression vector.

**26.** The method of claim 24 or 25 wherein DNA encoding both an inhibin $\alpha$ and an inhibin $\beta$ chain is inserted into the vector.

**27.** The method of claim 24 or 25 wherein DNA encoding an inhibin $\beta$ chain but not the inhibin $\alpha$ chain is inserted into the vector.

**28.** A host cell transformed with a replicable vector comprising DNA encoding the human or porcine inhibin $\alpha$ and/or an inhibin $\beta$ chain the amino acid sequences of which are as depicted in Fig 1B (porcine $\alpha$ chain), Fig 2B (porcine $\beta$ chains), Fig 6A (human $\alpha$ chain) and Figs 8 and 9 (human $\beta$ chains) or an amino acid sequence variant by way of insertion, deletion or substitution of a polypeptide of a said depicted sequence, the variant being substantially homologous with a polypeptide of a depicted sequence and 1) being cross reactive with antibodies raised against a polypeptide of a depicted sequence; or 2) having like hormonal activity to a polypeptide of a depicted sequence.

**29.** A host cell according to claim 28 wherein the DNA encodes a human or porcine inhibin α and/or an inhibin β chain, the amino acid sequences of which are as depicted in Fig 1B (porcine α chain), Fig 2B (porcine β chains), Fig 6A (human α chain) and Figs 8 and 9 (human β chains).

**30.** The cell of claim 28 or 29 which is a eukaryotic cell.

**31.** A method according to claim 4 wherein the recovered inhibin chain is a cell-free composition free of mature α chain sequences containing a human or porcine inhibin α chain prodomain polypeptide sequence as depicted in Fig 1B (porcine α chain) or Fig 6A (human α chain) or an amino acid sequence variant of a said prodomain sequence, the variant being substantially homologous with a polypeptide of a said sequence and being cross reactive with antibodies raised against a polypeptide of a depicted sequence.

**32.** A method according to claim 4 wherein the recovered inhibin chain is a cell-free composition containing

a) a polypeptide comprising the human inhibin $\beta_A$ chain prodomain sequence

HSAAPDCPSCALAALPKDVPNSQPEMVEAVKKHILNMLHL (amino acids 1-40 of Fig 8, PDVTQPVPKAALLNAIRKLHVGKVGENGYVEIEDDIG (amino acids 44-80 of Fig 8).

AEMNELMEQTSEIITFAESGTARKTLHFEISKEGSDLSVVERAEVWLFLKVPKAN-TRTKVTIRLFQQQKHPQGSLDTGEEAEEVGLKGERSELLLSEKVVDA, amino acids 83-185 of Fig 8),

STWHVFPVSSSIQRLLDQGKSSLDVRIACEQCQESGASLVLLG (amino acids 188-230 of Fig 8),

or naturally occurring mammalian amino acid sequence variants thereof;

b) a polypeptide comprising the human inhibin $\beta_B$ chain prodomain sequence

CTSCGGFRRPEELGRVDGDFLEAV, (amino acids 7-30 of Fig 9),

HILSRLQMRGRPNITHAVPKAAMVTALRKLHAGKVREDGRVEIPHLDGHASPGAD-GQERVSEIISFAETDGLASSRVRLYFFISNEGNQNLFVVQASLWLYLKLLPYV-LEKGS (amino acids 33-145 of Fig 9),

VRVKVYFQEQGHDRWNMVEKRVDLKRSGWHTFPLTEAIQALFERGE (amino acids 149 - 195 of Fig 9),

LNLDVQCDSCQELAVVPVFVDPGEESHRPFVVVQARLGDSRHRI (amino acids 198-241 of Fig 9),

or naturally occurring mammalian amino acid sequence variants thereof; or
c) a polypeptide free of the mature α chain amino acid sequence comprising the human inhibin α chain prodomain sequences

KVRALFLDALGPPAVTREGGPGV (amino acids 1 - 24 of Fig 6),

HALGGFTHRGSEPEEEEDVSQAILFPATDASCEDKSAARGLAQEAEEGLFRYMFR-PSQHTRSRQVTSAQLWFHTGLDRQGTAASNSSEPLLGLLALSPGGPVAVPMSLGH-APPHWAVLHLATSALSLLTHPVLVLLLRCPLCTCSARPEATPFLVAHTRTRPPSG-GERA (amino acids 32 - 199 of Fig 6),

or naturally occurring mammalian amino acid sequence variants thereof.

**33.** A method according to claim 4 wherein the recovered inhibin chain is a cell-free composition containing a human or porcine inhibin β chain prodomain polypeptide of a sequence as depicted in Fig 2B (porcine β chains) and Figs 8 and 9 (human β chains) or an amino acid sequence variant by way of insertion, deletion or substitution of a said prodomain polypeptide of a sequence, the variant being substantially homologous with a polypeptide of a said sequence and being cross reactive with antibodies raised against a polypeptide of a depicted sequence.

**34.** The method of claim 33 wherein the β chain is the $\beta_A$ chain and the composition is free of mature $\beta_A$ chain sequence.

**35.** The method of claim 33 wherein the β chain is the $\beta_B$ chain and the composition is free of mature $\beta_B$ chain sequence.

**36.** The method of claim 32 wherein the variant is the corresponding porcine amino acid sequence, the composition containing:

a) a polypeptide comprising the porcine inhibin $\beta_A$ chain prodomain sequence depicted at: amino acids 28 to 58; amino acids 61 to 87; amino acids 90 to 108; amino acids 11 to 179; amino acids 182 to 213; amino acids 216 to 258; or amino acids 28 to 87 of Fig 2B; or
b) a polypeptide free of the mature α chain amino acid sequence comprising the porcine inhibin α chain prodomain polypeptide sequences depicted at: amino acids 20 to 54; or amino acids 70 to 228 of Fig 1B.

**37.** The method of any of claims 31 to 36 wherein the polypeptide is unaccompanied by native glycosylation.

**38.** The method of any one of claims 31 to 37 wherein the composition is sterile and further comprising coupling the polypeptide to an immunogenic polypeptide.

**39.** The use of a polypeptide of any of claims 31 to 38 in the preparation of an antibody capable of binding said polypeptide.

**40.** The method of any of claims 31 to 38 further comprising conjugating the polypeptide to a detectable group.

**41.** The method of claim 40 wherein the group is an enzyme, fluorophore or radioisotope.

**42.** The method of any of claims 31 to 38, 40 or 41 wherein the composition is insolubilized by non-covalent absorption or covalent cross-linking to a water insoluble support.

**43.** The method of any of claims 31 to 38 or 40 or. 41 further comprising mixing the composition with a physiologically acceptable implantable matrix for controlled release of the polypeptide into the tissues of an animal.

**Patentansprüche**

**Patentanspruch für folgende Vertragsstaaten : BE, CH, DE, FR, GB, IT, LI, NL, SE**

**1.** Verfahren, umfassend das Kultivieren einer Wirtszelle, die mit einem Vektor transformiert wurde, der Nukleinsäure enthält, die für eine menschliche oder Schweine-Inhibin-α-Kette und/oder eine menschliche oder Schweine-Inhibin-β-Kette, deren Aminosäuresequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind, oder eine durch Einfügung, Löschung oder Substitution einer der dargestellten Sequenzen erhaltene Aminosäuresequenz-Variante kodiert, wobei die Variante im wesentlichen homolog zu einem Polypeptid mit einer dargestellten Sequenz ist, jedoch unter Ausschluß der Rinder-Inhibin-α-Kette und der partiellen Rinder-Inhibin-β-Kette der Sequenz

Gly-Leu-Glu-Cys-Asp-Gly-Lys-Val-Asn-Ile-Cys-
Cys-Lys-Lys-Gln-Phe-Phe-Val-Ser-Phe-Lys-Asp-Ile-Gly-Trp-
Asn-Asp-Trp-Ile-Ile-Ala-Pro-Ser-Gly-Tyr-His-Ala-Asn-Tyr-
Cys-Glu-Gly-Glu-Cys-Pro-Ser-His-Ile-Ala-Gly-Thr-Ser-Gly-
Ser-Ser-Leu-Ser-Phe-His-Ser-Thr-Val-Ile-Asn-His-Tyr-Arg-
Met-Arg-Gly-His-Ser

und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt.

**2.** Verfahren nach Anspruch 1, worin die Nukleinsäure für eine menschliche oder Schweine-Inhibin-a-Kette und/oder eine menschliche oder Schweine-Inhibin-β-Kette kodiert, deren Aminosäuresequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind.

**3.** Verfahren nach Anspruch 1, worin der Vektor für eine Inhibin-β-Kette oder eine Aminosäuresequenzvariante davon mit Ausnahme der nativen menschlichen oder Schweine-$\beta_A$- oder der partiellen Rinder-Inhibin-β-Kette, wie in Anspruch 1 dargelegt, kodiert.

**4.** Verfahren nach Anspruch 1, worin die Nukleinsäure für eine Variante kodiert, ausgewählt aus: den Schweine-Inhibinderivaten ($Asn_{266}$→Gln]Inhα; [$Cys_{325}$ oder $Cys_{324}$→Δ]Inhα; [$Cys_{361}$ oder $Cys_{363}$→Δ]Inhα; [$Lys_{321}$ oder $Lys_{322}$→Δ]$Inh\beta_A$; [$Lys_{322}$→His oderSer] $Inh\beta_A$; [$Lys_{315}$→Arg; $Val_{316}$→Thr]$Inh\beta_A$; [$Cys_{388}$ oder $Cys_{390}$→Δ]$Inh\beta_A$, [$Lys_{411}$→Gln]$Inh\beta_A$; [$Arg_{315}$→Lys; $Val_{316}$→Thr]$Inh\beta_B$; [$Cys_{319}$ oder $Cys_{320}$→Δ]$Inh\beta_B$; [$Pro_{381}$ $Gly_{382}$→Pro Phe Gly] $Inh\beta_B$; [$Arg_{395}$→Gln]$Inh\beta_B$,
worin Inh eine Abkürzung für Inhibin ist und die Nummern der Reste für $Inh\beta_B$ die für den entsprechenden $Inh\beta_A$-Rest verwendeten sind (siehe Fig. 2B); den menschliche Inhibin-$\beta_A$-Ketten-Varianten, die Variationen aufweisen, die Substitution oder Löschung eines Restes oder Einfügung neben einem Rest, ausgewählt aus den Resten 293 - 297, 364 - 376 und 387 - 398, der in Fig. 8 dargestellten Sequenz sind; und den Varianten menschlicher α-Ketten, die zu mehr als 90% homolog zu der in Fig. 6A dargestellten. Sequenz sind.

**5.** Verfahren nach Anspruch 1, worin die Nukleinsäure für eine Variante einer menschlichen $\beta_A$-Inhibin-Kette, ausgewählt aus

$Phe_{302}$→Ile oder Leu; $Gln_{297}$→Asp oder Lys; $Trp_{307}$→Tyr oder Phe; $Trp_{310}$→Tyr oder Phe; $Ile_{311}$→Phe oder Val; $Tyr_{317}$→Trp oder Thr; $His_{318}$→Lys; $Ala_{319}$→Ser; $Asn_{320}$→Gln, Tyr oder His; $Tyr_{321}$→Thr oder Asp, $Phe_{340}$→Tyr; $His_{353}$→Asp; $His_{353}$→Lys (ein $\beta_A/\beta_B$-Hybrid); $Phe_{356}$→Tyr; $Val_{364}$→Phe; $Val_{364}$→Leu; $Tyr_{375}$→Thr; $Tyr_{376}$→Trp; $Asn_{389}$→Gln, His oder Lys; $Ile_{391}$→Leu oder Thr; $Met_{390}$ →Leu oder Ser; $Val_{392}$→Phe, Glu, Thr oder Ile; oder eine vergleichbar modifizierte menschliche $\beta_B$-Kette kodiert.

**6.** Verfahren nach einem der vorangegangenen Ansprüche, worin die Nukleinsäure, die für die Inhibin-α-Kette und/oder -β-Kette kodiert, mit einem von der Wirtszelle erkannten Promotor operabel verbunden ist und das den weiteren Schritt der Gewinnung von Inhibin oder einem β-Ketten-Dimer aus dem Kulturmedium umfaßt.

**7.** Verfahren nach einem der vorangegangenen Ansprüche, worin die Zelle ein Prokaryot ist.

**8.** Verfahren nach einem der vorangegangenen Ansprüche, worin der Vektor Nukleinsäure umfaßt, die für die Präpro-Form einer Inhibin-α-Kette oder einer Inhibin-β-Kette kodiert.

**9.** Verfahren nach Anspruch 8, worin der Vektor eine Nukleinsäure umfaßt, die sowohl für eine Präpro-Form einer Inhibin-α-Kette als auch einer Inhibin-β-Kette kodiert.

**10.** Verfahren nach einem der Ansprüche 1-6, 8 oder 9, worin die Zelle eine Zelle aus einem vielzelligen Organismus ist und hormonell aktives Inhibin produziert wird.

**11.** Verfahren nach Anspruch 6, worin der Promotor ein viraler Promotor ist.

**12.** Verfahren nach Anspruch 11, worin der Promotor ein SV40-Promotor ist.

**13.** Verfahren nach einem der vorangegangenen Ansprüche, worin reifes Schweine- oder menschliches Inhibin gewonnen wird.

**14.** Verfahren nach einem der Ansprüche 1 bis 7 oder 10-12, worin der Vektor Nukleinsäure umfaßt, die für die Präpro-Form einer Inhibin-β-Kette kodiert, und ein reifes β-Ketten-Dimer frei von der α-Kette gewonnen wird.

**15.** Verfahren nach Anspruch 10 oder 13, worin die β-Kette die $\beta_A$-Kette ist und das Inhibin in einer Konzentration von über etwa 20 ng/ml im Kulturmedium vorliegt.

**16.** Zusammensetzung, umfassend menschliches oder Schweine-Inhibin, das aus einer α- und einer β-Kette besteht, wobei die Aminosäuresequenzen der α- und β-Ketten aus jenen in Fig. 1B (Schweine-α-Kette), Fig. 2B (Schweine-β-Ketten), Fig. 6A (menschliche α-Kette) und Fig. 8 und 9 (menschliche β-Ketten) dargestellten ausgewählt sind, und durch Einfügung, Löschung oder Substitution eines Polypeptids mit einer der dargestellten Sequenzen erhaltenen Aminosäuresequenz-Varianten, wobei die Varianten im wesentlichen homolog zu einem Polypeptid mit einer dargestellten Sequenz sind, jedoch unter Ausschluß der Rinder-Inhibin-α-Kette, und 1) kreuzreaktiv mit gegen ein Polypeptid mit einer dargestellten Sequenz gebildeten Antikörpern sind; oder 2) ähnliche hormonelle Aktivität wie ein Polypeptid mit einer dargestellten Sequenz besitzen; wobei die Zusammensetzung vollständig frei von nicht identifizierten menschlichen oder Schweine-Proteinen ist.

**17.** Zusammensetzung nach Anspruch 16, worin die Sequenzen der α- und β-Ketten aus jenen ausgewählt sind, die in Fig. 1B (Schweine-α-Kette), Fig. 2B (Schweine-β-Ketten), Fig. 6A (menschliche α-Kette) und Fig. 8 und 9 (menschliche β-Ketten) dargestellt sind.

**18.** Zusammensetzung nach Anspruch 16, worin die Varianten ausgewählt werden aus: den Schweine-Inhibinderivaten

($Asn_{266}$→In]GInhα; [$Cys_{325}$ oder $Cys_{324}$→Δ]Inhα; ($Cys_{361}$ oder $Cys_{363}$→Δ]Inhα; [$Lys_{321}$ oder $Lys_{322}$→Δ]Inh$\beta_A$; [$Lys_{322}$→His oder Ser]Inh$\beta_A$; [$Lys_{315}$→Arg; $Val_{316}$→Thr] Inh$\beta_A$; [$Cys_{388}$ oder $Cys_{390}$→Δ]Inh$\beta_A$, [$Lys_{411}$→Gln]Inh$\beta_A$; [$Arg_{315}$→Lys; $Val_{316}$→Thr]Inh$\beta_B$; [$Cys_{319}$ oder $Cys_{320}$→Δ]Inh$\beta_B$ [$Pro_{381}$ $Gly_{382}$→ Pro Phe Gly]Inh$\beta_B$; [$Arg_{395}$→Gln] Inn$\beta_B$,

worin Inh eine Abkürzung für Inhibin ist und die Nummern der Reste für Inh$\beta_B$ die für den entsprechenden Inh$\beta_A$-Rest verwendeten sind (siehe Fig. 2B); den menschlichen Inhibin-$\beta_A$-Ketten-Varianten, die Variationen aufweisen, die Substitution oder Löschung eines Restes oder Einfügung neben einem Rest, ausgewählt aus den Resten 293 - 297, 364 - 376 und 387 - 398, der in Fig. 8 dargestellten Sequenz, sind; und den Varianten menschlicher α-Ketten, die zu mehr als 90% homolog zu der in Fig. 6A dargestellten Sequenz sind.

**19.** Zusammensetzung nach Anspruch 16, umfassend eine Variante einer menschlichen $\beta_A$-Inhibin-Kette, ausgewählt aus $Phe_{302}$→Ile oder Leu; $Gln_{297}$→Asp oder Lys; $Trp_{307}$→Tyr oder Phe; $Trp_{310}$→Tyr oder Phe; $Ile_{311}$→Phe oder Val; $Tyr_{317}$→Trp oder Thr; $His_{318}$→Lys; $Ala_{319}$→Ser; $Asn_{320}$→Gln, Tyr oder His; $Tyr_{321}$→Thr oder Asp, $Phe_{340}$→Tyr; $His_{353}$→Asp; $His_{353}$→Lys (a $\beta_A/\beta_B$ hybrid); $Phe_{356}$→Tyr; $Val_{364}$→Phe; $Val_{364}$→Leu; $Tyr_{375}$→Thr; $Tyr_{376}$→Trp; $Asn_{389}$→Gln, His oder Lys; $Ile_{391}$→Leu oder Thr; $Met_{390}$ →Leu oder Ser; $Val_{392}$→Phe, Glu, Thr oder Ile; oder eine vergleichbar modifizierte menschliche $\beta_B$-Kette.

**20.** Zusammensetzung, umfassend eine Prodomäne von menschlichem oder Schweine-α- oder -$\beta_B$-Inhibin, wie in Fig. 1B (Schweine-α-Kette), Fig. 2B (Schweine-$\beta_B$-Ketten), Fig. 6A (menschliche α-Kette) und Fig. 9 (menschliche $\beta_B$-Kette) dargestellt, ist, die nicht mit nativer Glykosylierung assoziiert ist.

**21.** Zusammensetzung, umfassend ein Homodimer von reifen menschlichen oder Schweine-Inhibin-$\beta_A$- oder -$\beta_B$-Ketten, wobei die Ketten wie in Fig. 2B (Schweine-β-Ketten) und Fig. 8 und 9 (menschliche β-Ketten) dargestellt sind, oder eine durch Einfügung, Löschung oder Substitution eines Polypeptids mit einer der dargestellten Sequenzen erhaltene Aminosäuresequenz-Variante ist, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt; wobei das Dimer frei von der Inhibin-α-Kette ist.

**22.** Zusammensetzung nach Anspruch 21, die ein Homodimer von reifen menschlichen oder Schweine-$\beta_B$-Ketten oder eine der Aminosäuresequenz-Varianten davon ist.

**23.** Zusammensetzung, umfassend ein Heterodimer von reifem menschlichem oder Schweine-Inhibin-$\beta_A$ mit reifem menschlichem oder Schweine-Inhibin-$\beta_B$, wobei die Ketten wie in Fig. 2B (Schweine-β-Ketten) und Fig. 8 und 9 (menschliche β-Ketten) dargestellt sind, oder einer durch Einfügung, Löschung oder Substitution eines Polypeptids mit einer der dargestellten Sequenzen erhaltene Aminosäuresequenz-Variante, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt; wobei die Zusammensetzung frei von der Inhibin-α-Kette ist.

**24.** Nicht-chromosomale DNA, die für eine menschliche oder Schweine-Inhibin-α-oder eine menschliche oder Schweine-Inhibin-β-Kette, wie in Fig. 1B (Schweine-α-Kette), Fig. 2B (Schweine-β-Ketten), Fig. 6A (menschliche α-Kette) und Fig. 8 und 9 (menschliche β-Ketten) dargestellt, oder für eine durch Einfügung, Löschung oder Substitution eines Polypeptids mit einer der dargestellten Sequenzen erhaltene Aminosäuresequenz-Variante kodiert, wobei die Variante im wesentlichen homolog zu einem Polypeptid mit einer dargestellten Sequenz ist, jedoch unter Ausschluss der Rinder-Inhibin-α-Kette und der partiellen Rinder-Inhibin-β-Kette mit der Sequenz

Gly-Leu-Glu-Cys-Asp-
Gly-Lys-Val-Asn-Ile-Cys-Cys-Lys-Lys-Gln-Phe-Phe-Val-Ser-
Phe-Lys-Asp-Ile-Gly-Trp-Asn-Asp-Trp-Ile-Ile-Ala-Pro-Ser-
Gly-Tyr-His-Ala-Asn-Tyr-Cys-Glu-Gly-Glu-Cys-Pro-Ser-His-
Ile-Ala-Gly-Thr-Ser-Gly-Ser-Ser-Leu-Ser-Phe-His-Ser-Thr-
Val-Ile-Asn-His-Tyr-Arg-Met-Arg-Gly-His-Ser

und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt.

**25.** DNA nach Anspruch 24, die für eine menschliche oder Schweine-Inhibin-α-oder eine menschliche oder Schweine-Inhibin-β-Kette kodiert, deren Aminosäuresequenz wie in Fig. 1B (Schweine-α-Kette), Fig. 2B (Schweine-β-Ketten), Fig. 6A (menschliche α-Kette) und Fig. 8 und 9 (menschliche β-Ketten) dargestellt ist.

**26.** DNA nach Anspruch 24, die für eine Variante kodiert, ausgewählt aus: den Schweine-Inhibinderivaten ($Asn_{266}$→Gln] Inhα; [$Cys_{325}$ oder $Cys_{324}$→Δ]Inhα; [$Cys_{361}$ oder $Cys_{363}$→Δ]Inhα; [$Lys_{321}$ oder $Lys_{322}$→Δ]$Inh\beta_A$; [$Lys_{322}$→His oder Ser]$Inh\beta_A$; [$Lys_{315}$→Arg; $Val_{316}$→Thr] $Inh\beta_A$; [$Cys_{388}$ oder $Cys_{390}$→Δ]$Inh\beta_A$, [$Lys_{411}$→Gln]$Inh\beta_A$; [$Arg_{315}$→Lys; $Val_{316}$→Thr]$Inh\beta_B$; [$Cys_{319}$ oder $Cys_{320}$→Δ]$Inh\beta_B$; [$Pro_{381}$ $Gly_{382}$→ Pro Phe Gly]$Inh\beta_B$; [$Arg_{395}$→Gln] $Inh\beta_B$,
worin Inh eine Abkürzung für Inhibin ist und die Nummern der Reste für $Inh\beta_B$ die für den entsprechenden $Inh\beta_A$-Rest verwendeten sind (siehe Fig. 2B); den menschlichen Inhibin-$\beta_A$-Ketten-Varianten, die Variationen aufweisen, die Substitution oder Löschung eines Restes oder Einfügung neben einem Rest, ausgewählt aus den Resten 293 - 297, 364 - 376 und 387 - 398, der in Fig. 8 dargestellten Sequenz sind; und den Varianten menschlicher α-Ketten, die zu mehr als 90% homolog zu der in Fig. 6A dargestellten Sequenz sind.

**27.** DNA nach Anspruch 24, die für eine Variante einer menschlichen β-Inhibin-Kette, ausgewählt aus $Phe_{302}$→Ile oder Leu; $Gln_{297}$→Asp oder Lys; $Trp_{307}$→Tyr oder Phe; $Trp_{310}$→Tyr oder Phe; $Ile_{311}$→Phe oder Val; $Tyr_{317}$→Trp oder Thr; $His_{318}$→Lys; $Ala_{319}$→Ser; $Asn_{320}$→Gln, Tyr oder His; $Tyr_{321}$→Thr oder Asp, $Phe_{340}$→Tyr; $His_{353}$→Asp; $His_{353}$→Lys (ein $\beta_A/\beta_B$ Hybrid); $Phe_{356}$→Tyr; $Val_{364}$→Phe; $Val_{364}$→Leu; $Tyr_{375}$→Thr; $Tyr_{376}$→Trp; $Asn_{389}$→Gln, His oderLys; $Ile_{391}$→Leu oder Thr; $Met_{390}$ →Leu oder Ser; $Val_{392}$→Phe, Glu, Thr oder Ile;
oder eine vergleichbar modifizierte menschliche $\beta_B$-Kette kodiert.

**28.** DNA nach einem der Ansprüche 24 bis 27, die frei von intervenierenden untranslatierten Sequenzen ist.

**29.** DNA nach einem der Ansprüche 24 bis 28, die mit einer detektierbaren Gruppe markiert ist.

**30.** Replizierbarer Vektor, der eine DNA nach einem der Ansprüche 21 bis 27 umfaßt.

**31.** Vektor nach Anspruch 30, der einen viralen Promotor aufweist, der mit der für die Inhibin-α- und/oder -β-Ketten kodierenden DNA operal verbunden ist.

**32.** Vektor nach Anspruch 30 oder 31, der DNA enthält, die sowohl für eine Inhibin-α- als auch eine Inhibin-β-Kette kodiert.

**33.** Vektor nach Anspruch 30 oder 31, der DNA enthält, die für eine Inhibin-β-Kette, nicht jedoch für die Inhibin-α-Kette kodiert.

**34.** Wirtszelle, die mit einem replizierbaren Vektor transformiert wurde, der DNA enthält, die für eine menschliche oder Schweine-Inhibin-α-Kette und/oder eine -Inhibin-β-Kette, deren Aminosäuresequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind, oder für eine durch Einfügung, Löschung oder Substitution eines Polypeptids mit einer der dargestellten Sequenzen erhaltene Aminosäuresequenz-Variante kodiert, wobei die Variante im wesentlichen homolog zu einem Polypeptid mit einer dargestellten Sequenz ist, jedoch unter Ausschluß der Rinder-Inhibin-α-Kette und der partiellen Rinder-Inhibin-ß-Kette mit der Sequenz

Gly-Leu-Glu-Cys-Asp-Gly-
Lys-Val-Asn-Ile-Cys-Cys-Lys-Lys-Gln-Phe-Phe-Val-Ser-Phe-
Lys-Asp-Ile-Gly-Trp-Asn-Asp-Trp-Ile-Ile-Ala-Pro-Ser-Gly-
Tyr-His-Ala-Asn-Tyr-Cys-Glu-Gly-Glu-Cys-Pro-Ser-His-Ile-
Ala-Gly-Thr-Ser-Gly-Ser-Ser-Leu-Ser-Phe-His-Ser-Thr-Val-
Ile-Asn-His-Tyr-Arg-Met-Arg-Gly-His-Ser

und wobei die Variante 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt.

**35.** Wirtszelle nach Anspruch 34, worin die DNA für eine menschliche oder Schweine-Inhibin-α- und/oder eine -Inhibin-β-Kette kodiert, deren Aminosäure-sequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind.

**36.** Wirtszelle nach Anspruch 34, worin die DNA für eine Variante kodiert, ausgewählt aus: den Schweine-Inhibinderivaten
(Asn$_{266}$→Gln] Inhα; [Cys$_{325}$ oder Cys$_{324}$→Δ] Inhα; [Cys$_{361}$ oder Cys$_{363}$→Δ]Inhα; [Lys$_{321}$ oder Lys$_{322}$→Δ] Inhβ$_A$; [Lys$_{322}$→His oder Ser]Inhβ$_A$; [Lys$_{315}$→Arg; Val$_{316}$→Thr] Inhβ$_A$; [Cys$_{388}$ oder Cys$_{390}$→Δ]Inhβ$_A$, (Lys $_{411}$→Gln] Inhβ$_A$; [Arg$_{315}$→Lys; Val$_{316}$→Thr]Inhβ$_B$; [Cys$_{319}$ oder Cys$_{320}$→Δ]Inhβ$_B$; [Pro$_{381}$ Gly$_{382}$→ Pro Phe Gly]Inhβ$_B$; [Arg$_{395}$→Gln]Inhβ$_B$,
worin Inh eine Abkürzung für Inhibin ist und die Nummern der Reste für Inhβ$_B$ die für den entsprechenden Inhβ$_A$-Rest verwendeten sind (siehe Fig. 2B); den menschlichen Inhibin-β$_A$-Ketten-Varianten, die Variationen aufweisen, die Substitution oder Löschung i eines Restes oder Einfügung neben einem Rest, ausgewählt aus den Resten 293 - 297, 364 - 376 und 387 - 398, der in Fig. 8 dargestellten Sequenz sind; und den Varianten menschlicher α-Ketten, die zu mehr als 90% homolog zu der in Fig. 6A dargestellten Sequenz sind.

**37.** Wirtszelle nach Anspruch 34, worin die DNA für eine Variante einer menschlichen β$_A$-Inhibin-Kette, ausgewählt aus:
Phe$_{302}$→Ile oder Leu; Gln$_{297}$→Asp oder Lys; Trp$_{307}$→Tyr oder Phe; Trp$_{310}$→Tyr oder Phe; Ile$_{311}$→Phe oder Val; Tyr$_{317}$→Trp oder Thr; His$_{318}$→Lys; Ala$_{319}$→Ser; Asn$_{320}$→Gln, Tyr oder His; Tyr$_{321}$→Thr oder Asp, Phe$_{340}$→Tyr; His$_{353}$→Asp; His$_{353}$→Lys (ein β$_A$/β$_B$-Hybrid); Phe$_{356}$→Tyr; Val$_{364}$→Phe; Val$_{364}$→Leu; Tyr$_{375}$→Thr; Tyr$_{376}$→Trp; Asn$_{389}$→Gln, His oder Lys; Ile$_{391}$→Leu oder Thr; Met$_{390}$ →Leu oder Ser; Val$_{392}$→Phe, Glu, Thr oder Ile; oder einer vergleichbar modifizierten menschlichen β$_B$-Kette kodiert.

**38.** Zelle nach irgendeinem der Ansprüche 34 - 37, die eine eukaryotische Zelle ist.

**39.** Zellfreie Zusammensetzung, die frei von reifem α-Ketten-Polypeptid ist, die eine menschliche oder Schweine-Inhibin-α-Ketten-Prodomänen-Polypeptidsequenz, wie in Fig. 1B (Schweine-α-Kette) oder Fig. 6A (menschliche α-Kette) dargestellt, oder eine durch Einfügung, Löschung oder Substitution einer dieser Prodomänen-Sequenzen erhaltene Aminosäuresequenz-Variante enthält, wobei die Variante im wesentlichen homolog zu einem Polypeptid mit einer der dargestellten Sequenzen und kreuzreaktiv mit gegen ein Polypeptid mit einer der dargestellten Sequenzen gebildeten Antikörpern ist.

**40.** Zellfreie Zusammensetzung, umfassend

a) ein Polypeptid, umfassend die menschliche Inhibin-β$_A$-Ketten-Prodomänensequenz

HSAAPDCPSCALAALPKDVPNSQPEMVEAVKKHILNMLHL

(Aminosäuren 1 - 40 aus Fig. 8)

PDVTQPVPKAALLNAIRKLHVGKVGENGYVEIEDDIG

(Aminosäuren 44 - 80 aus Fig. 8)

AEMNELMEQTSEIITFAESGTARKTLHFEISKEGSDLSVVERAEVWLFLKVPKAN-

RTRTKVTIRLFQQQKHPQGSLDTGEEAEEVGLKGERSELLLSEKVVDA

(Aminosäuren 83 - 185 aus Fig. 8) STWHVFPVSSSIQRLLD-

QGKSSLDVRIACEQCQESGASLVLLG

(Aminosäuren 188 - 230 aus Fig. 8)

oder natürlich vorkommende Säugetier-Aminosäuresequenz-Varianten davon;
b) ein Polypeptid, umfassend die menschliche Inhibin-$\beta_B$-Ketten-Prodomänensequenz

CTSCGGFRRPEELGRVDGDFLEAV

(Aminosäuren 7 - 30 aus Fig. 9)

HILSRLQMRGRPNITHAVPKAAMVTALRKLHAGKVREDGRVEIPHLDGHASPGAD-

GQERVSEIISFAETDGLASSRVRLYFFISNEGNQNLFVVQASLWLYLKLLPYVLEKGS

(Aminosäuren 33 - 145 aus Fig. 9)

VRVKVYFQEQGHGDRWNMVEKRVDLKRSGWHTFPLTEAIQALFERGE

(Aminosäuren 149 - 195 aus Fig. 9)

LNLDVQCDSCQELAVVPVFVDPGEESHRPFVVVQARLGDSRHRI

(Aminosäuren 198 - 241 aus Fig. 9)

oder natürlich vorkommende Säugetier-Aminosäuresequenz-Varianten davon; oder
c) ein Polypeptid, das frei von der reifen $\alpha$-Ketten-Aminosäuresequenz ist, umfassend die menschlichen Inhibin-$\alpha$-Ketten-Prodomänensequenzen

KVRALFLDALGPPAVTREGGDPGV

(Aminosäuren 1 - 24 aus Fig. 6)

HALGGFTHRGSEPEEEEDVSQAILFPATDASCEDKSAARGLAQEAEEGLFRYMFR-

PSQHTRSRQVTSAQLWFHTGLDRQGTAASNSSEPLLGLLALSPGGPVAVPMSLGH-

APPHWAVLHLATSALSLLTHPVLVLLLRCPLCTCSARPEATPFLVAHTRTRPPSGGERA

(Aminosäuren 32 - 199 aus Fig. 6)

oder natürlich vorkommende Säugetier-Aminosäuresequenz-Varianten davon.

**41.** Zellfreie Zusammensetzung umfassend ein menschliches oder Schweine-Inhibin-$\beta$-Ketten-Prodomänen-Polypeptid mit einer Sequenz wie in Fig. 2B (Schweine-$\beta$-Ketten) und Fig. 8 und 9 (menschliche $\beta$-Ketten) dargestellt, oder eine durch Einfügung, Löschung oder Substitution einer dieser Prodomänen-Polypeptide mit einer dieser Sequenzen erhaltene Aminosäuresequenz-Variante, wobei die Variante im wesentlichen homolog zu einem Polypeptid mit einer der dargestellten Sequenzen und kreuzreaktiv mit gegen ein Polypeptid mit einer dargestellten Sequenz gebildeten Antikörpern ist.

**42.** Zusammensetzung nach Anspruch 41, worin die $\beta$-Kette die $\beta_A$-Kette ist und die Zusammensetzung frei von reifer $\beta_B$-Ketten-Sequenz ist.

**43.** Zusammensetzung nach Anspruch 41, worin die $\beta$-Kette die $\beta_B$-Kette ist und die Zusammensetzung frei von reifer

$\beta_B$-Ketten-Sequenz ist.

**44.** Zusammensetzung nach Anspruch 40, worin die Variante die entsprechende Schweine-Aminosäuresequenz ist, wobei die Zusammensetzung enthält:

a) ein Polypeptid, umfassend die Schweine-Inhibin-$\beta_A$-Ketten-Prodomänensequenz, die dargestellt wird durch die Aminosäuren: 28-58; 61-87; 90-108; 111-179; 182-213; 216-258; oder 28-87 aus Fig. 2B; oder
b) ein Polypeptid, das frei von der reifen $\alpha$-Ketten-Aminosäuresequenz ist, umfassend die Schweine-Inhibin-$\alpha$-Ketten-Prodomänen-Polypeptidsequenzen, die dargestellt werden durch die Aminosäuren: 20-54 oder 70-228 aus Fig. 1 B.

**45.** Zusammensetzung nach einem der Ansprüche 39 bis 44, worin das Polypeptid nicht von nativer Glykosylierung begleitet wird.

**46.** Zusammensetzung nach einem der Ansprüche 39 bis 45, die steril ist und worin das Polypeptid weiters ein immunogenes Polypeptid umfaßt.

**47.** Verwendung einer Zusammensetzung nach einem der Ansprüche 39 bis 46 zur Herstellung eines Antikörpers, der in der Lage ist, das Polypeptid zu binden.

**48.** Zusammensetzung nach einem der Ansprüche 39 bis 46, worin das Polypeptid an eine detektierbare Gruppe konjugiert ist.

**49.** Zusammensetzung nach Anspruch 48, worin die Gruppe ein Enzym, Fluorophor oder Radioisotop ist.

**50.** Zusammensetzung nach einem der Ansprüche 39 bis 44, 48 oder 49, die durch nichtkovalente Absorption oder kovalente Vernetzung an einen wasserunlöslichen Träger unlöslich gemacht ist.

**51.** Zusammensetzung nach einem der Ansprüche 39 bis 46, 48 oder 49, weiters umfassend eine physiologisch annehmbare, implantierbare Matrix zur gesteuerten Freisetzung des Polpypeptids in die Gewebe eines Tieres.

**Patentansprüche für folgenden Vertragsstaat : LU**

**1.** Verfahren, umfassend das Kultivieren einer Wirtszelle, die mit einem Vektor transformiert wurde, der Nukleinsäure enthält, die für eine menschliche oder Schweine-Inhibin-$\alpha$-Kette und/oder eine menschliche oder Schweine-Inhibin-$\beta$-Kette, deren Aminosäuresequenzen wie in Fig. 1B (Schweine-$\alpha$-Kette), Fig. 2B (Schweine-$\beta$-Ketten), Fig. 6A (menschliche $\alpha$-Kette) und Fig. 8 und 9 (menschliche $\beta$-Ketten) dargestellt sind, oder eine durch Einfügung, Löschung oder Substitution einer der dargestellten Sequenzen erhaltene Aminosäuresequenz-Variante kodiert, wobei die Variante im wesentlichen homolog zu einem Polypeptid mit einer dargestellten Sequenz ist, jedoch unter Ausschluss der Rinder-Inhibin-$\alpha$-Kette, und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt.

**2.** Verfahren nach Anspruch 1, worin die Nukleinsäure für eine menschliche oder Schweine-Inhibin-$\alpha$-Kette und/oder eine menschliche oder Schweine-Inhibin-$\beta$-Kette kodiert, deren Aminosäuresequenzen wie in Fig. 1B (Schweine-$\alpha$-Kette), Fig. 2B (Schweine-$\beta$-Ketten), Fig. 6A (menschliche $\alpha$-Kette) und Fig. 8 und 9 (menschliche $\beta$-Ketten) dargestellt sind.

**3.** Verfahren nach Anspruch 1, worin der Vektor für eine Inhibin-$\beta$-Kette oder eine Aminosäuresequenzvariante davon mit Ausnahme der nativen menschlichen oder Schweine-$\beta_A$ kodiert.

**4.** Verfahren nach Anspruch 1, worin die Nukleinsäure für eine Variante, ausgewählt aus: den Schweine-Inhibinderivaten
($Asn_{266}\rightarrow Gln$]Inh$\alpha$; [$Cys_{325}$ oder $Cys_{324}\rightarrow\Delta$]Inh$\alpha$; [$Cys_{361}$ oder $Cys_{363}\rightarrow\Delta$]Inh$\alpha$; [$Lys_{321}$ oder $Lys_{322}\rightarrow\Delta$]Inh$\beta_A$; [$Lys_{322}\rightarrow$His oder Ser]Inh$\beta_A$; [$Lys_{315}\rightarrow$Arg; $Val_{316}\rightarrow$Thr]Inh$\beta_A$; [$Cys_{388}$ oder $Cys_{390}\rightarrow\Delta$]Inh$\beta_A$, [$Lys_{411}\rightarrow$Gln]Inh$\beta_A$; [$Arg_{315}\rightarrow$Lys ; $Val_{316}\rightarrow$Thr]Inh$\beta_B$; [$Cys_{319}$ oder $Cys_{320}\rightarrow\Delta$]Inh$\beta_B$; [$Pro_{381}$ $Gly_{382}\rightarrow$ Pro Phe Gly]Inh$\beta_B$; [$Arg_{395}\rightarrow$Gln] Inh$\beta_B$,

worin Inh eine Abkürzung für Inhibin ist und die Nummern der Reste für Inh$\beta_B$ die für den entsprechenden Inh$\beta_A$-Rest verwendeten sind (siehe Fig. 2B); den menschlichen Inhibin-$\beta_A$-Ketten-Varianten, die Variationen aufweisen, die Substitution oder Löschung eines Restes oder Einfügung neben einem Rest, ausgewählt aus den Resten 293 - 297, 364 - 376 und 387 - 398, der in Fig. 8 dargestellten Sequenz sind; und den Varianten menschlicher $\alpha$-Ketten, die zu mehr als 90% homolog zu der in Fig. 6A dargestellten Sequenz sind, kodiert.

**5.** Verfahren nach Anspruch 1, worin die Nukleinsäure für eine Variante einer menschlichen $\beta_A$-Inhibin-Kette, ausgewählt aus

Phe$_{302}$→Ile oder Leu; Gln$_{297}$→Asp oder Lys; Trp$_{307}$→Tyr oder Phe; Trp$_{310}$→Tyr oder Phe; Ile$_{311}$→Phe oder Val; Tyr$_{317}$→Trp oder Thr; His$_{318}$→Lys; Ala$_{319}$→Ser; Asn$_{320}$→Gln, Tyr oder His; Tyr$_{321}$→Thr oder Asp, Phe$_{340}$→Tyr; His$_{353}$→Asp; His$_{353}$→Lys (ein $\beta_A$/$\beta_B$-Hybrid); Phe$_{356}$→Tyr; Val$_{364}$→Phe; Val$_{364}$→Leu; Tyr$_{375}$→Thr; Tyr$_{376}$→Trp; Asn$_{389}$→Gln, His oder Lys; Ile$_{391}$→Leu oder Thr; Met$_{390}$ →Leu oder Ser; Val$_{392}$→Phe, Glu, Thr oder Ile;
oder eine vergleichbar modifizierte menschliche $\beta_B$-Kette kodiert.

**6.** Verfahren nach einem der vorangegangenen Ansprüche, worin die Nukleinsäure, die für die Inhibin-$\alpha$-Kette und/oder -$\beta$-Kette kodiert, mit einem von der Wirtszelle erkannten Promotor operabel verbunden ist und das den weiteren Schritt der Gewinnung von Inhibin oder eines $\beta$-Ketten-Dimers aus dem Kulturmedium umfaßt.

**7.** Verfahren nach einem der vorangegangenen Ansprüche, worin die Zelle ein Prokaryot ist.

**8.** Verfahren nach einem der vorangegangenen Ansprüche, worin der Vektor Nukleinsäure umfaßt, die für die Präpro-Form einer Inhibin-$\alpha$-Kette oder einer Inhibin-$\beta$-Kette kodiert.

**9.** Verfahren nach Anspruch 8, worin der Vektor eine Nukleinsäure umfaßt, die sowohl für eine Präpro-Form einer Inhibin-$\alpha$-Kette als auch einer Inhibin-$\beta$-Kette kodiert.

**10.** Verfahren nach einem der Ansprüche 1 bis 6, 8 oder 9, worin die Zelle eine Zelle aus einem vielzelligen Organismus ist und hormonell aktives Inhibin produziert wird.

**11.** Verfahren nach Anspruch 6, worin der Promotor ein viraler Promotor ist.

**12.** Verfahren nach Anspruch 11, worin der Promotor ein SV40-Promotor ist.

**13.** Verfahren nach einem der vorangegangenen Ansprüche, worin reifes Schweine- oder menschliches Inhibin gewonnen wird.

**14.** Verfahren nach einem der Ansprüche 1 bis 7 oder 10 bis 12, worin der Vektor Nukleinsäure umfaßt, die für die Präpro-Form einer Inhibin-$\beta$-Kette kodiert, und ein reifes $\beta$-Ketten-Dimer frei von der $\alpha$-Kette gewonnen wird.

**15.** Verfahren nach Anspruch 10 oder 13, worin die $\beta$-Kette die $\beta_A$-Kette ist und das Inhibin in einer Konzentration von über etwa 20 ng/ml im Kulturmedium vorliegt.

**16.** Zusammensetzung, umfassend menschliches oder Schweine-Inhibin, das aus einer $\alpha$- und einer $\beta$-Kette besteht, wobei die Aminosäuresequenzen der $\alpha$- und $\beta$-Ketten aus jenen in Fig. 1B (Schweine-$\alpha$-Kette), Fig. 2B (Schweine-$\beta$-Ketten), Fig. 6A (menschliche $\alpha$-Kette) und Fig. 8 und 9 (menschliche $\beta$-Ketten) dargestellten ausgewählt sind, und durch Einfügung, Löschung oder Substitution eines Polypeptids mit einer der dargestellten Sequenzen erhaltenen Aminosäuresequenz-Varianten, wobei die Varianten im wesentlichen homolog zu einem Polypeptid mit einer dargestellten Sequenz sind, jedoch unter Ausschluß der Rinder-Inhibin-a-Kette, und 1) kreuzreaktiv mit gegen ein Polypeptid mit einer dargestellten Sequenz gebildeten Antikörpern sind; oder 2) ähnliche hormonelle Aktivität wie ein Polypeptid mit einer dargestellten Sequenz besitzen; wobei die Zusammensetzung vollständig frei von nicht identifizierten menschlichen oder Schweine-Proteinen ist.

**17.** Zusammensetzung nach Anspruch 16, worin die Sequenzen der $\alpha$- und $\beta$-Ketten aus jenen ausgewählt sind, die in Fig. 1B (Schweine-$\alpha$-Kette), Fig. 2B (Schweine-$\beta$-Ketten), Fig. 6A (menschliche $\alpha$-Kette) und Fig. 8 und 9 (menschliche $\beta$-Ketten) dargestellt sind.

**18.** Zusammensetzung nach Anspruch 16, worin die Varianten ausgewählt werden aus: den Schweine-Inhibinderivaten (Asn$_{266}$→In] GInh$\alpha$; [Cys$_{325}$ oder Cys$_{324}$→$\Delta$]Inh$\alpha$; [Cys$_{361}$ oder Cys$_{363}$→$\Delta$] Inh$\alpha$; [Lys$_{321}$ oder Lys$_{322}$→$\Delta$]Inh$\beta_A$;

[$Lys_{322}$→His oder Ser]Inh$\beta_A$; [$Lys_{315}$→Arg; $Val_{316}$→Thr] Inh$\beta_A$; [$Cys_{388}$ oder $Cys_{390}$→Δ]Inh$\beta_A$, [$Lys_{411}$→Gln]Inh$\beta_A$; [$Arg_{315}$→Lys; $Val_{316}$→Thr]Inh$\beta_B$; [$Cys_{319}$ oder $Cys_{320}$→Δ]Inh$\beta_B$ [$Prc_{381}$ $Gly_{382}$→ Pro Phe Gly]Inh$\beta_B$; [$Arg_{395}$→Gln] Inh$\beta_B$,
worin Inh eine Abkürzung für Inhibin ist und die Nummern der Reste für Inh$\beta_B$ die für den entsprechenden Inh$\beta_A$-Rest verwendeten sind (siehe Fig. 2B); den menschlichen Inhibin-$\beta_A$-Ketten-Varianten, die Variationen aufweisen, die Substitution oder Löschung eines Restes oder Einfügung neben einem Rest, ausgewählt aus den Resten 293 - 297, 364 - 376 und 387 - 398, der in Fig. 8 dargestellten Sequenz sind; und den Varianten menschlicher α-Ketten, die zu mehr als 90% homolog zu der in Fig. 6A dargestellten Sequenz sind.

**19.** Zusammensetzung nach Anspruch 16, umfassend eine Variante einer menschlichen $\beta_A$-Inhibin-Kette, ausgewählt aus $Phe_{302}$→Ile oder Leu; $Gln_{297}$→Asp oder Lys; $Trp_{307}$→Tyr oder Phe; $Trp_{310}$→Tyr oder Phe; $Ile_{311}$→Phe oder Val; $Tyr_{317}$→Trp oder Thr; $His_{318}$→Lys; $Ala_{319}$→Ser; $Asn_{320}$→Gln, Tyr oder His;
$Tyr_{321}$→Thr oder Asp, $Phe_{340}$→Tyr; $His_{353}$→Asp; $His_{353}$→Lys ein $\beta_A/\beta_B$ Hybrid) ; $Phe_{356}$→Tyr; $Val_{364}$→Phe; $Val_{364}$→Leu; $Tyr_{375}$→Thr; $Tyr_{376}$→Trp; $Asn_{389}$→Gln, His oder Lys; $Ile_{391}$→Leu oder Thr; $Met_{390}$ →Leu oder Ser; $Val_{392}$→Phe, Glu, Thr oder Ile;
oder einer vergleichbar modifizierten menschlichen $\beta_B$-Kette.

**20.** Zusammensetzung, umfassend eine Prodomäne von menschlichem oder Schweine-α- oder -$\beta_B$-Inhibin, wie in Fig. 1B (Schweine-α-Kette), Fig. 2B (Schweine-$\beta_B$-Kette), Fig. 6A (menschliche α-Kette) und Fig. 9 (menschliche $\beta_B$-Kette) dargestellt, die nicht mit nativer Glykosylierung assoziiert ist.

**21.** Zusammensetzung, umfassend ein Homodimer von reifen menschlichen oder Schweine-Inhibin-$\beta_A$- oder -$\beta_B$-Ketten, wobei die Ketten wie in Fig. 2B (Schweine-β-Ketten) und Fig. 8 und 9 (menschliche β-Ketten) dargestellt sind, oder eine durch Einfügung, Löschung oder Substitution eines Polypeptids mit einer der dargestellten Sequenzen erhaltene Aminosäuresequenz-Variante ist, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt; wobei das Dimer frei von der Inhibin-α-Kette ist.

**22.** Zusammensetzung nach Anspruch 21, die ein Homodimer von reifen menschlichen oder Schweine-$\beta_B$-Ketten oder einer der Aminosäuresequenz-Varianten davon ist.

**23.** Zusammensetzung, umfassend ein Heterodimer von reifem menschlichem oder Schweine-Inhibin-$\beta_A$ mit reifem menschlichem oder Schweine-Inhibin-$\beta_B$, wobei die Ketten wie in Fig. 2B (Schweine-β-Ketten) und Fig. 8 und 9 (menschliche β-Ketten) dargestellt sind, oder eine durch Einfügung, Löschung oder Substitution eines Polypeptids mit einer der dargestellten Sequenzen erhaltene Aminosäuresequenz-Variante ist, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt; wobei die Zusammensetzung frei von der Inhibin-α-Kette ist.

**24.** Nicht-chromosomale DNA, die für eine menschliche oder Schweine-Inhibin-α-oder eine menschliche oder Schweine-Inhibin-β-Kette, wie in Fig. 1B (Schweine-α-Kette), Fig. 2B (Schweine-β-Ketten), Fig. 6A (menschliche α-Kette) und Fig. 8 und 9 (menschliche β-Ketten) dargestellt, oder für eine durch Einfügung, Löschung oder Substitution eines Polypeptids mit einer der dargestellten Sequenzen erhaltene Aminosäuresequenz-Variante kodiert, wobei die Variante im wesentlichen homolog zu einem Polypeptid mit einer dargestellten Sequenz ist, jedoch unter Ausschluss der Rinder-Inhibin-α-Kette, und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt.

**25.** DNA nach Anspruch 24, die für eine menschliche oder Schweine-Inhibin-α-oder eine menschliche oder Schweine-Inhibin-β-Kette kodiert, deren Aminosäuresequenz wie in Fig. 1B (Schweine-α-Kette), Fig. 2B (Schweine-β-Ketten), Fig. 6A (menschliche α-Kette) und Fig. 8 und 9 (menschliche β-Ketten) dargestellt ist.

**26.** DNA nach Anspruch 24, die für eine Variante kodiert, ausgewählt aus: den Schweine-Inhibinderivaten ($Asn_{266}$→Gln]Inhα; [$Cys_{325}$ oder $Cys_{324}$→Δ]Inhα; [$Cys_{361}$ oder $Cys_{363}$→Δ]Inhα; [$Lys_{321}$ oder $Lys_{322}$→Δ]Inh$\beta_A$; [$Lys_{322}$→His oder Ser]Inh$\beta_A$; [$Lys_{315}$→Arg; $Val_{316}$→Thr] Inh$\beta_A$; [$Cys_{388}$ oder $Cys_{390}$→Δ]Inh$\beta_A$, [$Lys_{411}$→Gln]Inh$\beta_A$; [$Arg_{315}$→Lys; $Val_{316}$→Thr]Inh$\beta_B$; [$Cys_{319}$ oder $Cys_{320}$→Δ]Inh$\beta_{B;}$ [$Pro_{381}$ $Gly_{382}$→ Pro Phe Gly]Inh$\beta_B$; [$Arg_{395}$→Gln] Inh$\beta_B$,

worin Inh eine Abkürzung für Inhibin ist und die Nummern der Reste für Inh$\beta_B$ die für den entsprechenden Inh$\beta_A$-Rest verwendeten sind (siehe Fig. 2B); den menschlichen Inhibin-$\beta_A$-Ketten-Varianten, die Variationen aufweisen, die Substitution oder Löschung eines Restes oder Einfügung neben einem Rest, ausgewählt aus den Resten 293 - 297, 364 - 376 und 387 - 398, der in Fig. 8 dargestellten Sequenz sind; und den Varianten menschlicher $\alpha$-Ketten, die zu mehr als 90% homolog zu der in Fig. 6A dargestellten Sequenz sind.

**27.** DNA nach Anspruch 24, die für eine Variante einer menschlichen $\beta$-Inhibin-Phe$_{302}\rightarrow$Ile oder Leu; Kette, ausgewählt aus Gln$_{297}\rightarrow$Asp oder Lys; Trp$_{307}\rightarrow$Tyr oder Phe; Trp$_{310}\rightarrow$Tyr oder Phe; Ile$_{311}\rightarrow$Phe oder Val; Tyr$_{317}\rightarrow$Trp oder Thr; His$_{318}\rightarrow$Lys; Ala$_{319}\rightarrow$Ser; Asn$_{320}\rightarrow$Gln, Tyr oder His; Tyr$_{321}\rightarrow$Thr oder Asp, Phe$_{340}\rightarrow$Tyr; His$_{353}\rightarrow$Asp; His$_{353}\rightarrow$Lys (ein $\beta_A$/$B_B$ Hybrid); Phe$_{356}\rightarrow$Tyr; Val$_{364}\rightarrow$Phe; Val$_{364}\rightarrow$Leu; Tyr$_{375}\rightarrow$Thr; Tyr$_{376}\rightarrow$Trp; Asn$_{389}\rightarrow$Gln, His oder Lys; Ile$_{391}\rightarrow$Leu oder Thr; Met$_{390}$ $\rightarrow$Leu oder Ser; Val$_{392}\rightarrow$Phe, Glu, Thr oder Ile;
oder einer vergleichbar modifizierten menschlichen $\beta_B$-Kette kodiert.

**28.** DNA nach einem der Ansprüche 24 bis 27, die frei von intervenierenden untranslatierten Sequenzen ist.

**29.** DNA nach einem der Ansprüche 24 bis 28, die mit einer detektierbaren Gruppe markiert ist.

**30.** Replizierbarer Vektor, der eine DNA nach einem der Ansprüche 21 bis 27 umfaßt.

**31.** Vektor nach Anspruch 30, der einen viralen Promotor umfaßt, der mit der für die Inhibin-$\alpha$- und/oder -$\beta$-Ketten kodierenden DNA operal verbunden ist.

**32.** Vektor nach Anspruch 30 oder 31, der DNA enthält, die sowohl für eine Inhibin-$\alpha$- als auch eine Inhibin-$\beta$-Kette kodiert.

**33.** Vektor nach Anspruch 30 oder 31, der DNA enthält, die für eine Inhibin-$\beta$-Kette, nicht jedoch für die Inhibin-$\alpha$-Kette kodiert.

**34.** Wirtszelle, die mit einem replizierbaren Vektor transformiert wurde, der DNA enthält, die für eine menschliche oder Schweine-Inhibin-$\alpha$-Kette und/oder eine -Inhibin-$\beta$-Kette, deren Aminosäuresequenzen wie in Fig. 1B (Schweine-$\alpha$-Kette), Fig. 2B (Schweine-$\beta$-Ketten), Fig. 6A (menschliche $\alpha$-Kette) und Fig. 8 und 9 (menschliche $\beta$-Ketten) dargestellt sind, oder für eine durch Einfügung, Löschung oder Substitution eines Polypeptids mit einer der dargestellten Sequenzen erhaltene Aminosäuresequenz-Variante kodiert, wobei die Variante im wesentlichen homolog zu einem Polypeptid mit einer dargestellten Sequenz ist, jedoch unter Ausschluß der Rinder-Inhibin-$\alpha$-Kette, und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt.

**35.** Wirtszelle nach Anspruch 34, worin die DNA für eine menschliche oder Schweine-Inhibin-$\alpha$- und/oder eine -Inhibin-$\beta$-Kette kodiert, deren Aminosäuresequenzen wie in Fig. 1B (Schweine-$\alpha$-Kette), Fig. 2B (Schweine-$\beta$-Ketten), Fig. 6A (menschliche $\alpha$-Kette) und Fig. 8 und 9 (menschliche $\beta$-Ketten) dargestellt sind.

**36.** Wirtszelle nach Anspruch 34, worin die DNA für eine Variante kodiert, ausgewählt aus: den Schweine-Inhibinderivaten

(Asn$_{266}\rightarrow$Gln)Inh$\alpha$; [Cys$_{325}$ oder Cys$_{324}\rightarrow\Delta$]Inh$\alpha$; [Cys$_{361}$ oder Cys$_{363}\rightarrow\Delta$]Inh$\alpha$; [Lys$_{321}$ oder Lys$_{322}\rightarrow\Delta$]Inh$\beta_A$; [Lys$_{322}\rightarrow$His oder Ser]Inh$\beta_A$; [Lys$_{315}\rightarrow$Arg; Val$_{316}\rightarrow$Thr] Inh$\beta_A$; [Cys$_{388}$ oder Cys$_{390}\rightarrow\Delta$]Inh$\beta_A$, [Lys $_{411}\rightarrow$Gln] Inh$\beta_A$; [Arg$_{315}\rightarrow$Lys; Val$_{316}\rightarrow$Thr]Inh$\beta_B$; [Cys$_{319}$ oder Cys$_{320}\rightarrow\Delta$]Inh$\beta_B$; [Pro$_{381}$ Gly$_{382}\rightarrow$ Pro Phe Gly]Inh$\beta_B$; [Arg$_{395}\rightarrow$Gln]Inh$\beta_B$,
worin Inh eine Abkürzung für Inhibin ist und die Nummern der Reste für Inh$\beta_B$ die für den entsprechenden Inh$\beta_A$-Rest verwendeten sind (siehe Fig. 2B); den menschlichen Inhibin-ß$_A$-Ketten-Varianten, die Variationen aufweisen, die Substitution oder Löschung eines Restes oder Einfügung neben einem Rest, ausgewählt aus den Resten 293 - 297, 364 - 376 und 387 - 398, der in Fig. 8 dargestellten Sequenz sind; und den Varianten menschlicher $\alpha$-Ketten, die zu mehr als 90% homolog zu der in Fig. 6A dargestellten Sequenz sind.

**37.** Wirtszelle nach Anspruch 34, worin die DNA für eine Variante einer menschlichen $\beta_A$-Inhibin-Kette, ausgewählt aus:
Phe$_{302}\rightarrow$Ile oder Leu; Gln$_{297}\rightarrow$Asp oder Lys; Trp$_{307}\rightarrow$Tyr oder Phe; Trp$_{310}\rightarrow$Tyr oder Phe; Ile$_{311}\rightarrow$Phe oder Val; Tyr$_{317}\rightarrow$Trp oder Thr; His$_{318}\rightarrow$Lys; Ala$_{319}\rightarrow$Ser; Asn$_{320}\rightarrow$Gln, Tyr oder His;
Tyr$_{321}\rightarrow$Thr oder Asp, Phe$_{340}\rightarrow$Tyr; His$_{353}\rightarrow$Asp; His$_{353}\rightarrow$Lys (ein $\beta_A$/$\beta_B$ Hybrid); Phe$_{356}\rightarrow$Tyr; Val$_{364}\rightarrow$Phe;

$Val_{364}$→Leu; $Tyr_{375}$→Thr; $Tyr_{376}$→Trp; $Asn_{389}$→Gln, His oder Lys; $Ile_{391}$→Leu oder Thr; $Met_{390}$ →Leu oder Ser; $Val_{392}$→Phe, Glu, Thr oder Ile;
oder eine vergleichbar modifizierte menschliche $\beta_B$-Kette kodiert.

**38.** Zelle nach irgendeinem der Ansprüche 34 - 37, die eine eukaryotische Zelle ist.

**39.** Zellfreie Zusammensetzung, die frei von reifem α-Ketten-Polypeptid ist, die eine menschliche oder Schweine-Inhibin-a-Ketten-Prodomänen-Polypeptidsequenz, wie in Fig. 1B (Schweine-α-Kette) oder Fig. 6A (menschliche α-Kette) dargestellt, oder eine durch Einfügung, Löschung oder Substitution einer dieser Prodomänen-Sequenzen erhaltene Aminosäuresequenz-Variante enthält, wobei die Variante im wesentlichen homolog zu einem Polypeptid mit einer der dargestellten Sequenzen und kreuzreaktiv mit gegen ein Polypeptid mit einer der dargestellten Sequenzen gebildeten Antikörpern ist.

**40.** Zellfreie Zusammensetzung, umfassend

a) ein Polypeptid, umfassend die menschliche Inhibin-$\beta_A$-Ketten-Prodomänensequenz

HSAAPDCPSCALAALPKDVPNSQPEMVEAVKKHILNMLHL
(Aminosäuren 1 - 40 aus Fig. 8)

PDVTQPVPKAALLNAIRKLHVGKVGENGYVEIEDDIG
(Aminosäuren 44 - 80 aus Fig. 8)

AEMNELMEQTSEIITFAESGTARKTLHFEISKEGSDLSVVERAEVWLFLKVPKAN-
RTRTKVTIRLFQQQKHPQGSLDTGEEAEEVGLKGERSELLLSEKVVDA
(Aminosäuren 83 - 185 aus Fig. 8) STWHVFPVSSSIQRLLD-

QGKSSLDVRIACEQCQESGASLVLLG
(Aminosäuren 188 - 230 aus Fig. 8)

oder natürlich vorkommende Säugetier-Aminosäuresequenz-Varianten davon;
b) ein Polypeptid, umfassend die menschliche Inhibin-$\beta_B$-Ketten-Prodomänensequenz

CTSCGGFRRPEELGRVDGDFLEAV
(Aminosäuren 7 - 30 aus Fig. 9)

HILSRLQMRGRPNITHAVPKAAMVTALRKLHAGKVREDGRVEIPHLDGHASPGAD-
GQERVSEIISFAETDGLASSRVRLYFFISNEGNQNLFVVQASLWLYLKLLPYVLEKGS
(Aminosäuren 33 - 145 aus Fig. 9)

VRVKVYFQEQGHGDRWNMVEKRVDLKRSGWHTFPLTEAIQALFERGE
(Aminosäuren 149 - 195 aus Fig. 9)

LNLDVQCDSCQELAVVPVFVDPGEESHRPFVVVQARLGDSRHRI

(Aminosäuren 198 - 241 aus Fig. 9)

oder natürlich vorkommende Säugetier-Aminosäuresequenz-Varianten davon; oder
c) ein Polypeptid, das frei von der reifen α-Ketten-Aminosäuresequenz ist, umfassend menschlichen Inhibin-α-Ketten-Prodomänensequenzen

KVRALFLDALGPPAVTREGGDPGV

(Aminosäuren 1 - 24 aus Fig. 6)

HALGGFTHRGSEPEEEEDVSQAILFPATDASCEDKSAARGLAQEAEEGLFRYMFR-

PSQHTRSRQVTSAQLWFHTGLDRQGTAASNSSEPLLGLLALSPGGPVAVPMSLGH-

APPHWAVLHLATSALSLLTHPVLVLLLRCPLCTCSARPEATPFLVAHTRTRPPSGGERA

(Aminosäuren 32 - 199 aus Fig. 6)

oder natürlich vorkommende Säugetier-Aminosäuresequenz-Varianten davon.

**41.** Zellfreie Zusammensetzung umfassend ein menschliches oder Schweine-Inhibin-β-Ketten-Prodomänen-Polypeptid mit einer Sequenz, wie in Fig. 2B (Schweine-β-Ketten) und Fig. 8 und 9 (menschliche β-Ketten) dargestellt, oder eine durch Einfügung, Löschung oder Substitution einer dieser Prodomänen-Polypeptide mit einer dieser Sequenzen erhaltene Aminosäuresequenz-Variante ist, wobei die Variante im wesentlichen homolog zu einem Polypeptid mit einer der dargestellten Sequenzen ist und kreuzreaktiv mit gegen ein Polypeptid mit einer dargestellten Sequenz gebildeten Antikörpern ist.

**42.** Zusammensetzung nach Anspruch 41, worin die β-Kette die $\beta_A$-Kette ist und die Zusammensetzung frei von reifer $\beta_A$-Ketten-Sequenz ist.

**43.** Zusammensetzung nach Anspruch 41, worin die β-Kette die $\beta_B$-Kette ist und die Zusammensetzung frei von reifer $\beta_B$-Ketten-Sequenz ist.

**44.** Zusammensetzung nach Anspruch 40, worin die Variante die entsprechende Schweine-Aminosäuresequenz ist, wobei die Zusammensetzung enthält:

a) ein Polypeptid, umfassend die Schweine-Inhibin-$ß_A$-Ketten-Prodomänensequenz, die beschrieben wird durch die Aminosäuren: 28-58; 61-87; 90-108; 111-179; 182-213; 216-258; oder 28-87 aus Fig. 2B; oder
b) ein Polypeptid, das frei von reifen α-Ketten-Aminosäuresequenz ist, umfassend die Schweine-Inhibin-α-Ketten-Prodomänen-Polypeptidsequenzen, die dargestellt werden durch die Aminosäuren: 20-54 oder 70-228 aus Fig. 1B.

**45.** Zusammensetzung nach einem der Ansprüche 39 bis 44, worin das Polypeptid nicht von nativer Glykosylierung begleitet wird.

**46.** Zusammensetzung nach einem der Ansprüche 39 bis 45, die steril ist und worin das Polypeptid weiters ein immunogenes Polypeptid umfaßt.

**47.** Verwendung einer Zusammensetzung nach einem der Ansprüche 39 bis 46 zur Herstellung eines Antikörpers, der in der Lage ist, das Polypeptid zu binden.

**48.** Zusammensetzung nach einem der Ansprüche 39 bis 46, worin das Polypeptid an eine detektierbare Gruppe konjugiert ist.

**49.** Zusammensetzung nach Anspruch 48, worin die Gruppe ein Enzym, Fluorophor oder Radioisotop ist.

**50.** Zusammensetzung nach einem der Ansprüche 39 bis 44, 48 oder 49, die durch nichtkovalente Absorption oder kovalente Vernetzung an einen wasserunlöslichen Träger unlöslich gemacht ist.

**51.** Zusammensetzung nach einem der Ansprüche 39 bis 46, 48 oder 49, weiters umfassend eine physiologisch annehmbare, implantierbare Matrix zur gesteuerten Freisetzung des Polpypeptids in die Gewebe eines Tieres.

**Patentansprüche für folgenden Vertragsstaat : GR**

**1.** Verfahren, umfassend das Kultivieren einer Wirtszelle, die mit einem Vektor transformiert wurde, der Nukleinsäure enthält, die für eine menschliche oder Schweine-Inhibin-α-Kette und/oder eine menschliche oder Schweine-Inhibin-β-Kette, deren Aminosäuresequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind, oder eine durch Einfügung, Löschung oder Substitution einer der dargestellten Sequenzen erhaltene Aminosäuresequenz-Variante kodiert, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt.

**2.** Verfahren nach Anspruch 1, worin die Nukleinsäure für eine menschliche oder Schweine-Inhibin-α-Kette und/oder eine menschliche oder Schweine-Inhibin-β-Kette kodiert, deren Aminosäuresequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind.

**3.** Verfahren nach Anspruch 1, worin der Vektor für eine allelische Variante von menschlicher oder Schweine-Inhibin-α- und/oder -β-Kette kodiert.

**4.** Verfahren nach einem der vorangegangenen Ansprüche, worin die Nukleinsäure, die für die Inhibin-α-Kette und/oder -β-Kette kodiert, mit einem von der Wirtszelle erkannten Promotor operabel verbunden ist, und das den weiteren Schritt der Gewinnung der Inhibin-α-Kette, Inhibin-β-Kette, von Inhibin oder einem β-Ketten-Dimer aus dem Kulturmedium umfaßt.

**5.** Verfahren nach einem der vorangegangenen Ansprüche, worin die Zelle ein Prokaryot ist.

**6.** Verfahren nach einem der vorangegangenen Ansprüche, worin der Vektor Nukleinsäure umfaßt, die für die Präpro-Form einer Inhibin-α-Kette oder einer Inhibin-β-Kette kodiert.

**7.** Verfahren nach Anspruch 6, worin der Vektor eine Nukleinsäure umfaßt, die sowohl für eine Präpro-Form einer Inhibin-α-Kette als auch einer Inhibin-β-Kette kodiert.

**8.** Verfahren nach einem der Ansprüche 1 bis 4, 6 oder 7, worin die Zelle eine Zelle aus einem vielzelligen Organismus ist und hormonell aktives Inhibin produziert wird.

**9.** Verfahren nach Anspruch 4, worin der Promotor ein viraler Promotor ist.

**10.** Verfahren nach Anspruch 9, worin der Promotor ein SV40-Promotor ist.

**11.** Verfahren nach einem der vorangegangenen Ansprüche, worin reifes Schweine- oder menschliches Inhibin gewonnen wird.

**12.** Verfahren nach einem der Ansprüche 1 bis 5 oder 8 bis 10, worin der Vektor Nukleinsäure umfaßt, die für die Präpro-Form einer Inhibin-β-Kette kodiert, und ein reifes β-Ketten-Dimer frei von der α-Kette gewonnen wird.

**13.** Verfahren nach Anspruch 8 oder 11, worin die β-Kette die $\beta_A$-Kette ist und das Inhibin in einer Konzentration von über etwa 20 ng/ml im Kulturmedium vorliegt.

**14.** Verfahren nach Anspruch 4, worin das Inhibin menschliches oder Schweine-Inhibin ist, das aus einer α- und einer β-Kette besteht, wobei die Aminosäuresequenzen der α- und β-Kette aus jenen in Fig. 1B (Schweine-α-Kette), Fig. 2B (Schweine-β-Ketten), Fig. 6A (menschliche α-Kette) und Fig. 8 und 9 (menschliche β-Ketten) dargestellten ausgewählt sind, und durch Einfügung, Löschung oder Substitution eines Polypeptids mit einer der dargestellten Sequenzen erhaltenen Aminosäuresequenz-Varianten, wobei die Varianten im wesentlichen homolog zu einem Polypeptid mit einer dargestellten Sequenz sind und 1) kreuzreaktiv mit gegen ein Polypeptid mit einer dargestellten Sequenz gebildeten Antikörpern sind; oder 2) ähnliche hormonelle Aktivität wie ein Polypeptid mit einer dargestellten Sequenz besitzen; wobei die Zusammensetzung vollständig frei von nicht identifizierten menschlichen oder Schweine-Proteinen ist.

**15.** Verfahren nach Anspruch 14, worin das Inhibin menschliches oder Schweine-Inhibin ist, das aus einer α- und einer β-Kette besteht, wobei die Sequenzen der α-und β-Ketten aus jenen ausgewählt sind, die in Fig. 1B (Schweine-α-Kette), Fig. 2B (Schweine-β-Ketten), Fig. 6A (menschliche α-Kette) und Fig. 8 und 9 (menschliche β-Ketten) dargestellt sind.

**16.** Verfahren nach Anspruch 4, worin die gewonnene Inhibin-Kette eine Prodomäne von menschlichem oder Schweine-α- oder -$\beta_B$-Inhibin, wie in Fig. 1B (Schweine-α-Kette), Fig. 2B (Schweine-$\beta_B$-Kette), Fig. 6A (menschliche α-Kette) und Fig. 9 (menschliche $\beta_B$-Kette) dargestellt, ist, die nicht mit nativer Glykosylierung assoziiert ist.

**17.** Verfahren nach Anspruch 4, worin ein β-Ketten-Dimer gewonnen wird, das ein Homodimer von reifen menschlichen oder Schweine-Inhibin-$\beta_A$- oder -$\beta_B$-Ketten, wobei die Ketten wie in Fig. 2B (Schweine-β-Ketten) und Fig. 8 und 9 (menschliche β-Ketten) dargestellt sind, oder eine durch Einfügung, Löschung oder Substitution eines Polypeptids mit einer der dargestellten Sequenzen erhaltene Aminosäuresequenz-Variante ist, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt; wobei das Dimer frei von der Inhibin-α-Kette ist.

**18.** Verfahren nach Anspruch 17, worin ein Homodimer von reifen menschlichen Schweine-$\beta_B$-Ketten oder einer der Aminosäuresequenz-Varianten davon gewonnen wird.

**19.** Verfahren nach Anspruch 4, worin ein β-Ketten-Dimer gewonnen wird, das ein Heterodimer von reifem menschlichem oder Schweine-Inhibin-$\beta_A$ mit reifem menschlichem oder Schweine-Inhibin-$\beta_B$, wobei die Ketten wie in Fig. 2B (Schweine-β-Ketten) und Fig. 8 und 9 (menschliche β-Ketten) dargestellt sind, oder einer durch Einfügung, Löschung oder Substitution eines Polypeptids mit einer der dargestellten Sequenzen erhaltenen Aminosäuresequenz-Variante ist, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt; wobei das Heterodimer frei von der Inhibin-α-Kette ist.

**20.** Nicht-chromosomale DNA, die für eine menschliche oder Schweine-Inhibin-α-oder eine menschliche oder Schweine-Inhibin-β-Kette, wie in Fig. 1B (Schweine-α-Kette), Fig. 2B (Schweine-β-Ketten), Fig. 6A (menschliche α-Kette) und Fig. 8 und 9 (menschliche β-Ketten) dargestellt, oder für eine durch Einfügung, Löschung oder Substitution eines Polypeptids mit einer der dargestellten Sequenzen erhaltene Aminosäuresequenz-Variante kodiert, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt.

**21.** DNA nach Anspruch 20, die für eine menschliche oder Schweine-Inhibin-α-oder eine menschliche oder Schweine-Inhibin-β-Kette kodiert, deren Aminosäuresequenz wie in Fig. 1B (Schweine-α-Kette), Fig. 2B (Schweine-β-Ketten), Fig. 6A (menschliche α-Kette) und Fig. 8 und 9 (menschliche β-Ketten) dargestellt ist.

**22.** DNA nach Anspruch 20 oder 21, die frei von intervenierenden untranslatierten Sequenzen ist.

**23.** DNA nach einem der Ansprüche 20 bis 22, die mit einer detektierbaren Gruppe markiert ist.

**24.** Replizierbarer Vektor, der eine DNA nach Anspruch 20 oder 21 umfaßt.

**25.** Vektor nach Anspruch 24, der einen viralen Promotor aufweist, der mit der für die Inhibin-α- und/oder -β-Ketten kodierenden DNA operal verbunden ist.

26. Vektor nach Anspruch 24 oder 25, der DNA umfaßt, die sowohl für eine Inhibin-α- als auch eine Inhibin-β-Kette kodiert.

27. Vektor nach Anspruch 24 oder 25, der DNA umfaßt, die für eine Inhibin-β-Kette, nicht jedoch für die Inhibin-α-Kette kodiert.

28. Wirtszelle, die mit einem replizierbaren Vektor transformiert wurde, der DNA enthält, die für eine menschliche oder Schweine-Inhibin-α-Kette und/oder eine -Inhibin-β-Kette, deren Aminosäuresequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind, oder für eine durch Einfügung, Löschung oder Substitution eines Polypeptids mit einer der dargestellten Sequenzen erhaltene Aminosäuresequenz-Variante kodiert, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt.

29. Wirtszelle nach Anspruch 28, worin die DNA für eine menschliche oder Schweine-Inhibin-α-Kette und/oder eine -Inhibin-β-Kette kodiert, deren Aminosäuresequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind.

30. Zelle nach Anspruch 28 oder 29, die eine eukaryotische Zelle ist.

31. Verfahren nach Anspruch 4, worin die gewonnene Inhibin-Kette eine zellfreie Zusammensetzung ist, die frei von reifer α-Ketten-Sequenz ist, die eine menschliche oder Schweine-Inhibin-α-Ketten-Prodomänen-Polypeptidsequenz, wie in Fig. 1B (Schweine-α-Kette) oder Fig. 6A (menschliche α-Kette) dargestellt, oder eine Aminosäuresequenz-Variante einer dieser Prodomänen-Sequenzen enthält, wobei die Variante im wesentlichen homolog zu einem Polypeptid mit einer der dargestellten Sequenzen ist und kreuzreaktiv mit gegen ein Polypeptid mit einer dargestellten Sequenz gebildeten Antikörpern ist.

32. Verfahren nach Anspruch 4, worin die gewonnene Inhibin-Kette eine zellfreie Zusammensetzung ist, umfassend

a) ein Polypeptid, umfassend die menschliche Inhibin-$\beta_A$-Ketten-Prodomänensequenz

HSAAPDCPSCALAALPKDVPNSQPEMVEAVKKHILNMLHL

(Aminosäuren 1 - 40 aus Fig. 8)

PDVTQPVPKAALLNAIRKLHVGKVGENGYVEIEDDIG

(Aminosäuren 44 - 80 aus Fig. 8)

AEMNELMEQTSEIITFAESGTARKTLHFEISKEGSDLSVVERAEVWLFLKVPKAN-

RTRTKVTIRLFQQQKHPQGSLDTGEEAEEVGLKGERSELLLSEKVVDA

(Aminosäuren 83 - 185 aus Fig. 8)

STWHVFPVSSSIQRLLDQGKSSLDVRIACEQCQESGASLVLLG

(Aminosäuren 188 - 230 aus Fig. 8)

oder natürlich vorkommende Säugetier-Aminosäuresequenz-Varianten davon;
b) ein Polypeptid, umfassend die menschliche Inhibin-$\beta_B$-Ketten-Prodomänensequenz

CTSCGGFRRPEELGRVDGDFLEAV

(Aminosäuren 7 - 30 aus Fig. 9)

HILSRLQMRGRPNITHAVPKAAMVTALRKLHAGKVREDGRVEIPHLDGHASPGAD-

GQERVSEIISFAETDGLASSRVRLYFFISNEGNQNLFVVQASLWLYLKLLPYV-

LEKGS (Aminosäuren 33 - 145 aus Fig. 9)

VRVKVYFQEQGHDRWNMVEKRVDLKRSGWHTFPLTEAIQALFERGE

(Aminosäuren 149 - 195 aus Fig. 9)

LNLDVQCDSCQELAVVPVFVDPGEESHRPFVVVQARLGDSRHRI

(Aminosäuren 198 - 241 aus Fig. 9)

oder natürlich vorkommende Säugetier-Aminosäuresequenz-Varianten davon; oder
c) ein Polypeptid, das frei von der reifen α-Ketten-Aminosäuresequenz ist, umfassend menschlichen Inhibin-α-Ketten-Prodomänensequenzen

KVRALFLDALGPPAVTREGGPGV

(Aminosäuren 1 - 24 aus Fig. 6)

HALGGFTHRGSEPEEEEDVSQAILFPATDASCEDKSAARGLAQEAEEGLFRYMFR-

PSQHTRSRQVTSAQLWFHTGLDRQGTAASNSSEPLLGLLALSPGGPVAVPMSLGH-

APPHWAVLHLATSALSLLTHPVLVLLLRCPLCTCSARPEATPFLVAHTRTRPPSG- GERA

(Aminosäuren 32 - 199 aus Fig. 6)

oder natürlich vorkommende Säugetier-Aminosäuresequenz-Varianten davon.

**33.** Verfahren nach Anspruch 4, worin die gewonnene Inhibin-Kette eine zellfreie Zusammensetzung ist, umfassend ein menschliches oder Schweine-Inhibin-β-Ketten-Prodomänen-Polypeptid mit einer Sequenz, wie in Fig. 2B (Schweine-β-Ketten) und Fig. 8 und 9 (menschliche β-Ketten) dargestellt, oder eine durch Einfügung, Löschung oder Substitution einer dieser Prodomänen-Polypeptide mit einer dieser Sequenzen erhaltene Aminosäuresequenz-Variante ist, wobei die Variante im wesentlichen homolog zu einem Polypeptid mit einer der dargestellten Sequenzen ist und kreuzreaktiv mit gegen ein Polypeptid mit einer dargestellten Sequenz gebildeten Antikörpern ist.

**34.** Verfahren nach Anspruch 33, worin die β-Kette die $\beta_A$-Kette ist und die Zusammensetzung frei von reifer $\beta_A$-Ketten-Sequenz ist.

**35.** Verfahren nach Anspruch 33, worin die β-Kette die $\beta_B$-Kette ist und die Zusammensetzung frei von reifer $\beta_B$-Ketten-Sequenz ist.

**36.** Verfahren nach Anspruch 32, worin die Variante die entsprechende Schweine-Aminosäuresequenz ist, wobei die Zusammensetzung enthält:

a) ein Polypeptid, umfassend die Schweine-Inhibin-$\beta_A$-Ketten-Prodomänensequenz, die dargestellt wird durch die Aminosäuren: 28-58; 61-87; 90-108; 111-179; 182-213; 216-258; oder 28-87 aus Fig. 2B; oder
b) ein Polypeptid, das frei von der reifen α-Ketten-Aminosäuresequenz ist, umfassend die Schweine-Inhibin-α-Ketten-Prodomänen-Polypeptidsequenzen, die dargestellt werden durch die Aminosäuren: 20-54 oder 70-228 aus Fig. 1B.

**37.** Verfahren nach einem der Ansprüche 31 bis 36, worin das Polypeptid nicht von nativer Glykosylierung begleitet wird.

**38.** Verfahren nach einem der Ansprüche 31 bis 37, worin die Zusammensetzung steril ist, und weiters umfassend das Verbinden des Polypeptids mit einem immunogenen Polypeptid.

**39.** Verwendung eines Polypeptids nach einem der Ansprüche 31 bis 38 zur Herstellung eines Antikörpers, der in der Lage ist, das Polypeptid zu binden.

**40.** Verfahren nach einem der Ansprüche 31 bis 38, welters umfassend das Konjugieren des Polypeptids an eine detektierbare Gruppe.

**41.** Verfahren nach Anspruch 40, worin die Gruppe ein Enzym, Fluorophor oder Radioisotop ist.

**42.** Verfahren nach einem der Ansprüche 31 bis 38, 40 oder 41, worin die Zusammensetzung durch nichtkovalente Absorption oder kovalente Vernetzung an einen wasserunlöslichen Träger unlöslich gemacht ist.

**43.** Verfahren nach einem der Ansprüche 31 bis 38, 40 oder 41, weiters umfassend das Mischen der Zusammensetzung mit einer physiologisch annehmbaren, implantierbaren Matrix zur gesteuerten Freisetzung des Polpypeptids in die Gewebe eines Tieres.

**Patentansprüche für folgende Vertragsstaaten : ES, AT**

**1.** Verfahren, umfassend das Kultivieren einer Wirtszelle, die mit einem Vektor transformiert wurde, der Nukleinsäure enthält, die für eine menschliche oder Schweine-Inhibin-α-Kette und/oder eine menschliche oder Schweine-Inhibin-β-Kette, deren Aminosäuresequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind, oder eine durch Einfügung, Löschung oder Substitution einer der dargestellten Sequenzen erhaltene Aminosäuresequenz-Variante kodiert, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt.

**2.** Verfahren nach Anspruch 1, worin die Nukleinsäure für eine menschliche oder Schweine-Inhibin-α-Kette und/oder eine menschliche oder Schweine-inhibin-β-Kette kodiert, deren Aminosäuresequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind.

**3.** Verfahren nach Anspruch 1, worin der Vektor für eine allelische Variante von menschlicher oder Schweine-Inhibin-α- und/oder -β-Kette kodiert.

**4.** Verfahren nach einem der vorangegangenen Ansprüche, worin die Nukleinsäure, die für die Inhibin-α-Kette und/oder -β-Kette kodiert, mit einem von der Wirtszelle erkannten Promotor operabel verbunden ist, und das den weiteren Schritt der Gewinnung der Inhibin-α-Kette, Inhibin-β-Kette, von Inhibin oder einem β-Ketten-Dimer aus dem Kulturmedium umfaßt.

**5.** Verfahren nach einem der vorangegangenen Ansprüche, worin die Zelle ein Prokaryot ist.

**6.** Verfahren nach einem der vorangegangenen Ansprüche, worin der Vektor Nukleinsäure umfaßt, die für die Präpro-Form einer Inhibin-α-Kette oder einer Inhibin-β-Kette kodiert.

**7.** Verfahren nach Anspruch 6, worin der Vektor eine Nukleinsäure umfaßt, die sowohl für eine Präpro-Form einer Inhibin-α-Kette als auch einer Inhibin-β-Kette kodiert.

**8.** Verfahren nach einem der Ansprüche 1 bis 4, 6 oder 7, worin die Zelle eine Zelle aus einem vielzelligen Organismus ist und hormonell aktives Inhibin produziert wird.

**9.** Verfahren nach Anspruch 4, worin der Promotor ein viraler Promotor ist.

**10.** Verfahren nach Anspruch 9, worin der Promotor ein SV40-Promotor ist.

**11.** Verfahren nach einem der vorangegangenen Ansprüche, worin reifes Schweine- oder menschliches Inhibin gewonnen wird.

**12.** Verfahren nach einem der Ansprüche 1 bis 5 oder 8 bis 10, worin der Vektor Nukleinsäure umfaßt, die für die Präpro-Form einer Inhibin-β-Kette kodiert, und ein reifes β-Ketten-Dimer frei von der α-Kette gewonnen wird.

**13.** Verfahren nach Anspruch 8 oder 11, worin die β-Kette die $\beta_A$-Kette ist und das Inhibin in einer Konzentration von über etwa 20 ng/ml im Kulturmedium vorliegt.

**14.** Verfahren nach Anspruch 4, worin das Inhibin menschliches oder Schweine-Inhibin ist, das aus einer α- und einer β-Kette besteht, wobei die Aminosäuresequenzen der α- und β-Kette aus jenen in Fig. 1B (Schweine-α-Kette), Fig. 2B (Schweine-β-Ketten), Fig. 6A (menschliche α-Kette) und Fig. 8 und 9 (menschliche β-Ketten) dargestellten ausgewählt sind, und durch Einfügung, Löschung oder Substitution eines Polypeptids mit einer der dargestellten Sequenzen erhaltenen Aminosäuresequenz-Varianten, wobei die Varianten im wesentlichen homolog zu einem Polypeptid mit einer dargestellten Sequenz sind und 1) kreuzreaktiv mit gegen ein Polypeptid mit einer dargestellten Sequenz gebildeten Antikörpern sind; oder 2) ähnliche hormonelle Aktivität wie ein Polypeptid mit einer dargestellten Sequenz besitzen; wobei die Zusammensetzung vollständig frei von nicht identifizierten menschlichen oder Schweine-Proteinen ist.

**15.** Verfahren nach Anspruch 14, worin das Inhibin menschliches oder Schweine-Inhibin ist, das aus einer α- und einer β-Kette besteht, wobei die Sequenzen der α-und β-Ketten aus jenen ausgewählt sind, die in Fig. 1B (Schweine-α-Kette), Fig. 2B (Schweine-β-Ketten), Fig. 6A (menschliche α-Kette) und Fig. 8 und 9 (menschliche β-Ketten) dargestellt sind.

**16.** Verfahren nach Anspruch 4, worin die gewonnene Inhibin-Kette eine Prodomäne von menschlichem oder Schweine-α- oder -$\beta_B$-Inhibin, wie in Fig. 1B (Schweine-α-Kette), Fig. 2B (Schweine-$\beta_B$-Kette), Fig. 6A (menschliche α-Kette) und Fig. 9 (menschliche $\beta_B$-Kette) dargestellt, ist, die nicht mit nativer Glykosylierung assoziiert ist.

**17.** Verfahren nach Anspruch 4, worin ein β-Ketten-Dimer gewonnen wird, das ein Homodimer von reifen menschlichen oder Schweine-Inhibin-$\beta_A$- oder -$\beta_B$-Ketten, wobei die Ketten wie in Fig. 2B (Schweine-β-Ketten) und Fig. 8 und 9 (menschliche β-Ketten) dargestellt sind, oder einer durch Einfügung, Löschung oder Substitution eines Polypeptids mit einer der dargestellten Sequenzen erhaltenen Aminosäuresequenz-Variante ist, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt; wobei das Dimer frei von der Inhibin-α-Kette ist.

**18.** Verfahren nach Anspruch 17, worin ein Homodimer von reifen menschlichen Schweine-$\beta_B$-Ketten oder einer der Aminosäuresequenz-Varianten davon gewonnen wird.

**19.** Verfahren nach Anspruch 4, worin ein β-Ketten-Dimer gewonnen wird, das ein Heterodimer von reifem menschlichem oder Schweine-Inhibin-$\beta_A$ mit reifem menschlichem oder Schweine-Inhibin-$\beta_B$, wobei die Ketten wie in Fig. 2B (Schweine-β-Ketten) und Fig. 8 und 9 (menschliche β-Ketten) dargestellt sind, oder einer durch Einfügung, Löschung oder Substitution eines Polypeptids mit einer der dargestellten Sequenzen erhaltenen Aminosäuresequenz-Variante ist, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt; wobei das Heterodimer frei von der Inhibin-α-Kette ist.

**20.** Verfahren zur Herstellung von nicht-chromosomaler DNA, die für eine menschliche oder Schweine-Inhibin-α- oder eine menschliche oder Schweine-Inhibin-β-Kette, wie in Fig. 1B (Schweine-α-Kette), Fig. 2B (Schweine-β-Ketten), Fig. 6A (menschliche α-Kette) und Fig. 8 und 9 (menschliche β-Ketten) dargestellt, oder für eine durch Einfügung, Löschung oder Substitution eines Polypeptids mit einer der dargestellten Sequenzen erhaltene Aminosäuresequenz-Variante kodiert, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt; wobei das Verfahren chemische Synthese, Screenen von mRNA aus dem Eierstock oder Screenen der genomischen Sammlungen jeder Zelle umfaßt.

**21.** Verfahren nach Anspruch 20, worin DNA für eine menschliche oder Schweine-Inhibin-α- oder eine menschliche oder Schweine-Inhibin-β-Kette kodiert, deren Aminosäuresequenz wie in Fig. 1B (Schweine-α-Kette), Fig. 2B (Schweine-β-Ketten), Fig. 6A (menschliche α-Kette) und Fig. 8 und 9 (menschliche β-Ketten) dargestellt ist.

**22.** Verfahren nach Anspruch 20 oder 21, worin die nicht-chromosomale DNA frei von intervenierenden untranslatierten Sequenzen ist.

**23.** Verfahren nach einem der Ansprüche 20 bis 22, weiters umfassend den Schritt des Markierens der nicht-chromosomalen DNA mit einer detektierbaren Gruppe.

**24.** Verfahren zur Herstellung eines replizierbaren Vektors, wobei das Verfahren das Einfügen einer DNA nach Anspruch 20 oder 21 in einen Klonungsvektor umfaßt.

**25.** Verfahren nach Anspruch 24, worin die DNA in einen Vektor eingefügt wird, der einen viralen Promotor aufweist, der mit der für die Inhibin-α- und/oder -β-Ketten kodierenden DNA operal verbunden ist, um dadurch einen Expressionsvektor zu produzieren.

**26.** Verfahren nach Anspruch 24 oder 25, worin DNA, die sowohl für eine Inhibin-α- als auch eine Inhibin-β-Kette kodiert, in den Vektor eingefügt wird.

**27.** Verfahren nach Anspruch 24 oder 25, worin DNA, die für eine Inhibin-β-Kette, nicht jedoch für die Inhibin-α-Kette kodiert, in den Vektor eingefügt wird.

**28.** Wirtszelle, die mit einem replizierbaren Vektor transformiert wurde, der DNA enthält, die für eine menschliche oder Schweine-Inhibin-α-Kette und/oder eine -Inhibin-β-Kette, deren Aminosäuresequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind, oder für eine durch Einfügung, Löschung oder Substitution eines Polypeptids mit einer der dargestellten Sequenzen erhaltene Aminosäuresequenz-Variante kodiert, wobei die Variante im wesentlichen homolog zu einem Polypeptid mit einer dargestellten Sequenz ist und 1) kreuzreaktiv mit gegen ein Polypeptid mit einer dargestellten Sequenz gebildeten Antikörpern ist; oder 2) ähnliche hormonelle Aktivität wie ein Polypeptid mit einer dargestellten Sequenz besitzt.

**29.** Wirtszelle nach Anspruch 28, worin die DNA für eine menschliche oder Schweine-Inhibin-α-Kette und/oder eine -Inhibin-β-Kette kodiert, deren Aminosäuresequenzen wie in Fig. 1B (Schweine-α-Kette), Fig. 2B (Schweine-β-Ketten), Fig. 6A (menschliche α-Kette) und Fig. 8 und 9 (menschliche β-Ketten) dargestellt sind.

**30.** Zelle nach Anspruch 28 oder 29, die eine eukaryotische Zelle ist.

**31.** Verfahren nach Anspruch 4, worin die gewonnene Inhibin-Kette eine zellfreie Zusammensetzung ist, die frei von reifer α-Ketten-Sequenz ist, die eine menschliche oder Schweine-Inhibin-α-Ketten-Prodomänen-Polypeptidsequenz, wie in Fig. 1B (Schweine-α-Kette) oder Fig. 6A (menschliche α-Kette) dargestellt, oder eine Aminosäuresequenz-Variante einer dieser Prodomänen-Sequenzen enthält, wobei die Variante im wesentlichen homolog zu einem Polypeptid mit einer der dargestellten Sequenzen ist und kreuzreaktiv mit gegen ein Polypeptid mit einer dargestellten Sequenz gebildeten Antikörpern ist.

**32.** Verfahren nach Anspruch 4, worin die gewonnene Inhibin-Kette eine zellfreie Zusammensetzung ist, umfassend

a) ein Polypeptid, umfassend die menschliche Inhibin-$\beta_A$-Ketten-Prodomänensequenz

HSAAPDCPSCALAALPKDVPNSQPEMVEAVKKHILNMLHL

(Aminosäuren 1 - 40 aus Fig. 8)

PDVTQPVPKAALLNAIRKLHVGKVGENGYVEIEDDIG

(Aminosäuren 44 - 80 aus Fig. 8)

AEMNELMEQTSEIITFAESGTARKTLHFEISKEGSDLSVVERAEVWLFLKVPKAN-

RTRTKVTIRLFQQQKHPQGSLDTGEEAEEVGLKGERSELLLSEKVVDA

(Aminosäuren 83 - 185 aus Fig. 8)

STWHVFPVSSSIQRLLDQGKSSLDVRIACEQCQESGASLVLLG

(Aminosäuren 188 - 230 aus Fig. 8)

oder natürlich vorkommende Säugetier-Aminosäuresequenz-Varianten davon;

b) ein Polypeptid, umfassend die menschliche Inhibin-$\beta_B$-Ketten-Prodomänensequenz

CTSCGGFRRPEELGRVDGDFLEAV

(Aminosäuren 7 - 30 aus Fig. 9)

HILSRLQMRGRPNITHAVPKAAMVTALRKLHAGKVREDGRVEIPHLDGHASPGAD-

GQERVSEIISFAETDGLASSRVRLYFFISNEGNQNLFVVQASLWLYLKLLPYV-

LEKGS (Aminosäuren 33 - 145 aus Fig. 9)

VRVKVYFQEQGHDRWNMVEKRVDLKRSGWHTFPLTEAIQALFERGE

(Aminosäuren 149 - 195 aus Fig. 9)

LNLDVQCDSCQELAVVPVFVDPGEESHRPFVVVQARLGDSRHRI

(Aminosäuren 198 - 241 aus Fig. 9)

oder natürlich vorkommende Säugetier-Aminosäuresequenz-Varianten davon; oder

c) ein Polypeptid, das frei von der reifen α-Ketten-Aminosäuresequenz ist, umfassend menschlichen Inhibin-α-Ketten-Prodomänensequenzen

KVRALFLDALGPPAVTREGGPGV

(Aminosäuren 1 - 24 aus Fig. 6)

HALGGFTHRGSEPEEEEDVSQAILFPATDASCEDKSAARGLAQEAEEGLFRYMFR-

PSQHTRSRQVTSAQLWFHTGLDRQGTAASNSSEPLLGLLALSPGGPVAVPMSLGH-

APPHWAVLHLATSALSLLTHPVLVLLLRCPLCTCSARPEATPFLVAHTRTRPPSG- GERA

(Aminosäuren 32 - 199 aus Fig. 6)

oder natürlich vorkommende Säugetier-Aminosäuresequenz-Varianten davon.

**33.** Verfahren nach Anspruch 4, worin die gewonnene Inhibin-Kette eine zellfreie Zusammensetzung ist, umfassend ein menschliches oder Schweine-Inhibin-ß-Ketten-Prodomänen-Polypeptid mit einer Sequenz, wie in Fig. 2B (Schweine-β-Ketten) und Fig. 8 und 9 (menschliche β-Ketten) dargestellt, oder eine durch Einfügung, Löschung oder Substitution einer dieser Prodomänen-Polypeptide mit einer dieser Sequenzen erhaltene Aminosäuresequenz-Variante ist, wobei die Variante im wesentlichen homolog zu einem Polypeptid mit einer der dargestellten Sequenzen ist und kreuzreaktiv mit gegen ein Polypeptid mit einer dargestellten Sequenz gebildeten Antikörpern ist.

**34.** Verfahren nach Anspruch 33, worin die β-Kette die $\beta_A$-Kette ist und die Zusammensetzung frei von reifer $\beta_A$-Ketten-Sequenz ist.

**35.** Verfahren nach Anspruch 33, worin die β-Kette die $\beta_B$-Kette ist und die Zusammensetzung frei von reifer $\beta_B$-Ketten-Sequenz ist.

**36.** Verfahren nach Anspruch 32, worin die Variante die entsprechende Schweine-Aminosäuresequenz ist, wobei die Zusammensetzung enthält:

a) ein Polypeptid, umfassend die Schweine-Inhibin-$\beta_A$-Ketten-Prodomänensequenz, die beschrieben wird durch die Aminosäuren: 28-58; 61-87; 90-108; 111-179; 182-213; 216-258; oder 28-87 aus Fig. 2B; oder
b) ein Polypeptid, das frei von der reifen α-Ketten-Aminosäuresequenz ist, umfassend die Schweine-Inhibin-α-Ketten-Prodomänen-Polypeptidsequenzen, die dargestellt werden durch die Aminosäuren: 20-54 oder 70-228 aus Fig. 1B.

**37.** Verfahren nach einem der Ansprüche 31 bis 36, worin das Polypeptid nicht von nativer Glykosylierung begleitet wird.

**38.** Verfahren nach einem der Ansprüche 31 bsi 37, worin die Zusammensetzung steril ist, und weiters umfassend das Verbinden des Polypeptids mit einem immunogenen Polypeptid.

**39.** Verwendung eines Polypeptids nach einem der Ansprüche 31 bis 38 zur Herstellung eines Antikörpers, der in der Lage ist, das Polypeptid zu binden.

**40.** Verfahren nach einem der Ansprüche 31 bis 38, weiters umfassend das Konjugieren des Polypeptids an eine detektierbare Gruppe.

**41.** Verfahren nach Anspruch 40, worin die Gruppe ein Enzym, Fluorophor oder Radioisotop ist.

**42.** Verfahren nach einem der Ansprüche 31 bis 38, 40 oder 41, worin die Zusammensetzung durch nichtkovalente Absorption oder kovalente Vernetzung an einen wasserunlöslichen Träger unlöslich gemacht ist.

**43.** Verfahren nach einem der Ansprüche 31 bis 38, 40 oder 41, weiters umfassend das Mischen der Zusammensetzung mit einer physiologisch annehmbaren, implantierbaren Matrix zur gesteuerten Freisetzung des Polpypeptids in die Gewebe eines Tieres.

## Revendications

**Revendications pour les Etats contractants suivants : BE, CH, DE, FR, GB, IT, LI, NL, SE**

1. **1.** Procédé comprenant la culture d'une cellule hôte transformée avec un vecteur qui renferme un acide nucléique codant pour une chaîne α d'inhibine humaine ou porcine et/ou pour une chaîne β d'inhibine humaine ou porcine dont les séquences d'aminoacides sont représentées sur la figure 1B (chaîne α porcine), la figure 2B (chaînes β porcine), la figure 6A (chaîne α humaine) et les figures 8 et 9 (chaînes β humaines), ou un variant de séquence d'aminoacides formé par insertion, délétion ou substitution d'une des séquences représentées, le variant étant fortement homologue avec un polypeptide d'une séquence représentée mais excluant la chaîne α d'inhibine bovine et la chaîne β partielle d'inhibine bovine de séquence Gly-Leu-Glu-Cys-Asp-Gly-Lys-Val-Asn-Ile-Cys-Cys-Lys-Lys-Gln-Phe-Phe-Val-Ser-Phe-Lys-Asp-Ile-Gly-Trp-Asn-Asp-Trp-Ile-Ala-Pro-Ser-Gly-Tyr-His-Ala-Asn-Tyr-Cys-Glu-Gly-Glu-Cys-Pro-Ser-His-Ile-Ala-Gly-Thr-Ser-Gly-Ser-Ser-Leu-Ser-Phe-His-Ser-Thr-Val-Ile-Asn-His-Tyr-Arg-Met-Arg-Gly-His-Ser-, et

   1) présentant une réactivité croisée avec des anticorps engendrés contre un polypeptide d'une séquence représentée ; ou 2) présentant une activité hormonale similaire à celle d'un polypeptide d'une séquence représentée.

**2.** Procédé suivant la revendication 1, dans lequel l'acide nucléique code pour une chaîne α d'inhibine humaine ou porcine et/ou une chaîne β d'inhibine ou porcine dont les séquences d'amino-acides sont représentées sur la figure 1B (chaîne α porcine), la figure 2B (chaînes β porcines), la figure 6A (chaîne α humaine) et les figures 8 et 9 (chaînes β humaines).

**3.** Procédé suivant la revendication 1, dans lequel le vecteur code pour une chaîne β d'inhibine, ou un de ses variants de séquence d'amino-acides, autre que la chaîne $\beta_A$ humaine ou porcine naturelle ou la chaîne β d'inhibine bovine partielle spécifiée dans la revendication 1.

**4.** Procédé suivant la revendication 1, dans lequel l'acide nucléique code pour un variant choisi entre : des dérivés d'inhibine porcine [$Asn_{266}$→Gln]Inhα ; [$Cys_{325}$ ou $Cys_{324}$→Δ]Inhα ; [$Cys_{361}$ ou $Cys_{363}$→Δ]Inhα ; [$Lys_{321}$ ou $Lys_{322}$→Δ]Inh$\beta_A$ ; [$Lys_{322}$→His ou Ser]Inh$\beta_A$ ; [$Lys_{315}$→Arg ; $Val_{316}$→Thr] Inh$\beta_A$ ; [$Cys_{388}$ ou $Cys_{390}$→Δ]Inh$\beta_A$, [$Lys_{411}$→Gln]Inh$\beta_A$ ; [$Arg_{315}$→Lys ; $Val_{316}$→Thr]Inh$\beta_B$ ; [$Cys_{319}$ ou $Cys_{320}$→Δ]Inh$\beta_B$ ; [$Pro_{381}$ $Gly_{382}$→Pro Phe Gly] Inh$\beta_B$ ; [$Arg_{395}$→Gln]Inh$\beta_B$, dans lesquel Inh est une abréviation d'inhibine et les numéros des résidus pour la Inh$\beta_B$ sont ceux utilisés pour le résidu de Inh$\beta_A$ correspondant (voir figure 2B) ; les variants de chaîne $\beta_A$ d'inhibine humaine possédant des variations qui consistent en une substitution ou une délétion au niveau d'un, ou une insertion après un, résidu choisi entre les résidus 293 à 297, 364 à 376 et 387 à 398 de la séquence représentée sur la figure 8 ; et des variants de la chaîne α humaine qui présentent une homologie de plus de 90 % avec la séquence représentée sur la figure 6A.

**5.** Procédé suivant la revendication 1, dans lequel l'acide nucléique code pour un variant d'une chaîne d'inhibine $\beta_A$ humaine choisi entre $Phe_{302}$→Ile ou Leu ; $Gln_{297}$→Asp ou Lys ; $Trp_{307}$→Tyr ou Phe ; $Trp_{310}$→Tyr ou Phe ; $Ile_{311}$→Phe ou Val ; $Tyr_{317}$→Trp ou Thr ; $His_{318}$→Lys ; $Ala_{319}$→Ser ; $Asn_{320}$→Gln, Tyr ou His ; $Tyr_{321}$→Thr ou Asp, $Phe_{340}$→Tyr ; $His_{353}$→Asp ; $His_{353}$→Lys (à un hybride $\beta_A/\beta_B$) ; $Phe_{356}$→Tyr ; $Val_{364}$→Phe ; $Val_{364}$→Leu ; $Tyr_{375}$→Thr ; $Tyr_{376}$→Trp ; $Asn_{389}$→Gln, His ou Lys ; $Ile_{391}$→Leu ou Thr ; $Met_{390}$→Leu ou Ser ; $Val_{392}$→Phe, Glu, Thr ou Ile ; ou une chaîne $\beta_B$ humaine modifiée de manière comparable.

**6.** Procédé suivant l'une quelconque des revendications précédentes, dans lequel l'acide nucléique codant pour la chaîne α et/ou la chaîne β d'inhibine est lié de manière fonctionnelle à un promoteur reconnu par la cellule hôte et contenant l'étape supplémentaire de séparation de l'inhibine ou d'un dimère de chaîne β du milieu de culture.

**7.** Procédé suivant l'une quelconque des revendications précédentes, dans lequel la cellule est un procaryote.

**8.** Procédé suivant l'une quelconque des revendications précédentes, dans lequel le vecteur comprend un acide nucléique codant pour la forme pré-pro d'une chaîne α d'inhibine ou d'une chaîne β d'inhibine.

**9.** Procédé suivant la revendication 8, dans lequel le vecteur comprend un acide nucléique codant pour la forme pré-pro de la chaîne α d'inhibine et de la chaîne β d'inhibine.

**10.** Procédé suivant l'une quelconque des revendications 1 à 6, 8 et 9, dans lequel la cellule est une cellule provenant d'un organisme pluricellulaire et une inhibine douée d'activité hormonale est produite.

**11.** Procédé suivant la revendication 6, dans lequel le promoteur est un promoteur viral.

**12.** Procédé suivant la revendicatioon 11, dans lequel le promoteur est un promoteur de SV40.

**13.** Procédé suivant l'une quelconque des revendications précédentes, dans lequel de l'inhibine porcine ou humaine mature est recueillie.

**14.** Procédé suivant l'une quelconque des revendications 1 à 7 ou 10 à 12, dans lequel le vecteur comprend un acide nucléique codant pour la forme pré-pro d'une chaîne β d'inhibine et un dimère de chaîne β mature est recueilli en l'absence de chaîne α.

**15.** Procédé suivant la revendication 10 ou 13, dans lequel la chaîne β est la chaîne $\beta_A$ et l'inhibine présente à une concentration supérieure à environ 20 ng/ml de milieu de culture.

**16.** Composition contenant de l'inhibine humaine ou porcine constituée d'une chaîne α et d'une chaîne β, les séquences d'amino-acides desdites chaînes α et β étant choisies entre celles représentées sur la figure 1B (chaîne α porcine), la figure 2B (chaînes β porcines), la figure 6A (chaîne α humaine) et les figures 8 et 9 (chaînes β humaines), et des variants de séquence d'amino-acides engendrés par insertion, délétion ou substitution d'un polypeptide d'une desdites séquences représentées, variants qui sont fortement homologues avec un polypeptide d'une séquence représentée, mais à l'exclusion de la chaîne α d'inhibine bovine, et 1) qui présentent une réactivité croisée avec des anticorps engendrés contre un polypeptide d'une séquence représentée ; ou 2) qui possèdent une activité hormonale similaire à celle d'un polypeptide d'une séquence représentée ; composition qui est totalement dépourvue de protéines humaines ou porcines non identifiées.

**17.** Composition suivant la revendication 16, dans laquelle les séquences des chaînes α et β sont choisies entre celles représentées sur la figure 1B (chaîne α porcine), la figure 2B (chaîne β porcine), la figure 6A (chaîne α humaine) et les figures 8 et 9 (chaînes β humaines).

**18.** Composition suivant la revendication 16, dans laquelle les variants sont choisis entre : des dérivés d'inhibine porcine ($Asn_{266}$→In)]GInhα ; [$Cys_{325}$ ou $Cys_{324}$→Δ]Inhα ; [$Cys_{361}$ ou $Cys_{363}$→Δ]Inhα ; [$Lys_{321}$ ou $Lys_{322}$→Δ] $Inh\beta_A$ ; [$Lys_{322}$→His ou Ser]$Inh\beta_A$ ; $Lys_{315}$→Arg ; $Val_{316}$→Thr] $Inh\beta_A$ ; [$Cys_{388}$ ou $Cys_{390}$→Δ] $Inh\beta_A$, [$Lys_{411}$→Gln]$Inh\beta_A$ ; [$Arg_{315}$→Lys ; $Val_{316}$→Thr]$Inh\beta_B$ ; [$Cys_{319}$ ou $Cys_{320}$→Δ] $Inh\beta_B$ [$Pro_{381}$ $Gly_{382}$→ Pro Phe Gly]$Inh\beta_B$ ; [$Arg_{395}$→Gln] $Inh\beta_B$, dans lesquels Inh représente une abréviation d'inhibine et les numéros des résidus pour $Inh\beta_B$ sont ceux utilisés pour le résidu $Inh\beta_A$ correspondant (voir figure 2B) ; des variants de chaîne $\beta_A$ d'inhibine humaine $\beta_A$ possédant des variations qui consistent en une substitution ou une délétion au niveau d'un, ou une insertion après un, résidu choisi entre les résidus 293 à 297, 364 à 376 et 387 à 398 de la séquence réprésentée sur la figure 8 ; et des variants de chaîne α humaine qui présentent une homologie de plus de 90 % avec la séquence représentée sur la figure 6A.

**19.** Composition suivant la revendication 16, comprenant un variant d'une chaîne d'inhibine $\beta_A$ humaine choisi entre $Phe_{302}$→Ile ou Leu ; $Gln_{297}$→Asp ou Lys ; $Trp_{307}$→Tyr ou Phe ; $Trp_{310}$→Tyr ou Phe ; $Ile_{311}$→Phe ou Val ; $Tyr_{317}$→Trp ou Thr ; $His_{318}$→Lys ; $Ala_{319}$→Ser ; $Asn_{320}$→Gln, Tyr ou His ; $Tyr_{321}$→Thr ou Asp, $Phe_{340}$→Tyr ; $His_{353}$→Asp ; $His_{353}$→Lys ; (un hybride $\beta_A/\beta_B$) ; $Phe_{356}$→Tyr ; $Val_{364}$→Phe ; $Val_{364}$→Leu ; $Tyr_{375}$→Thr ; $Tyr_{376}$→Trp ; $Asn_{389}$→Gln, His ou Lys ; $Ile_{391}$→Leu ou Thr ; $Met_{390}$→Leu ou Ser ; $Val_{392}$→Phe, Glu, Thr ou Ile ; ou une chaîne $\beta_B$ humaine modifiée de manière comparable.

**20.** Composition comprenant un prodomaine d'inhibine α ou $\beta_B$ humaine ou porcine représentée sur la figure 1B (chaîne α porcine), la figure 2B (chaîne $\beta_B$ porcine), la figure 6A (chaîne α humaine) et la figure 9 (chaîne $\beta_B$ humaine) non associé à une glycosylation naturelle.

**21.** Composition comprenant un homodimère de chaînes $\beta_A$ ou $\beta_B$ d'inhibine humaine ou porcine mature, lesdites chaînes étant représentées sur la figure 2B (chaînes β porcines) et les figures 8 et 9 (chaînes β humaines), ou d'un variant de séquence d'amino-acides engendré par insertion, délétion ou substitution d'un polypeptide d'une des séquences représentées, variant qui présente une forte homologie avec un polypeptide d'une séquence représentée et 1) qui présente une réactivité croisée avec des anticorps engendrés contre un polypeptide d'une séquence représentée ; ou 2) qui présente une activité hormonale similaire à celle d'un polypeptide d'une séquence représentée ; composition qui est dépourvue de la chaîne α d'inhibine.

**22.** Composition suivant la revendication 21, qui est un homodimère!de chaînes β humaine ou porcine mature ou d'un de ses variants de séquence d'amino-acides.

**23.** Composition comprenant un hétérodimère d'inhibine $\beta_A$ humaine ou porcine mature avec l'inhibine $\beta_B$ humaine ou porcine mature, lesdites chaînes étant représentées sur la figure 2B (chaînes β porcines) et les figures 8 et 9 (chaînes β humaines), ou d'un variant de séquence d'aminoacides engendré par insertion, délétion ou substitution d'une des séquences représentées, le variant présentant une forte homologie avec un polypeptide d'une séquence représentée et 1) présentant une réactivité croisée avec des anticorps engendrés contre un polypeptide d'une séquence représentée ; ou 2) présentant une activité hormonale similaire à celle d'un polypeptide d'une séquence représentée ; composition qui est dépourvue de la chaîne α d'inhibine.

**24.** ADN non chromosomique codant pour une chaîne α d'inhibine humaine ou porcine ou d'une chaîne β d'inhibine humaine ou porcine dont les séquences d'amino-acides sont décrites sur les figures 1B (chaîne α porcine), 2B (chaînes β porcines), 6A (chaîne α humaine) et 8 et 9 (chaînes β humaines) ou pour un variant de séquence d'amino-acides engendré par insertion, délétion ou substitution d'un polypeptide d'une desdites séquences représentées, le variant présentant une forte homologie avec un polypeptide d'une séquence représentée, à l'exclusion de la chaîne α d'inhibine bovine et de la chaîne β d'inhibine bovine partielle de séquence Gly-Leu-Glu-Cys-Asp-Gly-Lys-Val-Asn-Ile-Cys-Cys-Lys-Lys-Gln-Phe-Phe-Val-Ser-Phe-Lys-Asp-Ile-Gly-Trp-Asn-Asp-Trp-Ile-Ile-Ala-Pro-Ser-Gly-Tyr-His-Ala-Asn-Tyr-Cys-Glu-Gly-Glu-Cys-Pro-Ser-His-Ile-Ala-Gly-Thr-Ser-Gly-Ser-Ser-Leu-Ser-Phe-His-Ser-Thr-Val-Ile-Asn-His-Tyr-Arg-Met-Arg-Gly-HisSer, et

1) présentant une réactivité croisée avec des anticorps engendrés contre un polypeptide d'une séquence représentée ; ou 2) présentant une activité hormonale similaire à celle d'un polypeptide d'une séquence représentée.

**25.** ADN suivant la revendication 24, codant pour une chaîne α d'inhibine humaine ou porcine ou une chaîne β d'inhibine humaine ou porcine dont la séquence d'amino-acides est représentée sur la figure 1B (chaîne α porcine), la figure 2B (chaînes β porcines), la figure 6A (chaîne α humaine) et les figures 8 et 9 (chaînes β humaines).

**26.** ADN suivant la revendication 24, qui code pour un variant choisi entre : des dérivés d'inhibine porcine [$Asn_{266}$→Gln] Inhα ; [$Cys_{325}$ ou $Cys_{324}$→Δ]Inhα ; [$Cys_{361}$ ou $Cys_{363}$→Δ]Inhα ; [$Lys_{321}$ ou $Lys_{322}$→Δ]$Inh\beta_A$ ; [$Lys_{322}$→His ou Ser] $Inh\beta_A$ ; [$Lys_{315}$→Arg ; $Val_{316}$→Thr] $Inh\beta_A$ ; [$Cys_{388}$ ou $Cys_{390}$→Δ]$Inh_A$, [$Lys_{411}$→Gln]$Inh\beta_A$ ; [$Arg_{315}$→Lys ; $Val_{316}$→Thr]$Inh\beta_B$ ; [$Cys_{319}$ ou $Cys_{320}$→Δ]$Inh\beta_B$ ; [$Pro_{381}$ $Gly_{382}$→Pro Phe Gly]$Inh\beta_B$ ; [$Arg_{395}$→Gln]$Inh\beta_B$, dans lesquel Inh est une abréviation d'inhibine et les numéros des résidus pour la $Inh\beta_B$ sont ceux utilisés pour le résidu de $Inh\beta_A$ correspondants (voir figure 2B) ; les variants de chaîne $\beta_A$ d'inhibine humaine possédant des variations qui consistent en une substitution ou une délétion au niveau d'un, ou une insertion après un, résidu choisi entre les résidus 293 à 297, 364 à 376 et 387 à 398 de la séquence représentée sur la figure 8 ; et des variants de la chaîne α humaine qui présentent une homologie de plus de 90 % avec la séquence représentée sur la figure 6A.

**27.** ADN suivant la revendication 24, qui code pour un variant d'une chaîne β d'inhibine humaine choisi entre $Phe_{302}$→Ile ou Leu ; $Gln_{297}$→Asp ou Lys ; $Trp_{307}$→Tyr ou Phe ; $Trp_{310}$→Tyr ou Phe ; $Ile_{311}$→Phe ou Val ; $Tyr_{317}$→Trp ou Thr ; $His_{318}$→Lys ; $Ala_{319}$→Ser ; $Asn_{320}$→Gln, Tyr ou His ; $Tyr_{321}$→Thr ou Asp, $Phe_{340}$→Tyr ; $His_{353}$→Asp ; $His_{353}$→Lys (à un hybride $\beta_A/\beta_B$) ; $Phe_{356}$→Tyr ; $Val_{364}$→Phe ; $Val_{364}$→Leu ; $Tyr_{375}$→Thr ; $Tyr_{376}$→Trp ; $Asn_{389}$→Gln, His ou Lys ; $Ile_{391}$→Leu ou Thr ; $Met_{390}$→Leu ou Ser ; $Val_{392}$→Phe, Glu, Thr ou Ile ; ou une chaîne $\beta_B$ humaine modifiée de manière comparable.

**28.** ADN suivant l'une quelconque des revendications 24 à 27, qui est dépourvu de séquences non traduites intermédiaires.

**29.** ADN suivant l'une quelconque des revendication 24 à 28, qui est marqué avec un groupement détectable.

**30.** Vecteur réplicable comprenant un ADN suivant l'une quelconque des revendications 21 à 27.

**31.** Vecteur suivant la revendication 30, comprenant un promoteur viral lié de manière fonctionnelle à l'ADN codant pour les chaînes α et/ou β d'inhibine.

**32.** Vecteur suivant la revendication 30 ou 31, qui contient un ADN codant à la fois pour une chaîne $\alpha$ d'inhibine et une chaîne $\beta$ d'inhibine.

**33.** Vecteur suivant la revendication 30 ou 31, qui contient un ADN codant pour une chaîne $\beta$ d'inhibine, mais pour la chaîne $\alpha$ d'inhibine.

**34.** Cellule hôte transformation avec un vecteur réplicable comprenant l'ADN codant pour la chaîne $\alpha$ et/ou la chaîne $\beta$ d'inhibine humaine ou porcine dont les séquences d'amino-acides sont représentées sur la figure 1B (chaîne $\alpha$ porcine), la figure 2B (chaînes $\beta$ porcines), la figure 6A (chaîne $\alpha$ humaine) et les figures 8 et 9 (chaînes $\beta$ humaines) ou un variant de séquence d'amino-acides engendré par insertion, délétion ou substitution d'un polypeptide d'une telle séquence représentée, le variant étant pratiquement homologue avec un polypeptide d'une séquence représentée, à l'exclusion de la chaîne $\alpha$ d'inhibine bovine et de la chaîne $\beta$ d'inhibine bovine partielle de séquence Gly-Leu-Glu-Cys-Asp-Gly-Lys-Val-Asn-Ile-Cys-Cys-Lys-Lys-Gln-Phe-Phe-Val-Ser-Phe-Lys-Asp-Ile-Gly-Trp-Asn-Trp-Ile-Ala-Pro-Ser-Gly-Tyr-His-Ala-Asn-Tyr-Cys-Glu-Gly-Glu-Cys-Pro-Ser-His-Ile-Ala-Gly-Thr-Ser-Gly-Ser-Ser-Leu-Ser-Phe-His- Ser-Thr-Val-Ile-Asn-His-Tyr-Arg-Met-Arg-Gly-His-Ser, et

1) présentant une réactivité croisée avec des anticorps engendrés contre un polypeptide d'une séquence représentée ; ou 2) présentant une activité hormonale similaire à celle d'un polypeptide d'une séquence représentée.

**35.** Cellule hôte suivant la revendication 34, dans laquelle l'ADN code pour une chaîne $\alpha$ et/ou une chaîne $\beta$ d'inhibine humaine porcine, dont les séquences d'amino-acides sont représentées sur la figure 1B (chaîne $\alpha$ porcine), la figure 2B (chaînes $\beta$ porcines), la figure 6A (chaîne $\alpha$ humaine) et les figures 8 et 9 (chaînes $\beta$ humaines).

**36.** Cellule hôte suivant la revendication 34, dans laquelle l'ADN code pour un variant choisi entre : des dérivés d'ihibine porcine [$Asn_{266}\rightarrow$Gln]Inh$\alpha$ ; [$Cys_{325}$ ou $Cys_{324}\rightarrow\Delta$] Inh$\alpha$ ; [$Cys_{361}$ ou $Cys_{363}\rightarrow\Delta$] Inh$\alpha$ ; [$Lys_{321}$ ou $Lys_{322}\rightarrow\Delta$) Inh$\beta_A$ ; [$Lys_{322}\rightarrow$His ou Ser] Inh$\beta_A$ ; [$Lys_{315}\rightarrow$Arg ; $Val_{316}\rightarrow$Thr] Inh$\beta_A$ ; [$Cys_{388}$ ou $Cys_{390}\rightarrow\Delta$] Inh$\beta_A$, [$Lys_{411}\rightarrow$Gln] Inh$\beta_A$ ; [$Arg_{315}\rightarrow$Lys ; $Val_{316}\rightarrow$Thr]Inh$\beta_B$ ; [$Cys_{319}$ ou $Cys_{320}\rightarrow\Delta$)Inh$\beta_B$ ; [$Pro_{381}$ $Gly_{382}\rightarrow$Pro Phe Gly]Inh$\beta_B$ ; [$Arg_{395}\rightarrow$Gln]Inh$\beta_B$, dans lesquel Inh est une abréviation d'inhibine et les numéros des résidus pour la Inh$\beta_B$ sont ceux utilisés pour le résidu de Inh$\beta_A$ correspondant (voir figure 2B) ; les variants de chaîne $\beta_A$ d'inhibine humaine possédant des variations qui consistent en une substitution ou une délétion au niveau d'un, ou une insertion après un, résidu choisi entre les résidus 293-297, 364-376 et 387-398 de la séquence représentée sur la figure 8 ; et des variants de la chaîne $\alpha$ humaine qui présentent une homologie de plus de 90 % avec la séquence représentée sur la figure 6A.

**37.** Cellule hôte suivant la revendication 34, dans laquelle l'ADN code pour un variant d'une chaîne $\beta_A$ d'inhibine humaine choisie entre $Phe_{302}\rightarrow$Ile ou Leu ; $Gln_{297}\rightarrow$Asp!ou Lys ; $Trp_{307}\rightarrow$Tyr ou Phe ; $Trp_{310}\rightarrow$Tyr ou Phe ; $Ile_{311}\rightarrow$Phe ou Val ; $Tyr_{317}\rightarrow$Trp ou Thr ; $His_{318}\rightarrow$Lys ; $Ala_{319}\rightarrow$Ser ; $Asn_{320}\rightarrow$Gln, Tyr ou His ; $Tyr_{321}\rightarrow$Thr ou Asp, $Phe_{340}\rightarrow$Tyr ; $His_{353}\rightarrow$Asp ; $His_{353}\rightarrow$Lys (à un hybride $\beta_A/\beta_B$) ; $Phe_{356}\rightarrow$Tyr ; $Val_{364}\rightarrow$Phe ; $Val_{364}\rightarrow$Leu ; $Tyr_{375}\rightarrow$Thr ; $Tyr_{376}\rightarrow$Trp ; $Asn_{389}\rightarrow$Gln, His ou Lys ; $Ile_{391}\rightarrow$Leu ou Thr ; $Met_{390}\rightarrow$Leu ou Ser ; $Val_{392}\rightarrow$Phe, Glu, Thr ou Ile ; ou une chaîne $\beta_B$ humaine modifiée de manière comparable.

**38.** Cellule suivant l'une quelconque des revendications 34 à 37, qui est une cellule eucaryotique.

**39.** Composition acellulaire qui est dépourvue d'un polypeptide de chaîne $\alpha$ mature, contenant une séquence polypeptidique de prodomaine de chaîne $\alpha$ d'inhibine humaine ou porcine représentée sur la figure 1B (chaîne $\alpha$ porcine) ou la figure 6A (chaîne $\alpha$ humaine) ou un variant de séquence d'amino-acides engendré par une insertion, délétion ou substitution d'une telle séquence de prodomaine, le variant présentant une forte homologie avec un polypeptide d'une desdites séquences présentant une réactivité croisée avec des anticorps engendrés contre un polypeptide d'une séquence représentée.

**40.** Composition acellulaire contenant

a) un polypeptide comprenant la séquence de prodomaine de chaîne $\beta$ d'inhibine humaine

HSAAPDCPSCALAALPKDVPNSQPEMVEAVKKHILNMLHL (aminoacides 1 à 40 de la figure 8), PDVTQPVPKAALLNAIRKLHVGKVGENGYVEIEDDIG (aminoacides 44 à 80 de la figure 8), .

AEMNELMEQTSEIITFAESGTARKTLHFEISKEGSDLSVVERAEVWLFLKVPKAN-RTRTKVTIRLFQQQKHPQGSLDTGEEAEEVGLKGERSELLLSEKVVDA (aminoacides 83 à 185 de la figure 8),

STWHVFPVSSSIQRLLDQGKSSLDVRIACEQCQESGASLVLLG (amino-acides 188 à 230 de la figure 8), ou un de ses variants de séquence d'amino-acides naturels de mammifère ;
b) un polypeptide comprenant la séquence de prodomaine de chaîne $\beta_B$ d'inhibine humaine CTSCGGFRRPEELGRVDGDFLEAV (amino-acides) 7-30 de la figure 9, (amino-acides 33 à 5 de la figure 9),

VRVKVYFQEQGHGDRWNMVEKRVDLKRSGWHTFPLTEAIQALFERGE (amino-acides 149 à 195 de la figure 9),

LNLDVQCDSCQELAVVPVFVDPGEESHRPFVVVQARLGDSRHRI

(amino-acides 149 à 195 de la figure 9), LNLDVQCDSCQELAVVPVFVDPGEESHRPFVVVQARLGDSRHRI (amino-acides 198 à 241 de la figure 9), ou un de leurs variants de séquence d'amino-acides naturels de mammifère ; ou
c) un polypeptide dépourvu de la séquence d'amino-acides de chaînes $\alpha$ mature comprenant les séquences de prodomaine de chaîne $\alpha$ d'inhibine humaine KVRALFLDALGPPAVTREGGDPGV (aminoacides 1 à 24 de la figure 6),
(amino-acides 32 à 199 de la figure 6), ou un de leurs variants de séquence d'amino-acides naturels de mammifère.

**41.** Composition acellulaire contenant un polypeptide de prodomaine de chaîne $\beta$ d'inhibine humaine ou porcine d'une séquence représentée sur la figure 2B (chaînes $\beta$ porcines) et les figures 8 et 9 (chaînes $\beta$ humaines) ou un variant de séquence d'amino-acides engendré par insertion, délétion ou substitution d'une desdites séquences de prodomaine, variant qui présente une forte homologie avec un polypeptide d'une desdites séquences et qui présente une réactivité croisée avec des anticorps engendrés contre un polypeptide d'une séquence représentée.

**42.** Composition suivant la revendication 41, dans laquelle la chaîne $\beta$ et la chaîne $\beta_A$ et la composition est dépourvue de séquence de chaîne $\beta_A$ mature.

**43.** Composition suivant la revendication 41, dans laquelle la chaîne $\beta$ est la chaîne $\beta_B$ et la composition est dépourvue de séquence de chaîne $\beta_B$ mature.

**44.** Composition suivant la revendication 40, dans laquelle le variant est la séquence d'amino-acides porcine correspondante, composition contenant :

a) un polypeptide comprenant la séquence de prodomaine de chaîne $\beta_A$ d'inhibine porcine représentée par : les amino-acides 28 à 58 ; les amino-acides 61 à 87 ; les aminoacides 90 à 108 ; les amino-acides 111 à 179 ; les aminoacides 182 à 213 ; les amino-acides 216 à 258 ; ou les amino-acides 28 à 87 de la figure 2B ; ou
b) un polypeptide dépourvu de la séquence d'amino-acides de chaîne $\alpha$ mature comprenant les séquences de polypeptides de prodomaine de chaîne $\alpha$ d'inhibine porcine représentées par : les amino-acides 20 à 54 ;

ou les amino-acides 70 à 228 de la figure 1B.

**45.** Composition suivant l'une quelconque des revendications 39 à 44, dans laquelle le polypeptide est non accompagné par une glycosylation naturelle.

**46.** Composition suivant l'une quelconque des revendications 39 à 45, qui est stérile et dans laquelle le polypeptide comprend en outre un polypeptide immunogène.

**47.** Utilisation d'une composition suivant l'une quelconque des revendications 39 à 46 dans la préparation d'un anticorps capable de se lier audit polypeptide.

**48.** Composition suivant l'une quelconque des revendications 39 à 46, dans laquelle le polypeptide est conjugué à un groupe détectable.

**49.** Composition suivant la revendication 48, dans laquelle le groupe est un enzyme, un fluorophore ou un radioisotope.

**50.** Composition suivant l'une quelconque des revendications 39 à 44, 48 et 49, qui est insolubilisée par une absorption non covalente ou une réticulation covalente à un support insoluble dans l'eau.

**51.** Composition suivant l'une quelconque des revendications 39 à 46, 48 et 49, comprenant en outre une matrice implantable physiologiquement acceptable pour la libération contrôlée du polypeptide dans les tissus d'un animal.

**Revendications pour l'Etat contractant suivant : LU**

**1.** Procédé comprenant la culture d'une cellule hôte transformée avec un vecteur qui renferme un acide nucléique codant pour une chaîne $\alpha$ d'inhibine humaine ou porcine et/ou pour une chaîne $\beta$ d'inhibine humaine ou porcine dont les séquences d'amino-acides sont représentées sur la figure 1B (chaîne $\alpha$ porcine), la figure 2B (chaînes $\beta$ porcines), la figure 6A (chaîne $\alpha$ humaine) et les figures 8 et 9 (chaînes $\beta$ humaines), ou un variant de séquence d'aminoacides formé par insertion, délétion ou substitution d'une des séquences représentées, le variant étant fortement homologue avec un polypeptide d'une séquence représentée mais excluant la chaîne $\alpha$ d'inhibine bovine et 1) qui présente une réactivité croisée avec des anticorps engendrés contre un polypeptide d'une séquence représentée ; ou 2) qui présente une activité hormonale similaire à celle d'un polypeptide d'une séquence représentée.

**2.** Procédé suivant la revendication 1, dans lequel l'acide nucléique code pour une chaîne $\alpha$ d'inhibine humaine ou porcine et/ou une chaîne $\beta$ d'inhibine ou porcine dont les séquences d'amino-acides sont représentées sur la figure 1B (chaîne $\alpha$ porcine), la figure 2B (chaînes $\beta$ porcines), la figure 6A (chaîne $\alpha$ humaine) et les figures 8 et 9 (chaînes $\beta$ humaines).

**3.** Procédé suivant la revendication 1, dans lequel le vecteur code pour une chaîne $\beta$ d'inhibine, ou un de ses variants de séquence d'amino-acides, autre que la $\beta_A$ humaine ou porcine naturelle.

**4.** Procédé suivant la revendication 1, dans lequel l'acide nucléique code pour un variant choisi entre : des dérivés d'inhibine porcine [$Asn_{266}$→Gln]Inh$\alpha$ ; [$Cys_{325}$ ou $Cys_{324}$→Δ]Inh$\alpha$ ; [$Cys_{361}$ ou $Cys_{363}$→Δ]Inh$\alpha$ ; [$Lys_{321}$ ou $Lys_{322}$→Δ]Inh$\beta_A$ ; [$Lys_{322}$→His ou Ser]Inh$\beta_A$; [$Lys_{315}$→Arg ; $Val_{316}$→Thr] Inh$\beta_A$ ; [$Cys_{388}$ ou $Cys_{390}$→Δ]Inh$\beta_A$, [$Lys_{411}$→Gln]Inh$\beta_A$ ; [$Arg_{315}$→Lys ; $Val_{316}$→Thr]Inh$\beta_B$ ; [$Cys_{319}$ ou $Cys_{320}$→Δ]Inh$\beta_B$ ; [$Pro_{381}$ $Gly_{382}$→Pro Phe Gly] Inh$\beta_B$ ; [$Arg_{395}$→Gln]Inh$\beta_B$, dans lesquel Inh est une abréviation d'inhibine et les numéros des résidus pour la Inh$\beta_B$ sont ceux utilisés pour le résidu de Inh$\beta_A$ correspondant (voir figure 2B) ; les variants de chaîne $\beta_A$ d'inhibine humaine possédant des variations qui consistent en une substitution ou une délétion au niveau d'un, ou une insertion après un, résidu choisi entre les résidus 293 à 297, 364 à 376 et 387 à 398 de la séquence représentée sur la figure 8 ; et des variants de la chaîne $\alpha$ humaine qui présentent une homologie de plus de 90 % avec la séquence représentée sur la figure 6A.

**5.** Procédé suivant la revendication 1, dans lequel l'acide nucléique code pour un variant d'une chaîne d'inhibine $\beta_A$ humaine choisi entre $Phe_{302}$→Ile ou Leu ; $Gln_{297}$→Asp ou Lys ; $Trp_{307}$→Tyr ou Phe ; $Trp_{310}$→Tyr ou Phe ; $Ile_{311}$→Phe ou Val ; $Tyr_{317}$→Trp ou Thr ; $His_{318}$→Lys ; $Ala_{319}$→Ser ; $Asn_{320}$→Gln, Tyr ou His ; $Tyr_{321}$→Thr ou Asp, $Phe_{34o}$→Tyr ; $His_{353}$→Asp ; $His_{353}$→Lys (à un hybride $\beta_A/\beta_B$) ; $Phe_{356}$→Tyr ; $Val_{364}$→Phe ; $Val_{364}$→Leu ;

$Tyr_{375}$→Thr ; $Tyr_{377}$→Trp ; $Asn_{389}$→Gln, His ou Lys ; $Ile_{391}$→Leu ou Thr ; $Met_{390}$→Leu ou Ser ; $Val_{392}$→Phe, Glu, Thr ou Ile ; ou une chaîne $\beta_B$ humaine modifiée de manière comparable.

**6.** Procédé suivant l'une quelconque des revendications précédentes, dans lequel l'acide nucléique codant pour la chaîne α et/ou la chaîne β d'inhibine est lié de manière fonctionnelle à un promoteur reconnu par la cellule hôte et contenant l'étape supplémentaire de séparation de l'inhibine ou d'un dimère de chaîne β du milieu de culture.

**7.** Procédé suivant l'une quelconque des revendications précédentes, dans lequel la cellule est un procaryote.

**8.** Procédé suivant l'une quelconque des revendications précédentes, dans lequel le vecteur comprend un acide nucléique codant pour la forme pré-pro d'une chaîne α d'inhibine d'une chaîne β d'inhibine.

**9.** Procédé suivant la revendication 8, dans lequel le vecteur comprend un acide nucléique codant pour la forme de pré-pro de la chaîne α d'inhibine et de la chaîne β d'inhibine.

**10.** Procédé suivant l'une quelconque des revendications 1 à 6, 8 et 9, dans lequel la cellule est une cellule provenant d'un organisme pluricellulaire et une inhibine douée d'activité hormonale est produite.

**11.** Procédé suivant la revendication 6, dans lequel le promoteur est un promoteur viral.

**12.** Procédé suivant la revendicatioon 11, dans lequel le promoteur est un promoteur de SV40.

**13.** Procédé suivant l'une quelconque des revendications précédentes, dans lequel de l'inhibine porcine ou humaine mature est recueillie.

**14.** Procédé suivant l'une quelconque des revendications 1 à 7 ou 10 à 12, dans lequel le vecteur comprend un acide nucléique codant pour la forme pré-pro d'une chaîne β d'inhibine et un dimère de chaîne β mature est recueilli en l'absence de chaîne α.

**15.** Procédé suivant la revendication 10 ou 13, dans lequel la chaîne β est la chaîne $\beta_A$ et l'inhibine présente à une concentration supérieure à environ 20 ng/ml de milieu de culture.

**16.** Composition contenant de l'inhibine humaine ou porcine constituée d'une chaîne α et d'une chaîne β, les séquences d'amino-acides desdites chaînes α et β étant choisies entre celles représentées sur la figure 1B (chaîne α porcine), la figure 2B (chaînes β porcines), la figure 6A (chaîne α humaine) et les figures 8 et 9 (chaînes β humaines), et des variants de séquence d'amino-acides engendrés par insertion, délétion ou substitution d'un polypeptide d'une desdites séquences représentées, variants qui sont fortement homologues avec un polypeptide d'une séquence représentée, mais à l'exclusion de la chaîne α d'inhibine bovine, et 1) qui présentent une réactivité croisée avec des anticorps engendrés contre un polypeptide d'une séquence représentée ; ou 2) qui possèdent une activité hormonale similaire à celle d'un polypeptide d'une séquence représentée ; composition qui est totalement dépourvue de protéines humaines ou porcines non identifiées.

**17.** Composition suivant la revendication 16, dans laquelle les séquences des chaînes α et β sont choisies entre celles représentées sur la figure 1B (chaîne α porcine), la figure 2B (chaîne β porcine), la figure 6A (chaîne α humaine) et les figures 8 et 9 (chaînes β humaines).

**18.** Composition suivant la revendication 16, dans laquelle les variants sont choisis entre : des dérivés d'inhibine porcine ($Asn_{266}$→In]GInhα ; [$Cys_{325}$ ou $Cys_{324}$→Δ] Inhα ; [$Cys_{361}$ ou $Cys_{363}$→Δ] Inhα ; [$Lys_{321}$ ou $Lys_{322}$→Δ]Inh$\beta_A$ ; [$Lys_{322}$→His ou Ser]Inh$\beta_A$ ; $Lys_{315}$→Arg ; $Val_{316}$→Thr] Inh$\beta_A$ ; [$Cys_{388}$ ou $Cys_{390}$→Δ]Inh$\beta_A$, [$Lys_{411}$→Gln]Inh$\beta_A$ ; [$Arg_{315}$→Lys ; $Val_{316}$→Thr]Inh$\beta_B$ ; [$Cys_{319}$ ou $Cys_{320}$→Δ]Inh$\beta_B$ [$Pro_{381}$ $Gly_{382}$→ Pro Phe Gly] Inh$\beta_B$ ; [$Arg_{395}$→Gln] Inh$\beta_B$, dans lesquels Inh est une abréviation d'inhibine et les numéros des résidus pour Inh$\beta_B$ sont ceux utilisés pour le résidu Inh$\beta_A$ correspondant (voir figure 2B) ; des variants de chaîne $\beta_A$ d'inhibine humaine possédant des variations qui consistent en une substitution ou une délétion au niveau d'un, ou une insertion après un, résidu choisi entre les résidus 293 à 297, 364 à 376 et 387 à 398 de la séquence réprésentée sur la figure 8 ; et des variants de chaîne α humaine qui présentent une homologie de plus de 90 % avec la séquence représentée sur la figure 6A.

**19.** Composition suivant la revendication 16, comprenant un variant d'une chaîne d'inhibine $\beta_A$ humaine choisi entre

$Phe_{302}$→Ile ou Leu ; $Gln_{297}$→Asp ou Lys ; $Trp_{307}$→Tyr ou Phe ; $Trp_{310}$→Tyr ou Phe ; $Ile_{311}$→Phe ou Val ; $Tyr_{317}$→Trp ou Thr ; $His_{318}$→Lys ; $Ala_{319}$→Ser ; $Asn_{320}$→Gln, Tyr ou His ; $Tyr_{321}$→Thr ou Asp, $Phe_{340}$→Tyr ; $His_{353}$→Asp ; $His_{353}$→Lys ; (un hybride $\beta_A/\beta_B$) ; $Phe_{356}$→Tyr ; $Val_{364}$→Phe ; $Val_{364}$→Leu ; $Tyr_{375}$→Thr ; $Tyr_{376}$→Trp ; $Asn_{389}$→Gln, His ou Lys ; $Ile_{391}$→Leu ou Thr ; $Met_{391}$→Leu ou Ser ; $Val_{392}$→Phe, Glu, Thr ou Ile ; ou une chaîne $\beta_B$ humaine modifiée de manière comparable.

**20.** Composition comprenant un prodomaine d'inhibine α ou $\beta_B$ humaine ou porcine représenté sur la figure 1B (chaîne α porcine), la figure 2B (chaîne $\beta_B$ porcine), la figure 6A (chaîne α humaine) et la figure 9 (chaîne $\beta_B$ humaine) non associé à une glycosylation naturelle.

**21.** Composition comprenant un homodimère de chaînes $\beta_A$ ou $\beta_B$ d'inhibine humaine ou porcine mature, lesdites chaînes étant représentées sur la figure 2B (chaînes β porcines) et les figures 8 et 9 (chaînes β humaines), ou d'un variant de séquence d'amino-acides engendré par insertion, délétion ou substitution d'un polypeptide d'une des séquences représentées, variant qui présente une forte homologie avec un polypeptide d'une séquence représentée et 1) qui présente une réactivité croisée avec des anticorps engendrés contre un polypeptide d'une séquence représentée ; ou 2) qui présente une activité hormonale similaire à celle d'un polypeptide d'une séquence représentée ; composition qui est dépourvue de la chaîne α d'inhibine.

**22.** Composition suivant la revendication 21, qui est un homodimère de chaînes $\beta_B$ humaine ou porcine mature ou d'un de ses variants de séquence d'amino-acides.

**23.** Composition comprenant un hétérodimère de chaîne $\beta_A$ d'inhibine humaine ou porcine mature avec une chaîne $\beta_B$ d'inhibine humaine ou porcine mature, lesdites chaînes étant représentées sur la figure 2B (chaînes β porcines) et les figures 8 et 9 (chaînes β humaines), ou d'un variant de séquence d'amino-acides engendré par insertion, délétion ou substitution d'une desdites séquences représentées, variant qui présente une forte homologie avec un polypeptide d'une séquence représentée et 1) qui présente une réactivité croisée avec des anticorps engendrés contre un polypeptide d'une séquence représentée ; ou 2) qui présente une activité hormonale similaire à celle d'un polypeptide d'une séquence représentée ; composition qui est dépourvue de la chaîne α d'inhibine.

**24.** ADN non chromosomique codant pour une chaîne α d'inhibine humaine ou porcine ou d'une chaîne β d'inhibine humaine ou porcine dont les séquences d'amino-acides sont représentées sur la figure 1B (chaîne α porcine), la figure 2B (chaînes β porcines), la figure 6A (chaîne α humaine) et les figures 8 et 9 (chaînes β humaines) ou un variant de séquence d'amino-acides engendré par insertion, délétion ou substitution d'un polypeptide d'une desdites séquences représentées, variant qui présente une forte homologie avec un polypeptide d'une séquence représentée à l'exclusion de la chaîne α d'inhibine bovine et 1) qui présente une réactivité croisée avec des anticorps!engendrés contre un polypeptide d'une séquence représentée ; ou 2) qui présente une réactivité croisée avec des récepteurs de surface cellulaire pour un polypeptide d'une séquence représentée ; ou 3) qui présente une activité hormonale similaire à celle d'un polypeptide d'une séquence représentée.

**25.** ADN suivant la revendication 24 codant pour une chaîne α d'inhibine humaine ou porcine ou une chaîne β d'inhibine humaine ou porcine dont la séquence d'aminoacides est représentée sur la figure 1B (chaîne α porcine), la figure 2B (chaînes β porcines), la figure 6A (chaîne α humaine) et les figures 8 et 9 (chaînes β humaines).

**26.** ADN suivant la revendication 24, qui code pour un variant choisi entre : des dérivés d'inhibine porcine [$Asn_{266}$→Gln] Inhα ; [$Cys_{325}$ ou $Cys_{324}$→Δ] Inhα ; [$Cys_{361}$ ou $Cys_{363}$→Δ] Inhα ; [$Lys_{321}$ ou $Lys_{322}$→Δ] Inh$\beta_A$ ; [$Lys_{322}$→His ou Ser] Inh$\beta_A$ ; [Lys315→Arg ; $val_{316}$→Thr] Inh$\beta_A$ ; [$Cys_{388}$ ou $Cys_{390}$→Δ] Inh$\beta_A$, [$Lys_{411}$→Gln] Inh$\beta_A$ ; [$Arg_{315}$→Lys ; $Val_{316}$→Thr]Inh$\beta_B$ ; [$Cys_{319}$ ou $Cys_{320}$→Δ]Inh$\beta_B$ ; [$Pro_{381}$ $Gly_{382}$→Pro Phe Gly]Inh$\beta_B$ ; [$Arg_{395}$→Gln]Inh$\beta_B$, dans lesquels Inh est une abréviation d'inhibine et les numéros des résidus pour la Inh$\beta_B$ sont ceux utilisés pour le résidu de Inh$\beta_A$ correspondant (voir figure 2B) ; des variants de chaîne $\beta_A$ d'inhibine humaine possédant des variations qui consistent en une substitution ou une délétion au niveau d'un, ou une insertion après un, résidu choisi entre les résidus 293 à 297, 364 à 376 et 387 à 398 de la séquence représentée sur la figure 8 ; et des variants de chaîne α humaine qui présentent une homologie de plus de 90 % avec la séquence représentée sur la figure 6A.

**27.** ADN suivant la revendication 24, qui code pour un variant d'une chaîne d'inhibine β humaine choisi entre : $Phe_{302}$→Ile ou Leu ; $Gln_{297}$→Asp ou Lys ; $Trp_{307}$→Tyr ou Phe ; $Trp_{310}$→Tyr ou Phe ; $Ile_{311}$→Phe ou Val ; $Tyr_{317}$→Trp ou Thr ; $His_{318}$→Lys ; $Ala_{319}$→Ser ; $Asn_{320}$→Gln, Tyr ou His ; $Tyr_{321}$→Thr ou Asp, $Phe_{340}$→Tyr ; $His_{353}$→Asp ; $His_{353}$→Lys (à un hybride $\beta_A/\beta_B$) ; $Phe_{356}$→Tyr ; $Val_{364}$→Phe ; $Val_{364}$→Leu ; $Tyr_{375}$→Thr ;

$Tyr_{376}$→Trp ; $Asn_{389}$→Gln, His ou Lys $Ile_{391}$→Leu ou Thr ; $Met_{390}$→Leu ou Ser ; $Val_{392}$→Phe, Glu, Thr ou Ile ; ou une chaîne $\beta_B$ humaine modifiée de manière comparable.

**28.** ADN suivant l'une quelconque des revendications 24 à 27, qui est dépourvue de séquences non traduites intermédiaires.

**29.** ADN suivant l'une quelconque des revendications 24 à 28, qui est marquée avec un groupement détectable.

**30.** Vecteur réplicable comprenant un ADN suivant l'une quelconque des revendications 21 à 27.

**31.** Vecteur suivant la revendication 30, comprenant un promoteur viral lié de manière fonctionnelle à l'ADN codant pour les chaînes α et/ou β d'inhibine.

**32.** Vecteur suivant la revendication 30 ou 31, qui contient un ADN codant à la fois pour une chaîne α d'inhibine et une chaîne β d'inhibine.

**33.** Vecteur suivant la revendication 30 ou 31, qui contient un ADN codant pour une chaîne β d'inhibine mais non pour la chaîne α d'inhibine.

**34.** Cellule hôte transformée avec un vecteur réplicable comprenant un ADN codant pour la chaîne α d'inhibine et/ou la chaîne β d'inhibine humaine ou porcine dont les séquences d'amino-acides sont représentées sur la figure 1B (chaîne α porcine), la figure 2B (chaînes β porcines), la figure 6A (chaîne α humaine) et les figures 8 et 9 (chaînes β humaines), ou un variant de séquence d'amino-acides engendré par insertion, délétion ou substitution d'un polypeptide d'une desdites séquences représentées, variant qui présente une forte homologie avec un polypeptide d'une séquence représentée, à l'exclusion de la chaîne α d'inhibine bovine, et 1) qui présente une réactivité croisée avec des anticorps engendrés contre un polypeptide d'une séquence représentée ; ou 2) qui présente une activité hormonale similaire à celle d'un polypeptide d'une séquence représentée.

**35.** Cellule hôte suivant la revendication 34, dans laquelle l'ADN code pour une chaîne α d'inhibine et/ou une chaîne β d'inhibine humaine ou porcine, dont les séquences d'amino-acides sont représentées sur la figure 1B (chaîne α porcine), la figure 2B (chaînes β porcines) la figure 6A (chaîne α humaine) et les figures 8 et 9 (chaînes β humaines).

**36.** Cellule hôte suivant la revendication 34, dans laquelle l'ADN code pour un variant choisi entre : des dérivés d'inhibine porcine [$Asn_{266}$→Gln]Inhα ; [$Cys_{325}$ ou $Cys_{324}$→Δ]Inhα ; [$Cys_{361}$ ou $Cys_{363}$→Δ]Inhα ; [$Lys_{321}$ ou $Lys_{322}$→Δ] $Inh\beta_A$ ; [$Lys_{322}$→His ou Ser] $Inh\beta_A$ ; [$Lys_{315}$→Arg; $Val_{316}$→Thr) $Inh\beta_A$ ; [$Cys_{388}$ ou $Cys_{390}$→Δ]$Inh\beta_A$, [$Lys_{411}$→Gln] $Inh\beta_A$ ; [$Arg_{315}$→Lys ; $Val_{316}$→Thr]$Inh\beta_B$ ; [$Cys_{319}$ ou $Cys_{320}$→Δ]$Inh\beta_B$ ; [$Pro_{381}$ $Gly_{382}$→Pro Phe Gly]InhβB ; [$Arg_{395}$→Gln]$Inh\beta_B$, dans lesquels Inh est une abréviation d'inhibine et les numéros des résidus pour $Inh\beta_B$ sont ceux utilisés pour le résidu de $Inh\beta_A$ correspondant (voir figure 2B) ; des variants de chaîne $\beta_A$ d'inhibine humaine possédant des variations qui consistent en une substitution ou une délétion au niveau d'un, ou une insertion après un, résidu choisi entre les résidus 293 à 297, 364 à 376 et 387 à 398 de la séquence représentée sur la figure 8 ; et des variants de chaîne α humaine qui présentent une homologie de plus de 90 % avec la séquence représentée sur la figure 6A.

**37.** Cellule hôte suivant la revendication 34, dans laquelle l'ADN code pour un variant d'une chaîne d'inhibine $\beta_A$ humaine choisi entre $Phe_{302}$→Ile ou Leu ; $Gln_{297}$→Asp ou Lys ; $Trp_{307}$→Tyr ou Phe ; $Trp_{310}$→Tyr ou Phe ; $Ile_{311}$→Phe ou Val ; $Tyr_{317}$→Trp ou Thr ; $His_{318}$→Lys ; $Ala_{319}$→Ser ; $Asn_{320}$→Gln, Tyr ou His ; $Tyr_{321}$→Thr ou Asp, $Phe_{340}$→Tyr ; $His_{353}$→Asp ; $His_{353}$→Lys (à un hybride $\beta_A/\beta_B$) ; $Phe_{356}$→Tyr ; $Val_{364}$→Phe ; $Val_{364}$→Leu ; $Tyr_{375}$→Thr ; $Tyr_{376}$→Trp ; $Asn_{389}$→Gln, His ou Lys ; $Ile_{391}$→Leu ou Thr ; $Met_{390}$→Leu ou Ser ; $Val_{392}$→Phe, Glu, Thr ou Ile ; ou une chaîne $\beta_B$ humaine modifiée de manière comparable.

**38.** Cellule suivant l'une quelconque des revendications 34 à 37, qui est une cellule eucaryotique.

**39.** Composition acellulaire qui est dépourvue de polypeptide de chaîne α mature, contenant une séquence polypeptidique de prodomaine de chaîne α d'inhibine humaine ou porcine représentée sur la figure 1B (chaîne α porcine) ou la figure 6A (chaîne α humaine) ou un variant de séquence d'amino-acides engendré par une insertion, délétion ou substitution d'une desdites séquences de prodomaine, variant qui présente une forte homologie avec un polypeptide d'une desdites séquences et qui présente une réactivité croisée avec des anticorps engendrés contre un polypeptide d'une séquence représentée.

**40.** Composition acellulaire contenant

a) un polypeptide comprenant la séquence de prodomaine de chaîne β d'inhibine humaine

HSAAPDCPSCALAALPKDVPNSQPEMVEAVKKHILNMLHL (amino-acides 1 à 40 de la figure 8), PDVTQPVPKAALLNAIRKLHVGKVGENGYVEIEDDIG (aminoacides 44 à 80 de la figure 8),

AEMNELMEQTSEIITFAESGTARKTLHFEISKEGSDLSVVERAEVWLFLKVPKAN-RTRTKVTIRLFQQQKHPQGSLDTGEEAEEVGLKGERSELLLSEKVVDA (amino-acides 83 à 185 de la figure 8),

STWHVFPVSSSIQRLLDQGKSSLDVRIACEQCQESGASLVLLG (amino-acides 188 à 230 de la figure 8), ou un de ses variants de séquence d'amino-acides naturels de mammifère ;

b) un polypeptide comprenant la séquence de prodomaine de chaîne $_B\beta$ d'inhibine humaine CTSCGGFRR-PEELGRVDGDFLEAV (amino-acides) 7 à 30 de la figure 9, (amino-acides 33 à 145 de la figure 9),

VRVKVYFQEQGHGDRWNMVEKRVDLKRSGWHTFPLTEAIQALFERGE (aminoacides 149 à 195 de la figure 9), LNLDVQCDSCQELAVVPVFVDPGEESHRPFVVVQARLGDSRHRI (amino-acides 198 à 241 de la figure 9), ou un de leurs variants de séquence d'amino-acides naturels de mammifère ; ou

c) un polypeptide dépourvu de la séquence d'amino-acides de chaîne α mature comprenant les séquences de prodomaine de chaîne α d'inhibine humaine

KVRALFLDALGPPAVTREGGDPGV (amino-acides 1 à 24 de la figure 6),

(amino-acides 32 à 199 de la figure 6), ou un de leurs variants de séquence d'aminoacides naturels de mammifère.

**41.** Composition acellulaire contenant un polypeptide de prodomaine de chaîne β d'inhibine humaine ou porcine d'une séquence représentée sur la figure 2B (chaînes β porcines) et les figures 8 et 9 (chaînes β humaines) ou un variant de séquence d'amino-acides engendré par insertion, délétion ou substitution d'une desdites séquences de prodomaine, variant qui présente une forte homologie avec un polypeptide d'une desdites séquences et qui présente une réactivité croisée avec des anticorps engendrés contre un polypeptide d'une séquence représentée.

**42.** Composition suivant la revendication 41, dans laquelle la chaîne β est la chaîne $\beta_A$ et la composition est dépourvue de séquence de chaîne $\beta_A$ mature.

**43.** Composition suivant la revendication 41, dans laquelle la chaîne β est la chaîne $\beta_B$ et la composition est dépourvue de séquence de chaîne $\beta_B$ mature.

**44.** Composition suivant la revendication 40, dans laquelle le variant est la séquence d'amino-acides porcine correspondante, composition contenant :

a) un polypeptide comprenant la séquence de prodomaine de chaîne $\beta_A$ d'inhibine porcine représentée par : les amino-acides 28 à 58 ; les amino-acides 61 à 87 ; les aminoacides 90 à 108 ; les amino-acides 111 à 179 ; les aminoacides 182 à 213 ; les amino-acides 216 à 258 ; ou les amino-acides 28 à 87 de la figure 2B ; ou
b) un polypeptide dépourvu de la séquence d'amino-acides de chaîne α mature comprenant les séquences polypeptidiques de prodomaine de chaîne α d'inhibine porcine représentées par : les amino-acides 20 à 54 ; ou les amino-acides 70 à 228 de la figure 1B.

**45.** Composition suivant l'une quelconque des revendications 39 à 44, dans laquelle le polypeptide est non accompagné par une glycosylation naturelle.

**46.** Composition suivant l'une quelconque des revendications 39 à 45, qui est stérile et dans laquelle le polypeptide comprend en outre un polypeptide immunogène.

**47.** Utilisation d'une composition suivant l'une quelconque des revendications 39 à 46 dans la préparation d'un anticorps capable de se lier audit polypeptide.

**48.** Composition suivant l'une quelconque des revendications 39 à 46, dans laquelle le polypeptide est conjugué à un groupe détectable.

**49.** Composition suivant la revendication 48, dans laquelle le groupe est un enzyme, un fluorophore ou un radioisotope.

**50.** Composition suivant l'une quelconque des revendications 39 à 44, 48 et 49, qui est insolubilisée par une absorption non covalente ou une réticulation covalente à un support insoluble dans l'eau.

**51.** Composition suivant l'une quelconque des revendications 39 à 46, 48 et 49, comprenant en outre une matrice implantable physiologiquement acceptable pour la libération contrôlée du polypeptide dans les tissus d'un animal.

**Revendications pour l'Etat contractant suivant : GR**

**1.** Procédé comprenant la culture d'une cellule d'une cellule hôte transformée avec un vecteur qui comprend un acide nucléique codant pour une chaîne α d'inhibine humaine ou porcine et/ou une chaîne β d'inhibine ou porcine dont les séquences d'amino-acides sont représentées sur la figure 1B ((chaîne α porcine), la figure 2B (chaînes β porcines), la figure 6A (chaîne α humaine) et les figures 8 et 9 (chaînes β humaines), ou un variant de séquence d'aminoacides formé par insertion, délétion ou substitution d'une des séquences représentées, le variant étant fortement homologue avec un polypeptide d'une séquence représentée et 1) qui présente une réactivité croisée avec des anticorps engendrés contre un polypeptide d'une séquence représentée ; ou 2) qui présente une activité hormonale similaire à celle d'un polypeptide d'une séquence représentée.

**2.** Procédé suivant la revendication 1, dans lequel l'acide nucléique code pour une chaîne α d'inhibine humaine ou porcine et/ou une chaîne β d'inhibine ou porcine dont les séquences d'amino-acides sont représentées sur la figure 1B (chaîne α porcine), la figure 2B (chaînes β porcines), la figure 6A (chaîne α humaine) et les figures 8 et 9 (chaînes β humaines).

**3.** Procédé suivant la revendication 1, dans lequel le vecteur code pour un variant allélique d'une chaîne α et/ou d'une chaîne β d'inhibine humaine ou porcine.

**4.** Procédé suivant l'une quelconque des revendications précédentes, dans lequel l'acide nucléique codant pour la chaîne α et/ou la chaîne β d'inhibine est liée de manière fonctionnelle à un promoteur reconnu par la cellule hôte et comprenant l'étape supplémentaire consistant à séparer la chaîne α d'inhibine, la chaîne β d'inhibine, l'inhibine ou un dimère de chaîne β du milieu de culture.

**5.** Procédé suivant l'une quelconque des revendications précédentes, dans lequel la cellule est un procaryote.

**6.** Procédé suivant l'une quelconque des revendications précédentes, dans lequel le vecteur comprend un acide nucléique codant pour la forme pré-pro d'une chaîne α d'inhibine ou d'une chaîne β d'inhibine.

**7.** Procédé suivant la revendication 6, dans lequel le vecteur comprend un acide nucléique codant pour les formes pré-pro de la chaîne $\alpha$ d'inhibine et d'une chaîne $\beta$ d'inhibine.

**8.** Procédé suivant l'une quelconque des revendications 1 à 4, 6 et 7, dans lequel la cellule est une cellule provenant d'un organisme pluricellulaire et de l'inhibine douée d'activité hormonale est produite.

**9.** Procédé suivant la revendication 4, dans lequel le promoteur est un promoteur viral.

**10.** Procédé suivant la revendication 9, dans lequel le promoteur est un promoteur de SV40.

**11.** Procédé suivant l'une quelconque des revendications précédentes, dans lequel de l'inhibine porcine ou humaine mature est recueillie.

**12.** Procédé suivant l'une quelconque des revendications 1 à 5 ou 8 à 10, dans lequel le vecteur comprend un acide nucléique codant pour la forme pré-pro d'une chaîne $\beta$ d'inhibine et un dimère de chaîne $\beta$ mature est recueilli en l'absence de chaîne $\alpha$.

**13.** Procédé suivant la revendication 8 ou 11, dans lequel la chaîne $\beta$ et la chaîne $\beta_A$ et l'inhibine est présente à une concentration supérieure à environ 20 ng/ml dans le milieu de culture.

**14.** Procédé suivant la revendication 4, dans lequel l'inhibine est une inhibine humaine ou porcine constituée d'une chaîne $\alpha$ et d'une chaîne $\beta$, les séquences d'amino-acides desdites chaînes $\alpha$ et $\beta$ étant choisies entre celles représentées sur la figure 1B (chaîne $\alpha$ porcine), la figure 2B (chaînes $\beta$ porcines), la figure 6A (chaîne $\alpha$ humaine) et les figures 8 et 9 (chaînes $\beta$ humaines), et des variants de séquence d'amino-acides engendrés par insertion, délétion ou substitution d'un polypeptide d'une desdites séquences représentées, variants qui sont fortement homologues avec un polypeptide d'une séquence représentée, et 1) qui présentent une réactivité croisée avec des anticorps engendrés contre un polypeptide d'une séquence représentée ; ou 2) qui possèdent une activité hormonale similaire à celle d'un polypeptide d'une séquence représentée ; composition qui est totalement dépourvue de protéines humaines ou porcines non identifiées.

**15.** Procédé suivant la revendication 14, dans lequel l'inhibine est une inhibine humaine ou porcine constituée d'une chaîne $\alpha$ et d'une chaîne $\beta$, les séquences d'amino-acides desdites chaînes $\alpha$ et $\beta$ étant choisies entre celles représentées sur la figure 1B (chaîne $\alpha$ porcine), la figure 2B (chaînes $\beta$ porcines), la figure 6A (chaîne $\alpha$ humaine) et les figures 8 et 9 (chaînes $\beta$ humaines).

**16.** Procédé suivant la revendication 4, dans lequel la chaîne d'inhibine recueillie est un prodomaine d'inhibine $\alpha$ ou $\beta_B$ humaine ou porcine représentée sur la figure 1B (chaîne $\alpha$ porcine), la figure 2B (chaînes $\beta_B$ porcines), la figure 6A (chaîne $\alpha$ humaine) et la figure 9 (chaînes $\beta$ humaines), non associé à une glycosylation naturelle.

**17.** Procédé suivant la revendication 4, dans lequel est recueilli un dimère de chaîne $\beta$ qui est un homodimère de chaînes $\beta$ ou $\beta_B$ d'inhibine humaine ou porcine mature, lesdites chaînes étant représentées sur la figure 2B (chaînes $\beta$ porcines) et les figures 8 et 9 (chaînes $\beta$ humaines) ou un variant de séquence d'amino-acides engendré par insertion, délétion ou substitution d'un polypeptide d'une desdites séquences représentées, variant qui présente une forte homologie avec un polypeptide d'une séquence représentée et 1) qui présente une réactivité croisée avec des anticorps engendrés contre un polypeptide d'une séquence représentée ; ou 2) qui présente une activité hormonale similaire à celle d'un polypeptide d'une séquence représentée ; dimère qui est dépourvu de la chaîne $\alpha$ d'inhibine.

**18.** Procédé suivant la revendication 17, dans lequel un homodimère de chaînes $\beta_B$ humaines ou porcines matures ou d'un d'un de leurs variants de séquence d'amino-acides est recueilli.

**19.** Procédé suivant la revendication 4, dans lequel est recueilli un dimère de chaîne $\beta$ qui est un hétérodimère de chaîne $\beta_A$ d'inhibine humaine ou porcine mature avec une chaîne $\beta_B$ d'inhibine ou porcine mature, lesdites chaînes étant représentées sur la figure 2B (chaînes $\beta$ porcines) et les figures 8 et 9 (chaînes $\beta$ humaines) ou un variant de séquence d'amino-acides engendré par insertion, délétion ou substitution d'un polypeptide d'une desdites séquences représentées, variant qui présente une forte homologie avec un polypeptide d'une séquence représentée et 1) qui présente une réactivité croisée avec des anticorps engendrés contre un polypeptide d'une séquence représentée ; ou .2) qui présente une activité hormonale similaire à celle d'un polypeptide d'une séquence représentée ; dimère qui est dépourvu de la chaîne $\alpha$ d'inhibine.

**20.** ADN non chromosomique codant pour une chaîne α d'inhibine humaine ou porcine ou une chaîne β d'inhibine humaine ou porcine, dont les séquences d'amino-acides sont représentées sur la figure 1B (chaîne α porcine), la figure 2B (chaînes β porcines), la figure 6A (chaîne α humaine) et les figures 8 et 9 (chaînes β humaines) ou un variant de séquence d'amino-acides engendré par insertion, délétion ou substitution d'un polypeptide d'une desdites séquences représentées, variant qui présente une forte homologie avec un polypeptide d'une séquence représentée et 1) qui présente une réactivité croisée avec des anticorps engendrés contre un polypeptide d'une séquence représentée ; ou 2) qui présente une activité hormonale similaire à celle d'un polypeptide d'une séquence représentée.

**21.** ADN suivant la revendication 20, codant pour une chaîne α d'inhibine humaine ou porcine ou une chaîne β d'inhibine humaine ou porcine, dont la séquence d'amino-acides est représentée sur la figure 1B (chaîne α porcine), la figure 2B (chaînes β porcines), la figure 6A (chaîne α humaine) et les figures 8 et 9 (chaînes β humaines).

**22.** ADN suivant la revendication 20 ou 21, qui est dépourvue de séquences non traduites intermédiaires.

**23.** ADN suivant l'une quelconque des revendications 20 à 22, qui est marqué avec un groupement détectable.

**24.** Vecteur réplicable comprenant un ADN suivant la revendication 20 ou 21.

**25.** Vecteur suivant la revendication 24, comprenant un promoteur viral lié de manière fonctionnelle à l'ADN codant pour les chaînes α et/ou β d'inhibine.

**26.** Vecteur suivant la revendication 24 ou 25, qui contient un ADN codant à la fois pour une chaîne α d'inhibine et une chaîne β d'inhibine.

**27.** Vecteur suivant la revendication 24 ou 25, qui contient un ADN codant pour une chaîne β d'inhibine, mais non pour la chaîne α d'inhibine.

**28.** Cellule hôte transformée avec un vecteur réplicable comprenant un ADN codant pour la chaîne α d'inhibine humaine ou porcine et/ou une chaîne β d'inhibine humaine ou porcine, dont les séquences d'amino-acides sont représentées sur la figure 1B (chaîne α porcine), la figure 2B (chaînes β porcines), la figure 6A (chaîne α humaine) et les figures 8 et 9 (chaînes β humaines) ou un variant de séquence d'amino-acides engendré par insertion, délétion ou substitution d'un polypeptide d'une desdites séquences représentées, variant qui présente une forte homologie avec un polypeptide d'une séquence représentée et 1) qui présente une réactivité croisée avec des anticorps engendrés contre un polypeptide d'une séquence représentée ; ou 2) qui présente une activité hormonale similaire à celle d'une séquence représentée.

**29.** Cellule hôte suivant la revendication 28, dans laquelle l'ADN code pour une chaîne α et/ou une chaîne β d'inhibine humaine ou porcine, dont les séquences d'aminoacides sont représentées sur la figure 1B (α porcine), la figure 2B (chaînes β porcines), la figure 6A (chaîne α humaine) et les figures 8 et 9 (chaînes β humaines).

**30.** Cellule suivant la revendication 28 ou 29, qui est une cellule eucaryotique.

**31.** Procédé suivant la revendication 4, dans lequel la chaîne d'inhibine recueillie est une composition cellulaire, dépourvue de séquence de chaîne α mature, contenant une séquence polypeptidique de prodomaine de chaîne α d'inhibine humaine ou porcine représentée sur la figure 1B (α porcine) ou la figure 6A (chaîne α humaine) ou un variant de séquence d'amino-acides engendré par insertion, délétion ou substitution d'une desdites séquences de prodomaine, variant qui présente une forte homologie avec un polypeptide d'une desdites séquences représentées et qui présente une réactivité croisée avec des anticorps engendrés contre un polypeptide d'une séquence représentée.

**32.** Procédé suivant la revendication 4, dans lequel la chaîne d'inhibine recueillie est une composition acellulaire contenant

a) un polypeptide comprenant la séquence de prodomaine de chaîne $\beta_A$ d'inhibine humaine

HSAAPDCPSCALAALPKDVPNSQPEMVEAVKKHILNMLHL (aminoacides 1 à 40 de la figure 8), PDVTQPVPKAALLNAIRKLHVGKVGENGYVEIEDDIG (aminoacides 44 à 80 de la figure 8),

AEMNELMEQTSEIITFAESGTARKTLHFEISKEGSDLSVVERAEVWLFLKVPKAN-RTRTKVTIRLFQQQKHPQGSLDTGEEAEEVGLKGERSELLLSEKVVDA (aminoacides 83 à 185 de la figure 8),

STWHVFPVSSSIQRLLDQGKSSLDVRIACEQCQESGASLVLLG (amino-acides 188 à 230 de la figure 8), ou un de ses variants de séquence d'amino-acides naturels de mammifère ;
b) un polypeptide comprenant la séquence de prodomaine de chaîne $\beta_B$ d'inhibine humaine CTSCGGFRRPEELGRVDGDFLEAV (amino-acides) 7 à 30 de la figure 9, (amino-acides 33-145 de la figure 9),

VRVKVYFQEQGHGDRWNMVEKRVDLKRSGWHTFPLTEAIQALFERGE (amino-acides 149 à 195 de la figure 9), LNLDVQCDSCQELAVVPVFVDPGEESHRPFVVVQARLGDSRHRI (amino-acides 198 à 241 de la figure 9), ou un de leurs variants de séquence d'amino-acides naturels de mammifère ; ou
c) un polypeptide dépourvu de la séquence d'amino-acides de chaînes $\alpha$ mature comprenant les séquences de prodomaine de chaîne $\alpha$ d'inhibine humaine

KVRALFLDALGPPAVTREGGDPGV (amino-acides 1 à 24 de la figure 6),

(amino-acides 32 à 199 de la figure 6), ou un de leurs variants de séquence d'amino-acides naturels de mammifère.

**33.** Procédé suivant la revendication 4, dans lequel la chaîne d'inhibine recueillie est une composition acellulaire contenant un polypeptide de prodomaine de chaîne $\beta$ d'inhibine humaine porcine d'une séquence représentée sur la figure 2B (chaînes $\beta$ porcines) et les figures 8 et 9 (chaînes $\beta$ humaines) ou un variant de séquence d'amino-acides engendré par insertion, délétion ou substitution d'une desdites séquences polypeptidiques de prodomaine, variant qui présente une forte homologie avec un polypeptide d'une desdites séquences et qui présente une réactivité croisée avec des anticorps engendrés contre un polypeptide d'une séquence représentée.

**34.** Procédé suivant la revendication 33, dans lequel la chaîne $\beta$ est la chaîne $\beta_A$ et la composition est dépourvue de séquence de chaîne $\beta_A$ mature.

**35.** Procédé suivant la revendication 33, dans lequel la chaîne $\beta$ est la chaîne $\beta_B$ et la composition est dépourvue de séquence de chaîne $\beta_B$ mature.

**36.** Procédé suivant la revendication 32, dans lequel le variant est la séquence d'amino-acides porcine correspondante, la composition contenant :

a) un polypeptide comprenant la séquence de prodomaine de chaîne $\beta_A$ d'inhibine porcine représentée par :

des amino-acides 28 à 58 ; les amino-acides 61 à 87 ; les aminoacides 90 à 108 ; les amino-acides 111 à 179 ; les aminoacides 182 à 213 ; les amino-acides 216 à 258 ou les aminoacides 28 à 87 de la figure 2B ; ou b) un polypeptide dépourvu de la séquence d'amino-acides de chaîne α mature, comprenant les séquences polypeptidiques de prodomaine de chaîne α d'inhibine porcine représentées par : les amino-acides 20 à 54 ; ou les amino-acides 70 à 228 de la figure 1B.

**37.** Procédé suivant l'une quelconque des revendications 31 à 36, dans lequel le polypeptide est non accompagné par une glycosylation naturelle.

**38.** Procédé suivant l'une quelconque des revendications 31 à 37, dans lequel la composition est stérile, et comprend en outre le couplage du polypeptide à un polypeptide immunogène.

**39.** Utilisation d'une composition suivant l'une quelconque des revendications 31 à 38 dans la préparation d'un anticorps capable de se lier au polypeptide.

**40.** Procédé suivant l'une quelconque des revendications 31 à 38, comprenant en outre la conjugaison du polypeptide à un groupe détectable.

**41.** Procédé suivant la revendication 40, dans lequel le groupe est un enzyme, un fluorophore ou un radioisotope.

**42.** Procédé suivant l'une quelconque des revendications 31 à 38, 40 et 41, dans lequel la composition est insolubilisée par une absorption non covalente ou une réticulation covalente à un support insoluble dans l'eau.

**43.** Procédé suivant l'une quelconque des revendications 31 à 38 ou 40 à 41, comprenant en outre le mélange de la composition à une matrice implantable physiologiquement acceptable pour la libération contrôlée du polypeptide dans les tissus d'un animal.

**Revendications pour les Etats contractants suivants : ES, AT**

**1.** Procédé comprenant la culture d'une cellule hôte transformée avec un vecteur qui renferme un acide nucléique codant pour une chaîne α d'inhibine humaine ou porcine et/ou pour une chaîne β d'inhibine humaine ou porcine dont les séquences d'aminoacides sont représentées sur la figure 1B (chaîne α porcine), la figure 2B (chaînes β porcines), la figure 6A (chaîne α humaine) et les figures 8 et 9 (chaînes β humaines), ou un variant de séquence d'aminoacides formé par insertion, délétion ou substitution d'une des séquences représentées, le variant étant fortement homologue avec un polypeptide d'une séquence représentée et 1) qui présente une activité croisée avec les anticorps engendrés contre un polypeptide d'une séquence représentée ; ou 2) qui présente une activité hormonale similaire à celle d'un polypeptide d'une séquence représentée.

**2.** Procédé suivant la revendication 1, dans lequel l'acide nucléique code pour une chaîne α d'inhibine humaine ou porcine et/ou une chaîne β d'inhibine humaine ou porcine dont les séquences d'amino-acides sont représentées sur la figure 1B (chaîne α porcine), la figure 2B (chaînes β porcines), la figure 6A (chaîne α humaine) et les figures 8 et 9 (chaînes β humaines).

**3.** Procédé suivant la revendication 1, dans lequel le vecteur code pour un variant allélique de chaîne α et/ou de chaîne β d'inhibine humaine ou porcine.

**4.** Procédé suivant l'une quelconque des revendications précédentes, dans lequel l'acide nucléique codant pour la chaîne α et/ou la chaîne β d'inhibine est lié de manière fonctionnelle à un promoteur reconnu par la cellule hôte, et comprenant l'étape supplémentaire de séparation de la chaîne α d'inhibine, de la chaîne β d'inhibine, de l'inhibine ou d'un dimère de chaîna β du milieu de culture.

**5.** Procédé suivant l'une quelconque des revendications précédentes, dans lequel la cellule est un procaryote.

**6.** Procédé suivant l'une quelconque des revendications précédentes, dans lequel le vecteur comprend un acide nucléique codant pour la forme pré-pro d'une chaîne α d'inhibine ou d'une chaîne β d'inhibine.

**7.** Procédé suivant la revendication 6, dans lequel le vecteur comprend un acide nucléique codant pour les formes pré-pro de la chaîne α d'inhibine et d'une chaîne β d'inhibine.

**8.** Procédé suivant l'une quelconque des revendications 1 à 4, 6 et 7, dans lequel la cellule est une cellule provenant d'un organisme pluricellulaire et une inhibine douée d'activité hormonale est produite.

**9.** Procédé suivant la revendication 4, dans lequel le promoteur est un promoteur viral.

**10.** Procédé suivant la revendication 9, dans lequel le promoteur est un promoteur de SV40.

**11.** Procédé suivant l'une quelconque des revendications précédentes, dans lequel une inhibine porcine humaine mature est recueillie.

**12.** Procédé suivant l'une quelconque des revendications 1 à 5 ou 8 à 10, dans lequel le vecteur comprend un acide nucléique codant pour la forme pré-pro d'une chaîne β d'inhibine et un dimère de chaîne β mature est recueilli en l'absence de chaîne α.

**13.** Procédé suivant la revendication 8 ou 11, dans lequel la chaîne β est la chaîne $\beta_A$ et l'inhibine est présente à une concentration supérieure à environ 20 ng/ml dans le milieu de culture.

**14.** Procédé suivant la revendication 4, dans lequel l'inhibine est une inhibine humaine ou porcine constituée d'une chaîne α et d'une chaîne β, les séquences d'amino-acides desdites chaînes α et β étant choisies entre celles représentées sur la figure 1B (chaîne α porcine), la figure 2B (chaînes β porcines), la figure 6A (chaîne α humaine), et les figures 8 et 9 (chaînes β humaines), et les variants de séquence d'amino-acides engendrés par insertion, délétion ou substitution d'un polypeptide d'une desdites séquences représentées, variants qui présentent une forte homologie avec un polypeptide d'une séquence représentée et 1) qui présentent une réactivité croisée avec des anticorps engendrés contre un polypeptide d'une séquence représentée ; ou 2) qui présentent une activité hormonale similaire à celle d'un polypeptide d'une séquence représentée ; composition qui est totalement dépourvue de protéines humaines ou porcines identifiées.

**15.** Procédé suivant la revendication 14, dans lequel l'inhibine est une inhibine humaine ou porcine constituée d'une chaîne α et d'une chaîne β, les séquences d'aminoacides desdites chaînes α et β étant choisies entre celles représentées sur la figure 1B (chaîne α porcine), la figure 2B (chaînes β porcines), la figure 6A (chaîne α humaine) et les figures 8 et 9 (chaînes β humaines).

**16.** Procédé suivant la revendication 4, dans lequel la chaîne d'inhibine recueillie est un prodomaine d'inhibine α ou $\beta_B$ humaine ou porcine représentée sur la figure 1B (chaîne α porcine), la figure 2B (chaîne $\beta_B$ porcine), la figure 6A (chaîne α humaine) et la figure 9 (chaîne $\beta_B$ humaine) non associé et à une glycosylation naturelle.

**17.** Procédé suivant la revendication 4, dans lequel est recueilli un dimère de chaîne β qui est un homodimère de chaîne $\beta_A$ ou $\beta_B$ d'inhibine humaine ou porcine mature, lesdites chaînes étant représentées sur la figure 2B (chaînes β porcines) et les figures 8 et 9 (chaînes β humaines) ou un variant de séquence d'amino-acides engendré par insertion, délétion ou substitution d'un polypeptide d'une desdites séquences représentées, variant qui présente une forte homologie avec un polypeptide d'une séquence représentée et 1) qui présente une réactivité croisée avec des anticorps engendrés contre un polypeptide d'une séquence représentée ; ou 2) qui présente une activité hormonale similaire à celle d'un polypeptide d'une séquence représentée ; dimère qui est dépourvu de la chaîne α d'inhibine.

**18.** Procédé suivant la revendication 17, dans lequel un homodimère de chaînes $\beta_B$ humaines ou porcines matures ou un de ses variants de séquence d'amino-acides est recueilli.

**19.** Procédé suivant la revendication 4, dans lequel est recueilli un dimère de chaîne β qui est un hétérodimère de chaîne $\beta_A$ d'inhibine humaine ou porcine mature avec la chaîne $\beta_B$ d'inhibine humaine ou porcine mature, lesdites chaînes étant présentées sur la figure 2B (chaînes β porcines) et les figures 8 et 9 (chaînes β humaines) ou un variant de séquence d'aminoacides engendré par insertion, délétion ou substitution d'un polypeptide d'une desdites séquences représentées, variant qui présente une forte homologie avec un polypeptide d'une séquence représentée et 1) qui présente une réactivité croisée avec des anticorps engendrés contre un polypeptide d'une séquence représentée ; 2) qui présente une réactivité croisée avec des récepteurs de surface cellulaire pour un polypeptide de séquence représenté ; ou 3) qui présente une activité hormonale similaire à celle d'un polypeptide d'une séquence représentée ; homodimère qui est dépourvu de la chaîne α d'inhibine.

**20.** Procédé de production d'un ADN non chromosomique codant pour une chaîne α d'inhibine humaine ou porcine ou une chaîne β d'inhibine humaine ou porcine représentée sur la figure 1B (chaîne α porcine), la figure 2B (chaînes β porcines), la figure 6A (chaîne α humaine) et les figures 8 et 9 (chaînes β humaines) ou un variant de séquence d'amino-acides engendré par insertion, délétion ou substitution d'un polypeptide d'une desdites séquences représentées, variant qui présente une forte homologie avec un polypeptide d'une séquence représentée et 1) qui présente une réactivité croisée avec des anticorps engendrés contre un polypeptide d'une séquence représentée ; ou 2) qui présente une activité hormonale similaire à celle d'un polypeptide d'une séquence représentée, procédé comprenant une synthèse chimique, une sélection d'ARNm d'ovaires ou une sélection de banques génomiques de n'importe quelle cellule.

**21.** Procédé suivant la revendication 20, dans lequel l'ADN code pour une chaîne α d'inhibine humaine ou porcine ou une chaîne β d'inhibine humaine ou porcine dont la séquence d'amino-acides est représentée sur la figure 1B (chaîne α porcine), la figure 2B (chaînes β porcine), la figure 6A (chaîne α humaine) ou la figure 8 ou 9 (chaînes β humaines).

**22.** Procédé suivant la revendication 20 ou 21, dans lequel l'ADN non chromosomique est dépourvu de séquences non traduites intermédiaires.

**23.** Procédé suivant l'une quelconque des revendications 1 à 22, comprenant en outre l'étape de marquage de l'ADN non chromosomique avec un groupement détectable.

**24.** Procédé de production d'un vecteur réplicable, procédé comprenant l'insertion d'un ADN suivant la revendication 20 ou 21 dans un vecteur de clonage.

**25.** Procédé suivant la revendication 24, dans lequel l'ADN est inséré dans un vecteur qui possède un promoteur viral lié de manière fonctionnelle à l'ADN codant pour la chaîne α et/ou la chaîne β d'inhibine afin de produire ainsi un vecteur d'expression.

**26.** Procédé suivant la revendication 24 ou 25, dans lequel un ADN codant à la fois pour une chaîne α d'inhibine et une chaîne β d'inhibine est insérée dans le vecteur.

**27.** Procédé suivant la revendication 24 ou 25, dans lequel un ADN codant pour une chaîne β d'inhibine mais non pour la chaîne α d'inhibine est inséré dans le vecteur.

**28.** Cellule hôte transformée avec un vecteur réplicable comprenant un ADN codant pour la chaîne α d'inhibine humaine ou porcine et/ou une chaîne β d'inhibine dont les séquences d'amino-acides sont représentées sur la figure 1B (chaîne α porcine), la figure 2B (chaînes β porcines), la figure 6A (chaîne α humaine) et les figures 8 et 9 (chaînes β humaines) ou un variant de séquence d'amino-acides engendré par insertion, délétion ou substitution d'un polypeptide d'une desdites séquences représentées, variant qui présente une forte homologie avec un polypeptide d'une séquence représentée et 1) qui présente une réactivité croisée avec des anticorps engendrés contre un polypeptide d'une séquence représentée ; ou 2) qui présente une activité hormonale similaire à celle d'un polypeptide dune séquence représentée.

**29.** Cellule hôte suivant la revendication 28, dans laquelle l'ADN code pour une chaîne α d'inhibine et/ou une chaîne β d'inhibine humaine ou porcine, dont les séquences d'amino-acides sont représentées sur la figure 1B (chaîne α porcine), la figure 2B (chaînes β porcines), la figure 6A (chaîne α humaine) et les figures 8 et 9 (chaînes β humaines).

**30.** Cellule suivant la revendication 28 ou 29, qui est une cellule eucaryotique.

**31.** Procédé suivant la revendicatioin 4, dans lequel la chaîne inhibine recueillie est une composition acellulaire dépourvue de séquences de chaîne α mature, contenant une séquence polypeptidique de prodomaine de chaîne α d'inhine humaine ou porcine représentée sur la figure 1B (chaîne α porcine) ou la figure 6A (chaîne α humaine) ou un variant de séquence d'amino-acides d'une desdites séquences de prodomaine, variant qui présente une forte homologie avec un polypeptide d'une desdites séquences et qui présente une réactivité croisée avec des anticorps engendrés contre un polypeptide d'une séquence représentée.

**32.** Procédé suivant la revendicatioin 4, dans lequel la chaîne d'inhibine recueillie est une composition acellulaire contenant

a) un polypeptide comprenant la séquence de prodomaine de chaîne $\beta_A$ d'inhibine humaine

HSAAPDCPSCALAALPKDVPNSQPEMVEAVKKHILNMLHL (amino-acides 1 à 40 de la figure 8), PDVTQPVPKAALLNAIRKLHVGKVGENGYVEIEDDIG (aminoacides 44 à 80 de la figure 8),

AEMNELMEQTSEIITFAESGTARKTLHFEISKEGSDLSVVERAEVWLFLKVPKAN-RTRTKVTIRLFQQQKHPQGSLDTGEEAEEVGLKGERSELLLSEKVVDA (amino-acides 83 à 185 de la figure 8),

STWHVFPVSSSIQRLLDQGKSSLDVRIACEQCQESGASLVLLG (amino-acides 188 à 230 de la figure 8), ou un de ses variants de séquence d'amino-acides naturels de mammifère ;
b) un polypeptide comprenant la séquence de prodomaine de chaîne $\beta_B$ d'inhibine humaine CTSCGGFRRPEELGRVDGDFLEAV (aminoacides) 7 à 30 de la figure 9, (amino-acides 33 à 145 de la figure 9),

VRVKVYFQEQGHGDRWNMVEKRVDLKRSGWHTFPLTEAIQALFERGE (amino-acides 149 à 195 de la figure 9), LNLDVQCDSCQELAVVPVFVDPGEESHRPFVVVQARLGDSRHRI (amino-acides 198 à 241 de la figure 9), ou un de leurs variants de séquence d'amino-acides naturels de mammifère ; ou
c) un polypeptide dépourvu de la séquence d'amino-acides de chaînes $\alpha$ mature comprenant les séquences de prodomaine de chaîne $\alpha$ d'inhibine humaine

KVRALFLDALGPPAVTREGGDPGV (amino-acides 1 à 24 de la figure 6),

(amino-acides 32 à 199 de la figure 6), ou un de leurs variants de séquence d'amino-acides naturels de mammifère.

**33.** Procédé suivant la revendication 4, dans lequel la chaîne d'inhibine recueillie est une composition acellulaire contenant un polypeptide de prodomaine de chaîne $\beta$ d'inhibine humaine porcine d'une séquence représentée sur la figure 2B (chaînes $\beta$ porcines) et les figures 8 et 9 (chaînes $\beta$ humaines) ou un variant de séquence d'amino-acides engendré par insertion, délétion ou substitution d'une desdites séquences polypeptidiques de prodomaine, variant qui présente une forte homologie avec un polypeptide d'une desdites séquences et qui présente une réactivité croisée avec des anticorps engendrés contre un polypeptide d'une séquence représentée.

**34.** Procédé suivant la revendication 33, dans lequel la chaîne $\beta$ est la chaîne $\beta_A$ et la composition est dépourvue de séquence de chaîne $\alpha_A$ mature.

**35.** Procédé suivant la revendication 33, dans lequel la chaîne $\beta$ est la chaîne $\beta_B$ et la composition est dépourvue de

séquence de chaîne $\beta_B$ mature.

**36.** Procédé suivant la revendication 32, dans lequel le variant est la séquence d'amino-acides porcine correspondante, la composition contenant :

a) un polypeptide comprenant la séquence de prodomaine de chaîne $\beta_A$ d'inhibine porcine représentée par : les amino-acides 28 à 58 ; les amino-acides 61 à 87 ; les aminoacides 90 à 108 ; les amino-acides 111 à 179 ; les aminoacides 182 à 213 ; les amino-acides 216 à 258 ou les aminoacides 28 à 87 de la figure 2B ; ou
b) un polypeptide dépourvu de la séquence d'amino-acides de chaîne $\alpha$ mature, comprenant les séquences polypeptidiques de prodomaine de chaîne $\alpha$ d'inhibine porcine représentées par : les amino-acides 20 à 54 ; ou les amino-acides 70 à 228 de la figure 1B.

**37.** Procédé suivant l'une quelconque des revendications 31 à 36, dans lequel le polypeptide est non accompagné par une glycosylation naturelle.

**38.** Procédé suivant l'une quelconque des revendications 31 à 37, dans lequel la composition est stérile, et comprenant en outre le couplage du polypeptide à un polypeptide immunogène.

**39.** Utilisation d'une composition suivant l'une quelconque des revendications 31 à 38 dans la préparation d'un anticorps capable de se lier au polypeptide.

**40.** Procédé suivant l'une quelconque des revendications 31 à 38, comprenant en outre la conjugaison du polypeptide à un groupe détectable.

**41.** Procédé suivant la revendication 40, dans lequel le groupe est un enzyme, un fluorophore ou un radioisotope.

**42.** Procédé suivant l'une quelconque des revendications 31 à 38, 40 et 41, dans lequel la composition est insolubilisée par une absorption non covalente ou une réticulation covalente à un support insoluble dans l'eau.

**43.** Procédé suivant l'une quelconque des revendications 31 à 38 ou 40 à 41, comprenant en outre le mélange de la composition à une matrice implantable physiologiquement acceptable pour la libération contrôlée du polypeptide dans les tissus d'un animal.

Fig.1A.

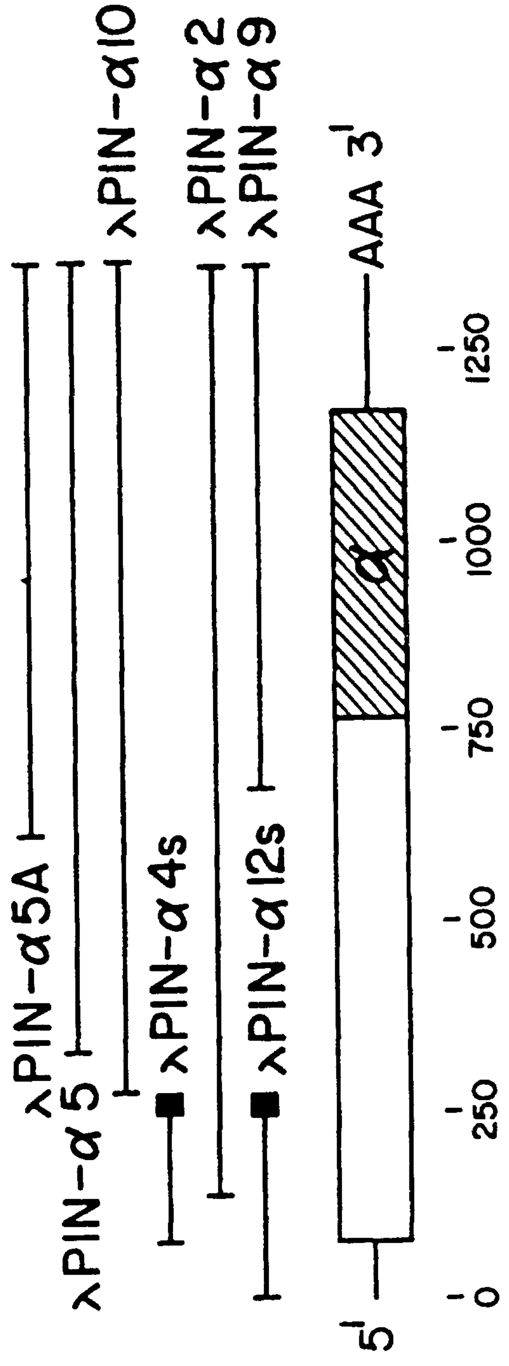
λPIN-α5A
λPIN-α5
λPIN-α10
λPIN-α4s
λPIN-α2
λPIN-α12s
λPIN-α9
5'
α
AAA 3'
0
250
500
750
1000
1250

# Fig. 1B(I)

```
                                                                                 1                                      10
                                                                                met trp pro gln leu leu leu leu leu leu ala pro
  1 TGTGGGGCAGACCCTGACAGAAGGGGCACAGGGCTGGGTGTGGGTTCACCGTTGGCAGGGCCAGGTGAGCT ATG TGG CCT CAG CTG CTC CTC TTG CTG TTG GCC CCA

                          20                                      30                                      40
    arg ser gly his gly cys gln gly pro glu leu asp arg glu leu val leu ala lys val arg ala leu phe leu asp ala leu gly pro
108 CGG AGT GGG CAT GGC TGC CAG GGC CCG GAG CTG GAC CGG GAG CTT GTC CTG GCC AAG GTG AGG GCT CTG TTC CTG GAT GCC TTG GGA CCC

                          50                                                                              70
    pro ala val thr gly glu gly gly asp pro gly val arg arg leu pro arg arg his ala val gly gly phe met arg arg gly ser glu
198 CCG GCA GTG ACT GGG GAA GGT GGG GAT CCT GGA GTC AGG CGT CTG CCC CGA AGA CAT GCT GTG GGG GGC TTC ATG CGC AGG GGC TCT GAG

                          80                                      90                                      100
    pro glu glu glu asp val ser gln ala ile leu phe pro ala thr gly ala arg cys gly asp glu pro ala ala gly glu leu ala arg
288 CCC GAG GAG GAG GAT GTC TCC CAG GCC ATC CTT TTC CCG GCT ACA GGT GCC CGC TGT GGG GAC GAG CCA GCT GCT GGA GAG CTG GCC CGG

                          110                                     120                                     130
    glu ala glu glu gly leu phe thr tyr val phe arg pro ser gln his thr his ser arg gln val thr ser ala gln leu trp phe his
378 GAG GCT GAG GAG GGC CTC TTC ACA TAT GTA TTC CGG CCG TCC CAG CAC ACA CAC AGC CGC CAG GTG ACT TCA GCT CAG CTG TGG TTC CAC

                          140                                     150                                     160
    thr gly leu asp arg gln gly met ala ala ala asn ser ser gly pro leu leu asp leu leu ala leu ser ser arg gly pro val ala
468 ACG GGA CTG GAC AGA CAG GGG ATG GCA GCC GCC AAT AGC TCT GGG CCC CTG CTG GAC CTG CTG GCA CTA TCA TCC AGG GGT CCT GTG GCT

                          170                                     180                                     190
    val pro met ser leu gly gln ala pro pro arg trp ala val leu his leu ala ala ser ala leu pro leu leu thr his pro val leu
558 GTG CCC ATG TCA CTG GGC CAG GCG CCC CCT CGC TGG GCT GTG CTG CAC CTG GCC GCC TCT GCC CTC CCT TTG TTG ACC CAC CCA GTC CTG
```

# Fig. 1B(II)

```
                                    200                                               210                                                  220
    val leu leu leu arg cys pro leu cys ser cys ser ala arg pro glu ala thr pro phe leu val ala his thr arg ala arg pro pro
648 GTG CTG CTG CTG CGC TGT CCT CTC TGT TCC TGC TCA GCC CGG CCC GAG GCC ACC CCC TTC CTG GTG GCC CAC ACT CGG GCC AGG CCA CCC
                                        α subunit                         240                                             250
    ser gly gly glu arg ala arg arg ser thr ala pro leu pro trp pro trp ser pro ala ala leu arg leu leu gln arg pro pro glu
738 AGC GGA GGG GAG AGG GCC CGA CGC TCC ACC GCC CCT CTG CCC TGG CCT TGG TCC CCC GCC GCG CTG CGC CTG CTG CAG AGG CCC CCG GAG

                                    260                                               270                                             280
    glu pro ala val his ala asp cys his arg ala ser leu asn ile ser phe gln glu leu gly trp asp arg trp ile val his pro pro
828 GAA CCC GCT GTG CAC GCC GAC TGC CAC AGA GCT TCC CTC AAC ATC TCC TTC CAG GAG CTG GGC TGG GAC CGG TGG ATC GTG CAC CCT CCC

                                    290                                               300                                             310
    ser phe ile phe his tyr cys his gly gly cys gly leu pro thr leu pro asn leu pro leu ser val pro gly ala pro pro thr pro
918 AGT TTC ATC TTC CAC TAC TGT CAC GGG GGC TGC GGG CTG CCG ACC CTG CCC AAC CTG CCC CTG TCT GTC CCT GGG GCC CCC CCT ACC CCT

                                    320                                               330                                             340
     val gln pro leu leu leu val pro gly ala gln pro cys cys ala ala leu pro gly thr met arg ser leu arg val arg thr thr ser
1008 GTC CAG CCC CTG TTG TTG GTG CCA GGG GCT CAG CCC TGC TGC GCT GCT CTC CCG GGG ACC ATG AGG TCC CTA CGC GTT CGC ACC ACC TCG

                                    350                                               360         364
     asp gly gly tyr ser phe lys tyr glu thr val pro asn leu leu thr gln his cys ala cys ile OC
1098 GAT GGA GGT TAC TCT TTC AAG TAC GAG ACG GTG CCC AAC CTT CTC ACC CAG CAC TGT GCC TGC ATC TAA GGGTGTCCCGCTGGTGGCCGAGCTCCC

1194 ACAGGCACCAGCCTGGAGGAAGGCAGAGTTCCCACCTCCCCTTTCCTTCCGCCTCTCCGCCTGGAGGCTCCCCTCCCTGTCCGCCCCTGTCCCATGGGTAATGTGACAATAAACAGCAT

1312 AGTGCAGATGACTCGGTGCGCAAAAAAAAAA
```

Fig.2A.

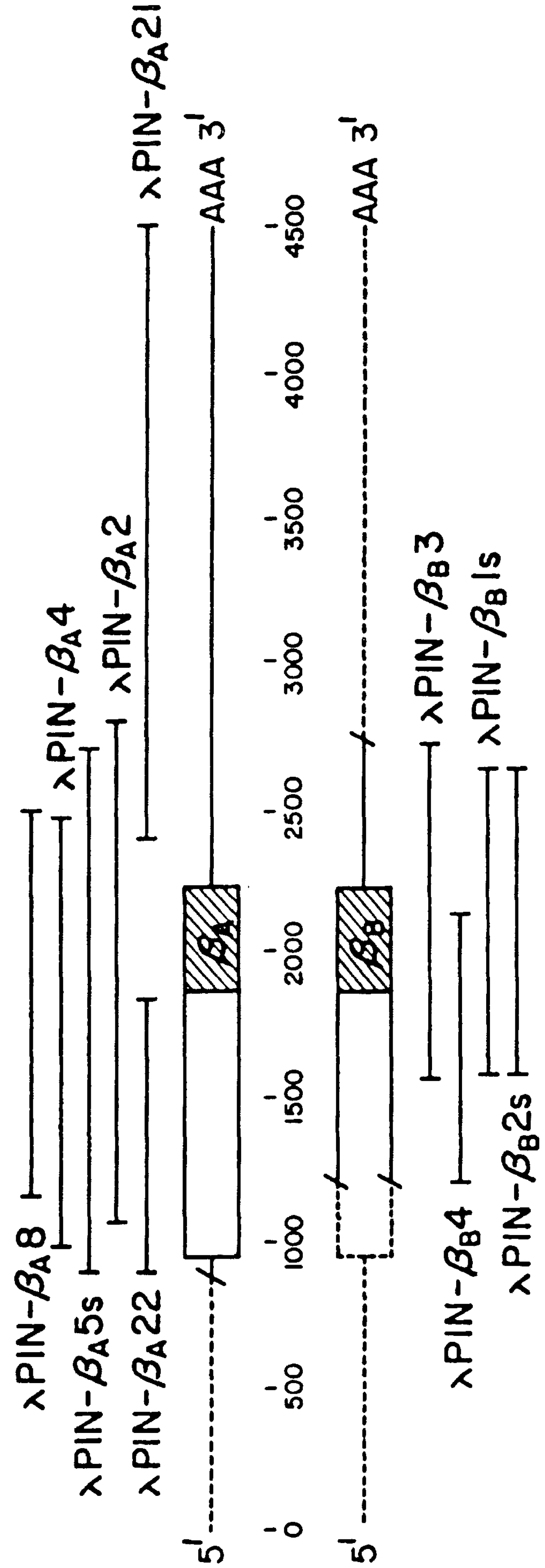
λPIN-βA8
λPIN-βA5s
λPIN-βA22
λPIN-βA4
λPIN-βA2
λPIN-βA21
5'
AAA 3'
βA
βB
0
500
1000
1500
2000
2500
3000
3500
4000
4500
λPIN-βB4
λPIN-βB2s
λPIN-βB3
λPIN-βB1s

# Fig. 2B(I)

```
                                       1                                       10                                      20
                                       met pro leu leu trp leu arg gly phe leu leu ala ser cys trp ile ile val arg ser ser
  1 AAAAGGGCCGTCACCACAACTTTGGCTGCCAGG   ATG CCC TTG CTT TGG CTG AGA GGA TTT TTG TTG GCG AGT TGC TGG ATT ATA GTG AGG AGT TCC

                                    30                                      40                                      50
    pro thr pro gly ser gly gly his ser ala ala pro asp cys pro ser cys ala leu ala thr leu pro lys asp val pro asn ser gln
 97 CCC ACC CCA GGA TCC GGG GGG CAC AGC GCA GCC CCG GAC TGC CCG TCC TGT GCG CTG GCC ACC CTC CCA AAG GAT GTA CCC AAC TCT CAG
                                                                    CGG GCG GCG GGG GCG GAG GAG GAG CTG GGC CGG CTG GAC
                                                                    arg ala ala gly ala glu glu glu leu gly arg leu asp

                                                                                                                   80
    pro glu met val glu ala val lys lys his ile leu asn met leu his leu lys lys arg pro asp val thr gln pro val pro lys ala
187 CCG GAG ATG GTG GAA GCC GTC AAG AAG CAC ATT TTA AAC ATG CTG CAC TTG AAG AAG AGA CCC GAT GTC ACC CAG CCG GTA CCC AAG GCG
    GGC GAC TTC CTG GAG GCG GTG AAG CGC CAC ATC TTG AAC CGC CTG CAG ATG CGG GGC CGA CCC AAC ATC ACC CAT GCC GTG CCC AAG GCC
    gly asp phe leu glu ala val lys arg his ile leu asn arg leu gln met arg gly arg pro asn ile thr his ala val pro lys ala

                                    90                                      100
    ala leu leu asn ala ile arg lys leu his val gly lys val gly glu asn gly tyr val glu leu glu asp asp ile gly arg arg ala
277 GCG CTT CTG AAC GCG ATC AGA AAG CTT CAT GTG GGC AAA GTG GGG GAG AAC GGG TAC GTG GAG CTG GAG GAC GAC ATC GGG AGG AGG GCG
    GCC ATG GTC ACG GCC CTG CGC AAA CTA CAT GCG GGC AAG GTG CGC GAG GAC GGC CGG GTG GAG ATC CCG CAC CTG GAC GGC CAC GCC AGC
    ala met val thr ala leu arg lys leu his ala gly lys val arg glu asp gly arg val glu ile pro his leu asp gly his ala ser

                                    120                                     130
    glu met asn glu leu met glu gln thr ser glu ile ile thr phe ala glu ala     gly thr ala         arg lys thr leu arg phe
367 GAA ATG AAT GAA CTC ATG GAG CAG ACC TCG GAG ATC ATC ACC TTC GCG GAA GCA --- GGC ACC GCC --- --- AGG AAG ACG CTG CGC TTT
    CCT GGC GCC GAC GGC CAA GAG CGG GTC TCC GAG ATC ATC AGC TTC GCA GAG ACA GAT GGC CTC GCC TCC TCC CGG GTC CGC CTG TAC TTC
    pro gly ala asp gly gln glu arg val ser glu ile ile ser phe ala glu thr asp gly leu ala ser ser arg val arg leu tyr phe
```

# Fig.2B(II)

```
         140                                          150                                                   160
     glu ile ser lys glu gly ser asp leu ser val val glu arg ala glu ile trp leu phe leu lys val pro lys ala asn arg thr arg
448  GAG ATC TCC AAA GAG GGC AGT GAC CTG TCC GTG GTG GAG CGC GCC GAA ATC TGG CTC TTC CTG AAA GTC CCC AAG GCC AAC CGG ACC AGG
     TTC ATC TCC AAC GAG GGT AAC CAG AAC CTG TTC GTG GTA CAG GCC AGT CTG TGG CTC TAC CTG AAG CTG CTG --- --- --- --- --- ---
     phe ile ser asn glu gly asn gln asn leu phe val val gln ala ser leu trp leu tyr leu lys leu leu

         170                                                                        190
     thr lys val ser ile arg leu phe gln gln gln arg arg pro gln gly ser ala asp ala gly glu glu ala glu asp val gly phe pro
538  ACC AAA GTC TCC ATC CGT CTC TTT CAA CAG CAG AGG CGC CCG CAA GGC AGC GCG GAC GCA GGG GAG GAG GCG GAG GAC GTG GGC TTC CCG
     --- --- --- CCT TAC GTT CTG GAG AAG GGC AGC CGG CGC --- --- --- --- --- --- AAG GTT CGG GTC AAG GTG TAC TTC CAG GAG CCG
                 pro tyr val leu glu lys gly ser arg arg                         lys val arg val lys val tyr phe gln glu pro

         200                                      210                                   220
     glu glu lys ser glu val leu ile ser glu lys val val asp ala arg lys ser thr trp his ile phe pro val ser ser ser ile gln
628  GAG GAG AAG AGC GAA GTG CTG ATT TCG GAG AAG GTG GTG GAT GCC CGG AAG AGC ACC TGG CAC ATC TTC CCC GTC TCC AGC AGC ATC CAG
     GGC CAC GGC GAC CGC TGG GAC GTG GTG GAG AAG CGT GTG GAC CTG AAG CGC AGC GGC TGG CAC ACC CTC CCG CTC ACC GAG GCC ATC CAG
     gly his gly asp arg trp asp val val glu lys arg val asp leu lys arg ser gly trp his thr leu pro leu thr glu ala ile gln

         230                                      240                                   250
     arg leu leu asp gln gly lys ser ala leu asp ile arg thr ala cys glu gln cys his glu thr gly ala ser leu val leu leu gly
718  CGC TTG CTG GAC CAG GGC AAG AGC GCC CTG GAC ATC CGG ACT GCC TGC GAG CAG TGC CAC GAG ACC GGC GCC AGC CTG GTG CTG CTG GGC
     GCC CTG TTT GAG CGG GGC GAG CGG CGC CTC AAC CTG GAC GTG CAG TGC GAC GGC TGC CAG GAG CTG GCC GTG GTG CCC GTG TTT --- ---
     ala leu phe glu arg gly glu arg arg leu asn leu asp val gln cys asp gly cys gln glu leu ala val val pro val phe

                                                                             280
     lys lys lys lys lys glu glu glu ala glu gly arg lys arg asp gly glu gly ala gly val asp glu glu lys glu gln ser his arg
808  AAG AAG AAG AAG AAG GAG GAG GAG GCG GAG GGG AGG AAG AGG GAC GGA GAG GGG GCG GGC GTG GAC GAG GAG AAG GAG CAG TCG CAC AGA
     --- --- --- --- --- --- --- --- --- --- --- --- --- --- --- --- --- --- --- --- GTG GAC CCG GGC --- GAG GAG TCA CAC CGG
                                                                                     val asp pro gly     glu glu ser his arg
```

Fig. 2B(III)

βA subunit
βB subunit

```
        290                                    300
     pro phe leu met leu gln ala arg gln ser glu glu his pro his arg arg arg arg arg gly leu glu cys asp gly lys val asn ile
898  CCT TTC CTC ATG CTG CAG GCC CGC CAG TCC GAA GAG CAC CCC CAC CGC CGC CGC CGG CGG GGC CTG GAG TGC GAC GGC AAG GTC AAC ATC
     CCC TTC GTG GTG GTG CAG GCG CGA CTG GGT GAC AGC AGG --- CAC CGC ATC CGC AAG CGG GGC CTG GAG TGT GAC GGC CGG ACC AAC CTC
     pro phe val val val gln ala arg leu gly asp ser arg     his arg ile arg lys arg gly leu glu cys asp gly arg thr asn leu

        320                                    330
     cys cys lys lys gln phe phe val ser phe lys asp ile gly trp asn asp trp ile ile ala pro ser gly tyr his ala asn tyr cys
988  TGC TGT AAG AAG CAG TTC TTT GTC AGT TTC AAG GAC ATC GGC TGG AAC GAC TGG ATC ATC GCT CCG TCC GGC TAC CAC GCC AAC TAC TGC
     TGT TGC AGG CAA CAG TTC TTC ATC GAC TTC CGC CTC ATT GGC TGG AGT GAC TGG ATC ATC GCG CCC ACC GGC TAC TAT GGG AAC TAC TGT
     cys cys arg gln gln phe phe ile asp phe arg leu ile gly trp ser asp trp ile ile ala pro thr gly tyr tyr gly asn tyr cys

        350                                    360                                 370
     glu gly glu cys pro ser his ile ala gly thr ser gly ser ser leu ser phe his ser thr val ile asn his tyr arg met arg gly
1078 GAG GGC GAG TGC CCC AGC CAC ATA GCG GGC ACG TCG GGC TCC TCG CTC TCG TTC CAC TCG ACG GTC ATC AAC CAC TAC CGC ATG CGC GGC
     GAG GGC AGC TGT CCG GCC TAC CTG GCA GGG GTG CCA GGC TCC GCC TCA TCC TTC CAC ACG GCC GTG GTC AAC CAG TAC CGC ATG CGG GGC
     glu gly ser cys pro ala tyr leu ala gly val pro gly ser ala ser ser phe his thr ala val val asn gln tyr arg met arg gly

        380                                    390                                 400
     his ser pro phe ala asn leu lys ser cys cys val pro thr lys leu arg pro met ser met leu tyr tyr asp asp gly gln asn ile
1168 CAC AGC CCC TTC GCC AAC CTC AAG TCG TGC TGC GTC CCC ACC AAG CTG AGG CCC ATG TCC ATG CTG TAC TAC GAC GAC GGG CAG AAC ATC
     CTG AAC CCG --- GGC ACA GTG AAC TCC TGC TGC ATC CCC ACC AAG CTG AGC ACC ATG TCC ATG CTC TAC TTC GAT GAC GAG TAC AAC ATC
     leu asn pro     gly thr val asn ser cys cys ile pro thr lys leu ser thr met ser met leu tyr phe asp asp glu tyr asn ile

        410                                    420     424
     ile lys lys asp ile gln asn met ile val glu glu cys gly cys ser AM
1258 ATC AAG AAG GAC ATC CAG AAC ATG ATC GTG GAG GAG TGC GGG TGC TCC TAG  AGCGCCGGCCGGGGCCCGGGGCCCGGGGCCCGGGGACGACGGCGGCCACGC
     GTC AAG CGG GAC GTG CCC AAC ATG ATC GTG GAG GAG TGT GGC TGT GCC TGA  AAGCATGGGCTCGGGACTGTCCCTGCGGGCACGGGGCACATGGCGGGGGG
     val lys arg asp val pro asn met ile val glu glu cys gly cys ala OP
```

Fig.2B(IV)

```
1360  GAAGACACGTTTACGGCCTCTGACCTAGGCGACCGCAAACATGGAAATGAACAAAAATAACCATAAACTAAAAACAAAACCTGAAACAGATGAAGGAAGACGTGGAAAAATTCCGTAGCC
      TGTGGTCTTGCCGCTGGGTGGCCCGGCAGGTGCCAGGGTGGGAGGCCTGAGATACTTTCCTACTTCTTTATTGAGCAATCAGTCGAAACCAGAGGGCGGACCCTCCGTGGACACGAAAGA

1480  AGGGCTCGGCGATGACACCGTGAAGGAGACGGGACTCGGGGGGGAGGGAGAGGCAGAACGTGGGGGGCGGGGCGGGGGGGGACGACCCTTCCTTTCTTCCTCCAGCATCGGAGTGGGGAC
      CTTGAAAATGCACACGTAGATGCCCGCAGCAGACGCCTCCTGCCACCCACACAGCAGCCTCCGGGATACCAGCAAATGGATGCAGTGACAAATGGCAGCTTAGCTACAAACGCCTGTCAG

1600  AGCAGTTGCTCCAACGGGAATATTGTCCTCTCCTTTTCAGTTCCCTGTCAGTGTGAGCCTCGAAGTCAGCTTGTCTGGTCTGCAGCCATGTGGGCTGGCACAACCCAAATAGCGTCTAGA
      TCGGAGAGAAAGGGTGAGCAGCCACCATTCCCACCAGCTGGCCCGGCCACTCTGAATCGCTCCTTTCGAGCACACAGAAAAGCACAAAGACAGAGACACCGAGAGAGAGAGAGAGAGAGA

1720  AAGCCATGAGTTTGAAAGGGCCAGTTATAGGCACTTTCCCACCCAGTAACCCAGGTCGTAAGGTATGTCTGTGTGACCCTCTCTCTGTGTATATCAGCCCATGCACACACCTACAAAGAC
      GAGACAGACAGACAGACAGAGAGAGAGAGCGAGAGAGAGAGCGAGAGAGAGAGAGAGAGAG

1840  ACACACACACACACACACACACACACACACACACACACACACACACACACACACACACACAACTTCCTCTGACTTTTCTGAGACAAAGAGGTGGGTATAAACTGACTCCAGGAAAACTCGAG
1960  TGGGAAAACGTGCCCTTTGGGTTGGGACAATTTAGATGGTGGAGCAAAGCAAAAAGGAGGCAACGGCAAGTATGTTCGTGATGGGCCTGTGCCCCTGAGGGAGGGGTGAGGAAGTCCCTA
2080  AGGGTGACCTTAGCCAGACAGTGACTCTAGAAGAAGGGGCTCGACAGGGTCATGTAAAGAGAGGAGCTAATTCAGTCAGAAAACCCCTGGCACTCAAGAGAACCACCGTGGGAGTTCCCG
2200  TCGTGGCGCAGTGGTTAACGAATCCGACTAGGAACCATGAGGTTGAGGGTTCGATCCCTGCCCTTACTCAGTGGGTTAACGATCCGGCGTTGCCGTGAGCTGTGGTGCAGGTTGCAGACG
2320  CGGCTCGGATCCTGCGTTGCTGTGGCTCTGGCGTAGGCGGTGGCTACAGCTCCGATTCAACCCCTAGCCTGGGAACCTCCATATGCCGCGGCCCAAGAAATAGCAAAAAAAAAAAAAAAG
2440  AGAACCACCGTGGAGGCCCGTAGCCAGAGCCGGTCCCTTTTAACCCAAGTAGGGAAGGGGAATGAGACTAAGAAGTGAATTTCTTGACAGTCGCAGGCCAGAAAGAGGCAGAGGGACGTC
2560  AGTGCCTCTTCCTGGGAGGCGGCCCCCTCCGTAGGCTGCACAGGAGTTCGCTGAGGGGCCGGCGAGGAAAGGTGTGGGACAGAGGTGGAGGCATGTATTCCACCTTTCGCTTTAGCAGTA
2680  TCTGAAGTCACGGCGAGACTAAGGGCTTCCATTCAGTCCCGTGTATTGCAAGAATCCATGAATTATCTGAATCATTTCGCCACTTAATCAACCCTACAGTTGTTTCACGTGTATCTTGTT
2800  TGCTGGTTAAACCCTACACTATTTGAGAACCAAAGCTGTGCTATTGCTCTAGCACCAGTCTCAGGGCCACGGGTCCCTCTTCCAGAGTCTCCTACCTTCAGTACCTCTTGCCAGGAACAC
2920  ATTCCTCTCCTGCCCAGTCACTCTCCAAGGAGATTCTGTCCCCTAAATATCTCTGGAAGCCATCTTTTCTCCAAGCTGTCATCACCGCTTGTCCAGACTGCTGCTTCCTCGCCAGGTCTC
3040  CCATCTCCCTTCCTGTCCTCCACACACAGCCGCGTGAGCTCTGAAAAACAAACCTAAACACCTGACTTTCCTCATTCAGATTCTTCAGTGGCTTCCCGTTGCTTTTGGAATAAAGTCCTA
3160  AATTCAAAGAGCTTGCATAAGTCAGCCTGTACCATGCATCGACCCCCTTGGTTCCCTAAGTTCCAGTCACATTGGCTGGCTTTCCGTCTTCCTGCCGCAAAGCCAGCACACGGACTGTTC
3280  TCTCCGCTTGTAACACTCCCATTTTCCACCTTTTAATCCTAAATGTTTCTTCCTCGGGGAGACCTTTTCTGATTTTGTGATGTAGGTCAAGACTTTTAGTTAAATCTTCTCTTAGCACCA
3400  TGCCTGTTTCATAGCACTTATTACAATCATAATGTTTACAGTAGAGACGTAATTGGCTGGCAGGCTGCTAGATTGTAAGCTCATGAGGGCAGAAATCACGTCCATCTTGTTCACTGCTGT
3520  ATTCCCAGTGTCGGGCACACAGTTGTTGCTCAATAAATTTGACTTAATGAACTCAAAAAAAAAAAAAAA
```

Fig.3.

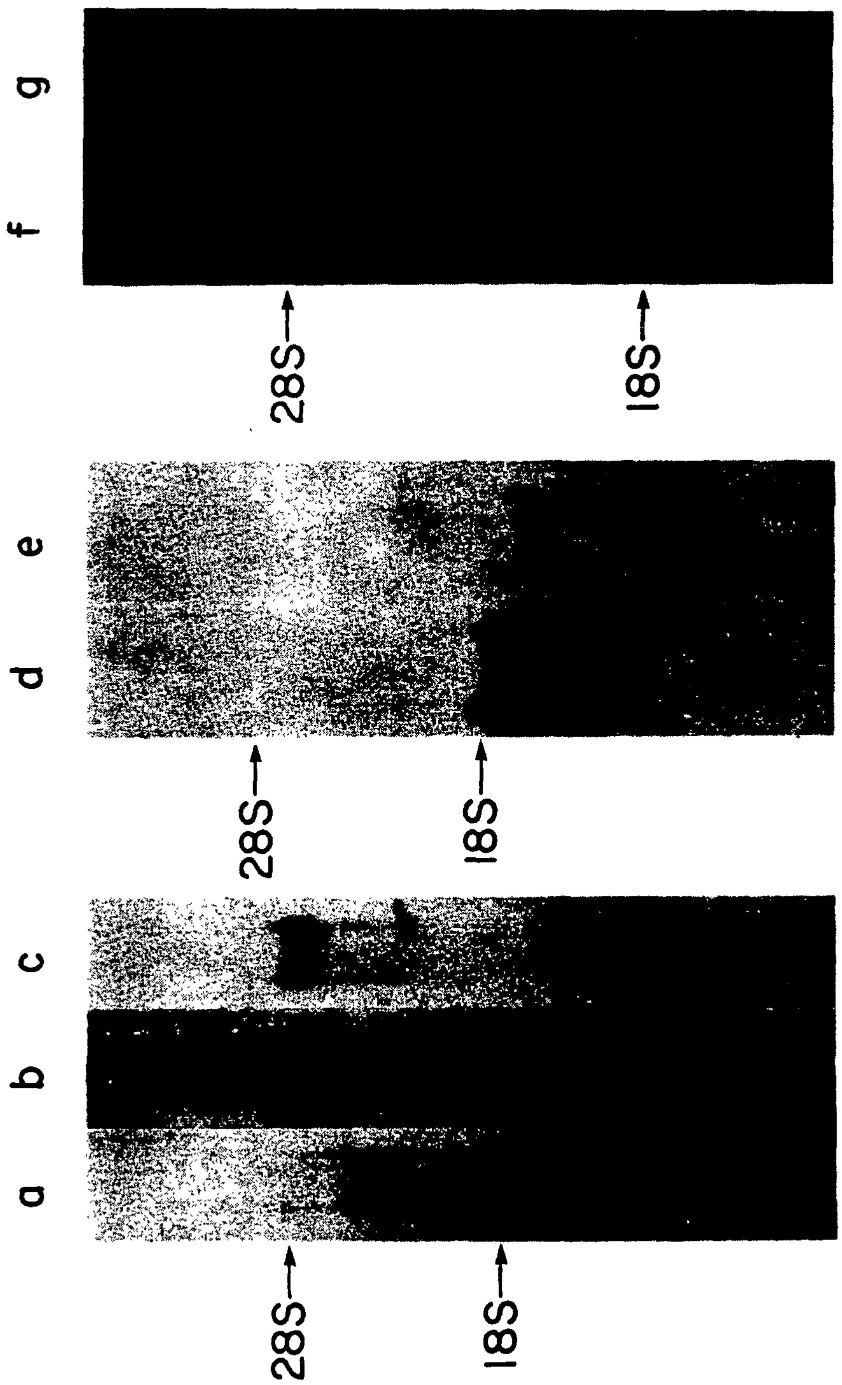

Fig.4A.

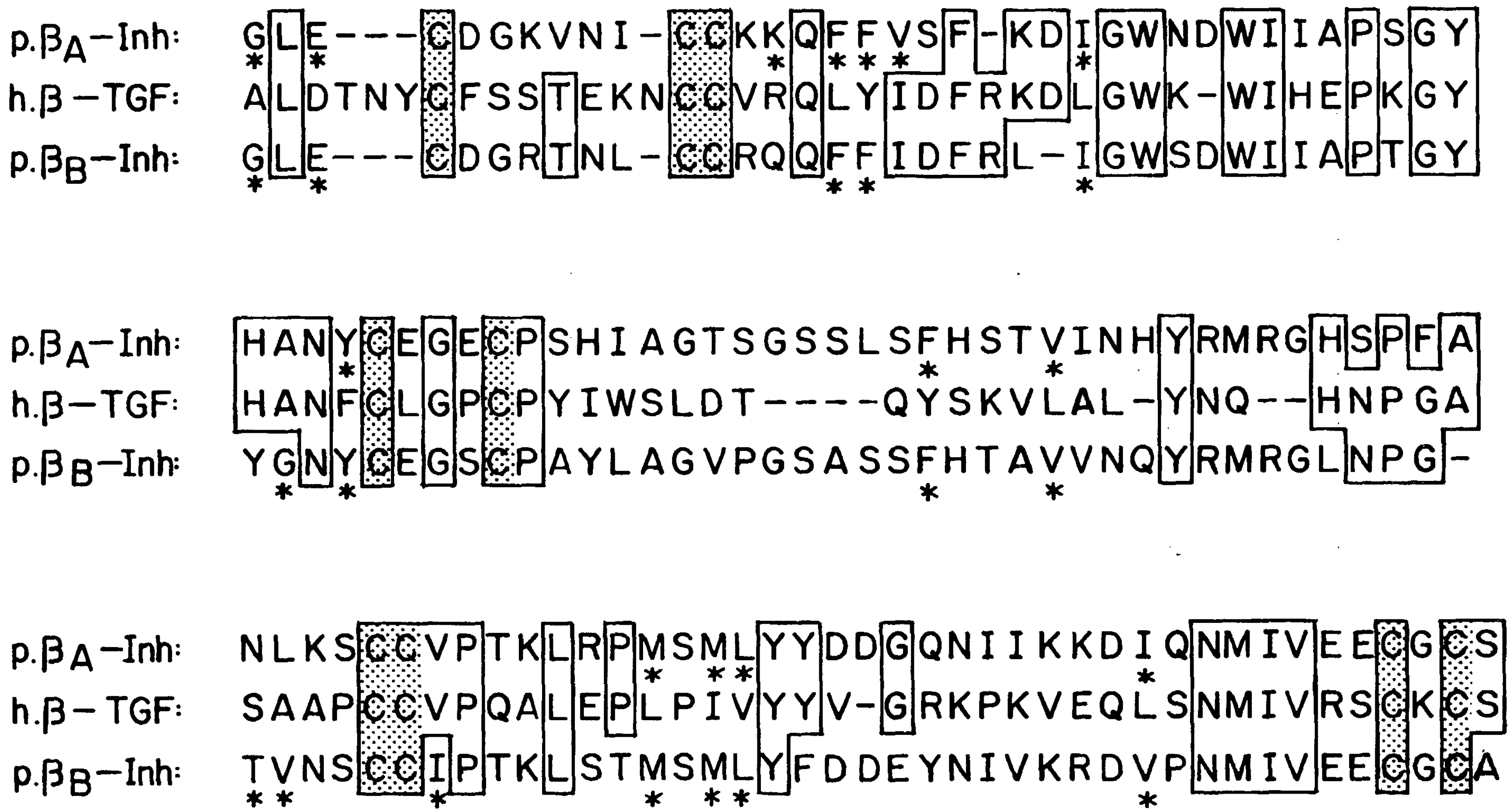
p.βA-Inh: GLE---CDGKVNI-CCKKQFFVSF-KDIGWNDWIIAPSGY
h.β-TGF: ALDTNYCFSSTEKNCCVRQLYIDFRKDLGWK-WIHEPKGY
p.βB-Inh: GLE---CDGRTNL-CCRQQFFIDFRL-IGWSDWIIAPTGY
p.βA-Inh: HANYCEGECPSHIAGTSGSSLSFHSTVINHYRMRGHSPFA
h.β-TGF: HANFCLGPCPYIWSLDT----QYSKVLAL-YNQ--HNPGA
p.βB-Inh: YGNYCEGSCPAYLAGVPGSASSFHTAVVNQYRMRGLNPG-
p.βA-Inh: NLKSCCVPTKLRPMSMLYYDDGQNIIKKDIQNMIVEECGCS
h.β-TGF: SAAPCCVPQALEPLPIVYYV-GRKPKVEQLSNMIVRSCKCS
p.βB-Inh: TVNSCCIPTKLSTMSMLYFDDEYNIVKRDVPNMIVEECGCA

Fig.4B.

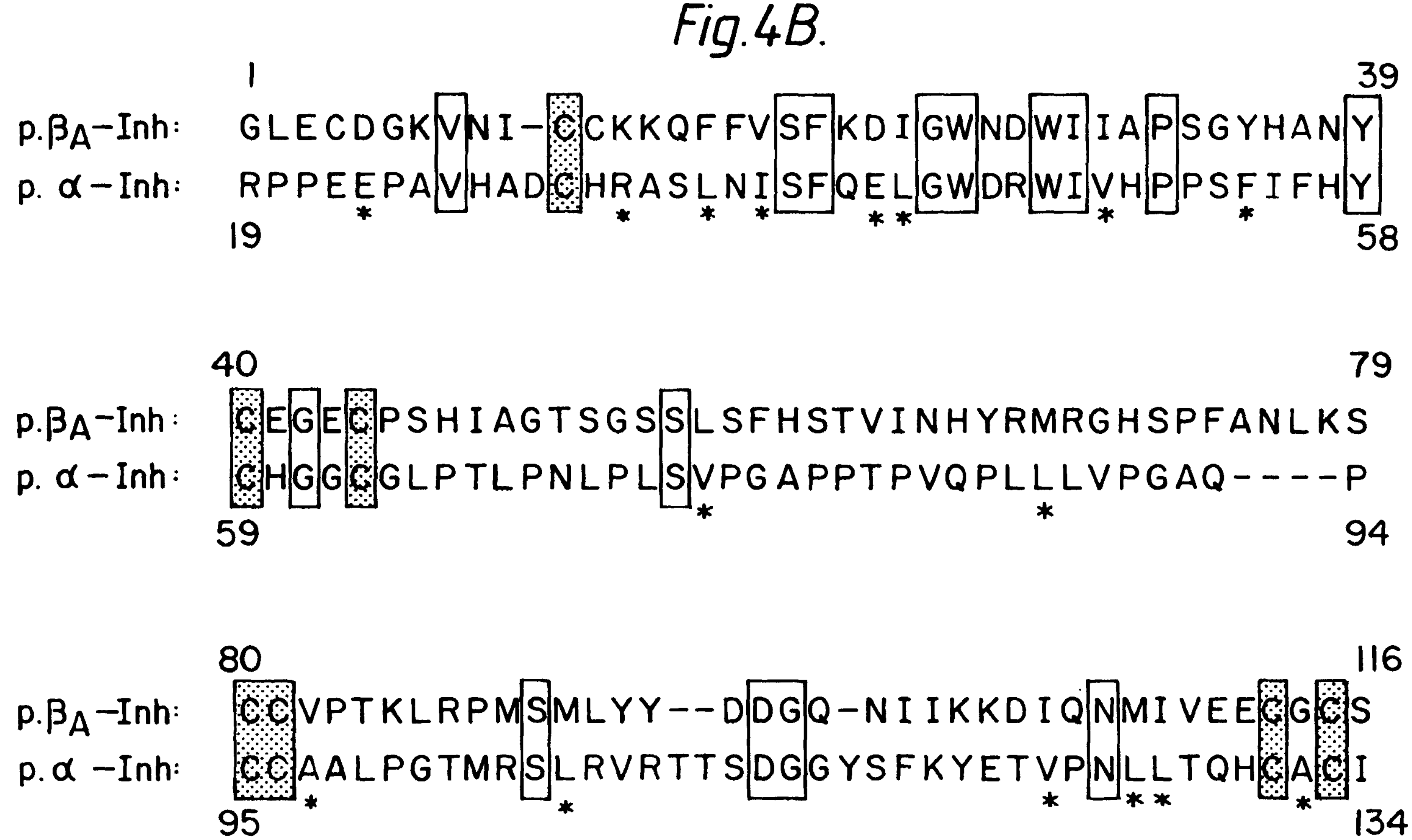
1
39
p.$\beta_A$-Inh: GLECDGKVNI-CCKKQFFVSFKDIGWNDWIIAPSGYHANY
p. α-Inh: RPPEEPAVHADCHRASLNISFQELGWDRWIVHPPSFIFHY
19
58
40
79
p.$\beta_A$-Inh: CEGECPSHIAGTSGSSLSFHSTVINHYRMRGHSPFANLKS
p. α-Inh: CHGGCGLPTLPNLPLSVPGAPPTPVQPLLLVPGAQ----P
59
94
80
116
p.$\beta_A$-Inh: CCVPTKLRPMSMLYY--DDGQ-NIIKKDIQNMIVEECGCS
p. α-Inh: CCAALPGTMRSLRVRTTSDGGYSFKYETVPNLLTQHCACI
95
134

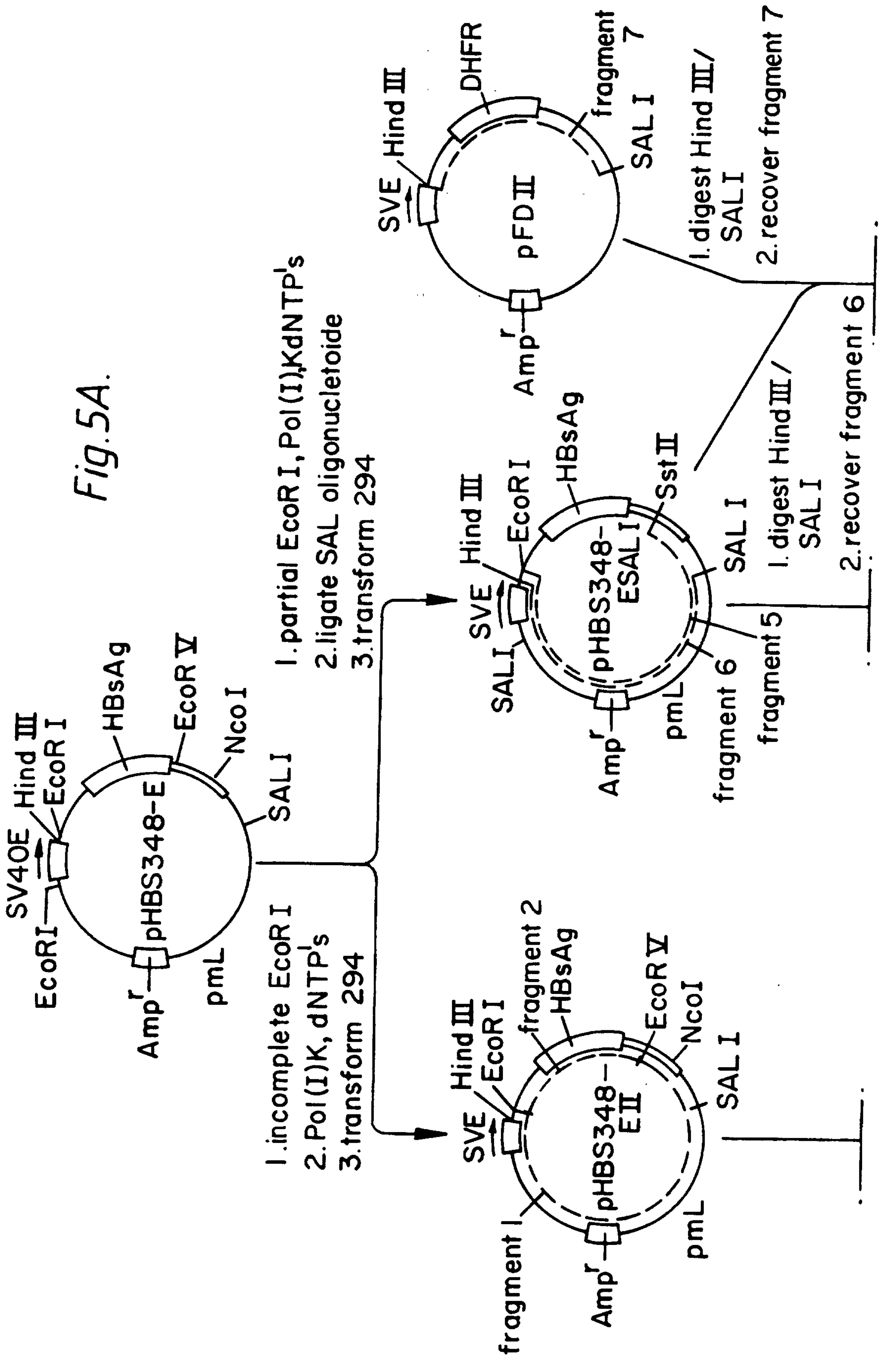
pHBS348-E
SV40E
EcoRI
Hind III
EcoR I
HBsAg
EcoR V
Nco I
SAL I
Amp^r
pmL
1. incomplete EcoR I
2. Pol(I)K, dNTP's
3. transform 294
1. partial EcoR I, Pol(I),KdNTP's
2. ligate SAL oligonucleotoide
3. transform 294
pHBS348-EII
SVE
Hind III
EcoR I
fragment 2
HBsAg
EcoR V
NcoI
SAL I
fragment I
Amp^r
pmL
pHBS348-ESAL I
SVE
Hind III
EcoR I
HBsAg
Sst II
SAL I
SALI
fragment 5
fragment 6
Amp^r
pmL
1. digest Hind III / SAL I
2. recover fragment 5
1. digest Hind III / SAL I
2. recover fragment 6
pFD II
SVE
Hind III
DHFR
fragment 7
SAL I
Amp^r
1. digest Hind III / SAL I
2. recover fragment 7

Fig.5A.

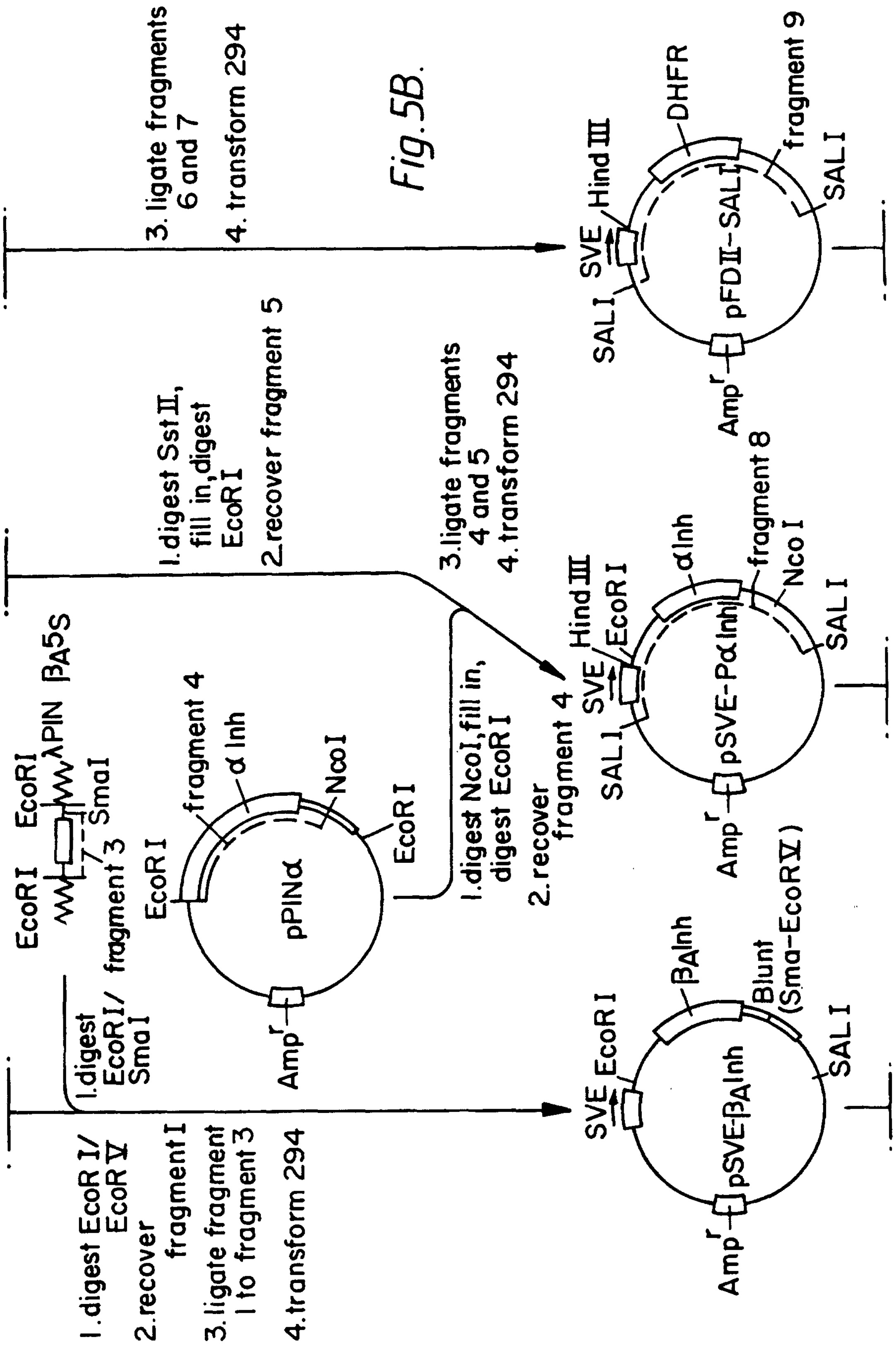
1. digest EcoR I/ EcoR V
2. recover fragment I
3. ligate fragment I to fragment 3
4. transform 294
1. digest EcoRI/ SmaI
EcoRI
EcoRI
λPIN βA5S
SmaI
fragment 3
EcoR I
fragment 4
α Inh
NcoI
EcoR I
pPINα
Ampr
1. digest Sst II, fill in, digest EcoR I
2. recover fragment 5
1. digest NcoI, fill in, digest EcoR I
2. recover fragment 4
3. ligate fragments 4 and 5
4. transform 294
3. ligate fragments 6 and 7
4. transform 294
SVE
EcoR I
βAInh
Blunt (Sma-EcoR V)
SAL I
pSVE-βAInh
Ampr
SAL I
SVE
Hind III
EcoR I
αInh
fragment 8
Nco I
SAL I
pSVE-PαInh
Ampr
SAL I
SVE
Hind III
DHFR
fragment 9
SAL I
pFDII-SALI
Ampr

Fig.5B.

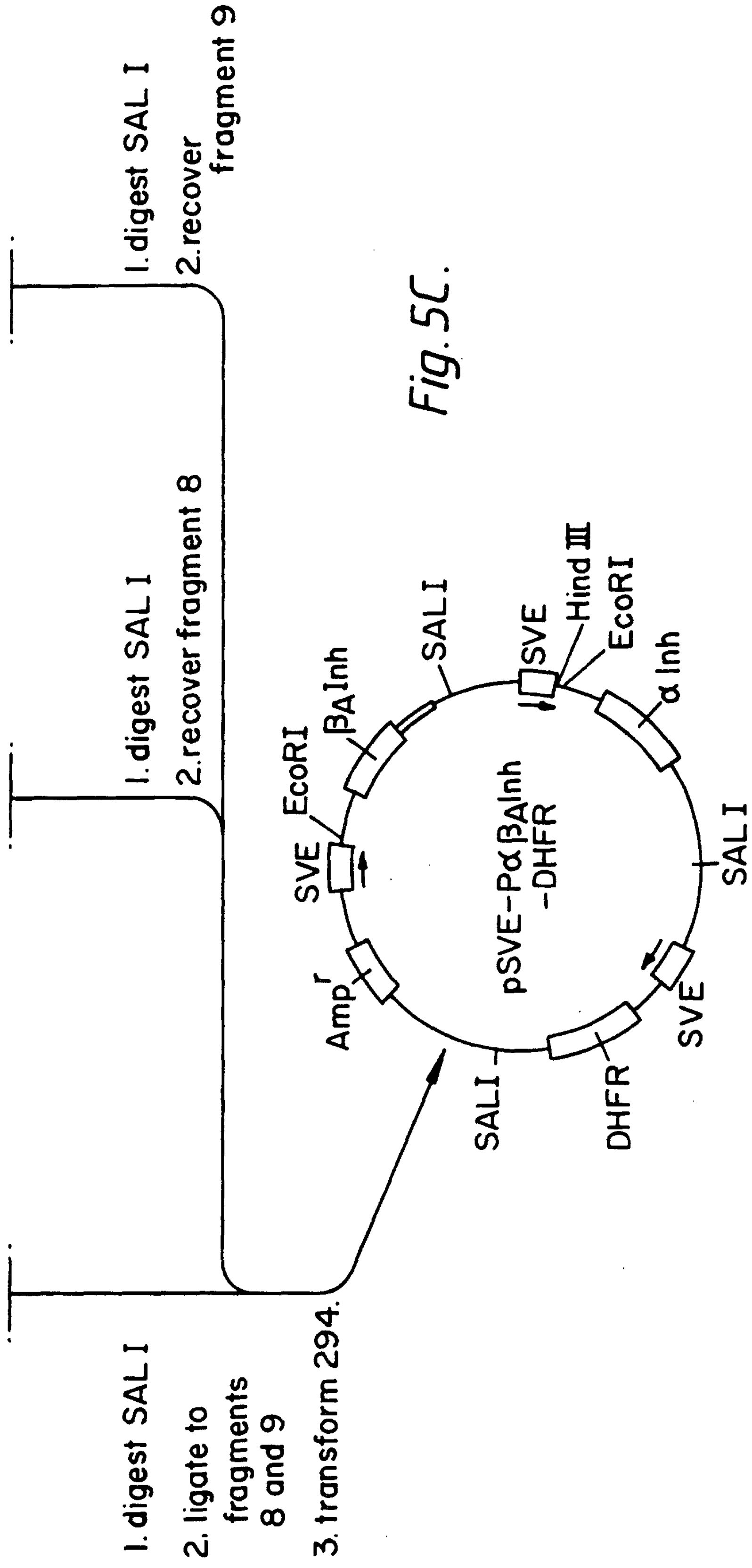
1. digest SAL I
2. recover fragment 9
1. digest SAL I
2. recover fragment 8
1. digest SAL I
2. ligate to fragments 8 and 9
3. transform 294.
pSVE-PαβAInh
-DHFR
SVE
EcoRI
βAInh
SAL I
SVE
Hind III
EcoRI
α Inh
SAL I
SVE
DHFR
SAL I
Amp r

Fig. 5C.

Fig.6A.

```
                                        -10                                             -1  +1
        -16
         Gly Val Ser Ser Gln Gly Leu Glu Leu Ala Arg Glu Leu Val Leu Ala Lys Val Arg Ala Leu Phe Leu Asp
  1 GT   GGG GTC AGC AGC CAG GGG CTG GAG CTG GCC CGG GAA CTT GTT CTG GCC AAG GTG AGG GCC CTG TTC TTG GAT

         10                                      20                                  30
     Ala Leu Gly Pro Pro Ala Val Thr Arg Glu Gly Gly Asp Pro Gly Val Arg Arg Leu Pro Arg Arg His Ala Leu
 75  GCC TTG GGG CCC CCC GCG GTG ACC AGG GAA GGT GGG GAC CCT GGA GTC AGG CGG CTG CCC CGA AGA CAT GCC CTG

                                 40                                  50
     Gly Gly Phe Thr His Arg Gly Ser Glu Pro Glu Glu Glu Glu Asp Val Ser Gln Ala Ile Leu Phe Pro Ala Thr
150  GGG GGC TTC ACA CAC AGG GGC TCT GAG CCC GAG GAA GAG GAG GAT GTC TCC CAA GCC ATC CTT TTC CCA GCC ACA

         60                                      70                                  80
     Asp Ala Ser Cys Glu Asp Lys Ser Ala Ala Arg Gly Leu Ala Gln Glu Ala Glu Glu Gly Leu Phe Arg Tyr Met
225  GAT GCC AGC TGT GAG GAC AAG TCA GCT GCC AGA GGG CTG GCC CAG GAG GCT GAG GAG GGC CTC TTC AGA TAC ATG

                                 90                                  100
     Phe Arg Pro Ser Gln His Thr Arg Ser Arg Gln Val Thr Ser Ala Gln Leu Trp Phe His Thr Gly Leu Asp Arg
300  TTC CGG CCA TCC CAG CAT ACA CGC AGC CGC CAG GTG ACT TCA GCC CAG CTG TGG TTC CAC ACC GGG CTG GAC AGG

         110                                     120                                 130
     Gln Gly Thr Ala Ala Ser Asn Ser Ser Glu Pro Leu Leu Gly Leu Leu Ala Leu Ser Pro Gly Gly Pro Val Ala
375  CAG GGC ACA GCA GCC TCC AAT AGC TCT GAG CCC CTG CTA GGC CTG CTG GCA CTG TCA CCG GGA GGA CCC GTG GCT

                                 140                                 150
     Val Pro Met Ser Leu Gly His Ala Pro Pro His Trp Ala Val Leu His Leu Ala Thr Ser Ala Leu Ser Leu Leu
450  GTG CCC ATG TCT TTG GGC CAT GCT CCC CCT CAC TGG GCC GTG CTG CAC CTG GCC ACC TCT GCT CTC TCT CTG CTG

         160                                     170                                 180
     Thr His Pro Val Leu Val Leu Leu Leu Arg Cys Pro Leu Cys Thr Cys Ser Ala Arg Pro Glu Ala Thr Pro Phe
525  ACC CAC CCC GTC CTG GTG CTG CTG CTG CGC TGT CCC CTC TGT ACC TGC TCA GCC CGG CCT GAG GCC ACG CCC TTC
```

# Fig.6B.

```
                                                              ┌─► α subunit
                        190
    Leu Val Ala His Thr Arg Thr Arg Pro Pro Ser Gly Gly Glu Arg Ala Arg Arg Ser Thr Pro Leu Met Ser Trp
600 CTG GTG GCC CAC ACT CGG ACC AGA CCA CCC AGT GGA GGG GAG AGA GCC CGA CGC TCA ACT CCC CTG ATG TCC TGG

        210                                     220                                     230
    Pro Trp Ser Pro Ser Ala Leu Arg Leu Leu Gln Arg Pro Pro Glu Glu Pro Ala Ala His Ala Asn Cys His Arg
675 CCT TGG TCT CCC TCT GCT CTG CGC CTG CTG CAG AGG CCT CCG GAG GAA CCG GCT GCC CAT GCC AAC TGC CAC AGA

                        240                                     250
    Val Ala Leu Asn Ile Ser Phe Gln Glu Leu Gly Trp Glu Arg Trp Ile Val Tyr Pro Pro Ser Phe Ile Phe His
750 GTA GCA CTG AAC ATC TCC TTC CAG GAG CTG GGC TGG GAA CGG TGG ATC GTG TAC CCT CCC AGT TTC ATC TTC CAC

        260                                     270                                     280
    Tyr Cys His Gly Gly Cys Gly Leu His Ile Pro Pro Asn Leu Ser Leu Pro Val Pro Gly Ala Pro Pro Thr Pro
825 TAC TGT CAT GGT GGT TGT GGG CTG CAC ATC CCA CCA AAC CTG TCC CTT CCA GTC CCT GGG GCT CCC CCT ACC CCA

                        290                                     300
    Ala Gln Pro Tyr Ser Leu Leu Pro Gly Ala Gln Pro Cys Cys Ala Ala Leu Pro Gly Thr Met Arg Pro Leu His
900 GCC CAG CCC TAC TCC TTG CTG CCA GGG GCC CAG CCC TGC TGT GCT GCT CTC CCA GGG ACC ATG AGG CCC CTA CAT

        310                                     320                                     330
    Val Arg Thr Thr Ser Asp Gly Gly Tyr Ser Phe Lys Tyr Glu Thr Val Pro Asn Leu Leu Thr Gln His Cys Ala
975 GTC CGC ACC ACC TCG GAT GGA GGT TAC TCT TTC AAG TAT GAG ACA GTG CCC AAC CTT CTC ACG CAG CAC TGT GCT

         335
     Cys Ile OC*
1050 TGT ATC TAA  GGGTGGGGGGTCTTCCTTCTTAATCCCATGGCTGGTGGCCACGCCCCCACCATCATCAGCTGGGAGGAAAGGCAGAGTTGGGAAATA

1146 GATGGCTCCCACTCCTCCCTCCTTTCACTTCTCTGCCTATGGGCTACCCTCCCCACCCCACTTCTATCTCAATAAAGAACACAGTGCATATG polyA
```

# Fig. 7A.

```
           -30                          +1                 20
pin.alpha  MWPQLLLLLLAPRSGHGCQGPELDRELVLAKVRALFLDALGPPAVTGEGG
hin.alpha                GVSSQGLELARELVLAKVRALFLDALGPPAVTREGG
                          ***  *  *                      *

                              40                  60
pin.alpha  DPGVRRLPRRHAVGGFMRRGSEPEEE-DVSQAILFPATGARCGDEPAAGE
hin.alpha  DPGVRRLPRRHALGGFTHRGSEPEEEEDVSQAILFPATDASCEDKSAARG
                       *   **        *           * * * **  **

                     80                  100
pin.alpha  LAREAEEGLFTYVFRPSQHTHSRQVTSAQLWFHTGLDRQGMAAANSSGPL
hin.alpha  LAQEAEEGLFRYMFRPSQHTRSRQVTSAQLWFHTGLDRQGTAASNSSEPL
             *       * *       *                   *  *   *

           120                 140                 160
pin.alpha  LDLLALSSRGPVAVPMSLGQAPPRWAVLHLAASALPLLTHPVLVLLLRCP
hin.alpha  LGLLALSPGGPVAVPMSLGHAPPHWAVLHLATSALSLLTHPVLVLLLRCP
            *     **          *   *       *   *
```

*Fig. 7B.*

```
                        180                 200
pin.alpha    LCSCSARPEATPFLVAHTRARPPSGGERARRSTAPLP-WPWSPAALRLLQ
hin.alpha    LCTCSARPEATPFLVAHTRTRPPSGGERARRST-PLMSWPWSPSALRLLQ
               *                *             *  **     *

              220                 240              260
pin.alpha    RPPEEPAVHADCHRASLNISFQELGWDRWIVHPPSFIFHYCHGGCGLPTL
hin.alpha    RPPEEPAAHANCHRVALNISFQELGWERWIVYPPSFIFHYCHGGCGLHIP
                    *  *   **          *    *               ***

                        280                 300
pin.alpha    PNLPLSVPGAPPTPVQPLLLVPGAQPCCAALPGTMRSLRVRTTSDGGYSF
hin.alpha    PNLSLPVPGAPPTPAQPYSLLPGAQPCCAALPGTMRPLHVRTTSDGGYSF
                * *        *  ** *               * *

              320           334
pin.alpha    KYETVPNLLTQHCACI
hin.alpha    KYETVPNLLTQHCACI
```

# Fig.8A.

```
  1 TGCTCCCTGACAGCCACAAACCTACAGCACTGACTGCATTCAGAGAGGAACCTGCAAACAAAACTTCACAGAAACTTTTTGTTCTTGTTCCAGAGAATT

101 TGCTGAAGAGGAGAAGGAAAAAAAAAACACCAAAAAAAAAAATAAAAAAATCCACACACACAAAAAACCTGCGCGTGAGGGGGGAGGAAAAGCAGGGCCT

                                          -28                                 -20
                                          Met Pro Leu Leu Trp Leu Arg Gly Phe Leu Leu Ala Ser Cys Trp
201 TTTAAAAAGGCAATCACAACAACTTTTGCTGCCAGG     ATG CCC TTG CTT TGG CTG AGA GGA TTT CTG TTG GCA AGT TGC TGG

                -10                                 -1  +1                                  10
    Ile Ile Val Arg Ser Ser Pro Thr Pro Gly Ser Glu Gly His Ser Ala Ala Pro Asp Cys Pro Ser Cys Ala Leu
282 ATT ATA GTG AGG AGT TCC CCC ACC CCA GGA TCC GAG GGG CAC AGC GCG GCC CCC GAC TGT CCG TCC TGT GCG CTG

                                20                                      30
    Ala Ala Leu Pro Lys Asp Val Pro Asn Ser Gln Pro Glu Met Val Glu Ala Val Lys Lys His Ile Leu Asn Met
357 GCC GCC CTC CCA AAG GAT GTA CCC AAC TCT CAG CCA GAG ATG GTG GAG GCC GTC AAG AAG CAC ATT TTA AAC ATG

            40                                      50                                      60
    Leu His Leu Lys Lys Arg Pro Asp Val Thr Gln Pro Val Pro Lys Ala Ala Leu Leu Asn Ala Ile Arg Lys Leu
432 CTG CAC TTG AAG AAG AGA CCC GAT GTC ACC CAG CCG GTA CCC AAG GCG GCG CTT CTG AAC GCG ATC AGA AAG CTT

                                70                                      80
    His Val Gly Lys Val Gly Glu Asn Gly Tyr Val Glu Ile Glu Asp Asp Ile Gly Arg Arg Ala Glu Met Asn Glu
507 CAT GTG GGC AAA GTC GGG GAG AAC GGG TAT GTG GAG ATA GAG GAT GAC ATT GGA AGG AGG GCA GAA ATG AAT GAA

            90                                      100                                     110
    Leu Met Glu Gln Thr Ser Glu Ile Ile Thr Phe Ala Glu Ser Gly Thr Ala Arg Lys Thr Leu His Phe Glu Ile
582 CTT ATG GAG CAG ACC TCG GAG ATC ATC ACG TTT GCC GAG TCA GGA ACA GCC AGG AAG ACG CTG CAC TTC GAG ATT
```

## Fig.8B.

```
                                 120                                               130
       Ser Lys Glu Gly Ser Asp Leu Ser Val Val Glu Arg Ala Glu Val Trp Leu Phe Leu Lys Val Pro Lys Ala Asn
   657 TCC AAG GAA GGC AGT GAC CTG TCA GTG GTG GAG CGT GCA GAA GTC TGG CTC TTC CTA AAA GTC CCC AAG GCC AAC

               140                                     150                                     160
       Arg Thr Arg Thr Lys Val Thr Ile Arg Leu Phe Gln Gln Gln Lys His Pro Gln Gly Ser Leu Asp Thr Gly Glu
   732 AGG ACC AGG ACC AAA GTC ACC ATC CGC CTC TTC CAG CAG CAG AAG CAC CCG CAG GGC AGC TTG GAC ACA GGG GAA

                                 170                                               180
       Glu Ala Glu Glu Val Gly Leu Lys Gly Glu Arg Ser Glu Leu Leu Leu Ser Glu Lys Val Val Asp Ala Arg Lys
   807 GAG GCC GAG GAA GTG GGC TTA AAG GGG GAG AGG AGT GAA CTG TTG CTC TCT GAA AAA GTA GTA GAC GCT CGG AAG

               190                                     200                                     210
       Ser Thr Trp His Val Phe Pro Val Ser Ser Ser Ile Gln Arg Leu Leu Asp Gln Gly Lys Ser Ser Leu Asp Val
   882 AGC ACC TGG CAT GTC TTC CCT GTC TCC AGC AGC ATC CAG CGG TTG CTG GAC CAG GGC AAG AGC TCC CTG GAC GTT

                                 220                                               230
       Arg Ile Ala Cys Glu Gln Cys Gln Glu Ser Gly Ala Ser Leu Val Leu Leu Gly Lys Lys Lys Lys Lys Glu Glu
   957 CGG ATT GCC TGT GAG CAG TGC CAG GAG AGT GGC GCC AGC TTG GTT CTC CTG GGC AAG AAG AAG AAG AAA GAA GAG

               240                                     250                                     260
       Glu Gly Glu Gly Lys Lys Lys Gly Gly Gly Glu Gly Gly Ala Gly Ala Asp Glu Glu Lys Glu Gln Ser His Arg
  1032 GAG GGG GAA GGG AAA AAG AAG GGC GGA GGT GAA GGT GGG GCA GGA GCA GAT GAG GAA AAG GAG CAG TCG CAC AGA
```

# Fig.8C.

```
                                          270                                                               ──►βA subunit
     Pro Phe Leu Met Leu Gln Ala Arg Gln Ser Glu Asp His Pro His Arg Arg Arg Arg Arg Gly Leu Glu Cys Asp
1107 CCT TTC CTC ATG CTG CAG GCC CGG CAG TCT GAA GAC CAC CCT CAT CGC CGG CGT CGG CGG GGC TTG GAG TGT GAT

             290                                     300                                     310
     Gly Lys Val Asn Ile Cys Cys Lys Lys Gln Phe Phe Val Ser Phe Lys Asp Ile Gly Trp Asn Asp Trp Ile Ile
1182 GGC AAG GTC AAC ATC TGC TGT AAG AAA CAG TTC TTT GTC AGT TTC AAG GAC ATC GGC TGG AAT GAC TGG ATC ATT

                                 320                                     330
     Ala Pro Ser Gly Tyr His Ala Asn Tyr Cys Glu Gly Glu Cys Pro Ser His Ile Ala Gly Thr Ser Gly Ser Ser
1257 GCT CCC TCT GGC TAT CAT GCC AAC TAC TGC GAG GGT GAG TGC CCG AGC CAT ATA GCA GGC ACG TCC GGG TCC TCA

             340                                     350                                     360
     Leu Ser Phe His Ser Thr Val Ile Asn His Tyr Arg Met Arg Gly His Ser Pro Phe Ala Asn Leu Lys Ser Cys
1332 CTG TCC TTC CAC TCA ACA GTC ATC AAC CAC TAC CGC ATG CGG GGC CAT AGC CCC TTT GCC AAC CTC AAA TCG TGC

                                 370                                     380
     Cys Val Pro Thr Lys Leu Arg Pro Met Ser Met Leu Tyr Tyr Asp Asp Gly Gln Asn Ile Ile Lys Lys Asp Ile
1407 TGT GTG CCC ACC AAG CTG AGA CCC ATG TCC ATG TTG TAC TAT GAT GAT GGT CAA AAC ATC ATC AAA AAG GAC ATT

             390                         398
     Gln Asn Met Ile Val Glu Glu Cys Gly Cys Ser AM*
1482 CAG AAC ATG ATC GTG GAG GAG TGT GGG TGC TCA TAG AGTTGCCCAGCCCAGGGGGAAAGGGAGCAAGAGTTGTCCAGAGAAGACAGTG

1570 GCAAAATGAAGAAATTTTTAAGGTTTCTGAGTTAACCAGAAAAATAGAAATTAAAAACAAAACA polyA
```

*Fig. 9A.*

```
           7          10                                                    20                                                30
        Cys Thr Ser Cys Gly Gly Phe Arg Arg Pro Glu Glu Leu Gly Arg Val Asp Gly Asp Phe Leu Glu Ala Val
  1  CC TGT ACG TCG TGC GGC GGC TTC CGG CGG CCA GAG GAG CTC GGC CGA GTG GAC GGC GAC TTC CTG GAG GCG GTG

                                         40                                        50
    Lys Arg His Ile Leu Ser Arg Leu Gln Met Arg Gly Arg Pro Asn Ile Thr His Ala Val Pro Lys Ala Ala Met
 75 AAG CGG CAC ATC TTG AGC CGC CTG CAG ATG CGG GGC CGG CCC AAC ATC ACG CAC GCC GTG CCT AAG GCC GCC ATG

                         60                                  70                                    80
    Val Thr Ala Leu Arg Lys Leu His Ala Gly Lys Val Arg Glu Asp Gly Arg Val Glu Ile Pro His Leu Asp Gly
150 GTC ACG GCC CTG CGC AAG CTG CAC GCG GGC AAG GTG CGC GAG GAC GGC CGC GTG GAG ATC CCG CAC CTC GAC GGC

                                         90                                  100
    His Ala Ser Pro Gly Ala Asp Gly Gln Glu Arg Val Ser Glu Ile Ile Ser Phe Ala Glu Thr Asp Gly Leu Ala
225 CAC GCC AGC CCG GGC GCC GAC GGC CAG GAG CGC GTT TCC GAA ATC ATC AGC TTC GCC GAG ACA GAT GGC CTC GCC

                        110                                 120                                   130
    Ser Ser Arg Val Arg Leu Tyr Phe Phe Ile Ser Asn Glu Gly Asn Gln Asn Leu Phe Val Val Gln Ala Ser Leu
300 TCC TCC CGG GTC CGC CTA TAC TTC TTC ATC TCC AAC GAA GGC AAC CAG AAC CTG TTT GTG GTC CAG GCC AGC CTG

                                        140                                  150
    Trp Leu Tyr Leu Lys Leu Leu Pro Tyr Val Leu Glu Lys Gly Ser Arg Arg Lys Val Arg Val Lys Val Tyr Phe
375 TGG CTT TAC CTG AAA CTC CTG CCC TAC GTC CTG GAG AAG GGC AGC CGG CGG AAG GTG CGG GTC AAA GTG TAC TTC

                        160                                 170                                   180
    Gln Glu Gln Gly His Gly Asp Arg Trp Asn Met Val Glu Lys Arg Val Asp Leu Lys Arg Ser Gly Trp His Thr
450 CAG GAG CAG GGC CAC GGT GAC AGG TGG AAC ATG GTG GAG AAG AGG GTG GAC CTC AAG CGC AGC GGC TGG CAT ACC
```

*Fig.9B.*

```
                                   190                                               200
    Phe Pro Leu Thr Glu Ala Ile Gln Ala Leu Phe Glu Arg Gly Glu Arg Arg Leu Asn Leu Asp Val Gln Cys Asp
525 TTC CCA CTC ACG GAG GCC ATC CAG GCC TTG TTT GAG CGG GGC GAG CGG CGA CTC AAC CTA GAC GTG CAG TGT GAC

                210                                               220                                 230
    Ser Cys Gln Glu Leu Ala Val Val Pro Val Phe Val Asp Pro Gly Glu Glu Ser His Arg Pro Phe Val Val Val
600 AGC TGC CAG GAG CTG GCC GTG GTG CCG GTG TTC GTG GAC CCA GGC GAA GAG TCG CAC CGG CCC TTT GTG GTG GTG

                                                            → βB subunit
                                   240
    Gln Ala Arg Leu Gly Asp Ser Arg His Arg Ile Arg Lys Arg Gly Leu Glu Cys Asp Gly Arg Thr Asn Leu Cys
675 CAG GCT CGG CTG GGC GAC AGC AGG CAC CGC ATT CGC AAG CGA GGC CTG GAG TGC GAT GGC CGG ACC AAC CTC TGT

                260                                               270                                 280
    Cys Arg Gln Gln Phe Phe Ile Asp Phe Arg Leu Ile Gly Trp Asn Asp Trp Ile Ile Ala Pro Thr Gly Tyr Tyr
750 TGC AGG CAA CAG TTC TTC ATT GAC TTC CGC CTC ATC GGC TGG AAC GAC TGG ATC ATA GCA CCC ACC GGC TAC TAC

                                   290                                               300
    Gly Asn Tyr Cys Glu Gly Ser Cys Pro Ala Tyr Leu Ala Gly Val Pro Gly Ser Ala Ser Ser Phe His Thr Ala
825 GGG AAC TAC TGT GAG GGC AGC TGC CCA GCC TAC CTG GCA GGG GTC CCC GGC TCT GCC TCC TCC TTC CAC ACG GCT

                310                                               320                                 330
    Val Val Asn Gln Tyr Arg Met Arg Gly Leu Asn Pro Gly Thr Val Asn Ser Cys Cys Ile Pro Thr Lys Leu Ser
900 GTG GTG AAC CAG TAC CGC ATG CGG GGT CTG AAC CCC GGC ACG GTG AAC TCC TGC TGC ATT CCC ACC AAG CTG AGC

                                   340                                               350
    Thr Met Ser Met Leu Tyr Phe Asp Asp Glu Tyr Asn Ile Val Lys Arg Asp Val Pro Asn Met Ile Val Glu Glu
975 ACC ATG TCC ATG CTG TAC TTC GAT GAT GAG TAC AAC ATC GTC AAG CGG GAC GTG CCC AAC ATG ATT GTG GAG GAG
```

Fig.9C.

```
                      359
     Cys  Gly  Cys  Ala  OP*
1050 TGC  GGC  TGC  GCC  TGA  CAGTGCAAGGCAGGGGCACGGTGGTGGGGCACGGAGGGCAGTCCCGGGTGGGCTTCTTCCAGCCCCCGCGGGAACGGGGT

1145 ACACGGTGGGCTGAGTACAGTCATTCTGTTGGGCTGTGGAGATAGTGCCAGGGTGCGGCCTGAGATATTTTTCTACAGCTTCATAGAGCAACCAGTCAAA
1245 ACCAGAGCGAGAACCCTCAACTGACATGAAATACTTTAAAATGCACACGTAGCCACGCACAGCCAGACGCATCCTGCCACCCACACAGCAGCCTCCAGGA
1345 TACCAGCAAATGGATGCGGTGACAAATGGCAGCTTAGCTACAAATGCCTGTCAGTCGGAGAGAATGGGGTGAGCAGCCACCATTCCACCAGCTGGCCCGG
1445 CCACGTCTCGAAGTTGCGCCTTCCCGAGCACACATAAAAGCACAAAGACAGAGACGCAGAGAGAGAGAGAGCCACGGAGAGGAAAAGCAGATGCAGGG
1545 GTGGGGAGCGCAGCTCGGCGGAGGCTGCGTGTGCCCCGTGGCTTTTACCAGGCCTGCTCTGCCTGGCTCGATGTCTGCTTCTTCCCAGCCTGGGATCCTT
1645 CGTGCTTCAAGGCCTGGGGAGCCTGTCCTTCCATGCCCTTGTCGAGGGAAAGAGACCCAGAAAGGACACAACCCGTCAGAGACCTGGGAGCAGGGGCAAT
1745 GACCGTTTGACTGTTTGTGGCTTGGGCCTCTGACATGACTTATGTGTGTGTGTGTTTTTGGGGTGGGGAGGGAGGGAGAGAAGAGGGGGCTAAATTTGAT
1845 GCTTTAACTGATCTCCAACAGTTGACAGGTCATCCTTGCCAGTTGTATAACTGAAAAAGGACTTTTCTACCAGGTATGACCTTTTAAGTGAAAATCTGAA
1945 TTGTTCTAAATGAAAAGAAAAA
```